# EUROPEAN PATENT APPLICATION

(11) **EP 1 466 973 A1**
(43) Date of publication of application: **13.10.2004**
(21) Application number: 02791924.0
(22) Date of filing: 16.12.2002
(51) Int. Cl.: C12N 15/09, C12N 5/10, C12Q 1/68, A01H 5/00

(54) **CHARACTERISTIC BASE SEQUENCES OCCURRING IN PLANT GENES AND METHOD OF UTILIZING THE SAME**

(30) Priority: 20.12.2001 JP 2001387059; 20.12.2001 JP 2001387131; 20.12.2001 JP 2001403299; 20.12.2001 JP 2001403300; 27.09.2002 JP 2002327515
(71) Applicant: JAPAN as represented by the President of Okayama University, Okayama-shi, Okayama 700-8530 (JP)
(72) Inventor: SATO, Kazuhiro, Kurashiki-shi, Okayama 710-0031 (JP); TAKEDA, Kazuyoshi, Kurashiki-shi, Okayama 710-0031 (JP); KOHARA, Yuji, Mishima-shi, Shizuoka 411-0025 (JP)
(74) Representative: Hoffmann, Jörg Peter, Dr. Ing.
(86) International application number: PCT/IB2002/005403
(87) International publication number: WO 2003/057877

(57) **Abstract**

By the mass sequencing of cDNA clones of barley strains including H. spontaneum, Haruna Nijo, and Akashinriki, it was found that single nucleotide polymorphism (SNP) was present at many sites among these different strains. These SNPs include nonsynonymous SNPs and are likely to be associated with the phonotype distinct to each strain. The SNPs are therefore useful in a wide range of applications, for example, in genetically identifying strains or isolating genes, or in producing and screening for novel recombinants.

## Description

### TECHNICAL FIELD

The present invention relates to genes of barley. Specifically, the invention finds variations in the base sequences of a gene among different strains of barley and thereby finds characteristic base sequences of the gene in these barley strains. The characteristic base sequences can be used for the testing or identification of barley strains to improve the efficiency of breeding in plants including barley.

The present invention finds characteristic base sequences of a gene among different strains of barley in relation to single nucleotide polymorphism (SNP) that confers variations among barley strains. The invention concerns nucleotides that take advantage of such gene characteristics, and a method using such nucleotides.

### BACKGROUND ART

For over centuries, humans have been cultivating cereal crops such as barley, wheat and rice as food sources. In Japan, wheat and barley are major winter crops that are categorized in the same group. In many other countries, major cereal crops include wheat, barley, rye and oats.

Barley belongs to the genus Hordeum, and its place of origin is thought to be central Asia. With later selection, barley has now been classified into hulled barley and naked barley, or six-rowed barley and two-rowed barley, according to the spike row types, hull form of grains, and brittleness of rachis. Generally, naked barley is known by the name "six-rowed naked barley," and two-rowed hulled barley is also known as malting barley.

Barley is most commonly used for feeding purposes. Many types of barley used for feeding are in the form of grains, but, in the Middle and Near East and inland Australia, barley is cultivated in grazing to provide feed. In one unique practice found in Cyprus, barley with kernel brittleness is selectively cultivated to provide grassland that regenerates naturally from mature fallen seeds. The purpose of breeding feed barley has been to improve crop-related traits such as disease resistance or lodging resistance, and there are not many examples of breeding that is concerned with quality traits. Asia is a major place where barley is cultivated for food. People living in areas of the Himalayas still feed on six-rowed naked barley as a main diet. Most strains cultivated in this part of the world are native to the areas, and the high altitude and dry climate help the barley grow without much influence from diseases, and accordingly a relatively high yield can be expected. For purposes where barley is processed into flour, mutants with a wide variety of ear shapes and grain colors are sometimes used because in this case there is substantially no need to screen the crop based on the appearance of the kernel.

In Japan, six-rowed barley has been consumed as food. Particularly in naked barley, semi-dwarf type barleys with shorter grains like rice have been used mainly in western Japan. One representative strain of such naked barley is Akashinriki, which excels in crop yield, and has flooding resistance and pest resistance. The cultivation of this particular strain was very common in the mid 50's, and the strain has continuously been used today as a crossing parent in breeding. For example, crossbreeding of Akashinriki with Kagawahadaka Ichigou has produced a superior strain Yunagihadaka, which has improved crop-yielding characteristics, and with flooding resistance and relatively strong pest resistance. The strain Yunagihadaka received a government endorsement in 1983.

Meanwhile, general requirements for malting barley include: (1) grains that are glossy and lemon-yellow in color; (2) strong and uniform germinability (germination rate of 95% or greater); (3) high starch value and suitable protein content; (4) strong enzyme activity; and (5) suitable content of β-glucan, polyphenol, and the like. Among different strains of malting barley that satisfy these requirements, Haruna Nijo is considered to be the best strain of all. This particular strain has set a world standard for all other strains of malting barley, and has widely been used as a crossing parent in many countries. The strain Haruna Nijo is an established strain that is produced by crossbreeding Seijo Jyugogou with the line derived from the cross between G65 and K-3, and it has become a standard of quality in malting barley. However, Haruna Nijo falls behind in ease of threshing, lodging strength, and grain yield. A further drawback of Haruna Nijo is susceptibility to diseases including barley yellow mosaic virus, fusarium head scab and powdery mildew. On this account, crossbreeding with a disease resistant strain is often carried out. For example, the disease resistant strain Nishino Gold was produced by first crossbreeding "Mokusekko-3", which is resistant to barley yellow mosaic virus, with Nankei B4718, which is obtained by backcrossing with Azuma Golden, and then by crossbreeding twice with the parental strain Haruna Nijo. This transfer of barley yellow mosaic virus resistant characteristic took 20 years to complete, even though it involved only a single gene.

Today, requirements for a new strain concern not only increased yield but also improved quality. In addition, with the standardization of quality, it has become common practice to grow and promote new strains by working with the consumers. This has encouraged improvement of various crops, such as rice, by means of gene transfer. However, owning to the fact that redifferentiation from the protoplast is difficult, and that the size of genome in barley is more than 10 times greater than that of rice, barley is one crop that needs a major technical breakthrough, particularly in the area of genetic analysis.

The number of chromosomes in barley is 2n = 14, which remains the same among different strains and thus allows for crossbreeding. By crossbreeding, many new cultivars have been released, including Amagi Nijo, Haruna Nijo, Nisaraki Nijo, Nasu Nijo, Tone Nijo, Myogi Nijo, Komaki Nijo, Kinuka Nijo, and Takaho Golden.

However, the conventional crossbreeding is essentially a method of combining new useful traits by crossing and selecting individuals carrying useful traits that complement one another. A drawback of this method, then, is that the traits conferred by genes other than a target gene are also transferred. It therefore takes a long time and tedious trial and error over many generations to remove unwanted traits and fix only superior genes. Another drawback is that it is impossible to predict which combination will appear, necessitating large numbers of individuals to be cultivated and thereby requiring a large cultivation area for breeding.

With a recent breeding method using genetic engineering, crops can be improved more suitably to their purposes and in a shorter period of time than conventionally, because it does not require crossbreeding. For example, a gene involved in a specific trait may be cloned and transferred into a plant to produce a recombinant plant. Theoretically, by knowing a clear relationship between functions of a gene and its phenotype, only desirable traits can be transferred, which, in turn, enables planned breeding to be carried out according to intended use.

Breeding using a genetic marker is one method of breeding employing genetic engineering. The genetic marker is linked to a useful gene, and always appears as a DNA band when the trait of the gene is expressed. Some of the methods available to obtain a genetic marker include RFLP, RAPD, AFLP, and PCR-RELP. For example, in RAPD, DNA of a given sequence is used as a primer to amplify DNA by PCR. In order to find the band pattern of DNA, the amplified DNA is dyed and electrophorased on agar gel, and the resulting DNA band pattern is analyzed. When a specific polymorphic band of interest confers a useful trait, this polymorphic band can be used as a genetic marker. The genetic marker can then be used to screen for individuals in crossbreeding. In this way, labor and time required for breeding can be reduced.

The genetic marker is for finding a gene polymorphism among different strains with different traits, and it is sometimes referred to as the first generation gene polymorphism marker. Finding such genetic marker one at a time is extremely laborious. Further, since the number of genetic markers in a genome is only about several hundreds of thousands, there is a limit in the amount of genetic information that can be obtained from these genetic markers.

Single nucleotide polymorphism (may be referred to simply as "SNP" hereinafter) has caught the attention as the next generation polymorphism marker. SNP is a base difference among different strains, and it is believed that the number of SNPs in a genome is on the order of several hundreds of thousands to several millions. Despite the large numbers, the SNP can be studied with considerable ease. Further, the results can be represented by a digital signal (0, 1), which is convenient in terms of information processing. Further, with the development of fast and large-capacity SNP typing technique for actual application, and with the automation of typing in sight, expectations are high for the SNP as a highly useful and easy-to-use polymorphism marker.

Not all SNPs in a genome necessarily have important meaning. SNPs (cSNP (coding SNP)) that bring about a change in amino acid in a translation region of a protein, or SNPs (rSNP (regulatory SNP)) in a regulatory region, such as a promoter region or intron, that affect the amount of gene expression are known to be important because these SNPs are very likely to be involved in phenotypic change. On the other hand, SNPs that are not strongly associated with the phenotypes are considered to be less important. Examples of such SNPs include sSNP (silent SNP) that do not bring about a change in amino acid in a translation region of a protein, and some gSNPs (genome SNP) occupying sites not associated with structural genes. Thus, despite large numbers, SNPs can be highly useful as a polymorphism marker among different strains, when only the cSNP and rSNP are considered.

In order to put into perspective a relation between an expressed gene in barley and a useful trait conferred by the gene, it is effective to study SNPs among different barley strains in sites associated with structural genes, e.g., between a strain with a useful trait, for example, such as Haruna Nijo or Akashinriki used as a crossing parent, and a wild-type barley. If cSNPs or rSNPs are stably found among different strains of barley, they can be used in gene mapping and breeding. For example, these cSNPs or rSNP can be used in crossbreeding as a marker to select strains with fewer undesirable traits and thereby find superior individuals.

By safe estimate, a set of SNPs obtained by the analysis of total expression genes contains SNPs that are associated with useful traits. Then, such a set of SNPs can be used as a marker for breeding.

However, owning to the fact that the genome size of barley (Hordeum) (about 5,000 Mbp) is greater than that of rice by more than 10 fold, and that the association of the genome with structural genes is unclear, it is difficult to analyze SNP in barley from the result of genome analysis.

Note that, some of the EST sequence data acquired by the inventors of the present invention in the EST project on barley have been registered in a data bank (described later) (see "Center for Information Biology and DNA Data Bank of Japan. National Institute of Genetics" (accession number: AV938080, etc.), available on the telecommunication line).

The present invention was made in view of the foregoing problems, and an object of the invention is to find characteristic base sequences of a gene among different strains of barley in sites associated with structural genes, more specifically, between a wild-type barley and particular strains used as a crossing parent, including Haruna Nijo and Akashinriki.

The present invention finds characteristics base sequences of a gene among different strains by finding single nucleotide polymorphism (SNP) in the gene. In this connection, the invention discloses the usefulness of oligonucleotides with SNP sites, and provides a method of use of SNP as a polymorphism marker among different strains in a wide range of applications. Another object of the invention is to provide a breeding method using the SNP.

### DISCLOSURE OF INVENTION

The genome size of barley (Hordeum vulgare) is about 5,000 Mbp, which is more than 10 times greater than that of rice, and the association of the genome with structural genes is unclear. This has made it difficult to analyze the SNP in barley from the result of genome analysis. From the result of EST (expressed sequence tag) project using the cDNA sequence stock in cDNA libraries of barley, the inventors of the present invention have conducted SNP analysis, and found that large numbers of SNPs stably exist in EST among different strains.

Specifically, in the analysis conducted by the inventors of the present invention in the EST project on barley (Hordeum vulgare), mRNA was extracted from tissues of different strains, and cDNA was synthesized using the mRNA as a template. The cDNA was cloned and groups of clones carrying overlapping inserts were assembled to obtain clone sequences (such assembled sequences will be called "CONTIGS" hereinafter). The result of sequence analysis, however accurate it was, revealed that the sequence contained a site whose base cannot be specified to a single particular base. This was identified as a base difference (SNP) among different barley strains. The CONTIG sequences are prepared from the cDNA libraries, and they correspond to expressed regions (part of or all of exons) of the barley genome. Therefore, the CONTIG sequences are very likely to contain cSNPs among different types of SNPs. The inventors of the present invention proceeded to carry out further analysis of the CONTIG sequences, and screened for a single nucleotide polymorphism at which nonsynonymous substitution occurred, and thereby found important SNPs among different barley strains.

Specifically, the present invention is characterized by the following features (A) through (J).
(A) A composition for testing or identifying single nucleotide polymorphism in a gene, the composition containing an oligonucleotide with a continuous DNA sequence of seven or more nucleotides, or a complementary sequence thereof, that contains at least one polymorphic site whose base is represented by a universal code, the oligonucleotide being identified by a DNA sequence set forth in any one of SEQ ID NOs: 1 through 17, 267 through 320, 990 through 1183, 4642 through 4763, and 9107 through 9640.
(B) A composition for testing or identifying single nucleotide polymorphism in a gene, the composition containing an oligonucleotide with a continuous DNA sequence of seven or more nucleotides, or a complementary sequence thereof, that contains at least one polymorphic site at which nonsynonymous substitution occurs and whose base is represented by a universal code, the oligonucleotide being identified by a DNA sequence set forth in any of SEQ ID NOs: 1 through 4, 267 through 278, 990 through 1016, 4642 through 4670, and 9107 through 9640.
(C) A method for testing or identifying single nucleotide polymorphism in a gene of a subject based on at least one polymorphic site, or a complementary site thereof, whose base is represented by a universal code, the polymorphic site being identified by a DNA sequence set forth in any one of SEQ ID NOs: 1 through 17, 267 through 320, 990 through 1183, 4642 through 4763, and 9107 through 9640.
(D) A method of testing or identifying single nucleotide polymorphism in a gene of a subject based on at least one polymorphic site, or a complementary site thereof, at which nonsynonymous substitution occurs and whose base is represented by a universal code, the polymorphic site being identified by a DNA sequence set forth in any one of SEQ ID NOs: 1 through 4, 267 through 278, 990 through 1016, 4642 through 4670, and 9107 through 9640.
(E) A method of producing a recombinant, and a recombinant produced thereby, the method including the steps of: isolating a gene that contains at least one polymorphic site, or a complementary site thereof, whose base is represented by a universal code, the polymorphic site being identified by a DNA sequence set forth in any one of SEQ ID NOs: 1 through 17, 267 through 320, 990 through 1183, 4642 through 4763, and 9107 through 9640; and introducing the gene so obtained in a target cell, using a genetic engineering technique.
(F) A method of producing a recombinant, and a recombinant produced thereby, the method including the steps of: isolating a gene that contains at least one polymorphic site, or a complementary site thereof, at which nonsynonymous substitution occurs and whose base is represented by a universal code, the polymorphic site being identified by a DNA sequence set forth in any one of SEQ ID NOs: 1 through 4, 267 through 278, 990 through 1016, 4642 through 4670, and 9107 through 9640; and introducing the gene so obtained in a target cell, using a genetic engineering technique.
(G) A method of producing a recombinant, and a recombinant produced thereby, the method including the steps of: producing hybrid plants by crossbreeding (i) a parent plant A having a gene that contains at least one polymorphic site, or a complementary site thereof, whose base is represented by a universal code, said at least one polymorphic site being identified by a DNA sequence set forth in any one of SEQ ID NO: 1 through 17, 267 through 320, 990 through 1183, 4642 through 4763, and 9107 through 9640, and (ii) a parent plant B without the gene or with an allele that confers single nucleotide polymorphism to the gene; and screening for a hybrid plant with the gene, using the oligonucleotide set forth in (A), or the testing method set forth in (C).
(H) A method of producing a recombinant, and a recombinant produced thereby, the method including the steps of: producing hybrid plants by crossbreeding (i) a parent plant A having a gene that contains at least one polymorphic site, or a complementary site thereof, at which nonsynonymous substitution occurs and whose base is represented by a universal code, the polymorphic site being identified by a DNA sequence set forth in any one of SEQ ID NOs: 1 through 4, 267 through 278, 990 through 1016, 4642 through 4670, and 9107 through 9640, and (ii) a parent plant B without the gene or with an allele that confers single nucleotide polymorphism to the gene; and screening for a hybrid plant with the gene, using the oligonucleotide set forth in (B), or the testing method set forth in (D).
(I) A primer designed to amplify a region of a polynucleotide with a DNA sequence set forth in any one of SEQ ID NOs: 1 through 17, 267 through 320, 990 through 1183, 4642 through 4763, and 9107 through 9640, the polynucleotide region containing at least one polymorphic site, and/or a complementary site thereof, whose base is represented by a universal code.
(J) A primer designed to amplify a region of a polynucleotide with a DNA sequence set forth in any one of SEQ ID NOs: 1 through 4, 267 through 278, 990 through 1016, 4642 through 4670, and 9107 through 9640, the polynucleotide region containing at least one polymorphic site, and/or a complementary site thereof, at which nonsynonymous substitution occurs and whose base is represented by a universal code.

As used herein, "polymorphic site at which nonsynonymous substitution occurs" may be:
(a) a polymorphic site that causes replacement of an amino acid encoded by a codon including the polymorphic site, or that changes a codon containing the polymorphic site to a stop codon, according to the base of the polymorphic site; or
(b) a polymorphic site that causes replacement of an amino acid encoded by a codon including a complementary site of the polymorphic site, or that changes a codon containing a complementary site of the polymorphic site to a stop codon.

That is, in case (a), the DNA sequences specified in the sequence table are the sense strand, and the polymorphic site itself causes nonsynonymous substitution of an amino acid. On the other hand, in case (b), the complementary sequences are the sense strand, and the complementary site of the polymorphic site causes nonsynonymous substitution of an amino acid.

As used herein, "universal code" is a single letter code that denotes a base of the assembled CONTIGS when the type of base cannot be specified to a specific base, and it indicates that the base is one of at least two kinds of bases selected from A, G, C, or T. Each letter is defined as follows.
R = A or G
Y = C or T
M = A or C
K = G or T
S = G or C
W = A or T
H = A, T, or C
B = G, T, or C
V = G, A, or C
D = G, A, or T
N = A, C, G, or T

In the description of the present invention (including the sequence table), base variations among Haruna Nijo, Akashinriki, and wild-type barley are indicated by the universal code. (Note: the sequence table denotes bases in small letters, including the universal code.)

Further, as used herein the term "polymorphism" means base variations among the different barley strains Haruna Nijo, Akashinriki, and wild-type barley at corresponding positions on DNA (cDNA or genomic DNA). In other words, the "polymorphism" confers a difference between a particular gene and its allele (or between complementary sequences of the gene and its allele). Particularly, in the present invention, "polymorphism" is a single nucleotide polymorphism that is stably preserved in a DNA sequence among different barley strains.

The "polymorphism" of the present invention was found from a combination of barley strains Haruna Nijo, Akashinriki, and wild-type barley (Hordeum vulgare ssp. spontaneum) (hereinafter simply "H. spontaneum") (accession number OUH602, Barley and Wild Plant Resource Center, Research Institute for Bioresources, Okayama University). Haruna Nijo is a widely cultivated strain of two-rowed barley for malting. Akashinriki is six-rowed barley cultivated for food. H. spontaneum is wild-type barley that is believed to be an ancestral form of barley that belongs to a group of two-rowed barley native to west Asia. In short, these different barley strains are genetically separated from one another, even though they are the same species that belongs to the same genus. Such genotypic differences characterize the useful traits of Haruna Nijo and Akashinriki. More specifically, base variations at the polymorphic site may be responsible for the expression of a gene that confers environmental resistance (pest resistance, hostile environment resistance, etc.) to H. spontaneum, and the expression of an allele of the gene in Haruna Nijo and Akashinriki, for example. Further, such base variations may account for the expression of a gene associated with the desirable malting characteristics in Haruna Nijo, and of a gene characteristic to Akashinriki and of its allele in H. spontaneum.

For a fuller understanding of the nature and advantages of the invention, reference should be made to the ensuing detailed description taken in conjunction with the accompanying drawings.

### BRIEF DESCRIPTION OF DRAWINGS

Fig. 1 through Fig. 20 represent base sequences (CONTIGS) represented by SEQ ID NOs: 1 through 17, 267 through 320, and 990 through 1183, in relation to SNP position (polymorphic site) on the CONTIG sequence, base type at the SNP position, evaluation for SNP, screened result of nonsynonymous SNP, and particularly suitable range for designing oligonucleotides.
Fig. 21 through Fig. 30 represent base sequences (CONTIGS) represented by SEQ ID NOs: 4642 through 4763, in relation to SNP position (polymorphic site) on the CONTIG sequence, base type at the SNP position, pseudo polymorphic site, evaluation for SNP, and screened result of nonsynonymous SNP.
Fig. 31 through Fig. 84 represent base sequences (CONTIGS) represented by SEQ ID NOs: 9107 through 9640, in relation to SNP position (polymorphic site) on the CONTIG sequence, base type at the SNP position, amino acid encoded by a codon including the SNP (polymorphic site or its complementary site), presence or absence of amino acid substitution caused by a base difference at the polymorphic site, result of determination whether a translation region (coding region) includes an SNP that causes amino acid substitution, and screened result of nonsynonymous SNP.
Fig. 85 represents base sequences (CONTIGS) represented by SEQ ID NOs: 1 through 17, in relation to reading frame (frame), pseudo polymorphic site, sequence number of amino acid sequence, sequence number of clone sequence, and sequence number of primer.
Fig. 86 through Fig. 93 represent base sequences (CONTIGS) represented by SEQ ID NOs: 267 through 320, and 990 through 1183, in relation to reading frame (frame), pseudo polymorphic site, restriction enzyme, sequence number of amino acid sequence, sequence number of clone sequence, and sequence number of primer.
Fig. 94 through Fig. 97 represent base sequences (CONTIGS) represented by SEQ ID NOs: 4642 through 4763, in relation to reading frame (frame), restriction enzyme, sequence number of amino acid sequence, sequence number of clone sequence, and sequence number of primer.
Fig. 98 through Fig. 135 represent base sequences (CONTIGS) represented by sequence numbers 9107 through 9640, in relation to reading frame (frame), start position of a homologous region with a known sequence, translation region, and translation region number.
Fig. 136 through Fig. 149 represent base sequences (CONTIGS) represented by SEQ ID NOs: 9107 through 9640, in relation to sequence number of amino acid sequence, sequence number of clone sequence, and the number of clone sequences.
Fig. 150 through Fig. 207 show homology search results for the base sequences (CONTIGS) represented by SEQ ID NOs: 1 through 17, 267 through 320, 990 through 1183, 4642 through 4763, and 9107 through 9640.
Fig. 208 and Fig. 209 show parameter settings for Phrap, which is used as an assembly tool for assembling inserts.

### BEST MODE FOR CARRYING OUT THE INVENTION

One embodiment of the present invention is described below with reference to the attached drawings.

The inventors of the present invention have developed cDNA libraries based on mRNA samples extracted from the respective tissues of barley strains Haruna Nijo, Akashinriki, and H. spontaneum, and sequence analysis was carried out for the cDNA clones, the number of which was on the order of several hundreds of thousands, as will be described later in more detail. From the result of sequence analysis, the clones of the respective strains were clustered and assembled into a total of 921 CONTIG sequences.

Based on the CONTIG sequences so prepared, 3048 single nucleotide polymorphisms (SNPs) with base variations among the different strains of barley were detected and identified. From these SNPs, 1039 single nucleotide polymorphisms that cause nonsynonymous substitution ("nonsynonymous substitution SNP" hereinafter) were found.

In the following, one embodiment of the present invention is described based on the foregoing 3048 single nucleotide polymorphisms (SNPs) with nonsynonymous substitution SNPs, and oligonucleotides that are synthesized from a region including at least one of these SNPs.

### (A) Nonsynonymous substitution SNPs among different barley strains

SEQ ID NOs: 1 through 17, 267 through 320, 990 through 1183, 4642 through 4763, and 9107 through 9640 represent 921 CONTIG sequences in which the 3048 single nucleotide polymorphisms (SNPs) were found.

For convenience of explanation, these 921 CONTIG sequences are assigned with numbers (CONTIG numbers), and will be called CONTIG (1) through CONTIG (921). For example, a CONTIG with a CONTIG number (ctg_ID) 1 will be called CONTIG (1).

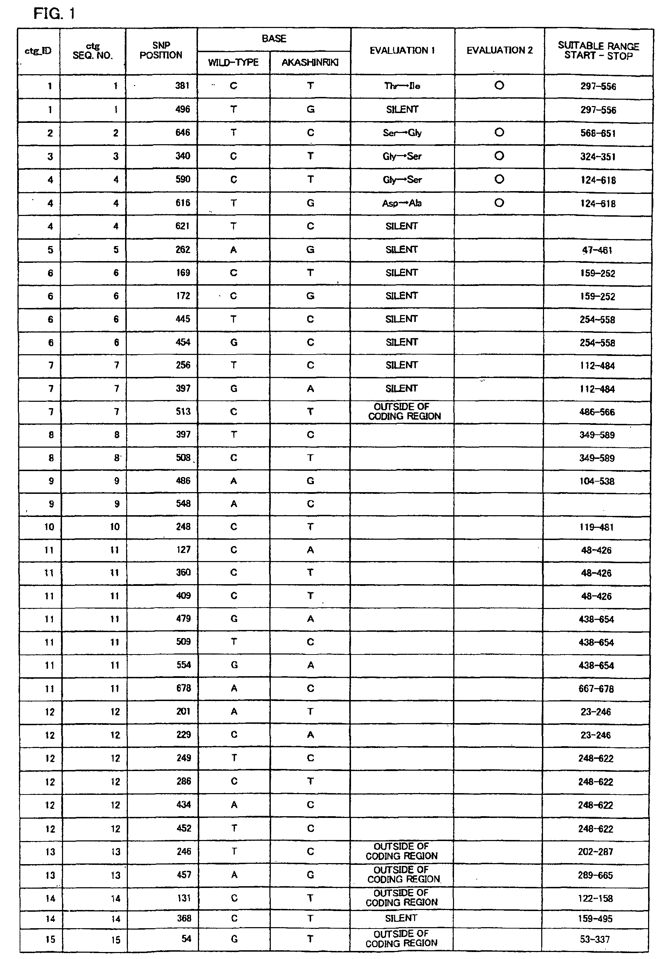

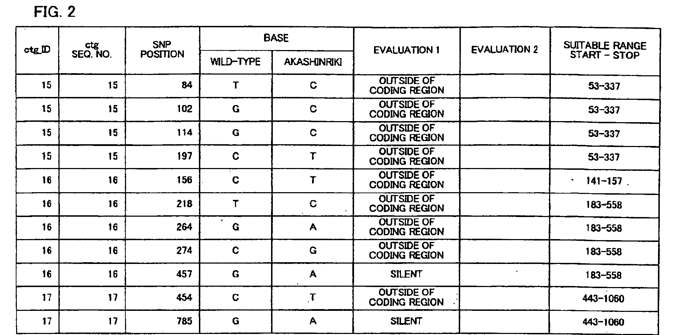

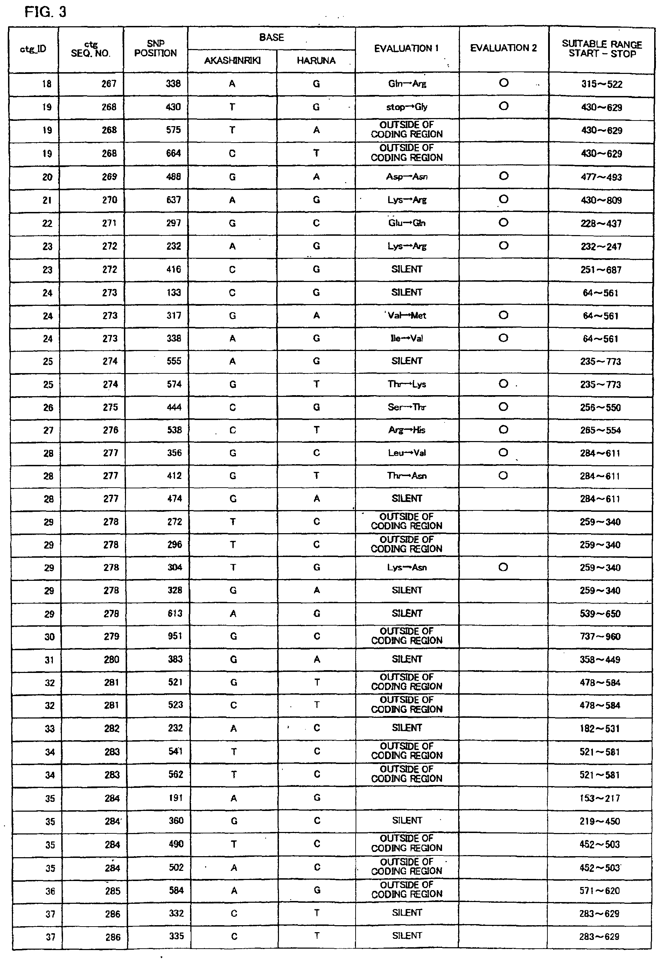

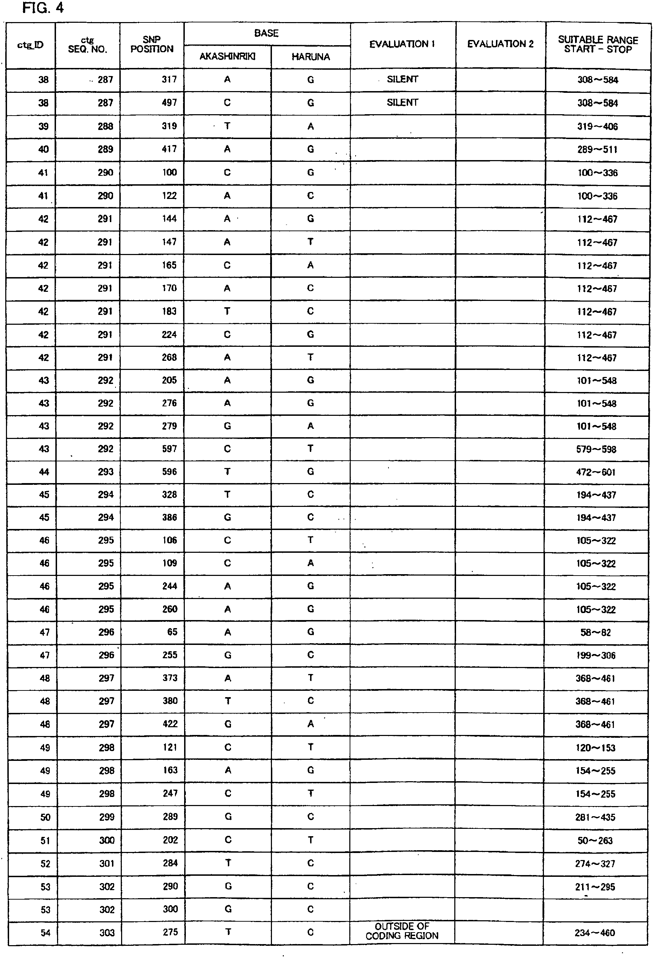

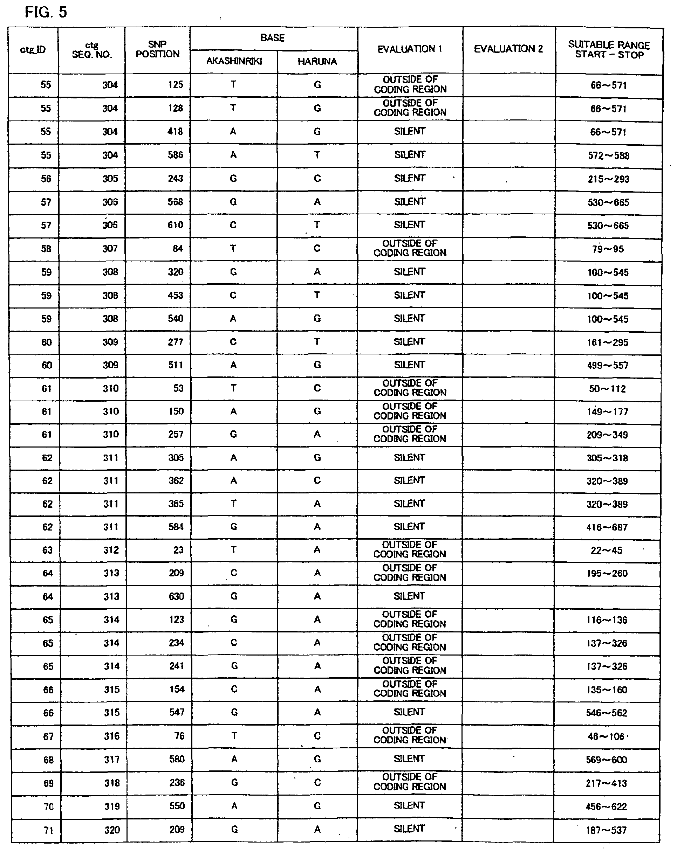

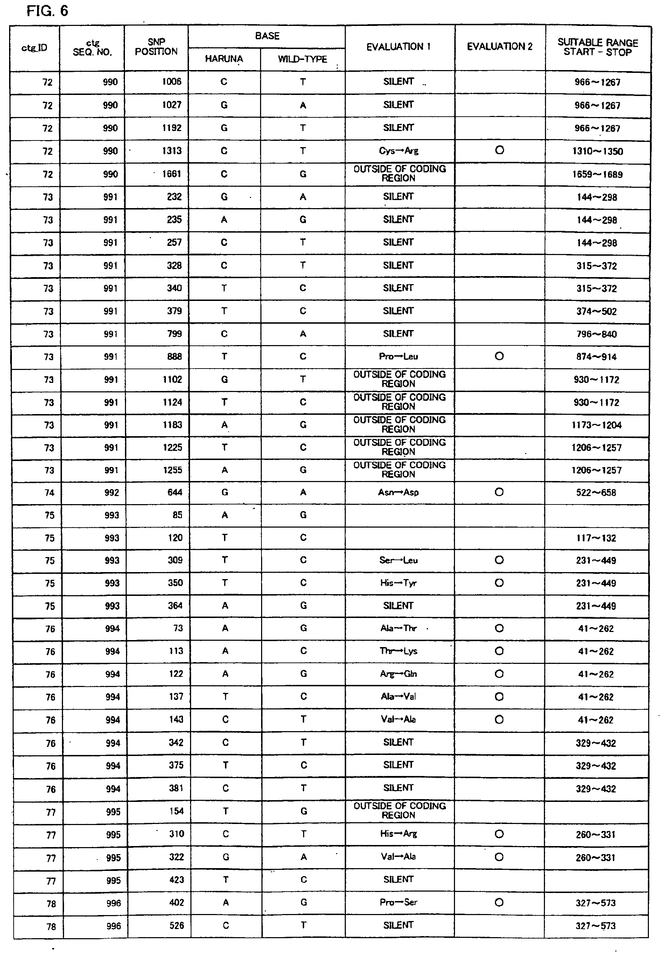

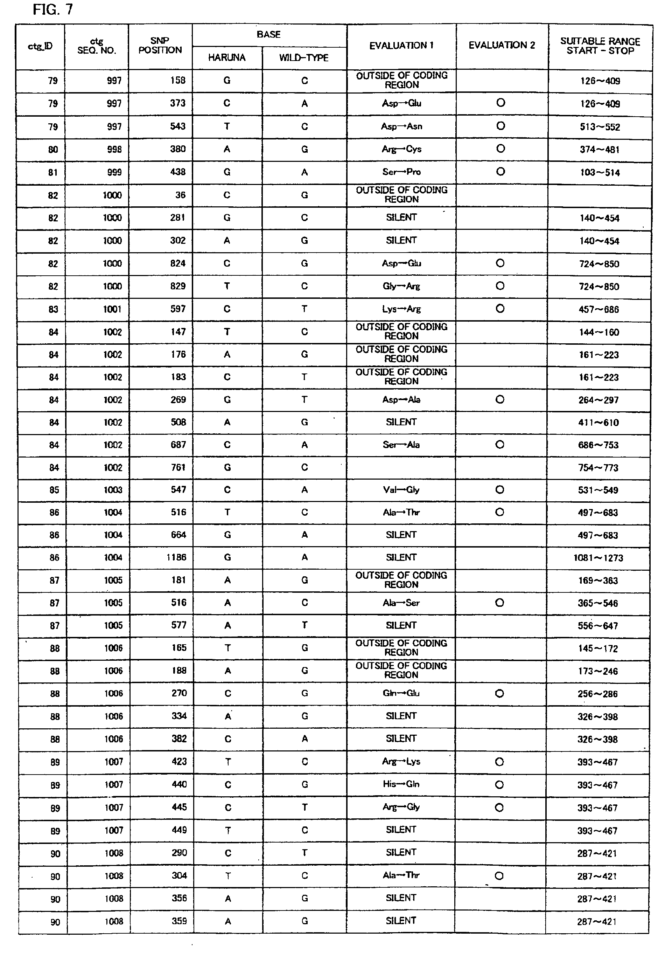

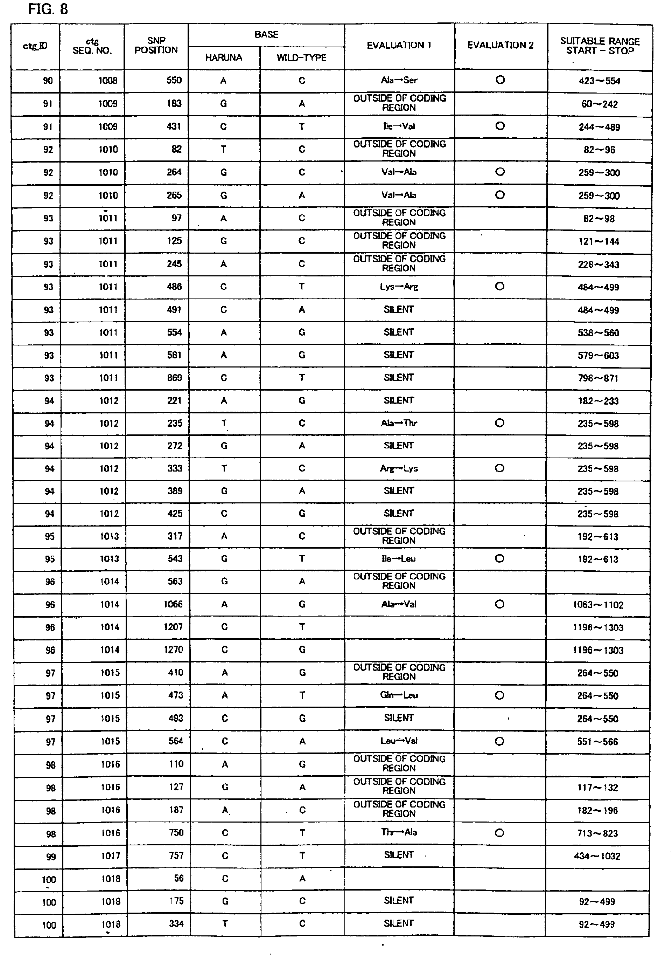

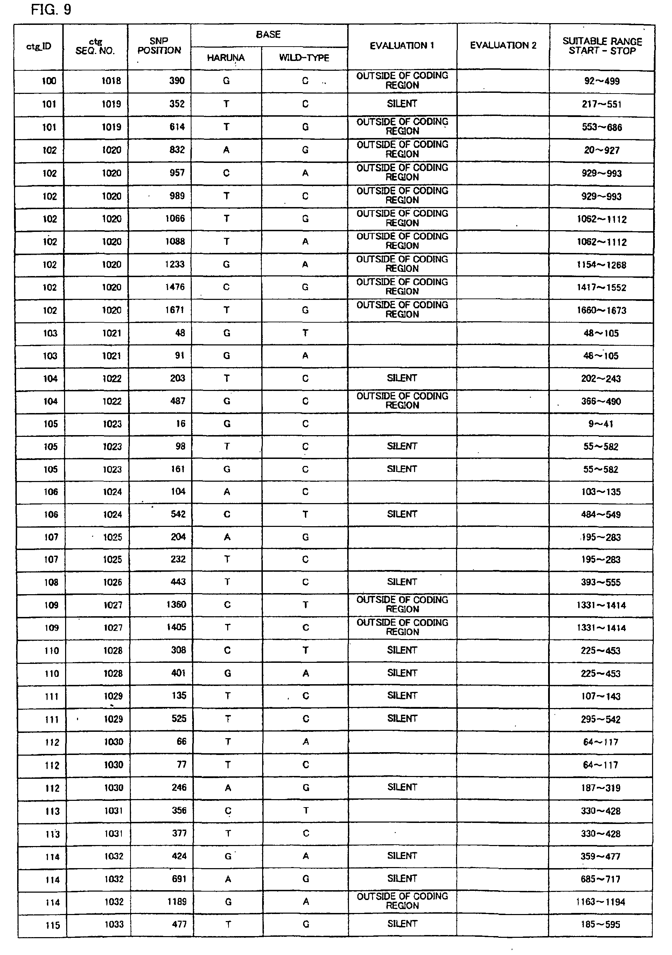

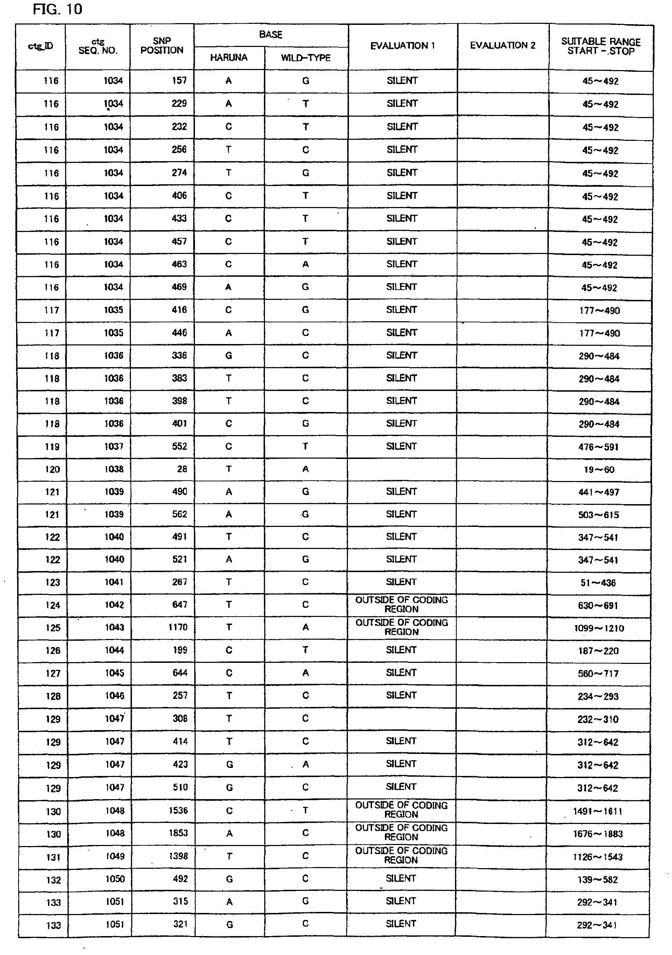

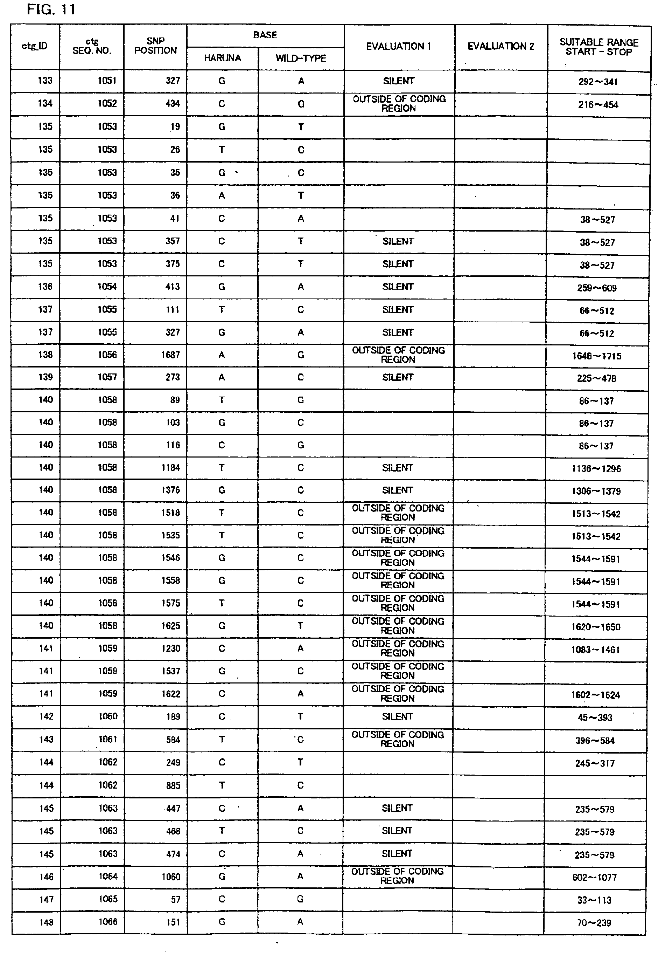

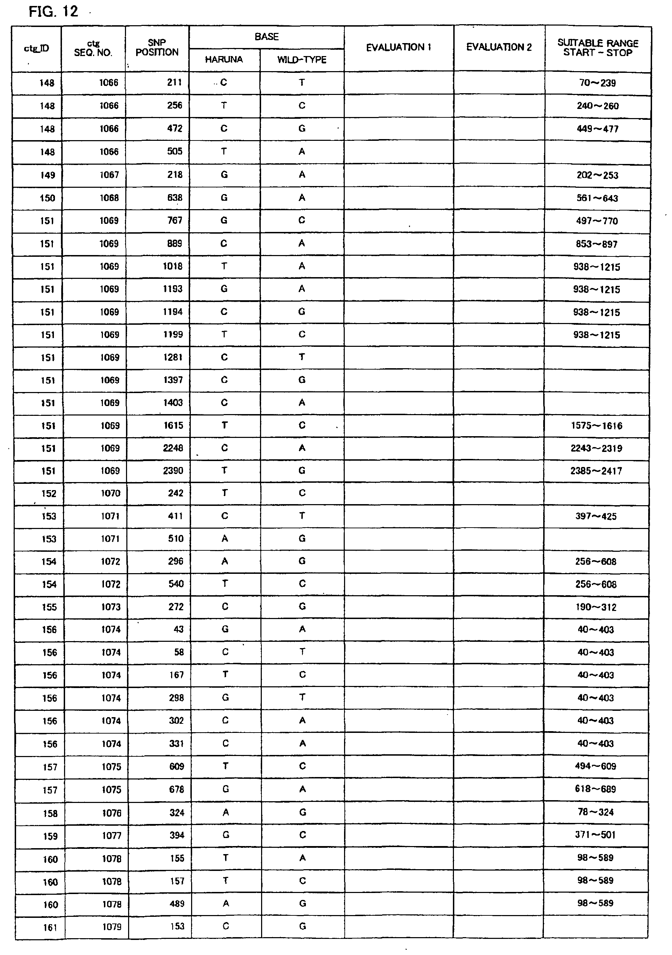

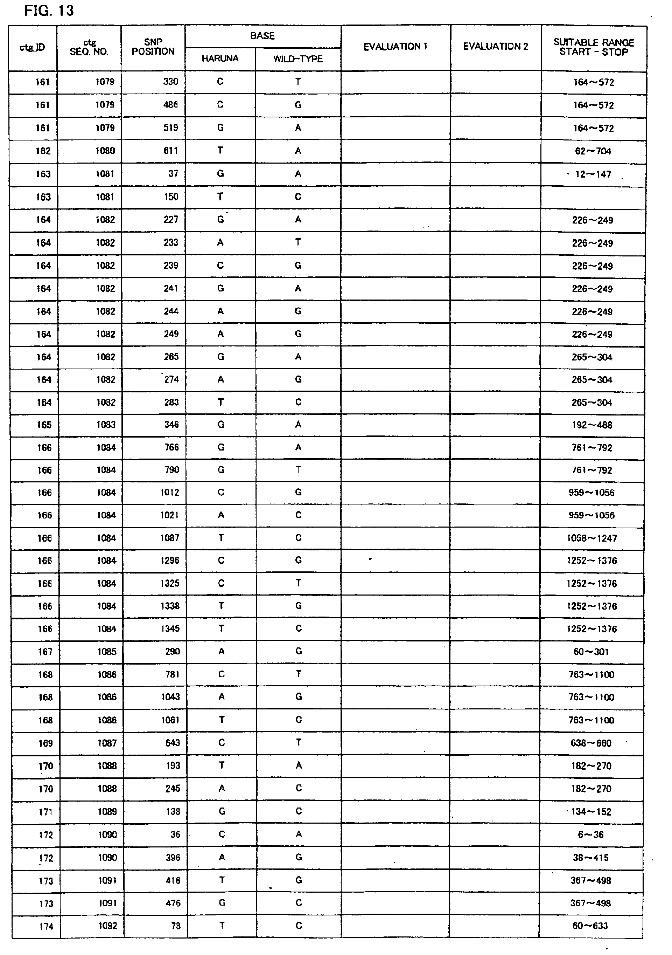

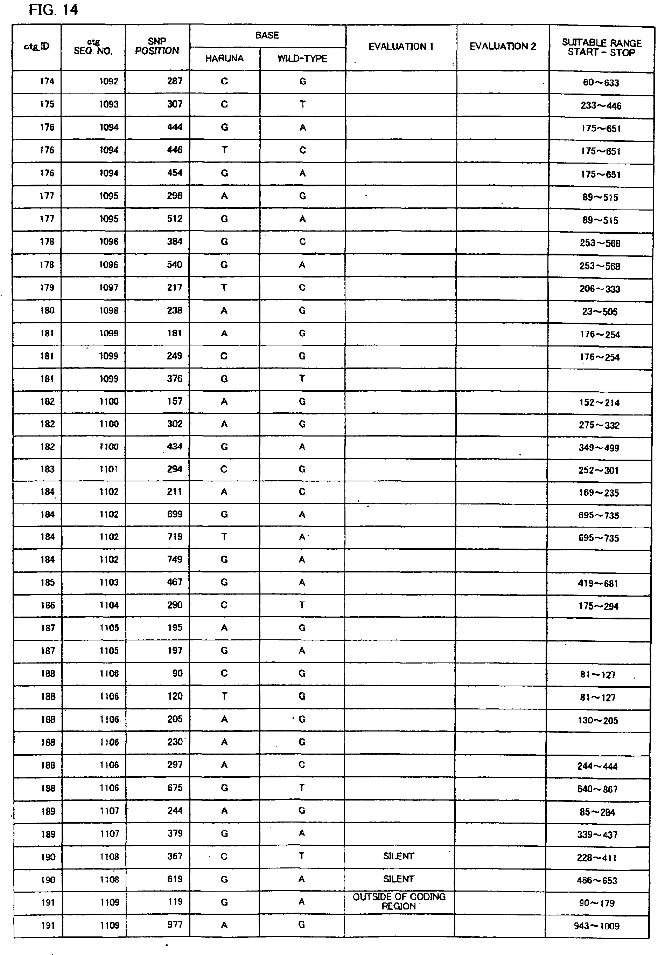

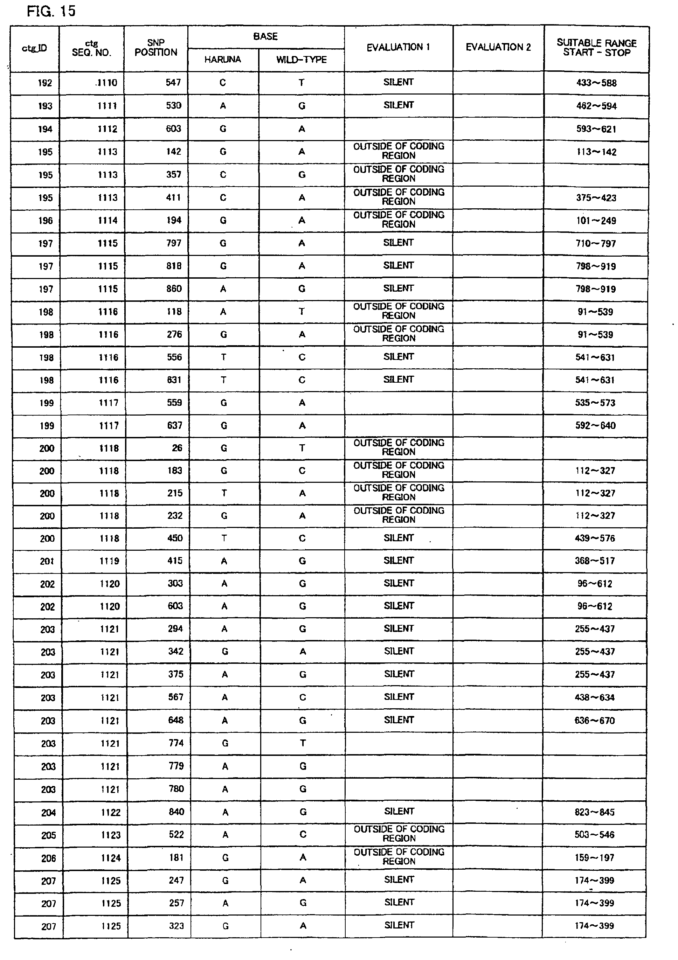

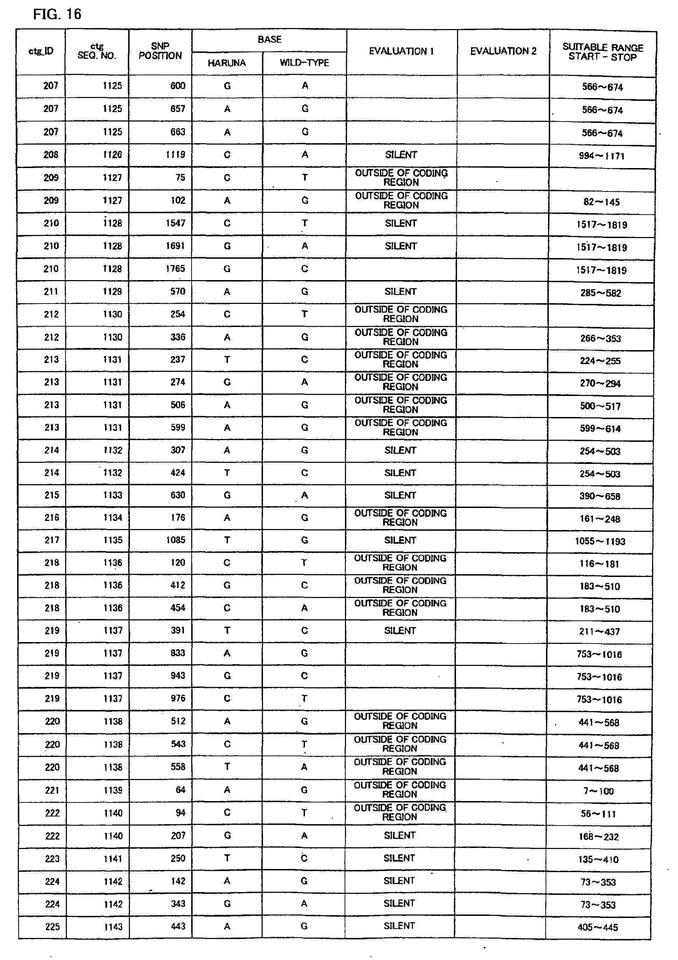

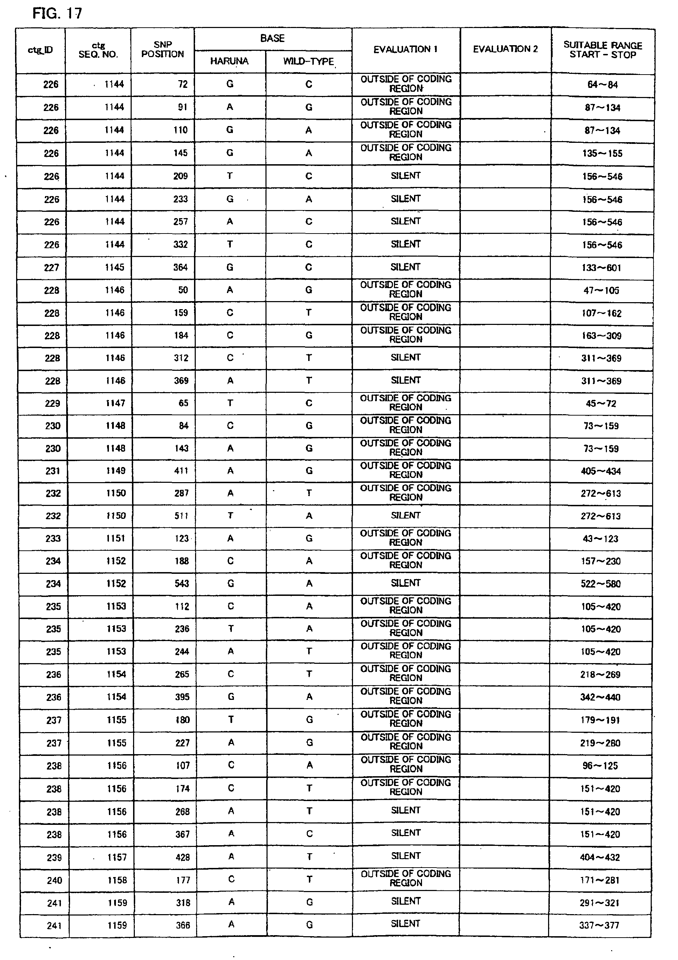

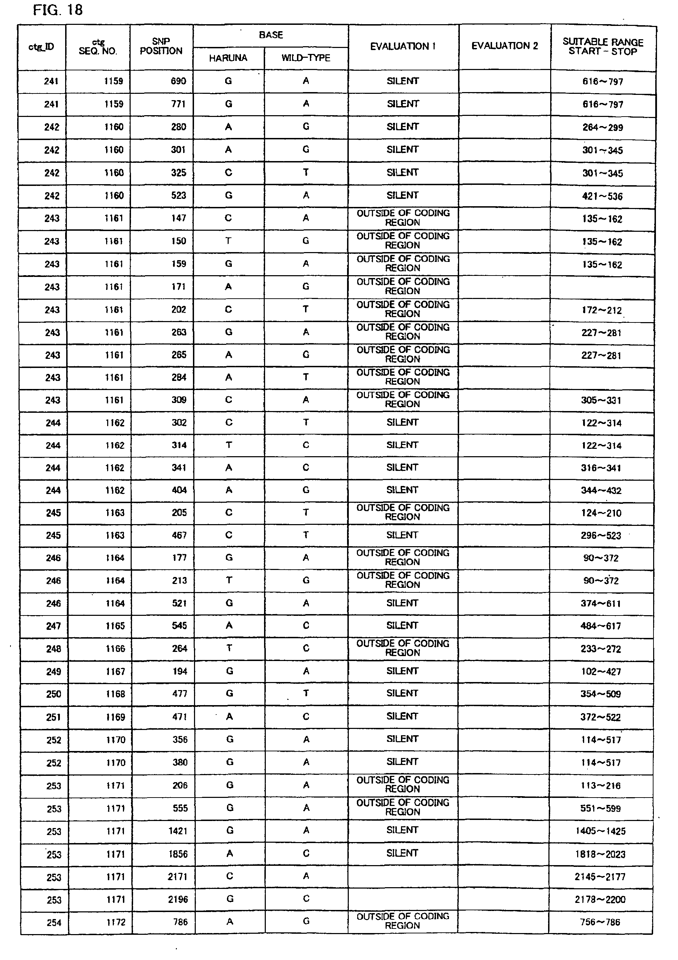

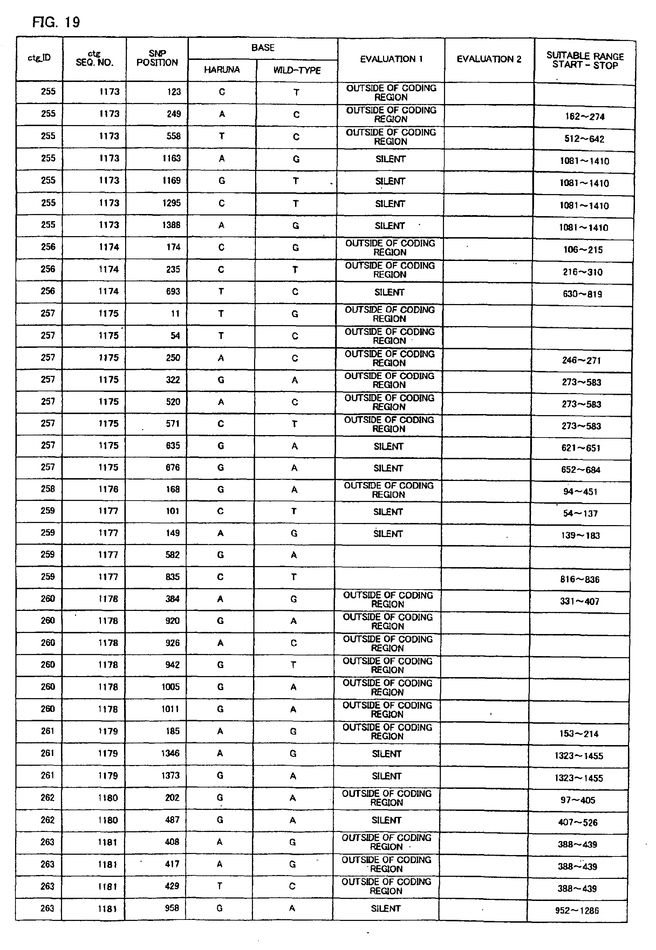

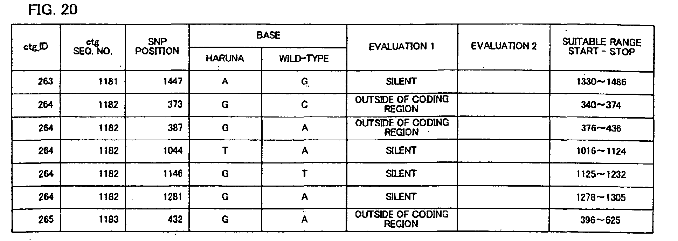

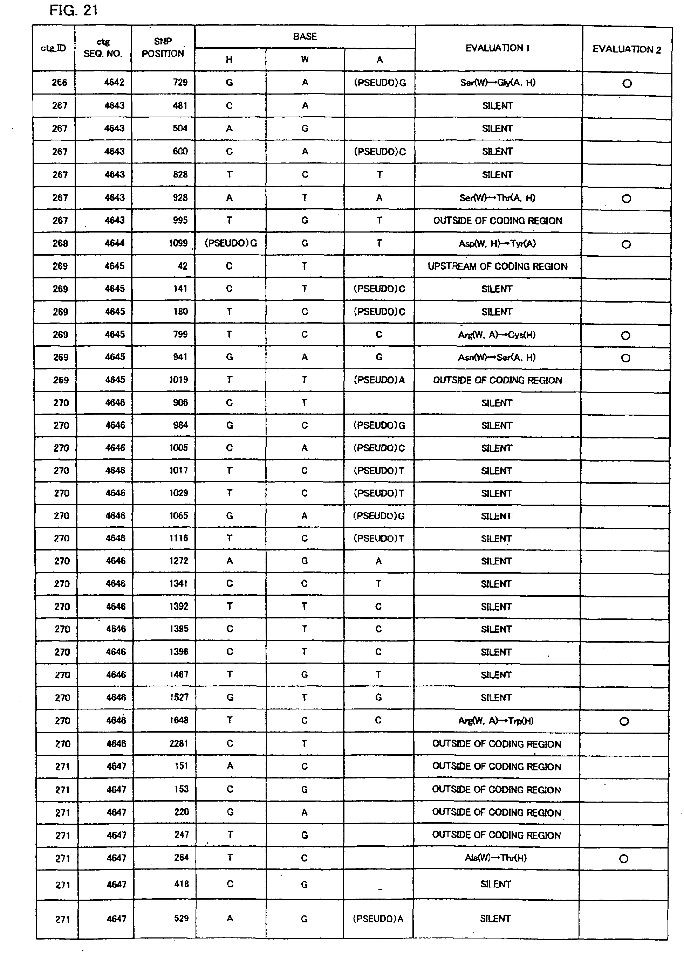

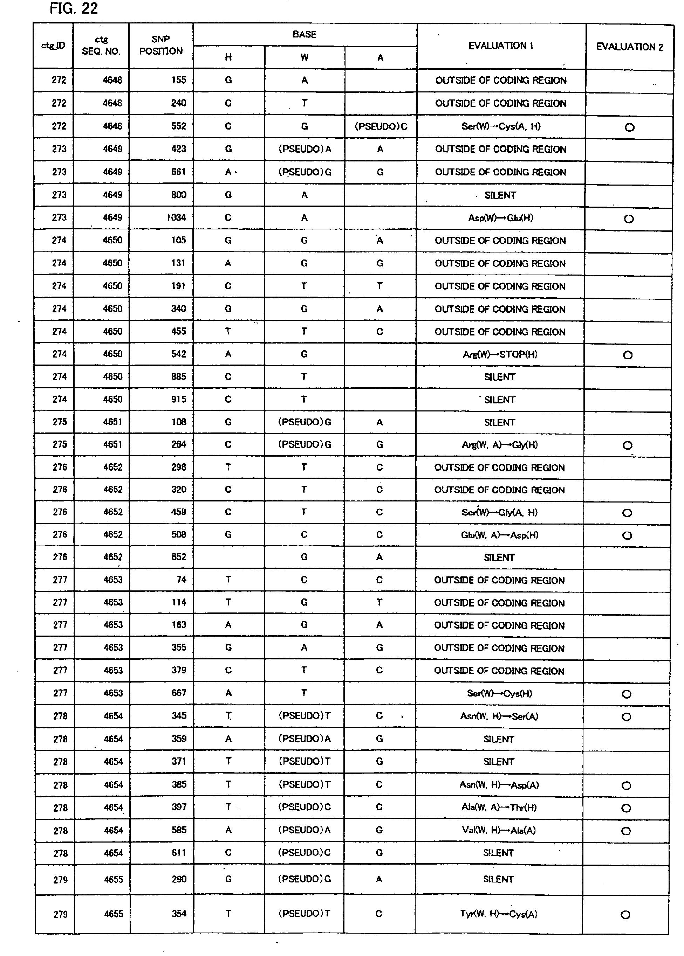

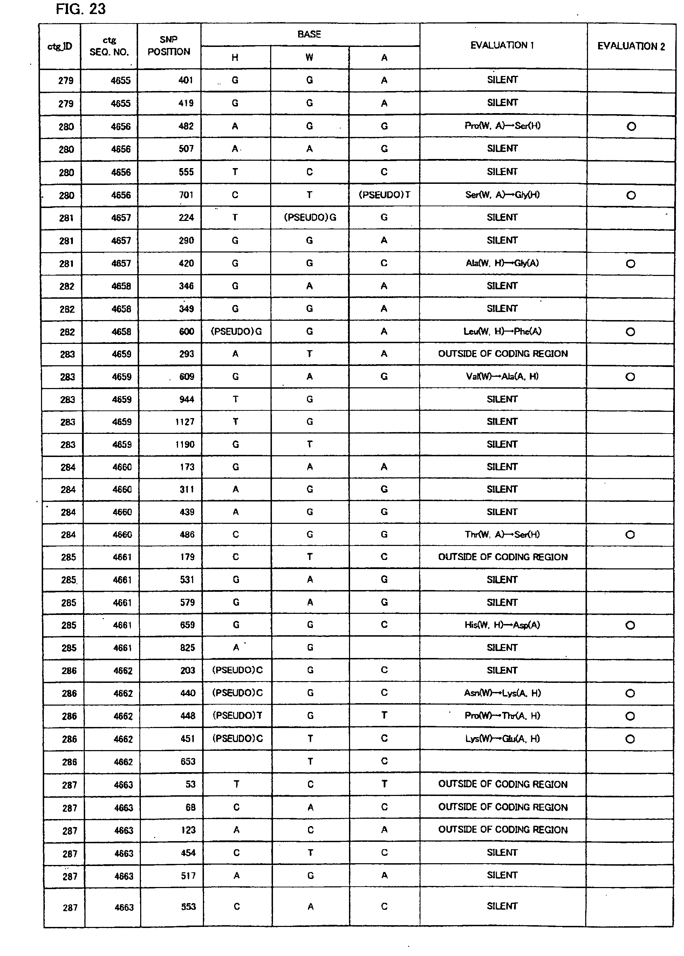

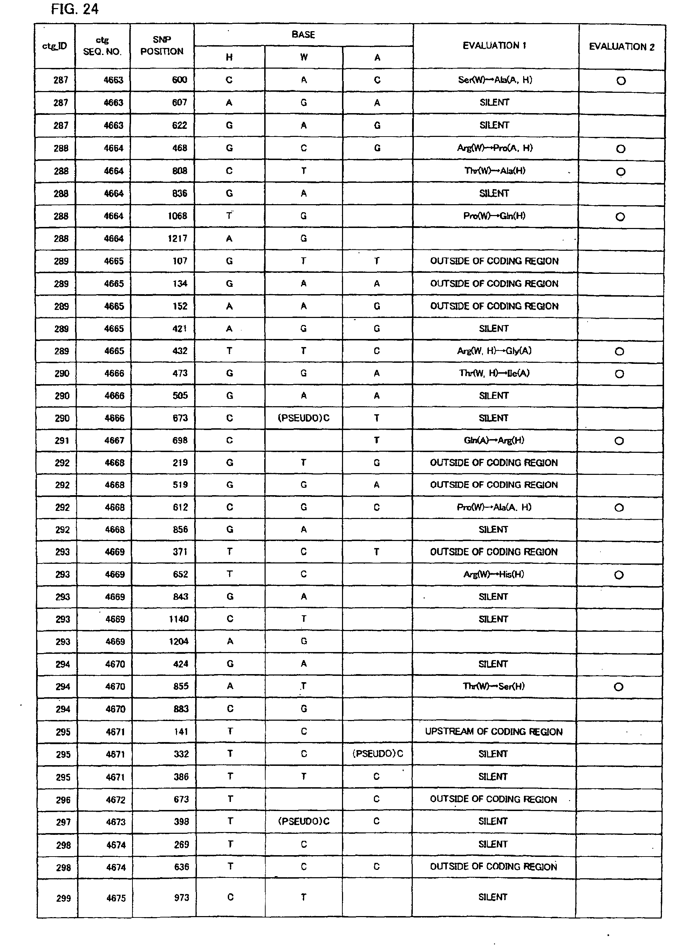

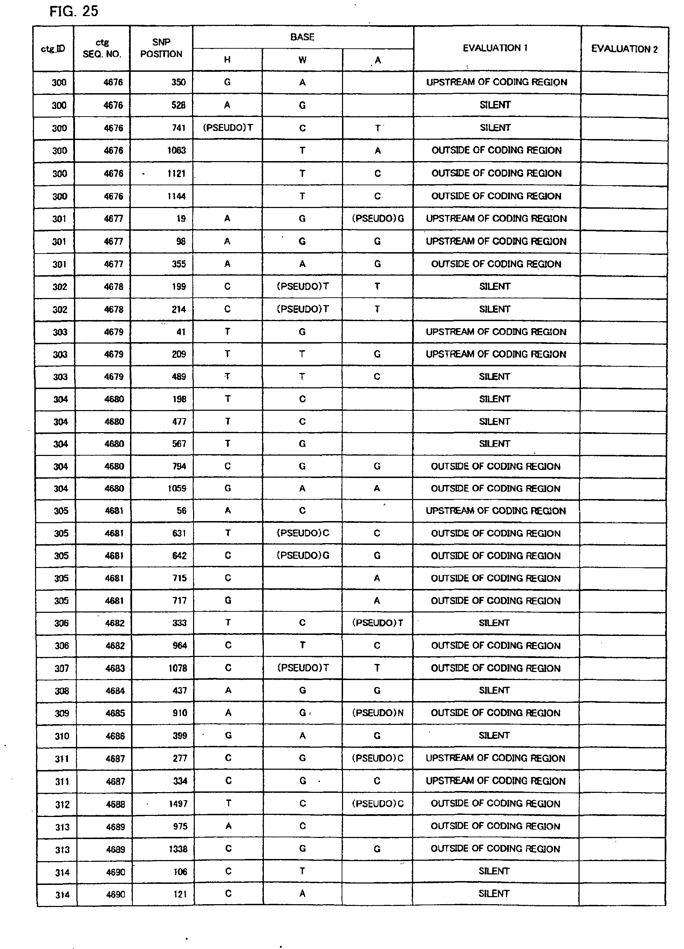

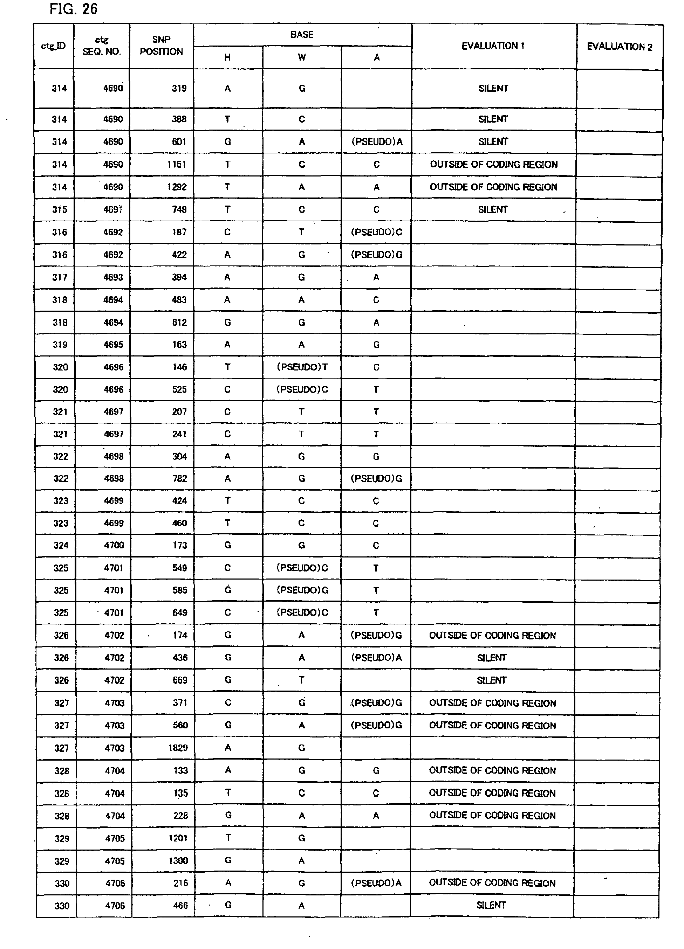

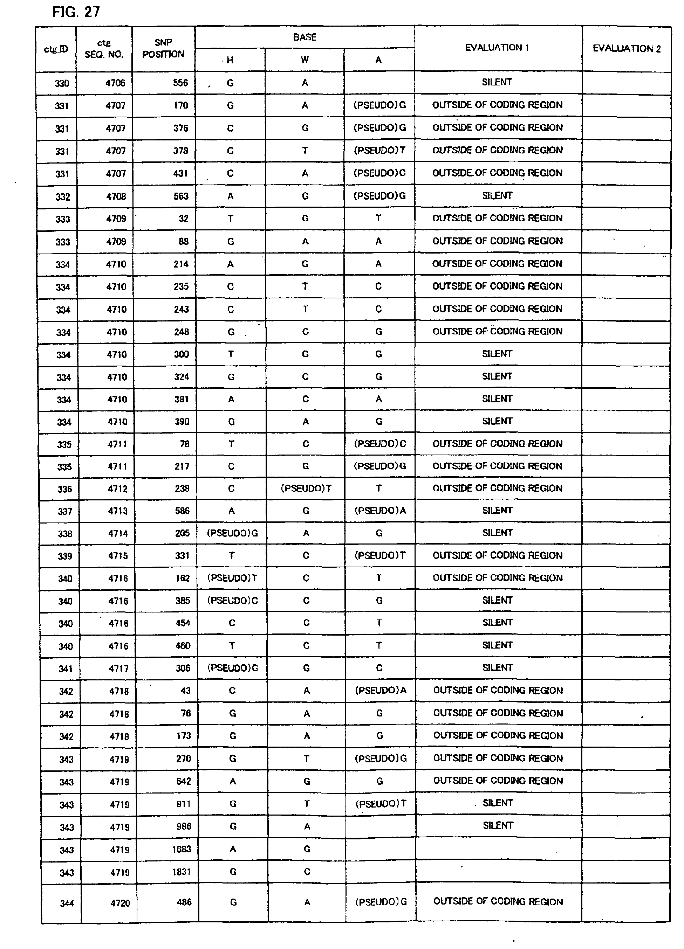

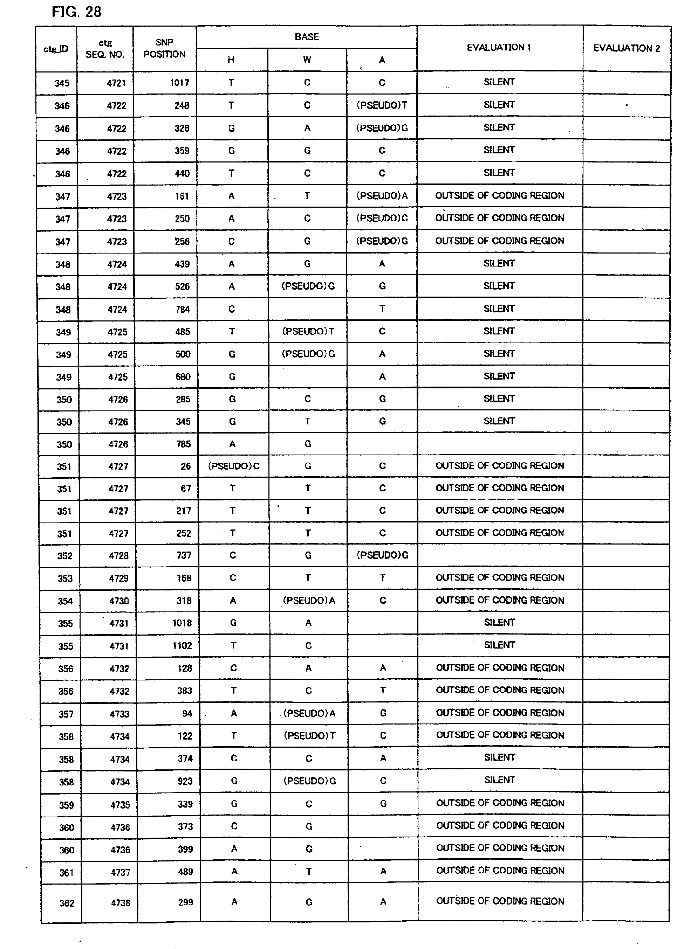

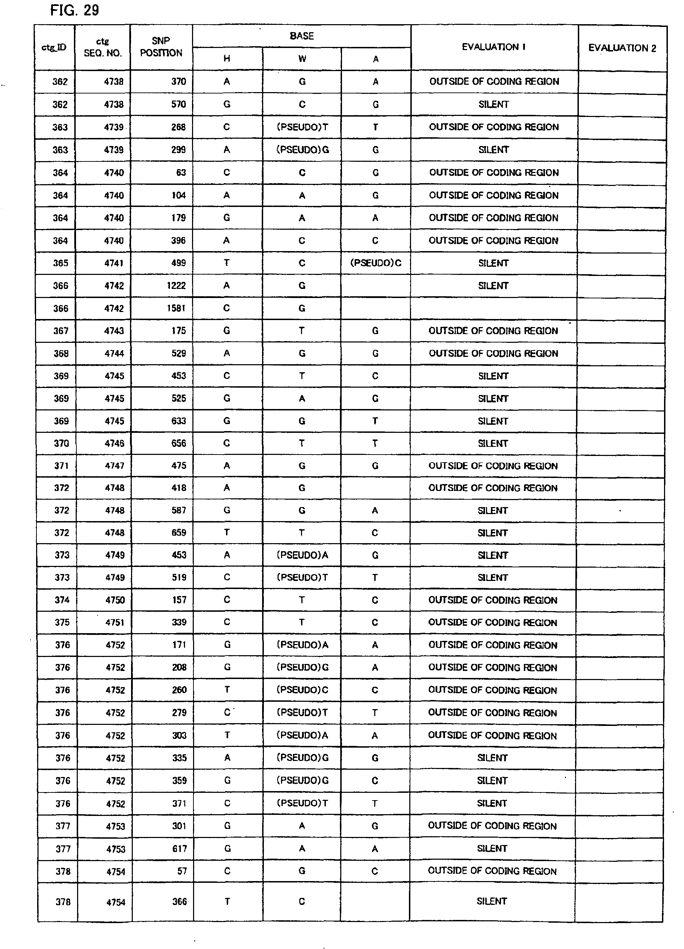

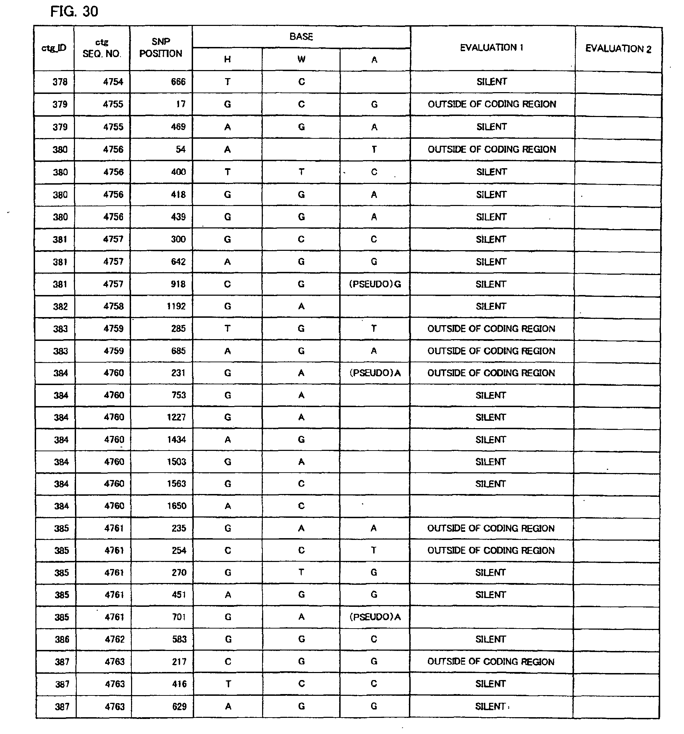

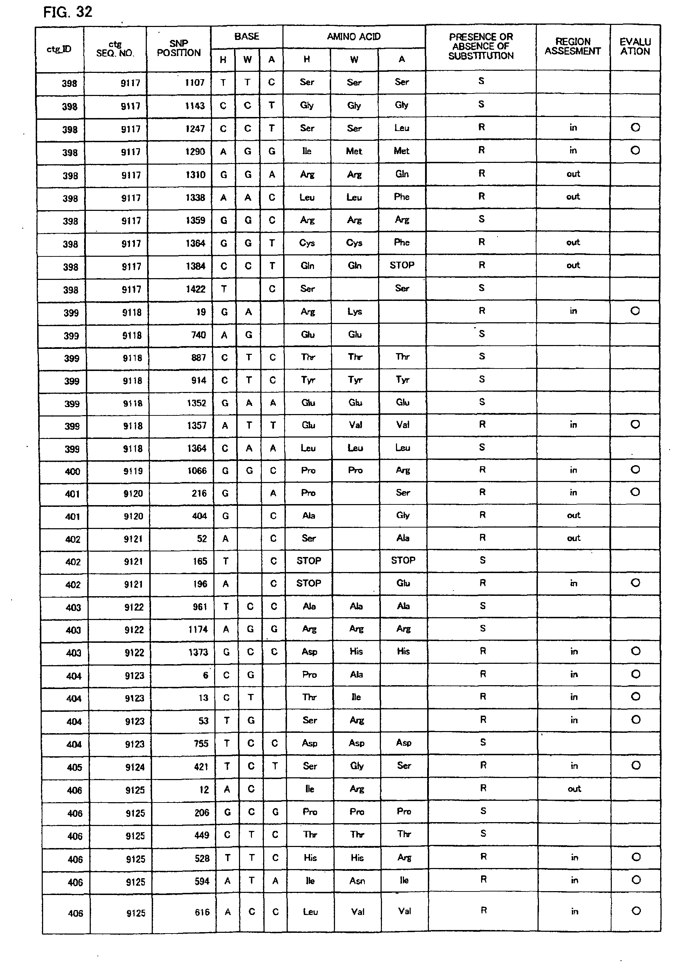

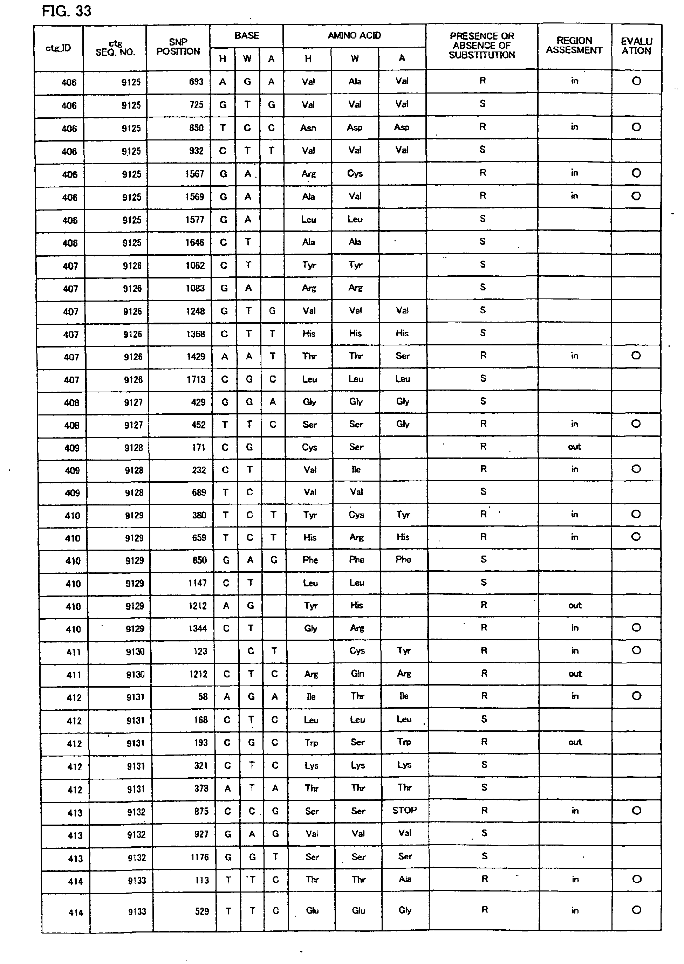

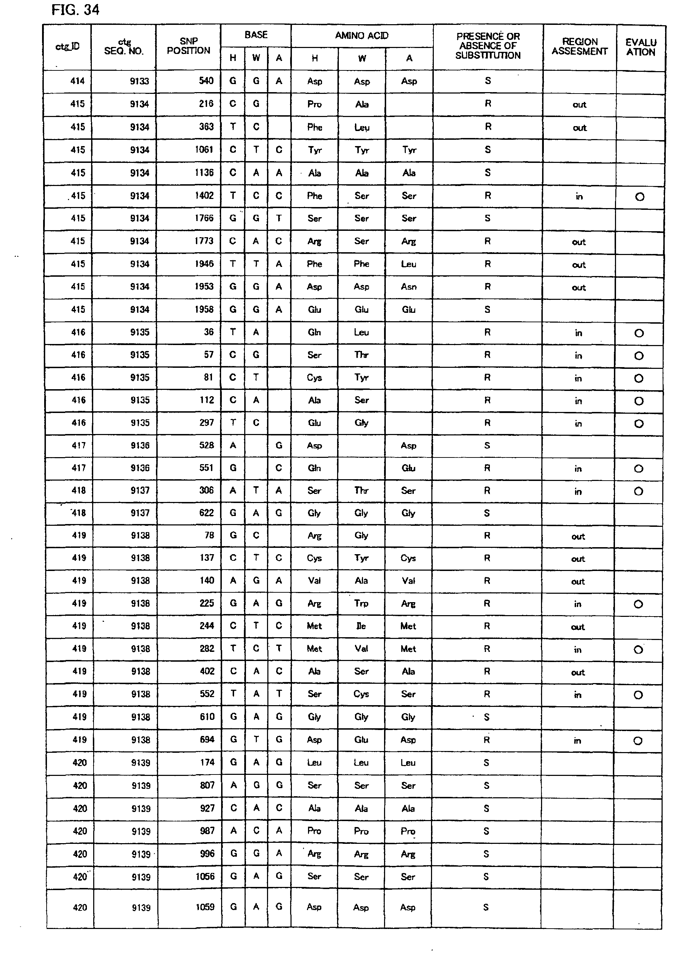

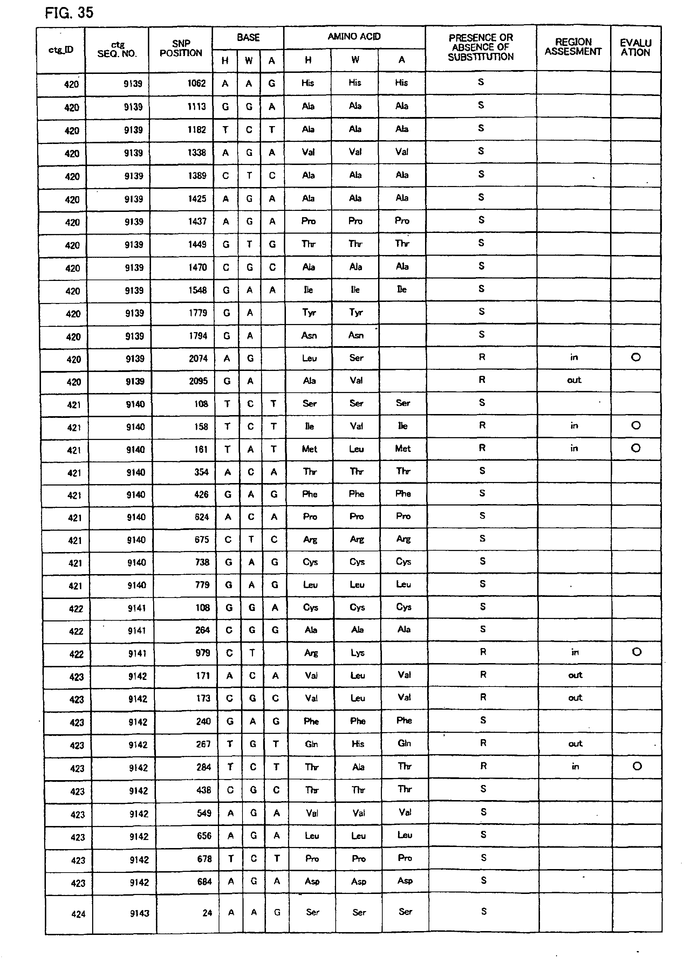

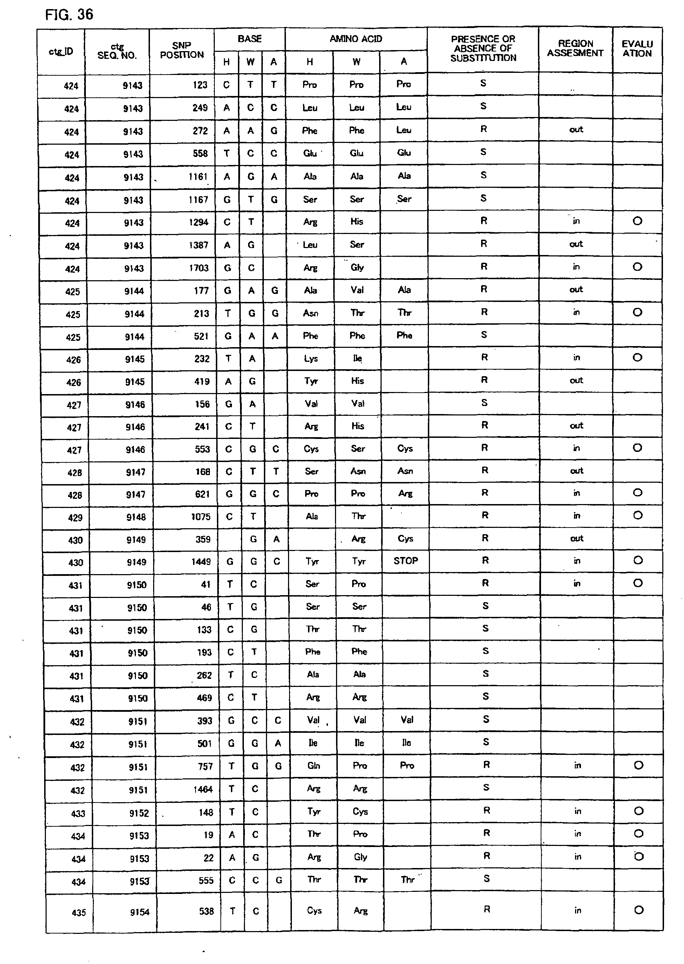

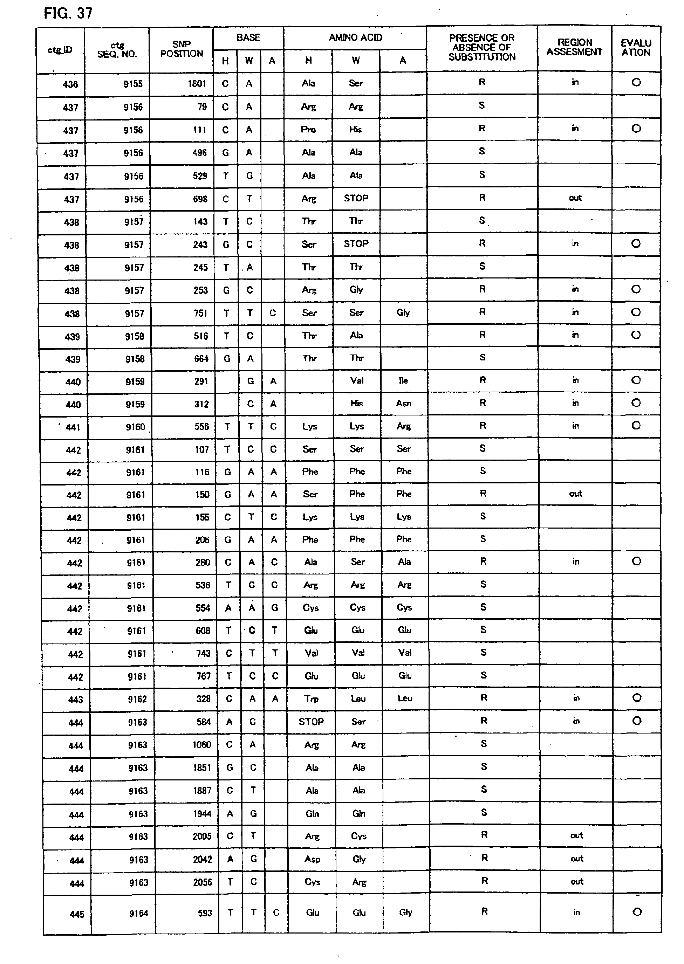

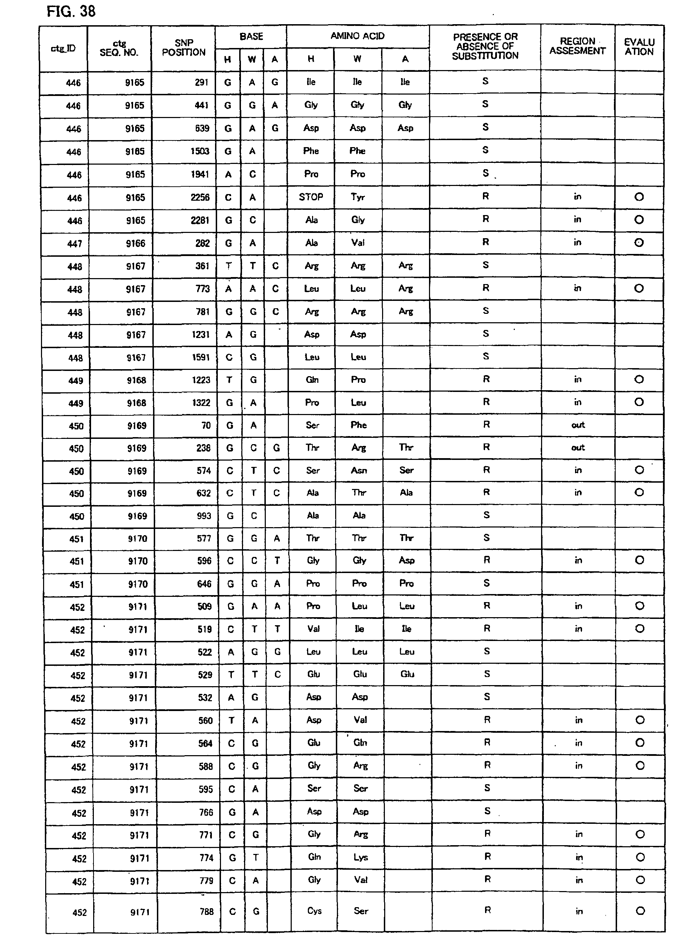

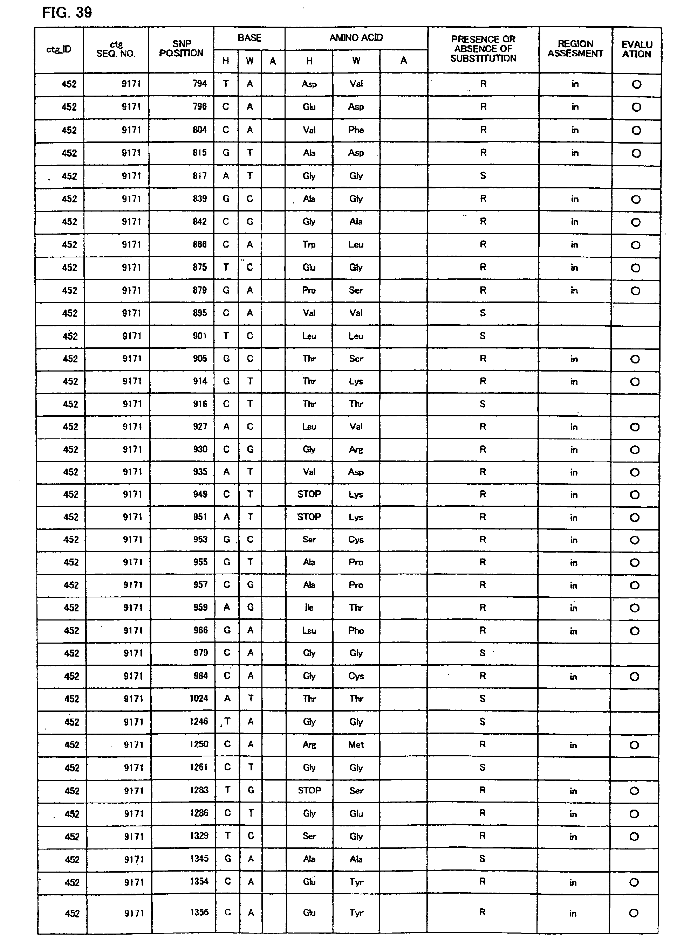

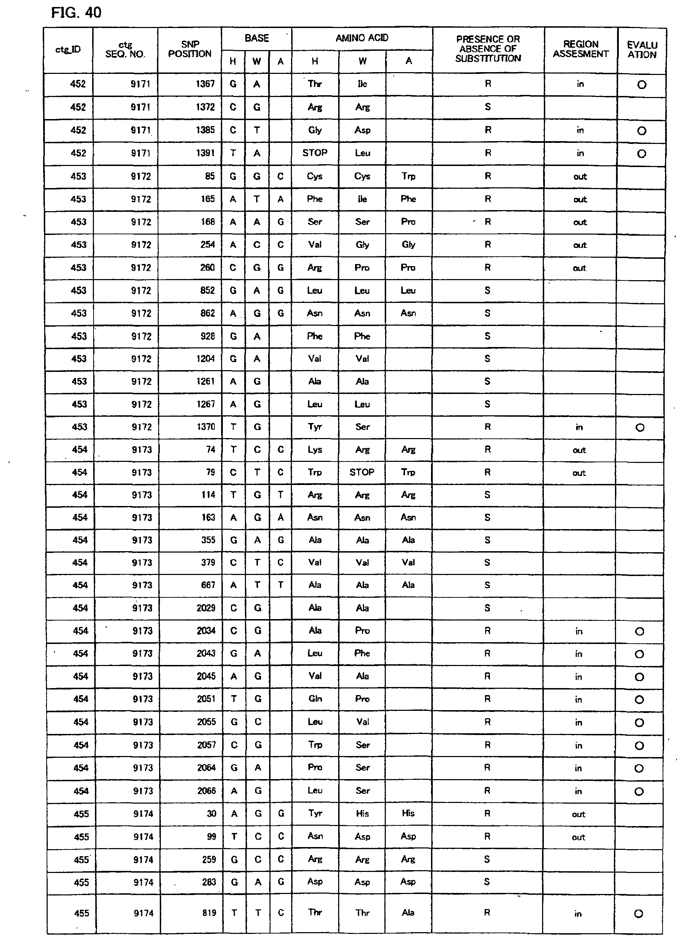

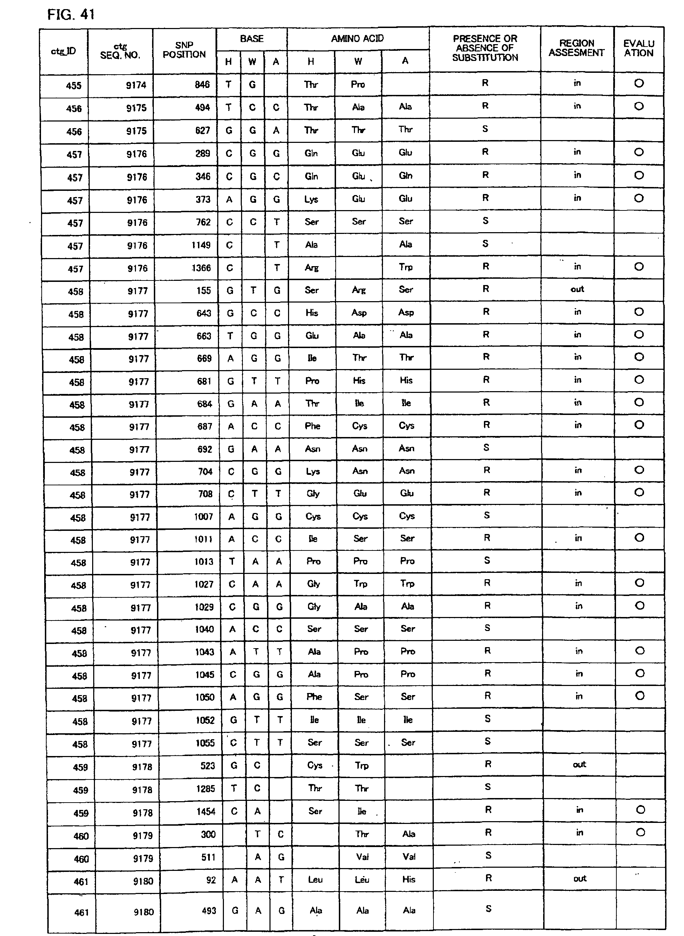

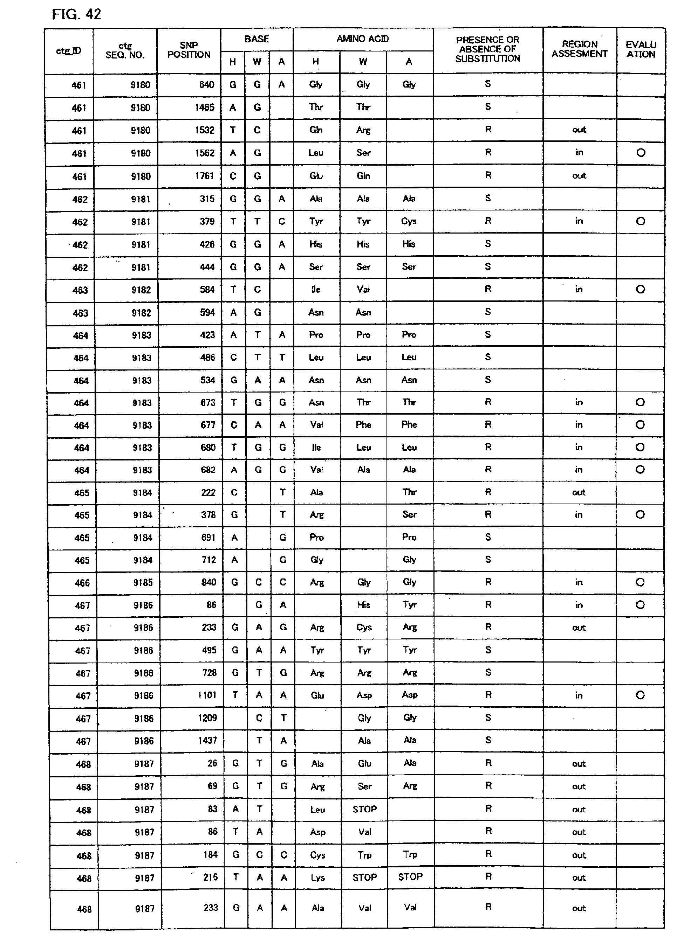

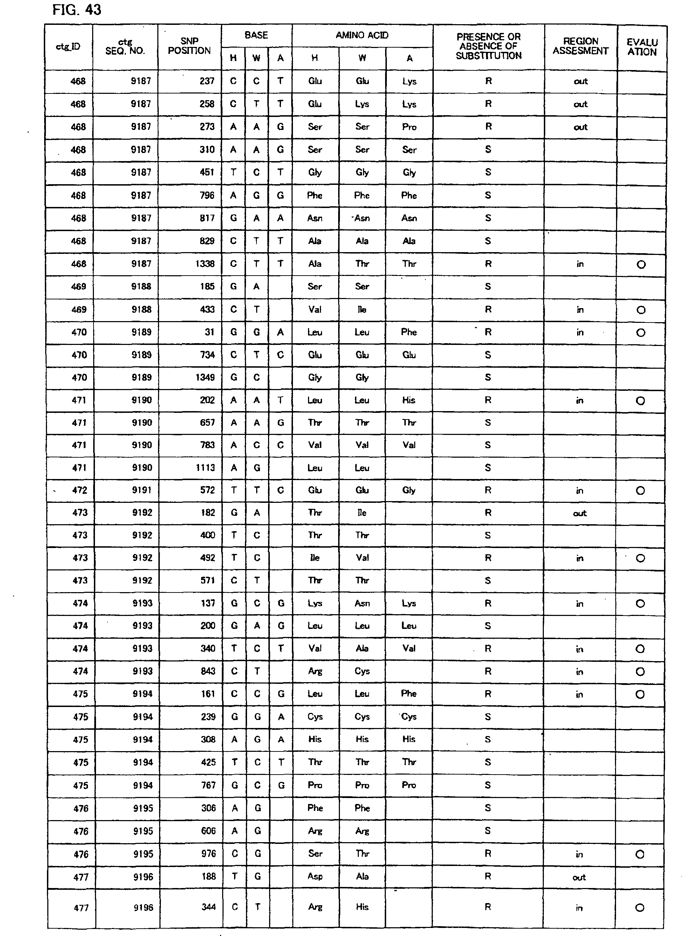

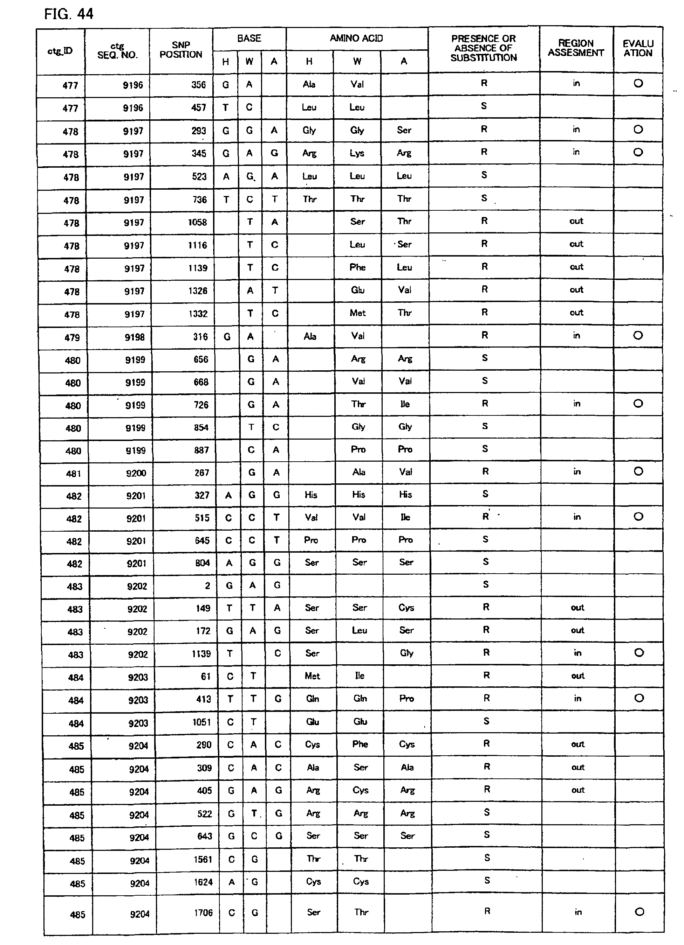

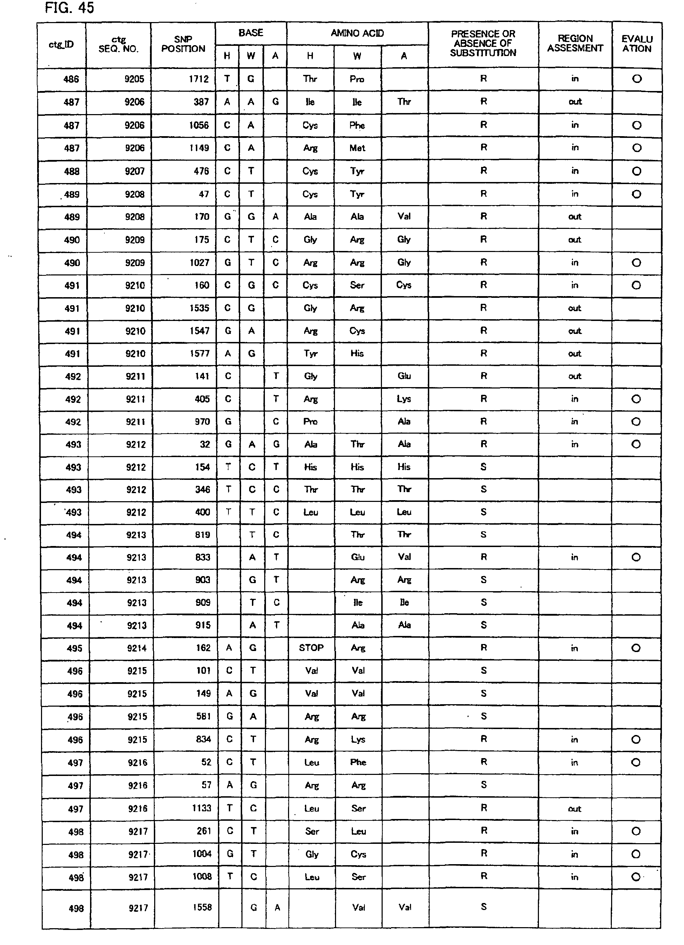

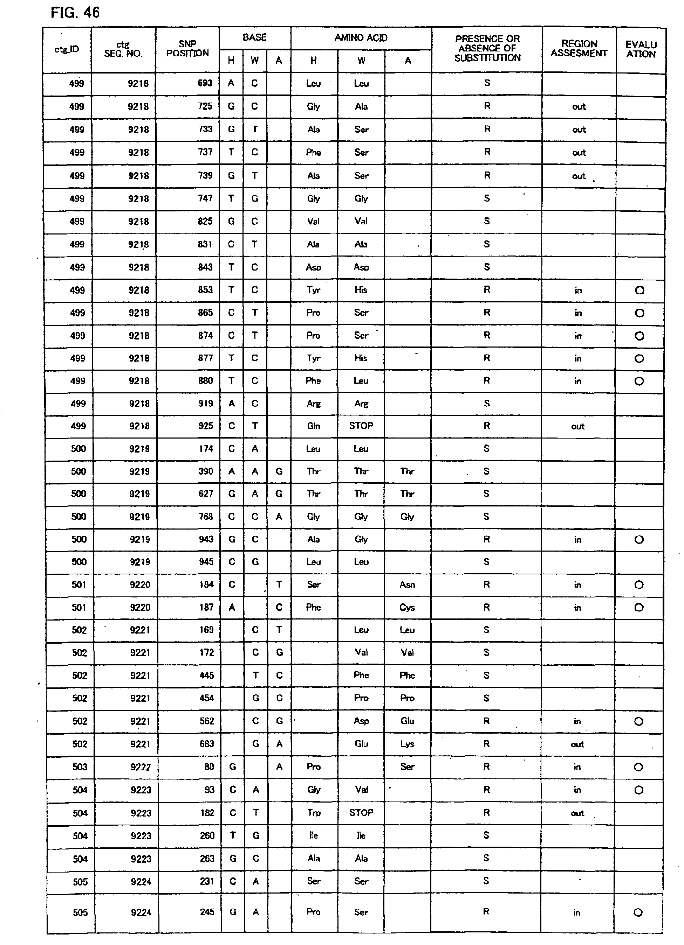

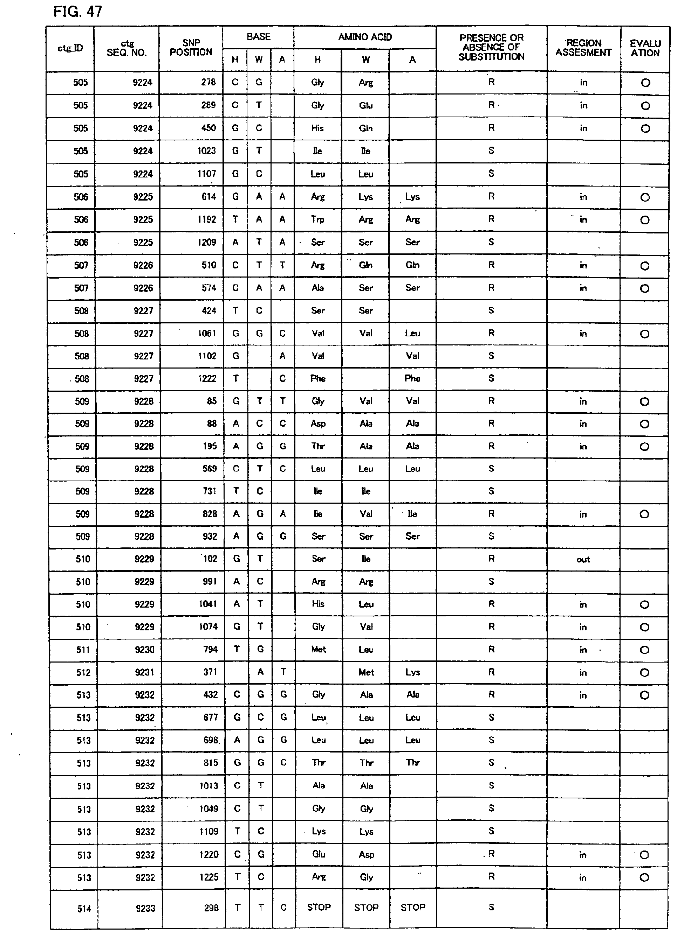

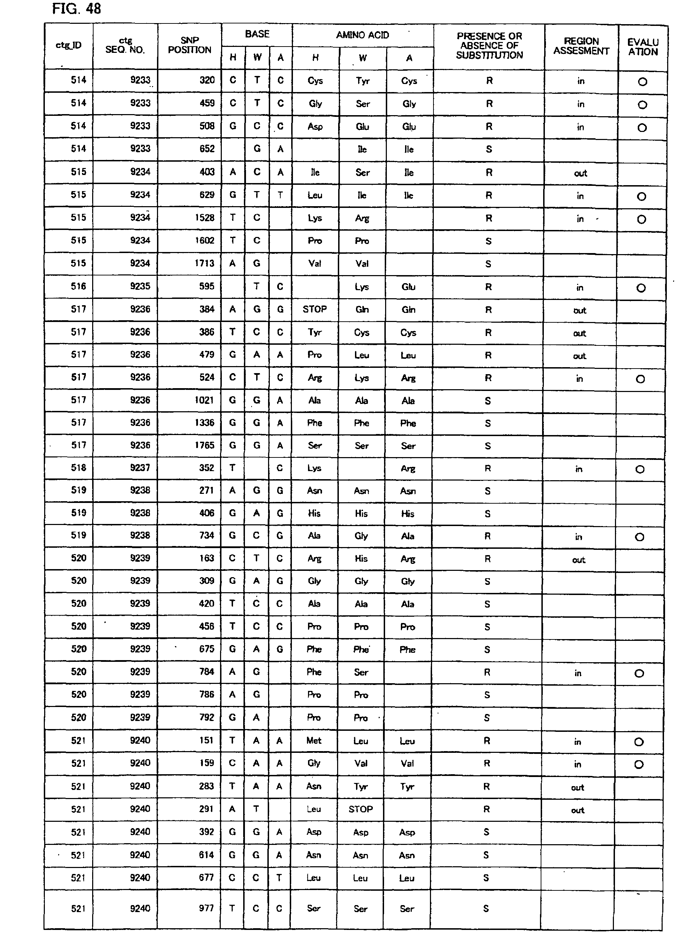

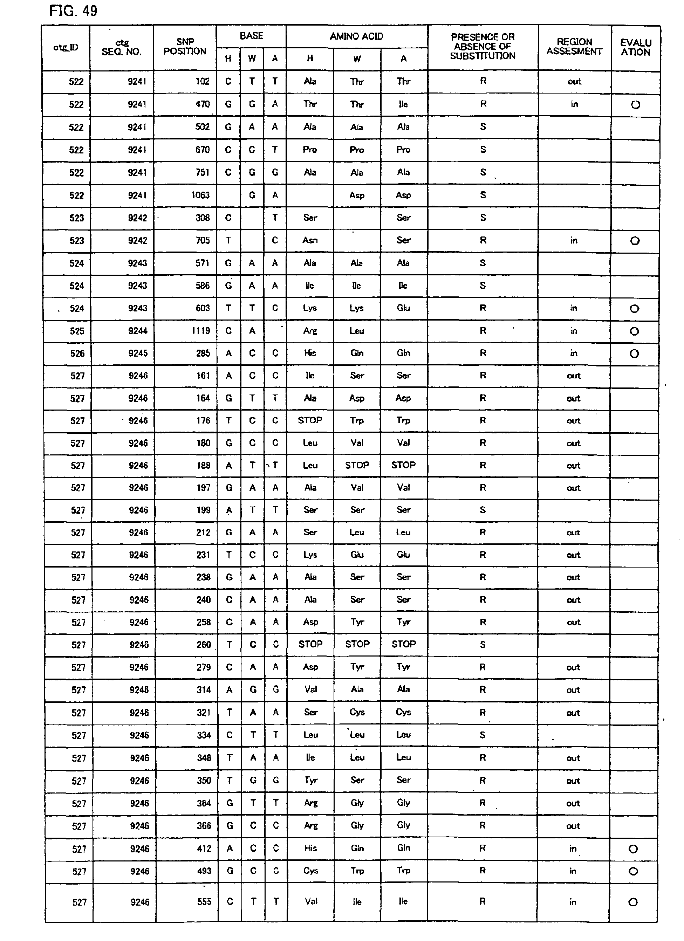

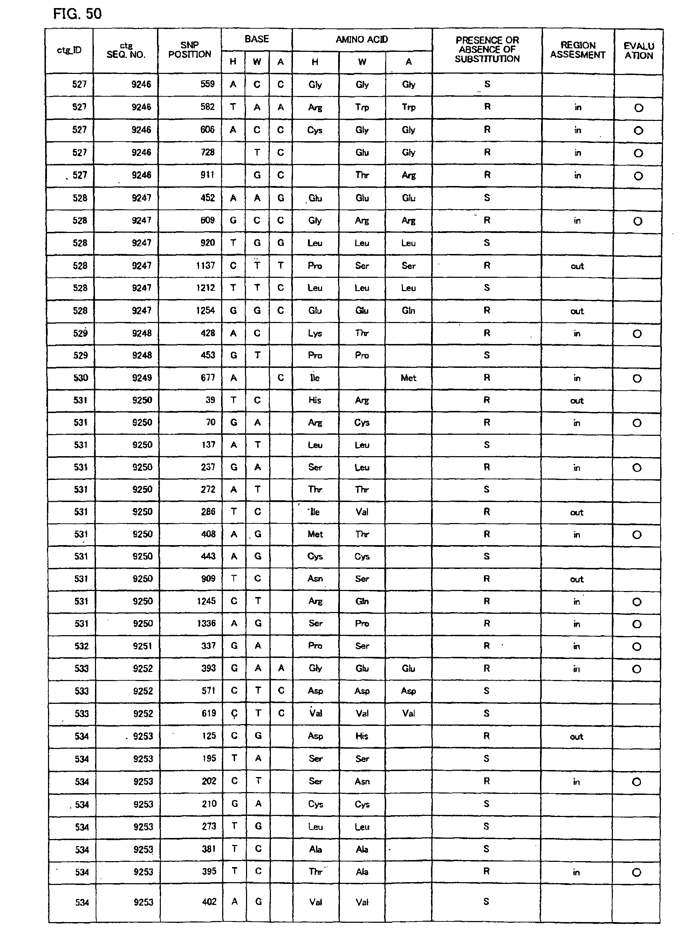

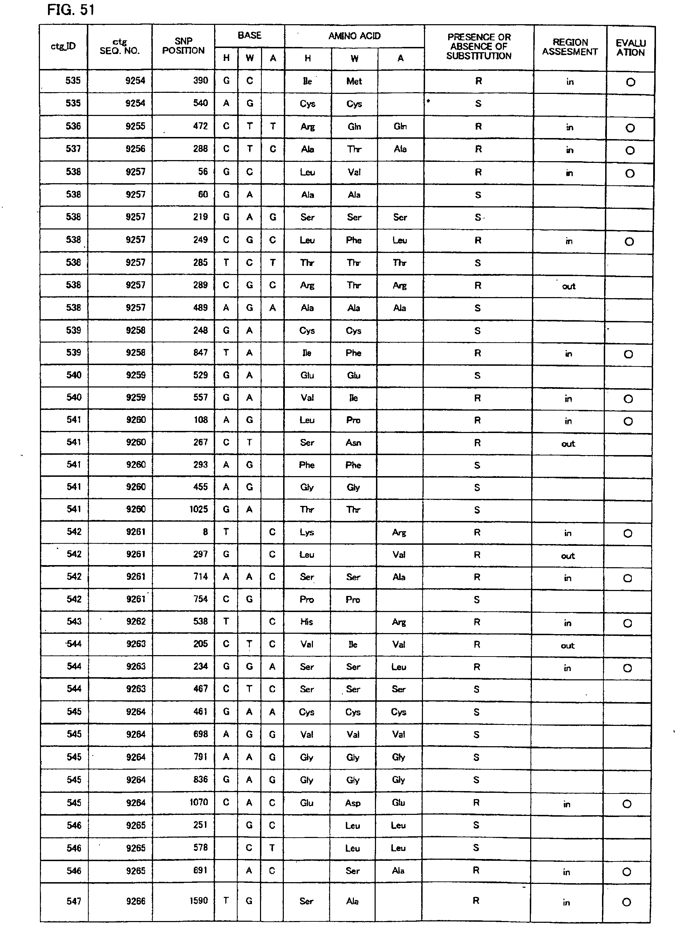

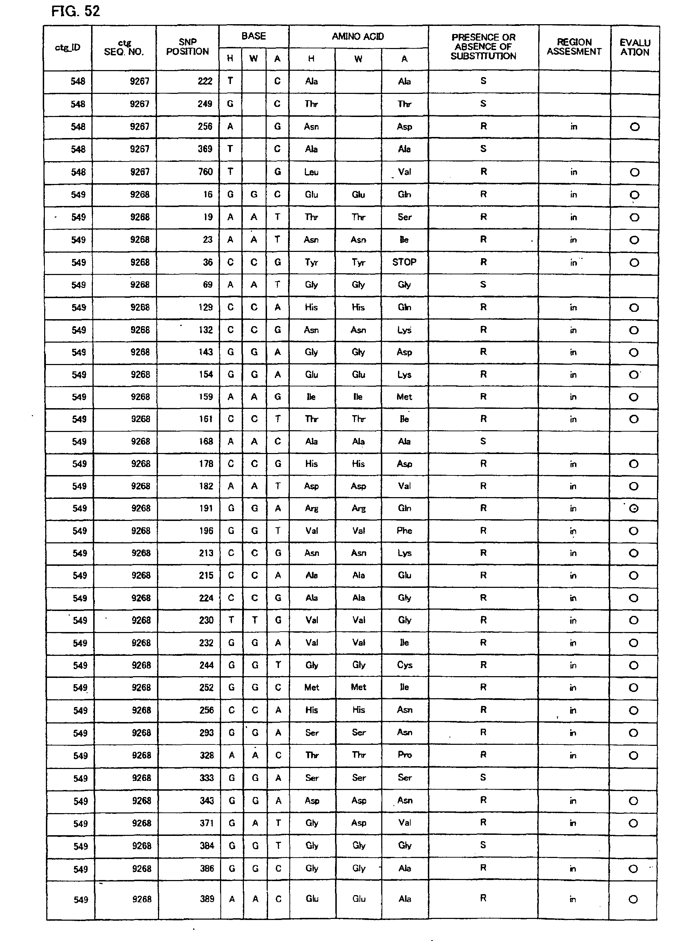

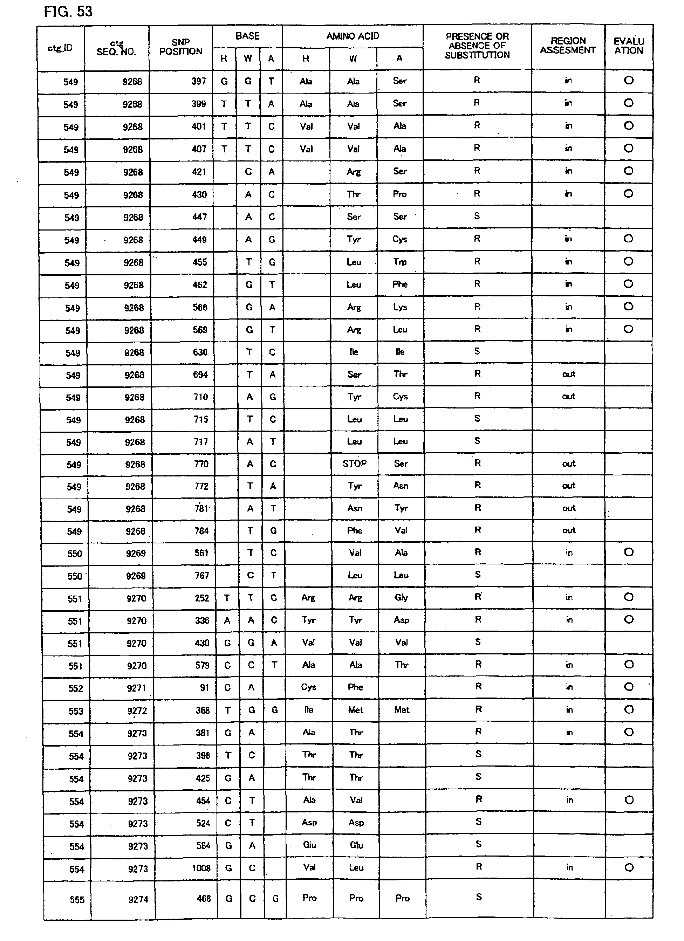

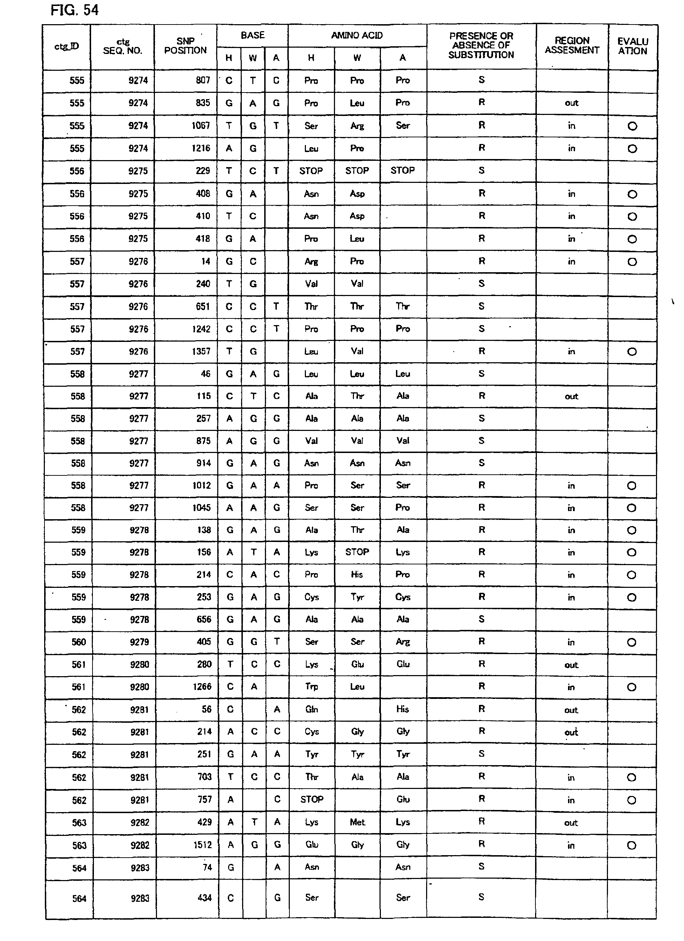

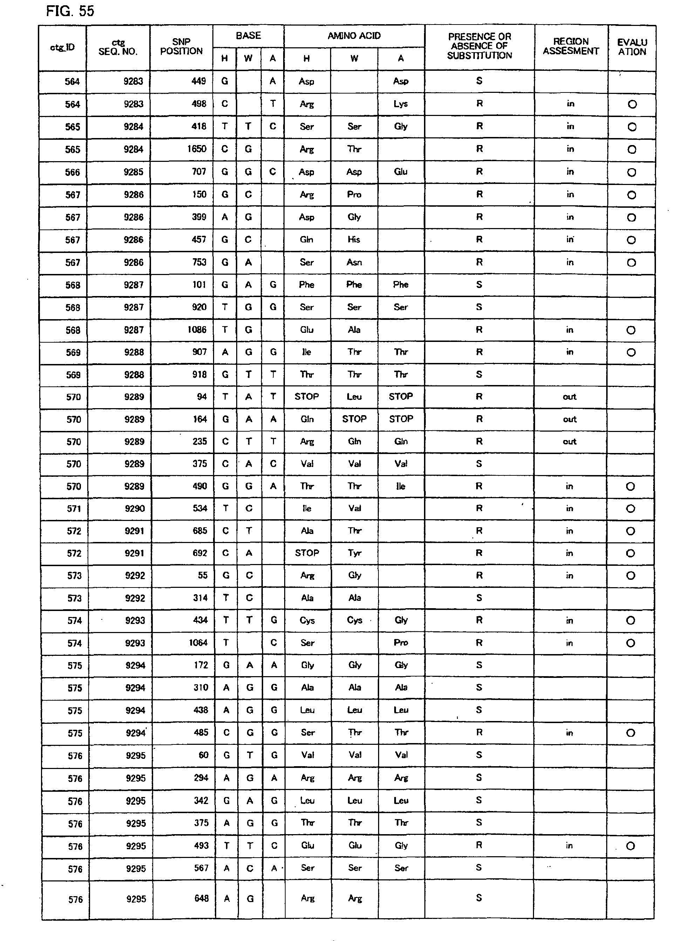

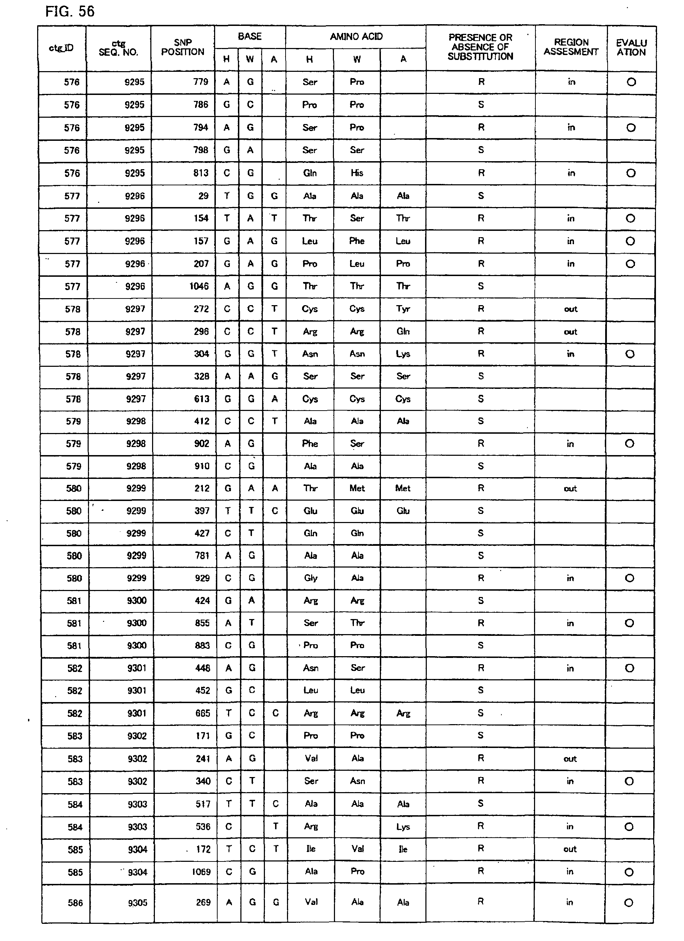

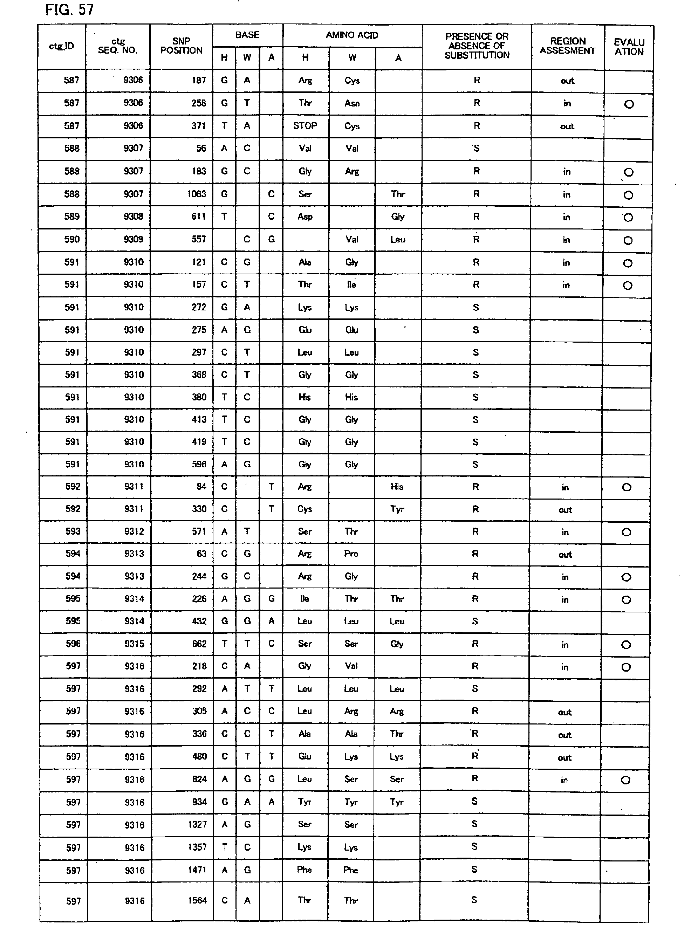

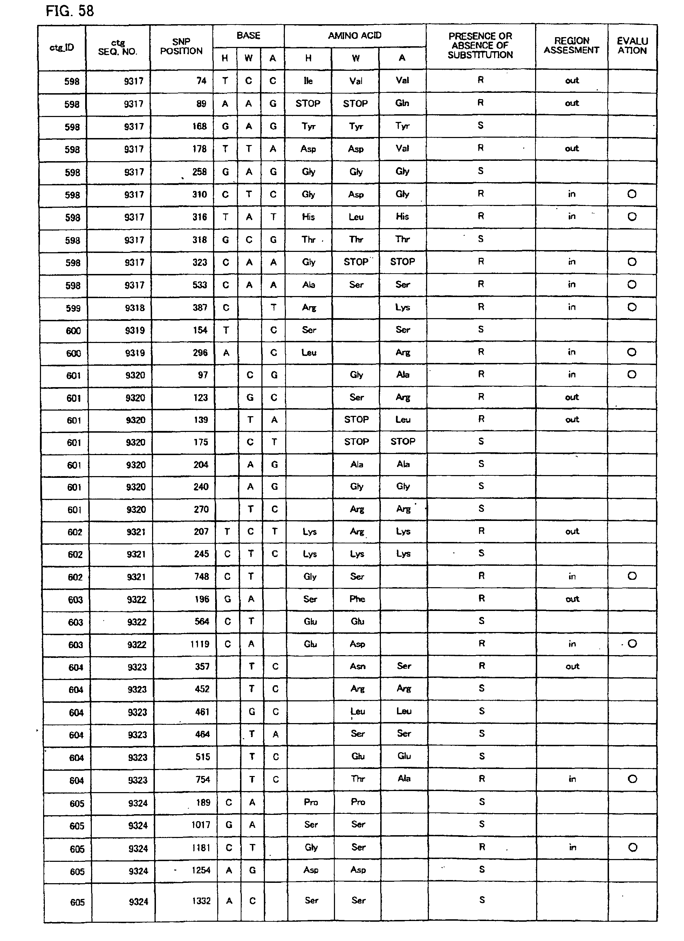

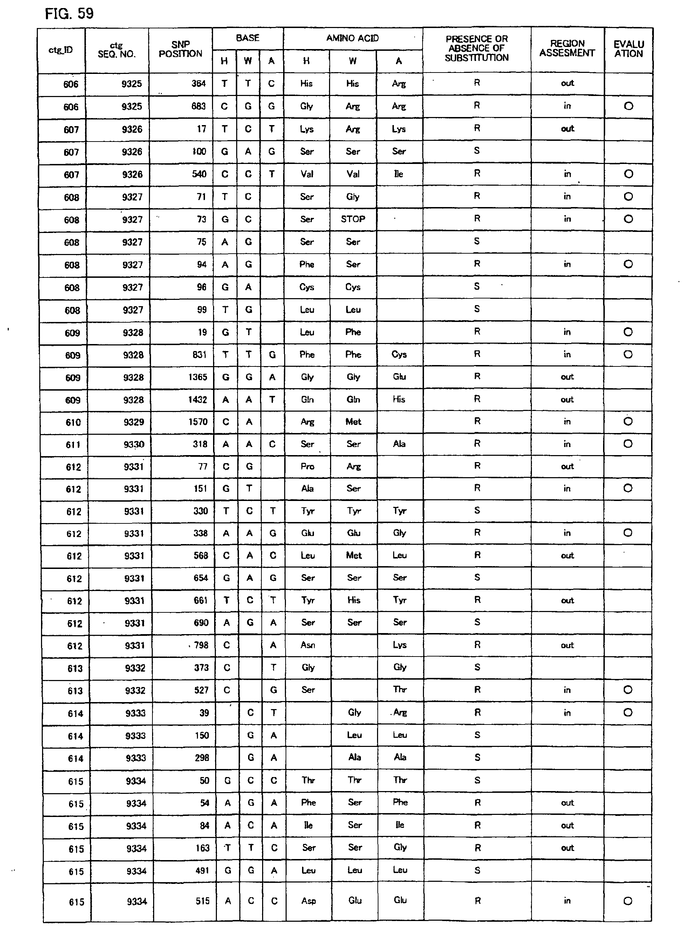

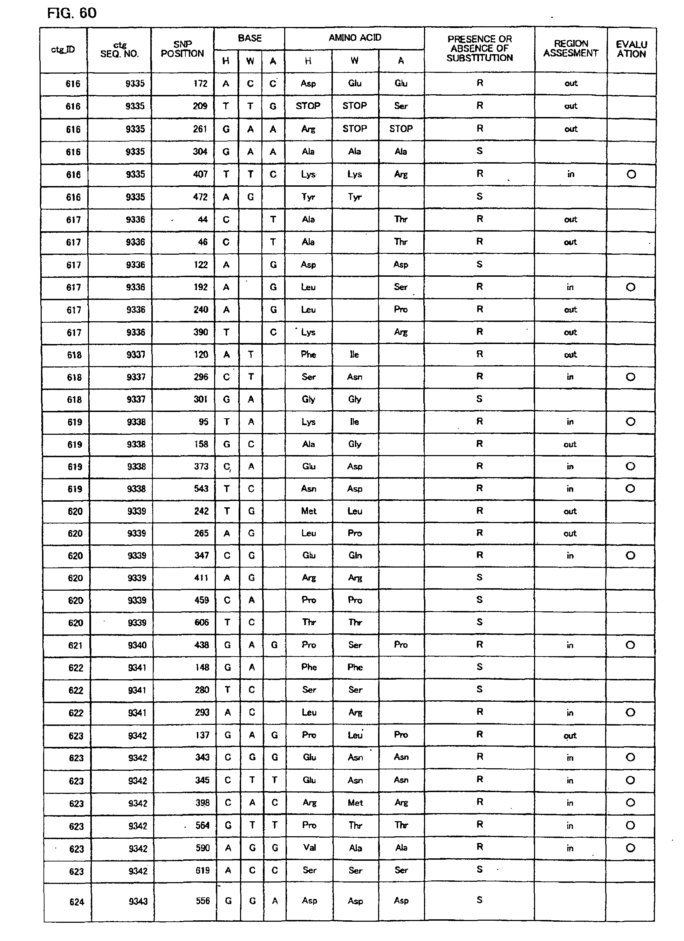

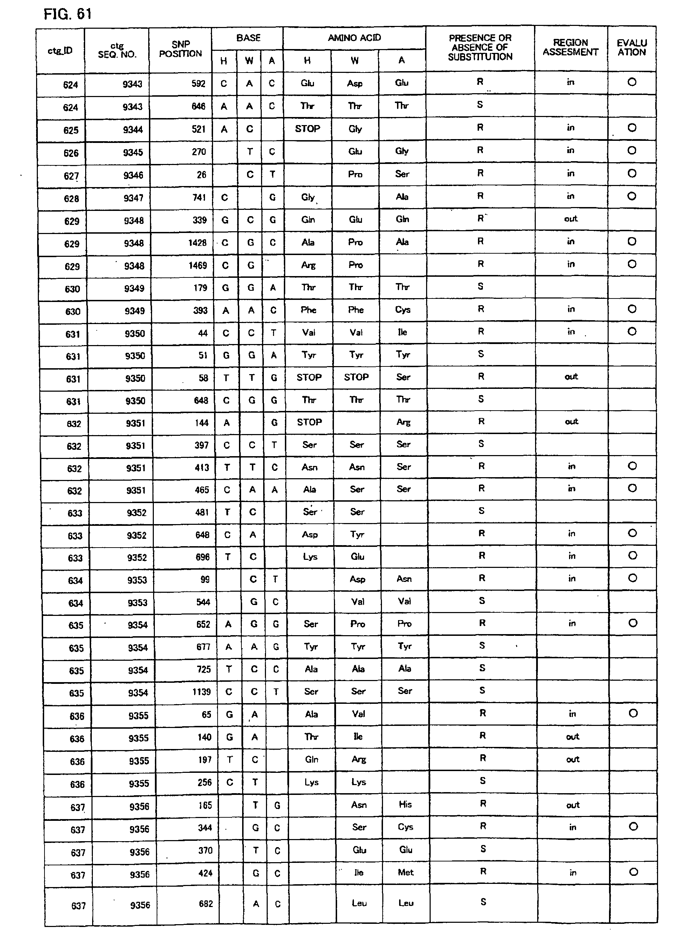

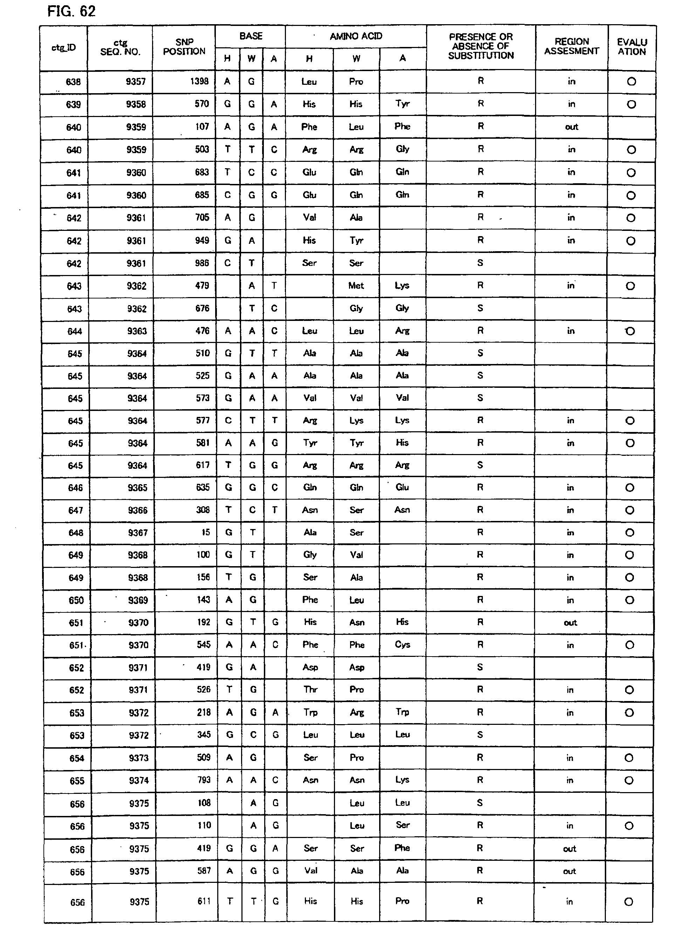

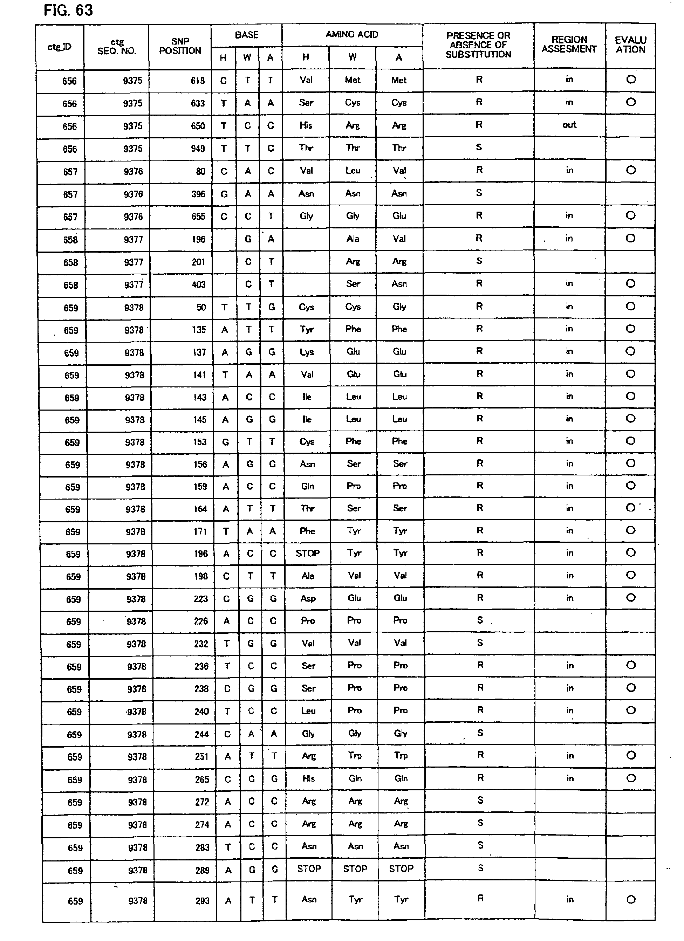

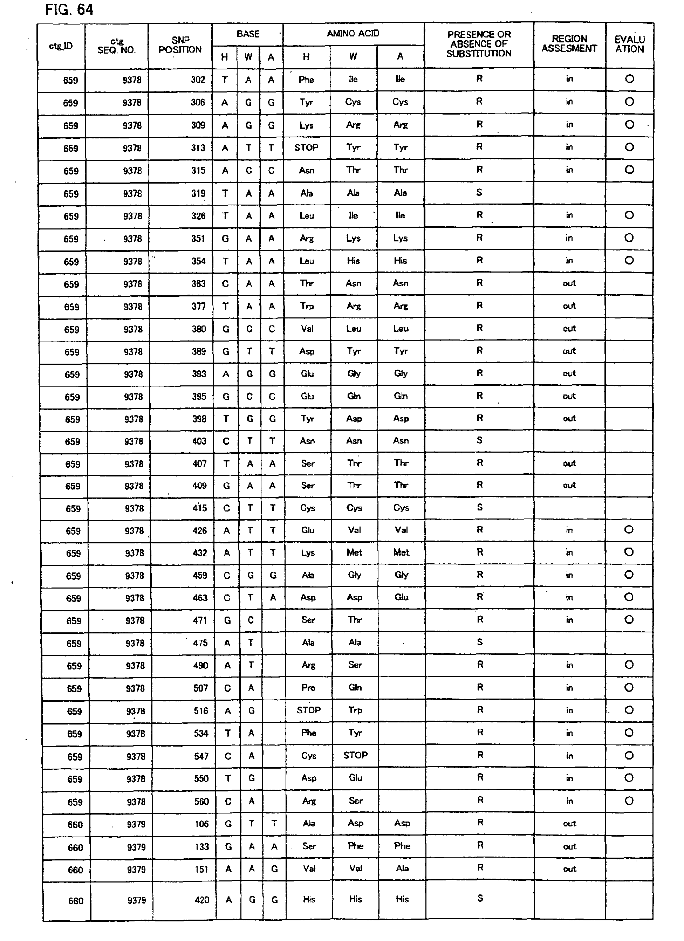

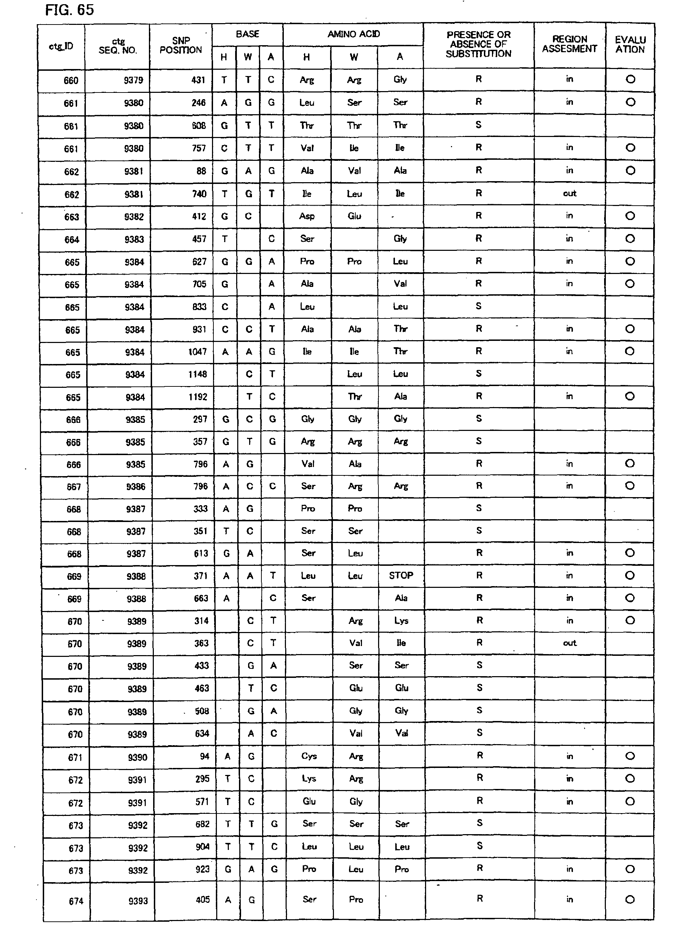

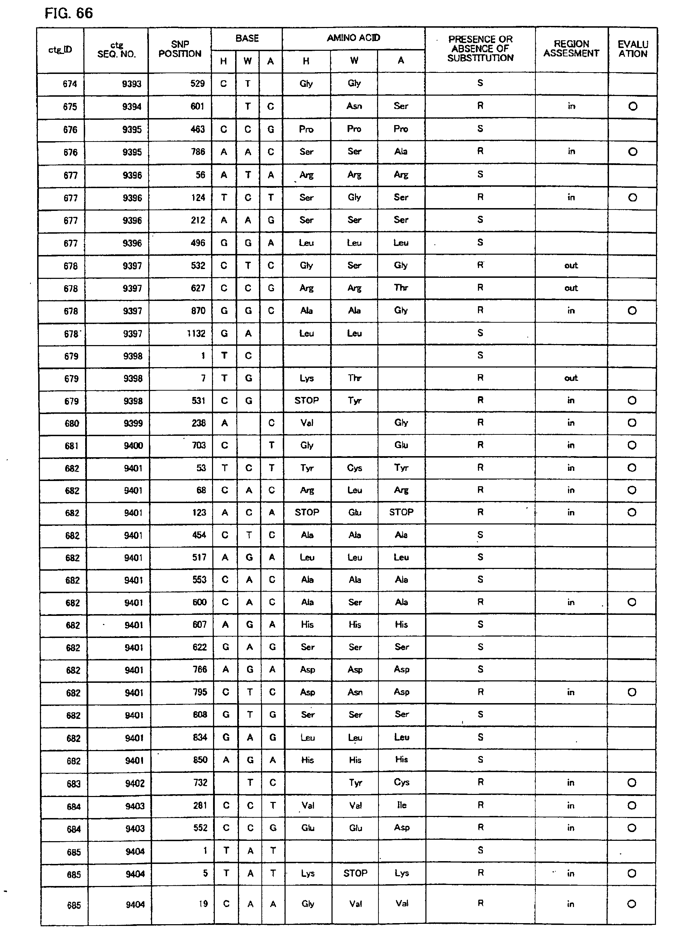

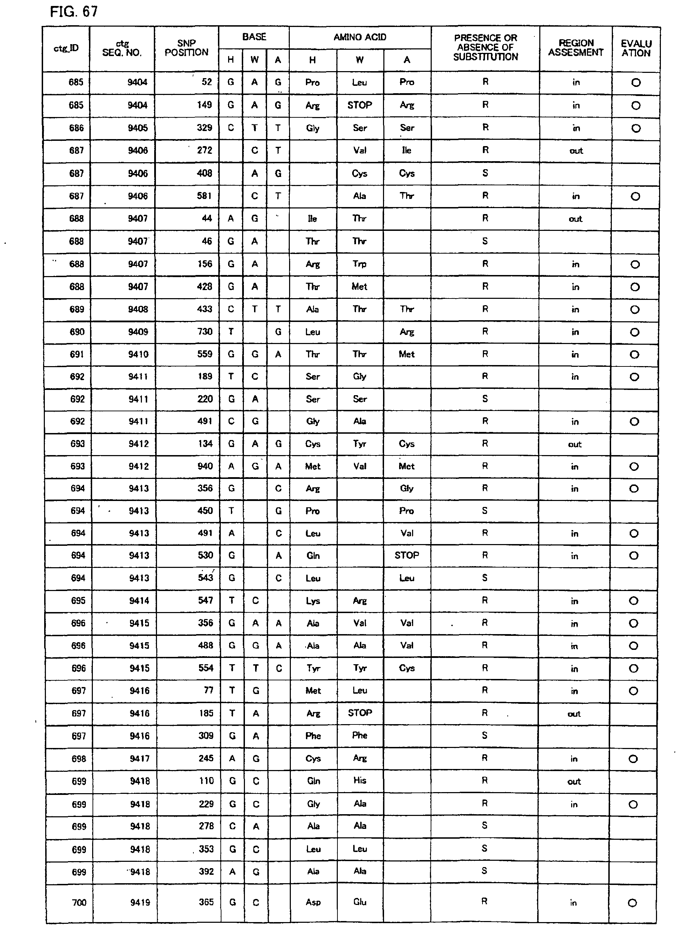

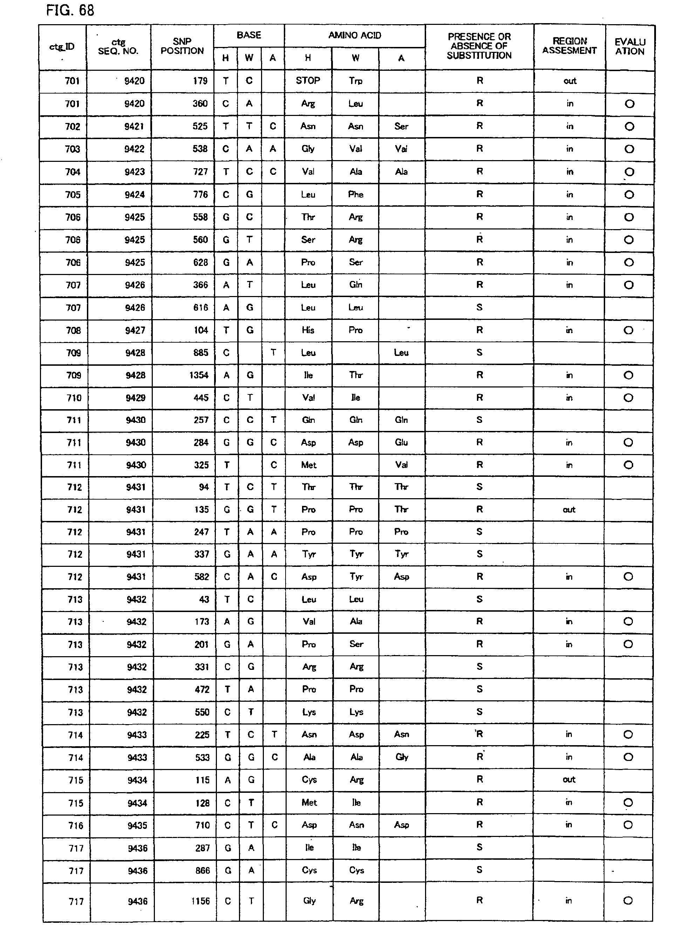

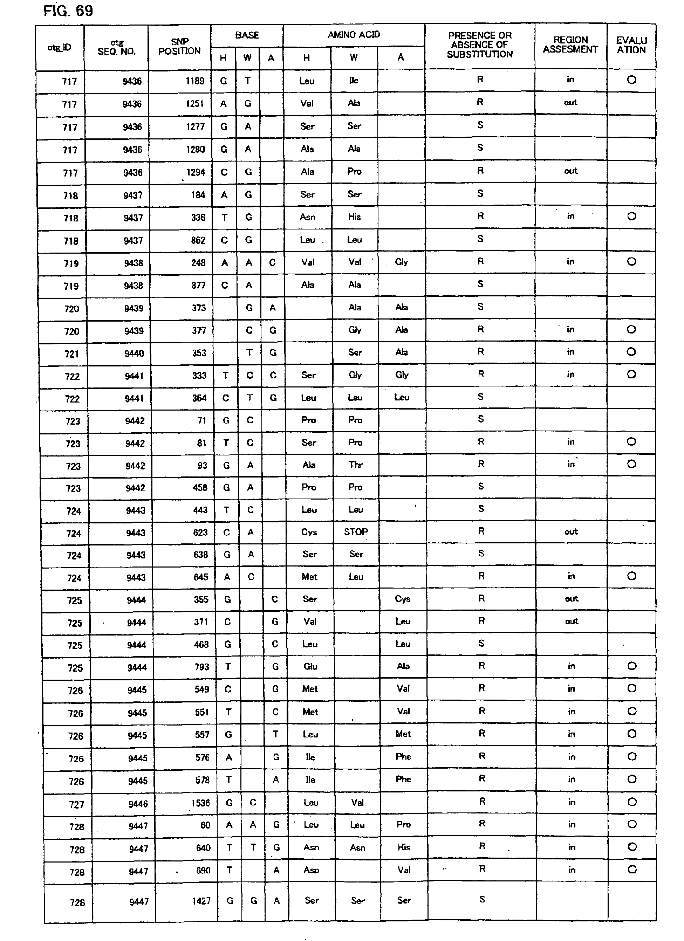

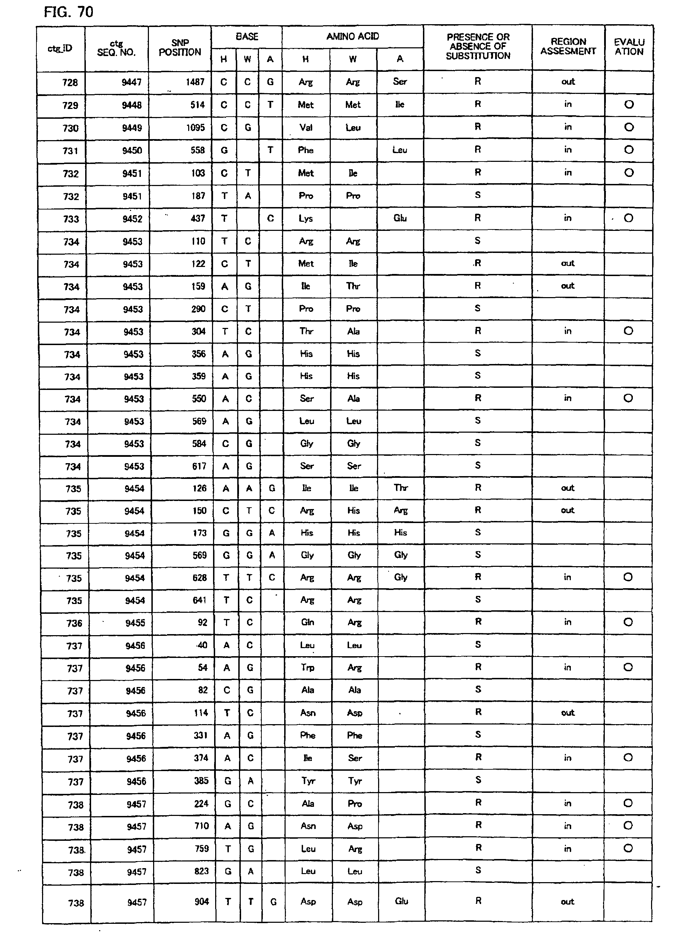

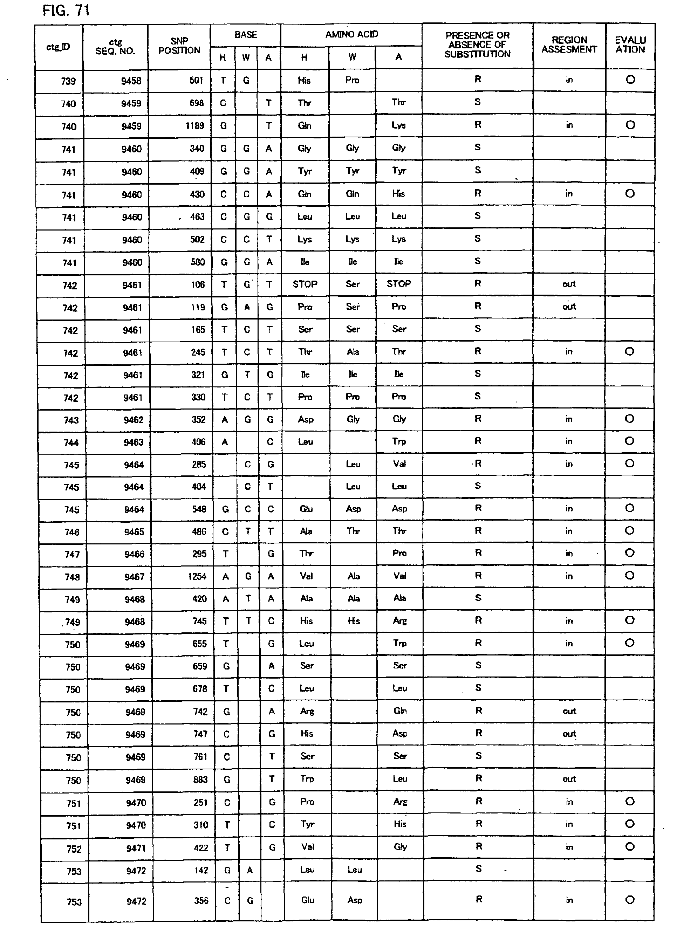

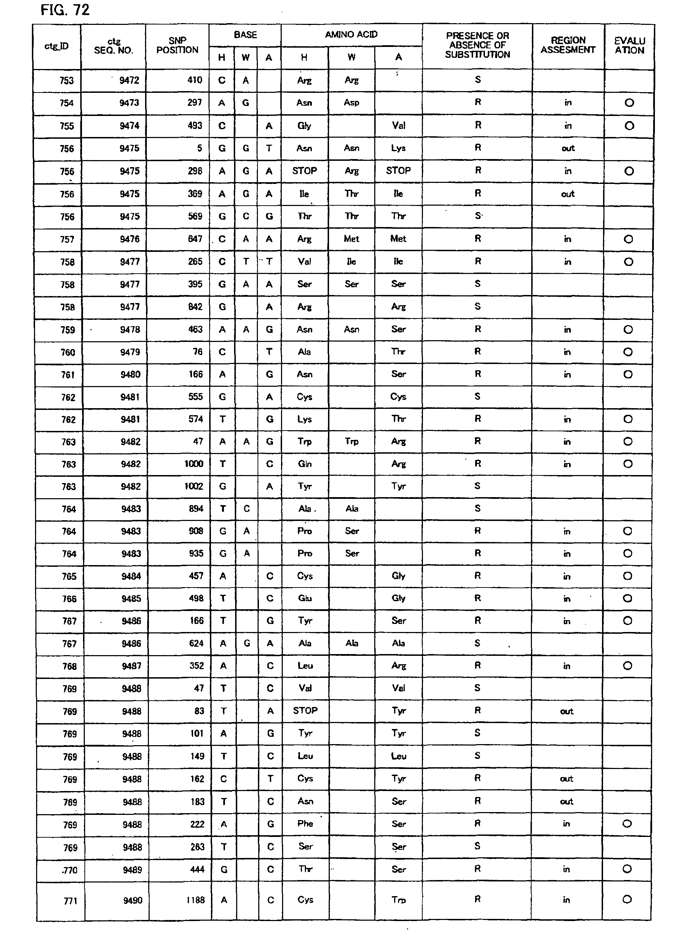

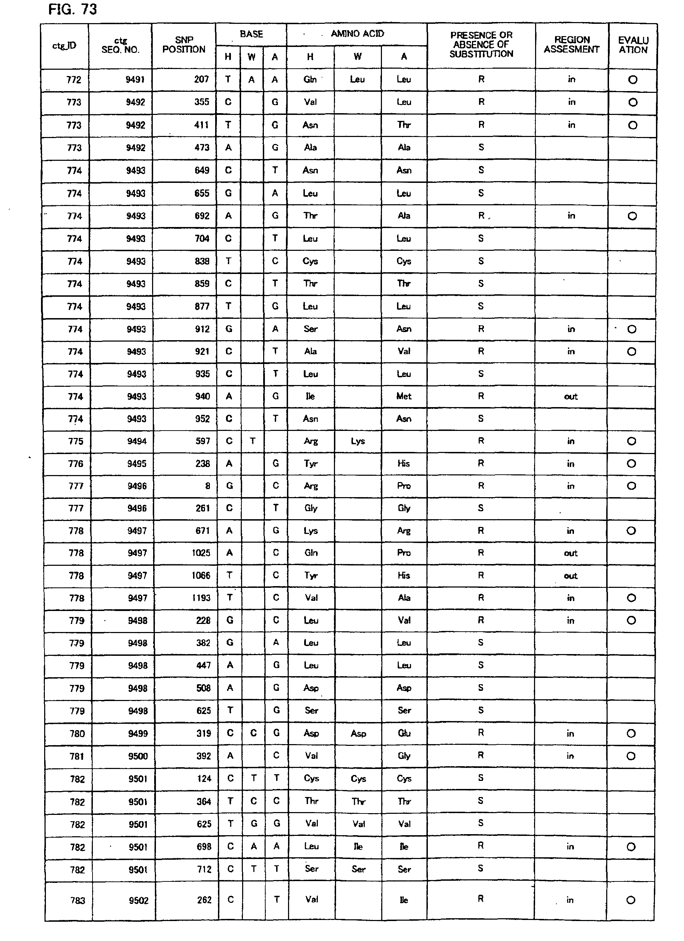

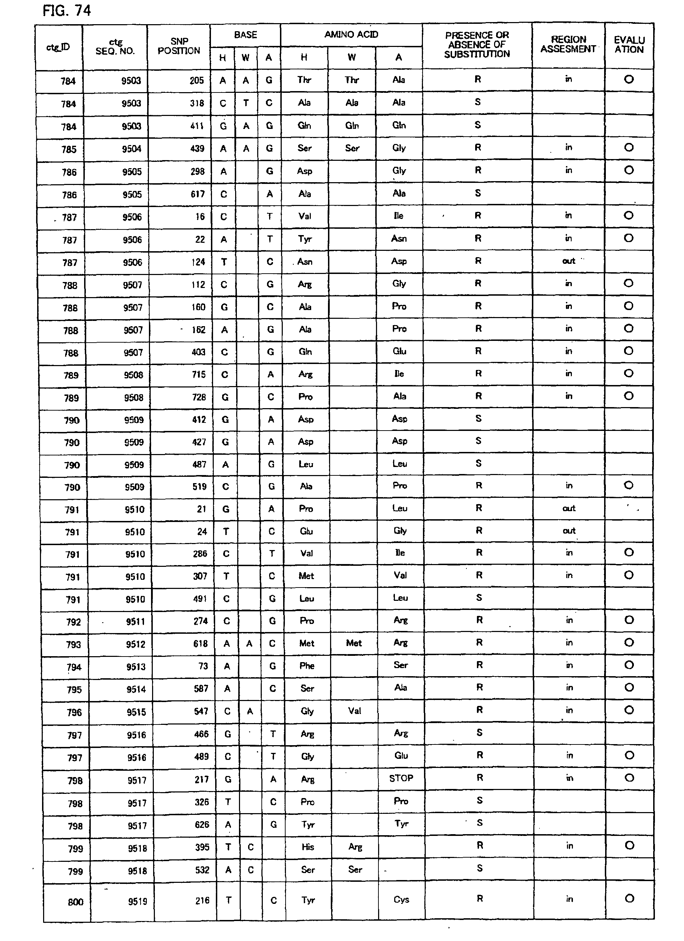

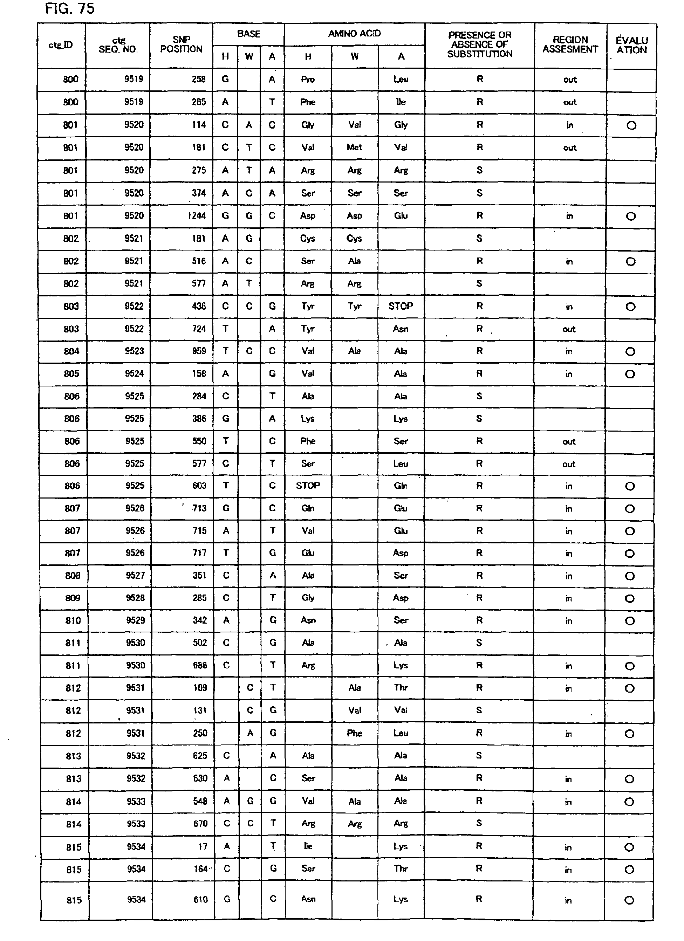

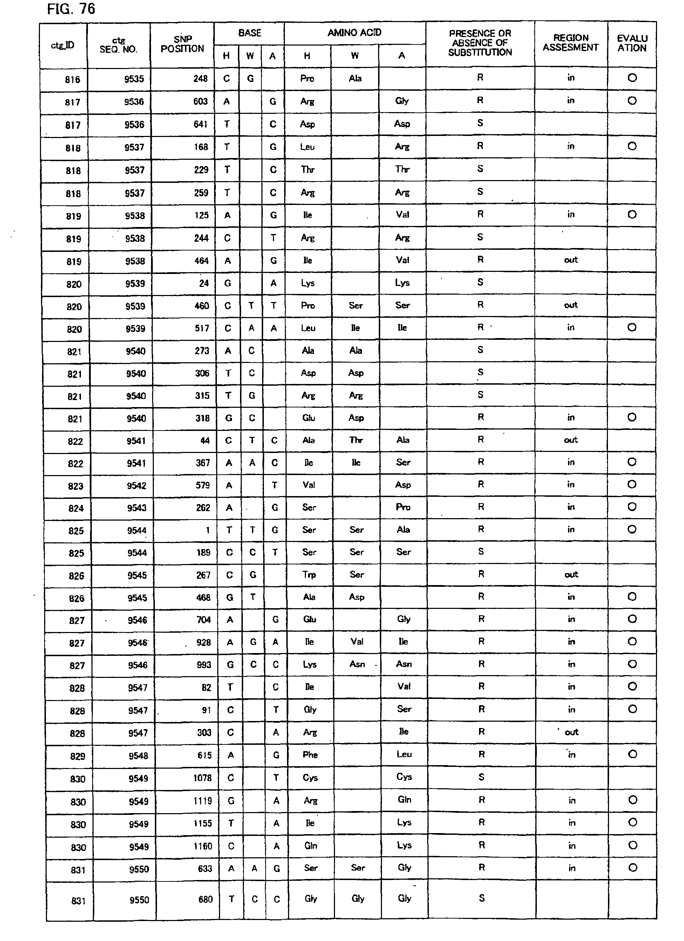

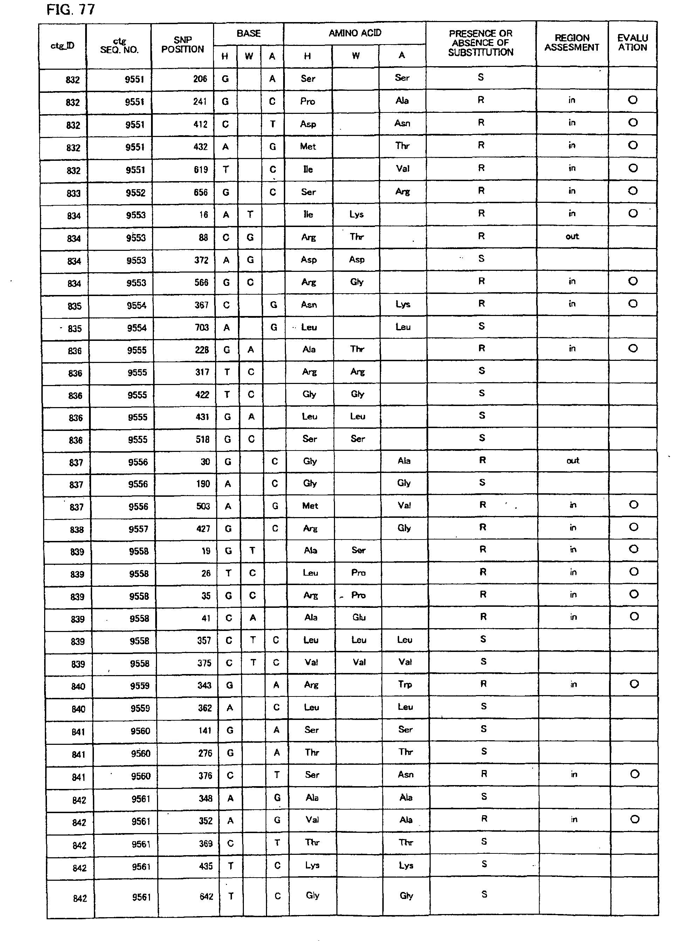

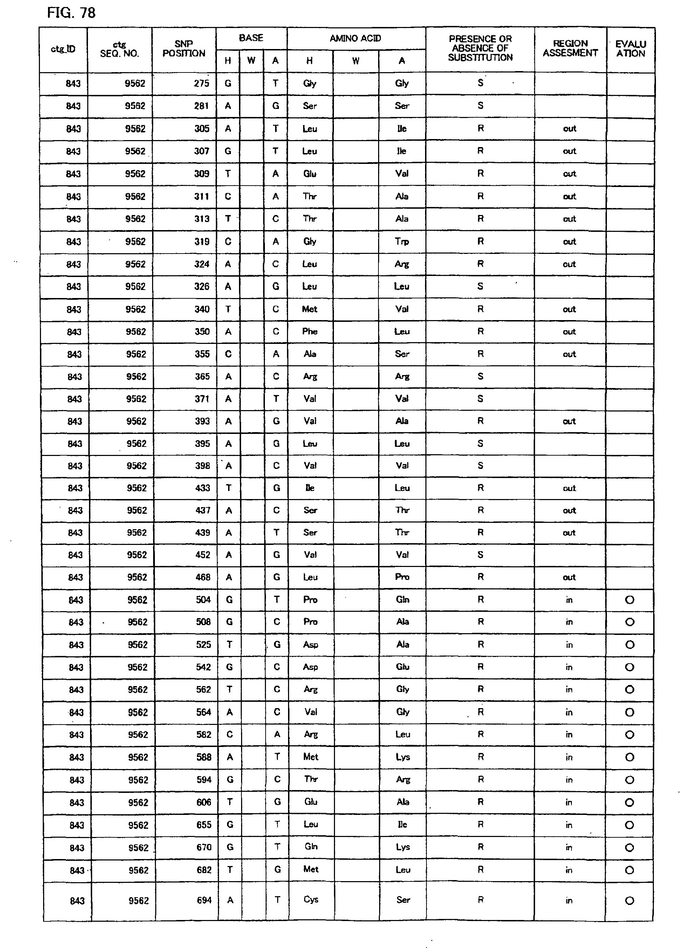

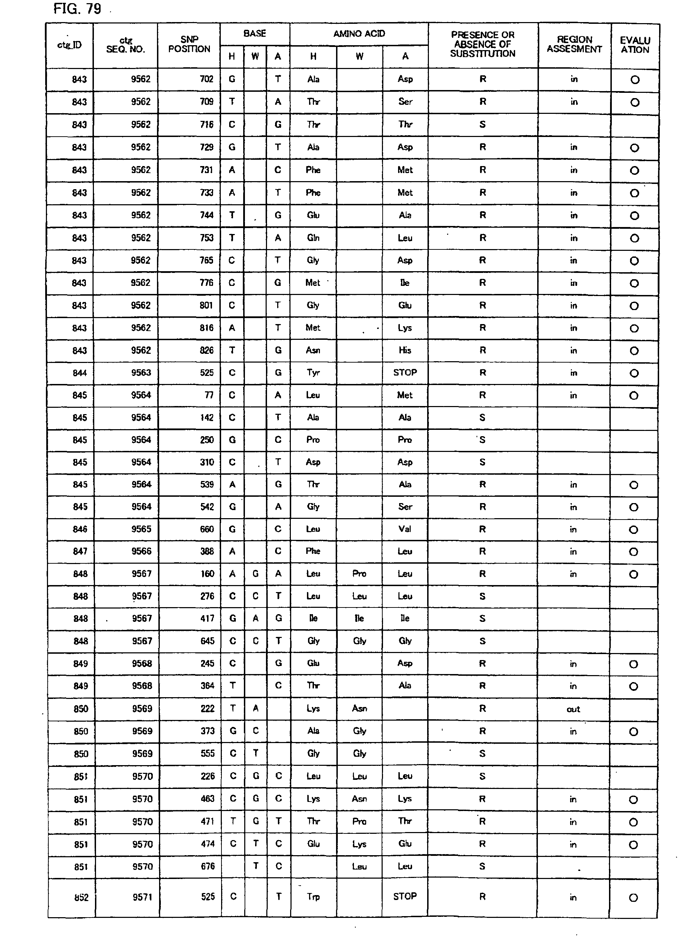

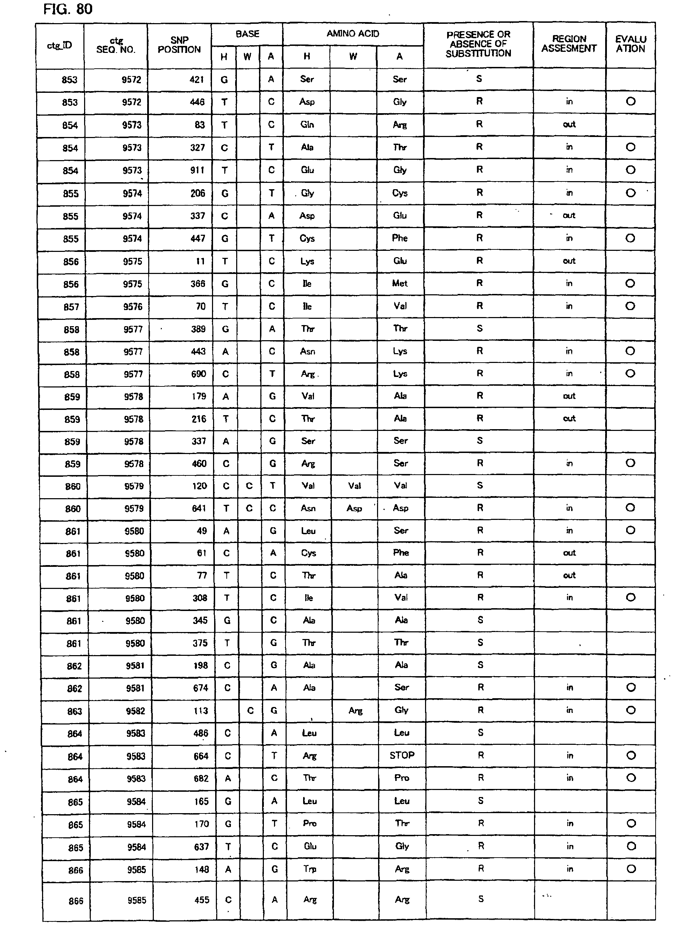

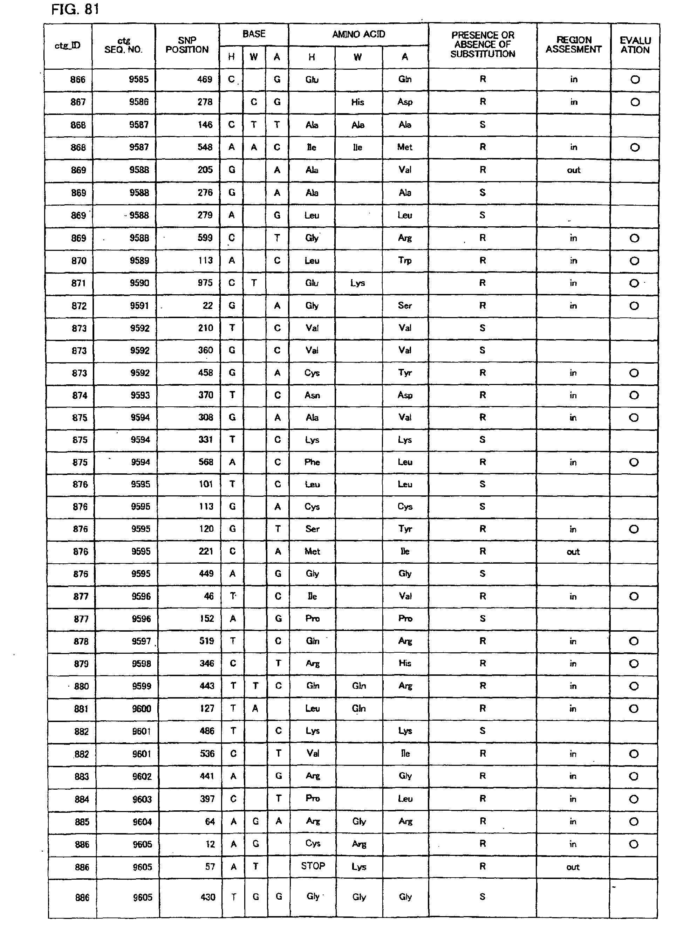

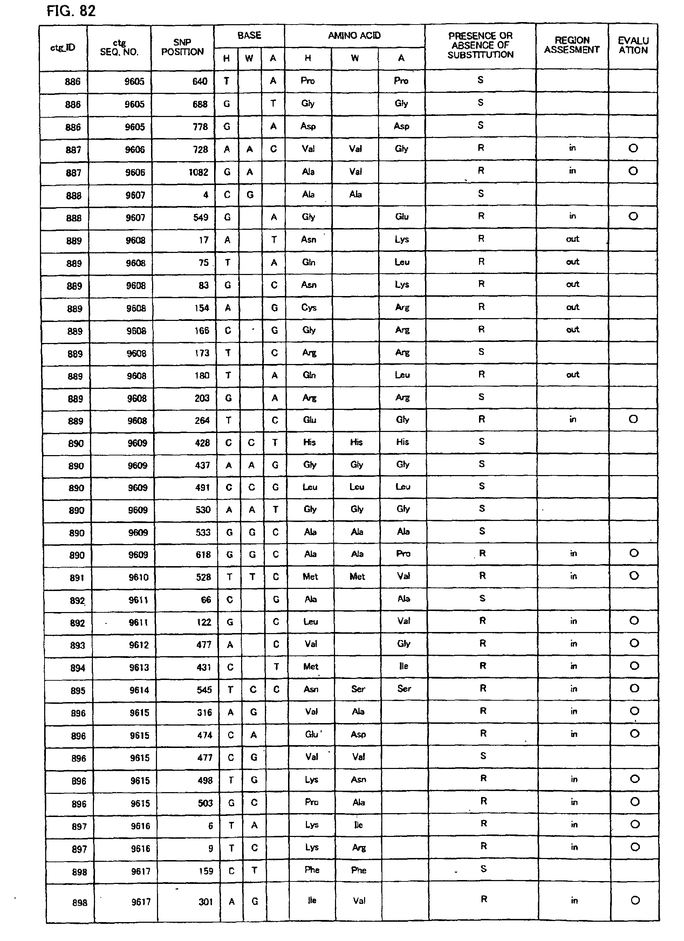

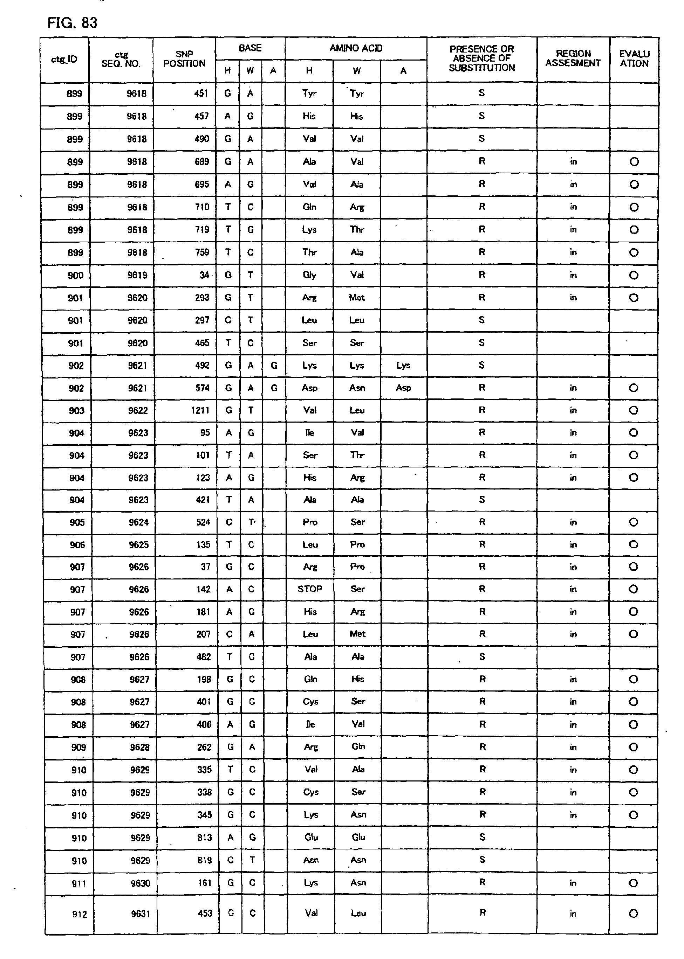

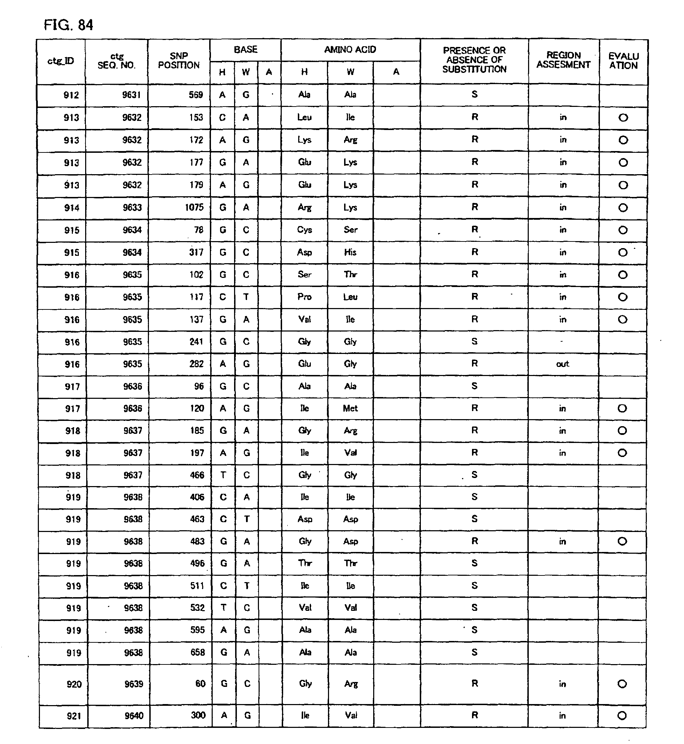

Fig. 1 through Fig. 84 represent a relationship between CONTIG numbers (ctg_ID) of the CONTIGS (1) through (921) and the sequence numbers (ctg sequence numbers) of the CONTIG sequences identified in a sequence table. Specifically, the base sequences indicated by the sequence numbers 1 through 17 respectively correspond to CONTIGS (1) through (17). The base sequences indicated by the sequence numbers 267 through 320 respectively correspond to CONTIGS (18) through (71). The base sequences indicated by the sequence numbers 990 through 1183 respectively correspond to CONTIGS (72) through (265). The base sequences indicated by the sequence numbers 642 through 4763 respectively correspond to CONTIGS (266) through (387). The base sequences indicated by the sequence numbers 9107 through 9640 respectively correspond to CONTIGS (388) through (921).

The sequence numbers 1 through 4, 267 through 278, 990 through 1016, 4642 through 4670, and 9107 through 9640 in the sequence table indicate 606 CONTIG sequences with the 1039 nonsynonymous substitution SNPs. The CONTIG sequences with the nonsynonymous substitution SNPs are CONTIGS (1) through (4), (18) through (29), (72) through (98), (266) through (294), and (388) through (921). Specifically, the base sequences with the sequence numbers 1 through 4 respectively correspond to CONTIGS (1) through (4). The base sequences with the sequence numbers 267 through 278 respectively correspond to CONTIGS (18) through (29). The base sequences with the sequence numbers 990 through 1016 respectively correspond to CONTIGS (72) through (98). The base sequences with the sequence numbers 4642 through 4670 respectively correspond to CONTIGS (266) through (294). The base sequences with the sequence numbers 9107 through 9640 respectively correspond to CONTIGS (388) through (921).

The base of CONTIGS (1) through (921) represented by a universal code is the site of single nucleotide polymorphism (SNP) that exists among the different strains of barley. In CONTIGS (1) through (921), the site of SNP is indicated by a universal code not only for the nonsynonymous substitution SNP but also for other types of SNPs (including rSNP and sSNP). Fig. 1 through Fig. 84 show which SNP is the nonsynonymous substitution SNP.

The CONTIGS (1) through (387) in Fig. 1 through Fig. 30 are classified under the headings as follows.

### Ctg_ID:

CONTIG number assigned to CONTIGS

### Ctg SEQUENCE NUMBER:

Sequence number in a sequence table, indicating a base sequence of the CONTIG

### SNP POSITION:

The POSITION of SNP on a CONTIG sequence (polymorphic site)

### BASE:

The type of base occupying the SNP site, in which the column under "Haruna" or "H" indicates a base in Haruna Nijo, "Wild-type" or "W" indicates a base in H. Spontaneum, and "Akashinriki" or "A" indicates a base in "Akashinriki."

Note that, for CONTIGS (266) through (387), those strains for which only one sequence data was obtained for the SNP are appended with "pseudo" before the type of base because they do not fall in the definition of SNP used in the present invention (described later). Note that, in the sequence table, a base at the site of SNP is indicated by a universal code.

The column under the heading "EVALUATION 1" contains evaluations of SNPs. The notation "SILENT" means that the SNP is a sSNP (silent SNP) that accompanies no change in the amino acid in a translation region of a protein. The notation "OUTSIDE OF CODING REGION" or "UPSTREAM OF CODING REGION" means that the SNP is a rSNP (regulatory SNP) in a regulatory region that affects the strength gene expression. When the SNP is non-silent (nonsynonymous substitution), the following indications are made.

For CONTIGS (1) through (4), a change in amino acid occurs from an amino acid encoded by a wild-type (left side of the arrow) to an amino acid encoded by Akashinriki (right side of the arrow).

For CONTIGS (18) through (29), a change in amino acid occurs from an amino acid encoded by Akashinriki (left side of the arrow) to an amino acid encoded by Haruna Nijo (right side of the arrow).

For CONTIGS (72) through (98), a change in amino acid occurs from an amino acid encoded by a wild-type (left side of the arrow) to an amino acid encoded by Haruna Nijo (right side of the arrow).

For CONTIGS (266) through (294), a change in amino acid is indicated by denoting the amino acids encoded by a wild-type, Haruna Nijo, and Akashinriki as "W", "H", and "A," respectively.

Note that, when the "homology search result" (described later) is "none", the column under "EVALUATION 1" is blanked, indicating that no translation region could be approximated in the CONTIG sequence.

The column under "EVALUATION 2" is indicated by "o" when the SNP is determined to be a nonsynonymous substitution SNP. That is, the column under "EVALUATION 2" is "o" for those SNPs that show a change in amino acid in "Evaluation 1". By a "change in amino acid," it means that a change in the base of the polymorphic site brings about a replacement of an amino acid encoded by a codon including the polymorphic site, or that a codon including the polymorphic site is changed to a stop codon. Alternatively, a "change in amino acid" means that a change in the base of the complementary site brings about a replacement of an amino acid encoded by a codon including the complementary site, or that a codon including the complementary site is changed to a stop codon.

For CONTIGS (1) through (265), specific ranges of CONTIG were set that are suitable for the designing of the oligonucleotides according to the present invention. Fig. 1 through Fig. 20 show the results.

The column under "SUITABLE RANGE" in Fig. 1 through Fig. 20 indicates reliable ranges of the base sequence including the SNP as indicated in the column under "SNP POSITION" in the table. These ranges are set by the position of a base or nucleotide in the base sequence, and they define a particularly suitable CONTIG range of the CONTIG sequence for designing a probe or primer for testing a single nucleotide polymorphism in the oligonucleotides or genes of the present invention, or for designing a probe for isolating the genes. Note that, the column under "SUITABLE RANGE" is blank for SNPs for which a suitable range was difficult to set or was not set at all. Further, the "SUITABLE RANGE" is a range in which basically no base variation is found among all corresponding sequence data.

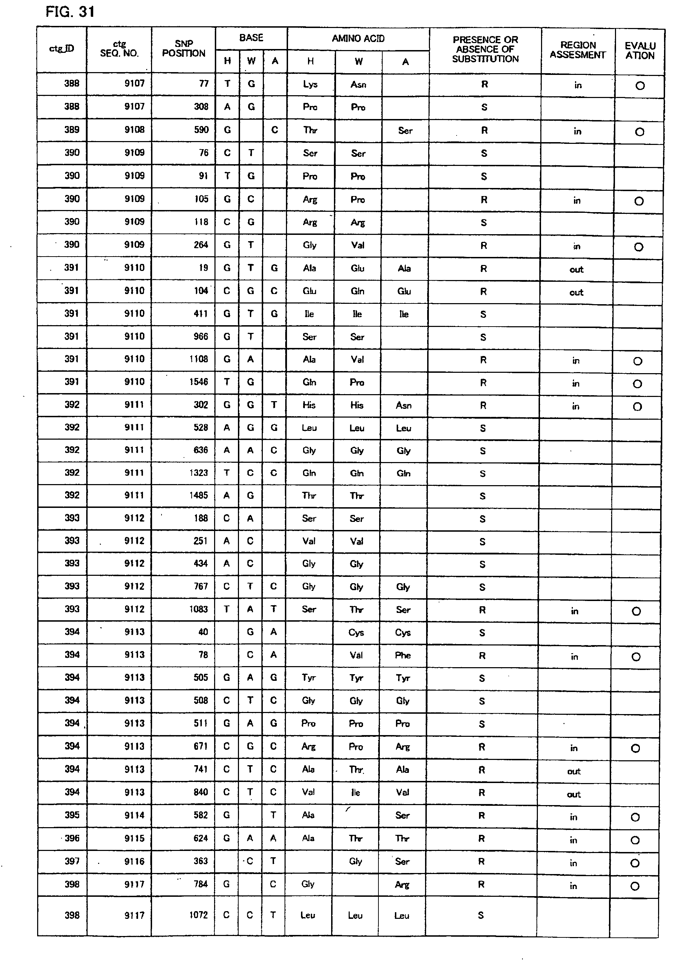

In Fig. 31 through Fig. 84, CONTIGS (388) through (921) are classified under the headings below.

The headings "ctg_ID", "ctg SEQUENCE NUMBER", "SNP POSITION", and "BASE" are the same as those used in Fig. 1 through Fig. 30.

### AMINO ACID:

For the amino acid encoded by a codon including the SNP (polymorphic site or its complementary site), the notations "H", "W", and "A" are used for "Haruna Nijo", "H. spontaneum", and "Akashinriki", respectively. (The determination of a reading frame (frame) is described later.)

### THE PRESENCE OR ABSENCE OF SUBSTITUTION:

The notation "S" is used when "Haruna", "wild-type", and "Akashinriki" all have the same amino acid, indicating that there was no replacement of amino acid. The notation "R" is used when at least two strains produce different amino acids, indicating that there was a replacement of amino acid.

### REGION ASSESMENT:

For SNPs with "R", the notation "in" is used when they are inside a translation region (coding region) as determined by a method to be described later. The notation "out" is used when they are outside of the coding region.

### EVALUATION:

The notation "o" is used for non-silent (nonsynonymous substitution) SNPs, i.e., the SNPs indicated by "R" under "PRESENCE OR ABSENCE OF SUBSTITUTION", and "in" under

### "REGION ASSESMENT".

The SNPs indicated by "o" under "EVALUATION 2" in Fig. 1 through Fig. 30 and under "EVALUATION" in Fig. 31 through Fig. 84 qualify as the nonsynonymous substitution SNPs of the present invention. Of the 3048 SNPs found in CONTIGS (1) through (921), 1039 were determined to be the nonsynonymous substitution SNPs.

It should be noted here that the single nucleotide polymorphism among the different barley strains is also found in the complementary sequences of the CONTIGS (1) through (921). Where necessary, the SNPs will be called CONTIG single nucleotide polymorphisms (CONTIG SNPs) when they are found on the CONTIGS. On the other hand, they will be called complementary single nucleotide polymorphisms (complementary SNPs) when they are on the complementary sequences of the CONTIGS. Further, when a nucleotide with a CONTIG SNP is called a polymorphic site, a nucleotide forming a base pair with the polymorphic site in a double-stranded DNA will be called a complementary site (complementary polymorphic site). The polymorphic site and complementary site are found on cDNA; however, unless otherwise noted, the corresponding regions of the genomic DNA will also be called a polymorphic site or a complementary site. Further, unless otherwise noted, the term "complementation" or any of its derivatives including "complementary", "complementary site", "complementary sequence", and "complementary region" used herein indicate a relation, site, sequence, or region that completely hybridizes with a target DNA sequence (DNA region), i.e., the base sequences that strictly follow the Watson-Crick base pairing.

One of the complementary SNP and CONTIG SNP is a sense (gene) SNP, and the other is an anti-sense SNP. The sense SNP and anti-sense SNP have many practical uses because (1) they are substantially absent within the same strain but are stably present among different strains; and (2) the sense SNP, which exists in ESP (cDNA fragment), is likely to be associated with the distinct phenotype (characteristic) of the strain. In particular, the nonsynonymous substitution SNP is very likely to be associated with the distinct phenotype (characteristic) of the strain.

Specifically, the SNP can be used to identify strains for example. Further, when the presence of SNP produces different phenotypes among different barley strains (i.e., when the phenotype of a gene is different from the allelic phenotype resulting from the single nucleotide polymorphism), the SNP can be used as (1) a marker for obtaining a gene associated with the phenotype, (2) a marker for selecting a candidate heterozygote to be crossed with a recurrent parent in backcrossing (candidate heterozygote selecting marker), (3) a marker for determining success or failure of gene transfer (transfer determining marker), and (4) a marker for selecting a new strain obtained by backcrossing or genetic engineering techniques (new strain selecting marker).

The oligonucleotides of the present invention including a polymorphic site or its complementary site may be used, for example, as (1) a probe or primer for testing (SNP typing) the base of the polymorphic site or its complementary site, and (2) a probe or primer for obtaining and/or identifying a gene including the polymorphic site or its complementary site. Methods of using and producing the oligonucleotides according to the present invention will be described later.

### (B) Preparation of CONTIG sequences and a method of detecting and identifying nonsynonymous substitution SNP

In the following, description is made as to a preparation method of the CONTIG sequence discussed above.

First, using an ordinary method, cDNA libraries are developed from the respective tissues of different barley strains (for example, "H. spontaneum", "Haruna Nijo", and "Akashinriki" in the present invention). For example, the total RNA of the tissue is coarsely extracted, and total mRNA (poly (A) + RNA) is purified from the RNA so obtained. Using the mRNA as a template, a first-strand cDNA is synthesized, followed by the synthesis of a second-strand cDNA. The resulting double-strand cDNA is then ligased to a suitable vector, which is then packaged into a host to create a cDNA library. Optionally, the double-strand cDNA may be cut into a suitable length.

In the next step, the resulting clones are extracted, and sequence analysis of the respective cDNA clones is performed. Based on the base sequence of the cDNA identified by the sequence analysis, the clones are clustered based on the presence or absence of an overlapping base sequence. Then, overlapping portions of the inserts (cDNA) in the clustered clones are found and assembled together to obtain CONTIGS. In addition, the inserts are aligned to enable detection of SNP (multiple alignment).

As the barley tissue from which the total RNA is extracted, tissues of a shoot or leaf are suitably used. However, the type of tissue is not just limited to these. In using tissues obtained from barley raised from a seed, the cultivation method or the growth stage of the seed is not particularly limited. Note that, a standard method that uses barley as an experiment material can be suitably used as a cultivation method of a barley seed (see Cell Technology, Separate Volume, Plant Cell Technology Series 14, Plant Genome Research Protocol, pp. 193-195 (Shujunsha (2000)).

Note that, the extraction of the total RNA is generally carried out using tissues obtained from barley shoot or leaf in the germination stage. This is because the shoot or leaf in this growth stage can be pestled relatively easily, and contains a relatively small amount of impurity such as polysaccharides. Another reason is that they can be grown from a seed in a short period of time. Alternatively, the total RNA may be extracted from the barley tissue when distinct characteristics of different barley strains are expressed.

The series of steps, from the step of extracting total RNA from the barley tissue to the step of performing sequence analysis of the clones, may be carried out by a common method. For example, a method described in Breeding Research, Volume 2, Separate Volume 1, page 28, 2000, Genome Analysis Laboratory Manual, pp. 63-77: Springer-Verlag Tokyo, 1988 may be conveniently used. In addition, in order to ensure reliability of analysis result, it is preferable to carry out at least one of the following procedures: (1) For each strain of barley, extract total RNA from more than one individual; (2) Use more than one clone for the sequence analysis; and (3) For an insert (cDNA) of a length no shorter than a predetermined length, analyze the sequence both from the 5' end and 3' end. It is more preferable that the inserts of the respective clones be re-sequenced to confirm the sequence.

Next, the base sequences of inserts obtained by the sequence analysis of the respective clones are assembled based on overlapping portions of the sequences, so as to create the CONTIGS. In addition, the inserts of the clones with the CONTIG sequences are multiple-aligned to enable detection of base variations (SNP in this example) among the different clones (strains).

Base-calling in the sequence analysis of inserts, assembling of overlapping inserts, and multiple-alignment may be carried out using, for example, known SNP-detecting software or sequence-editing software. Examples of a program installed in such software are Trace-Diff (Bonfield. J.K. et al.: Nucleic acids Research 26: pp. 3404-3408, 1998), Polybayes (Marth. G.T. et al.: Nature Genet. 23: PP. 452-456, 1999), Polyphred (Nickerson. D.A: Nucleic acids Research 25: pp. 2745-2751, 1997). All of these programs are of the Phred/Phrap type, in which Phred is used as a base-calling tool, and Phrap as an assembly tool (see Experimental Medicine, Vol. 18, No. 14 (September), 2000: pp. 1890-1892).

Alternatively, assuming that the probability of error in reading the bases in base-calling is constant (setting the Phred score (quality value) constant), overlapping inserts may be assembled using Phrap as an assembly tool.

In preparing CONTIGS, the base sequences of inserts are compared between all the clones making up the CONTIGS. (1) For a site that shows base variations, a base that has produced the highest quality accuracy (for example, a base with the highest Phrap score (quality value)) after the assembly is selected as a representative base of the CONTIGS. (2) When base variations are found between at least two of three groups of sequence data derived from Akashinriki, H. spontaneum, and Haruna Nijo, a universal code that denotes these bases is used as the base of the CONTIGS.

In the present invention, SNP among different strains is recognized only in case (2) which is considered to be particularly reliable. It should be noted here that the criteria that apply to case (2) include not only when base variations are found at a corresponding base among at least two of three groups of inserts derived from H. spontaneum, Akashinriki, and Haruna Nijo, but also when there are base variations at a corresponding base among at least two of three groups of sequence data obtained from H. spontaneum, Akashinriki, and Haruna Nijo by reading from the 3' end and 5' end the sequence of single inserts derived from the respective strains. Evidently, case (2) is also applied when there are base variations at a corresponding base between the sequence data obtained from any one of the three different strains of barley by reading from the 3' end and 5' end the sequence of a single insert derived from a selected strain, and the sequence data obtained from the single inserts derived from the other strains. However, when three or more SNPs are found consecutively on the CONTINGS by these methods, these SNPs are not regarded as the SNP of the present invention for reliability consideration.

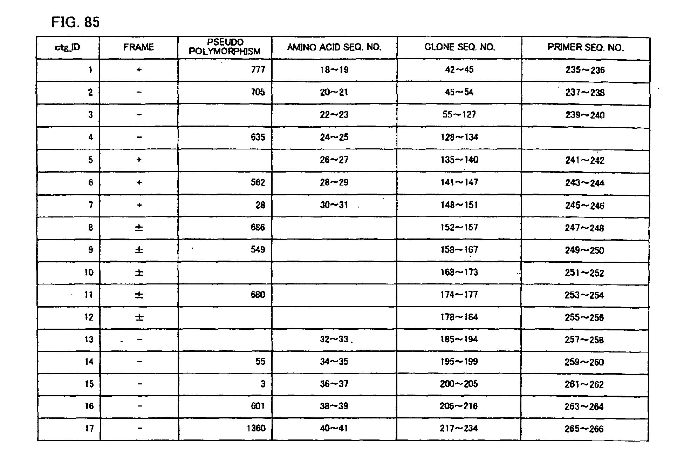

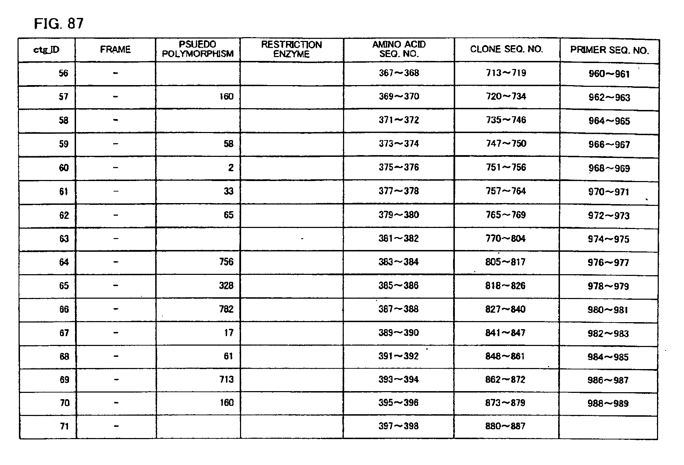

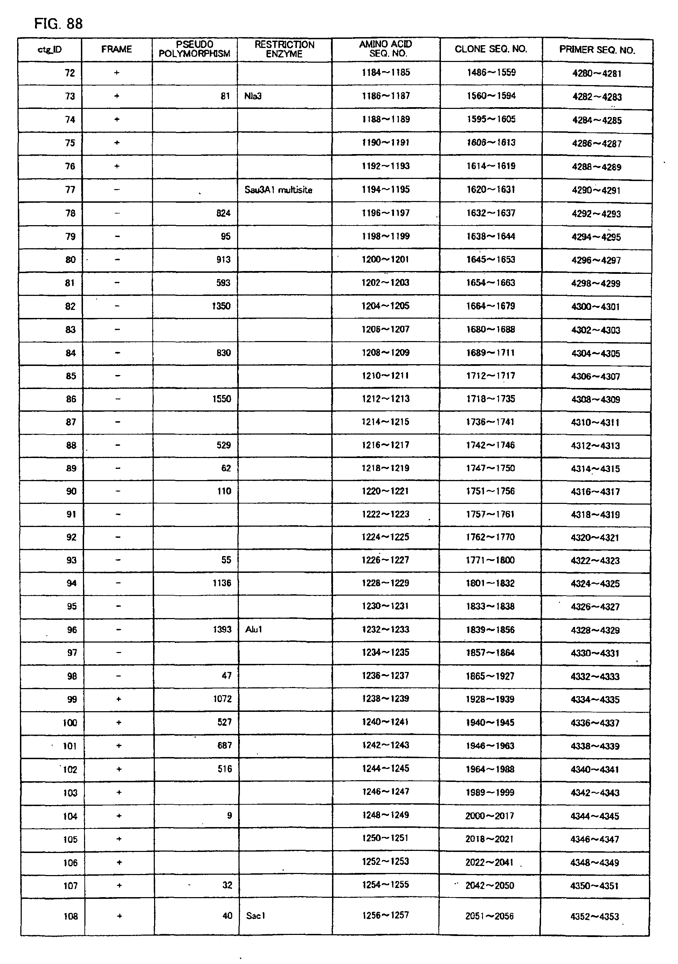

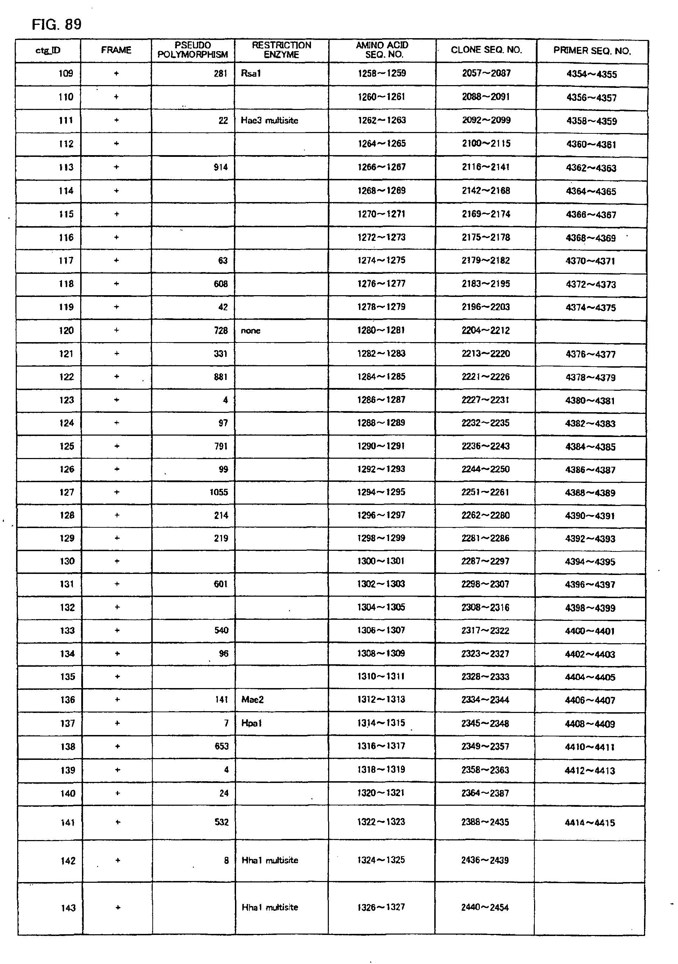

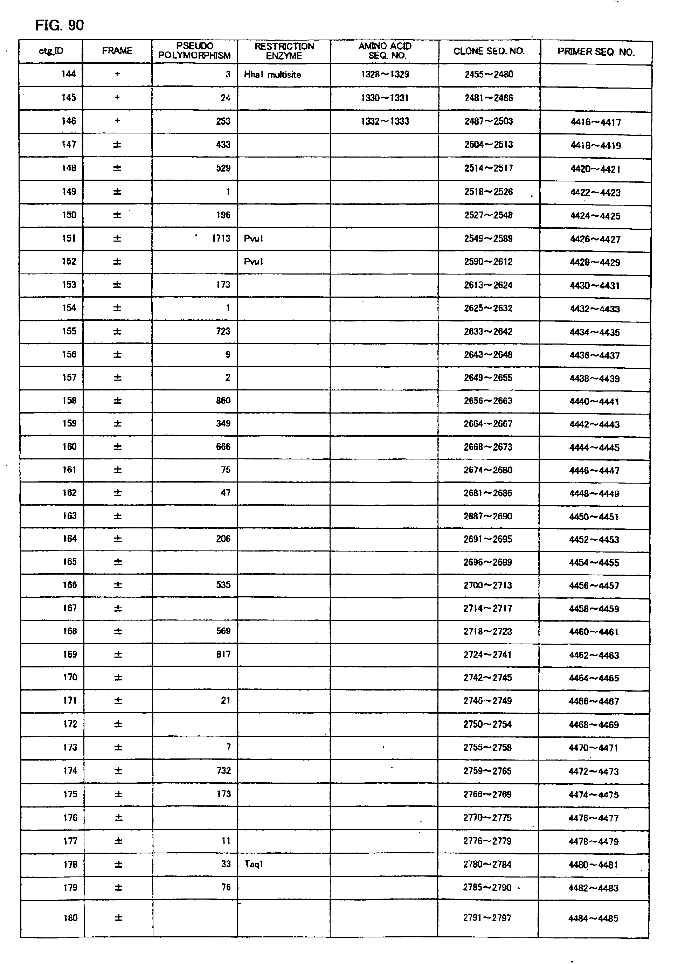

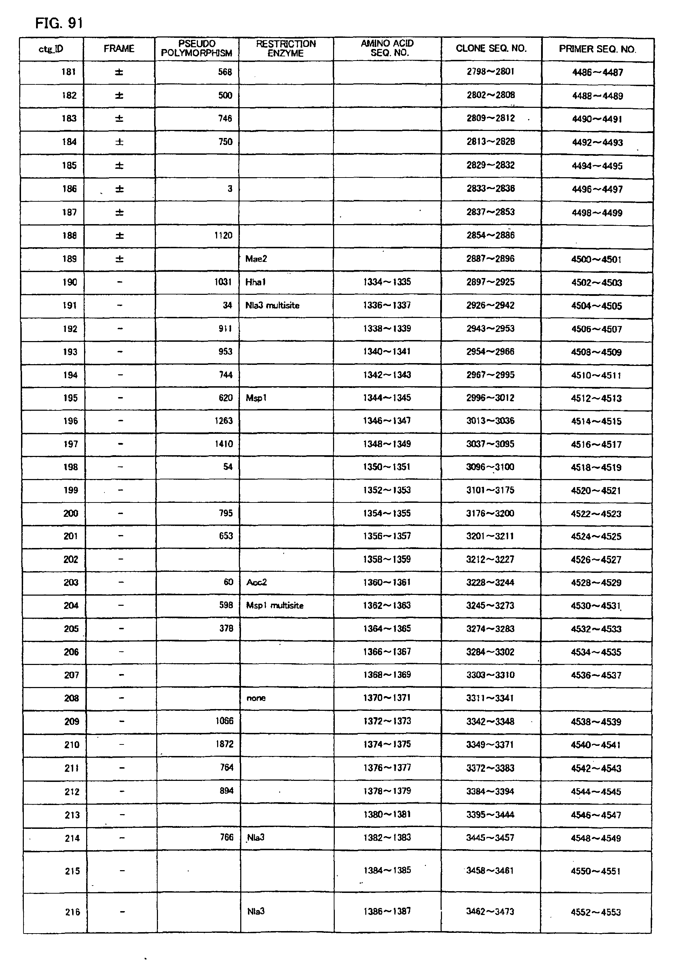

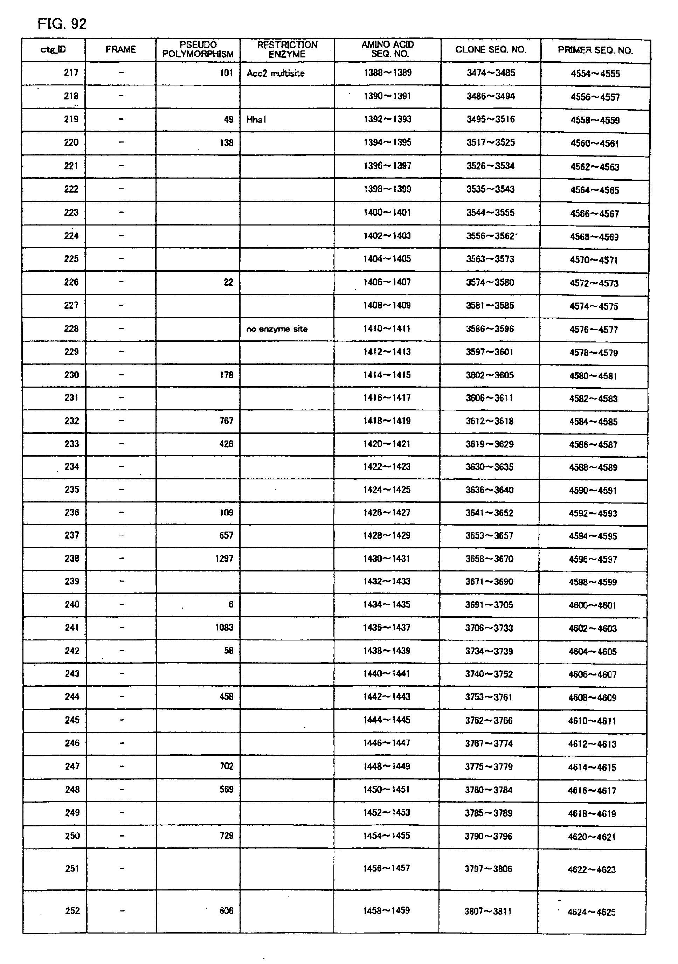

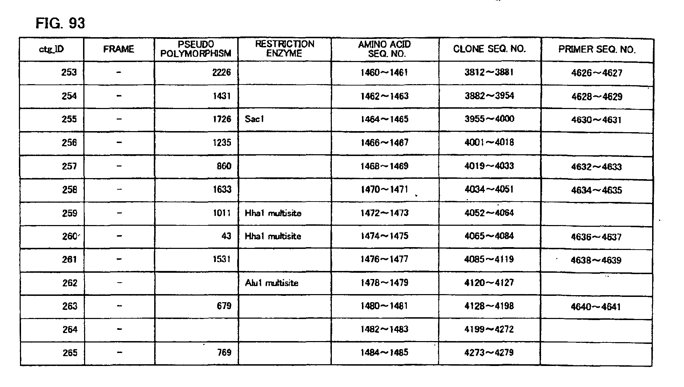

When an SNP is found not according to case (2) but case (1), the SNP is termed a "pseudo SNP" because it cannot definitely be regarded as the SNP among different strains. Fig. 85 through Fig. 93 show pseudo polymorphic sites for the CONTIGS (1) through (265).

In the CONTIGS (1) through (387), the SNPs found on the CONTIGS as above were screened for a nonsynonymous SNP in the manner described below. Since the method of screening for a nonsynonymous substitution SNP is different between CONTIGS (1) through (387) and CONTIGS (388) through (921), the following only deals with the former, and the latter will be described later.

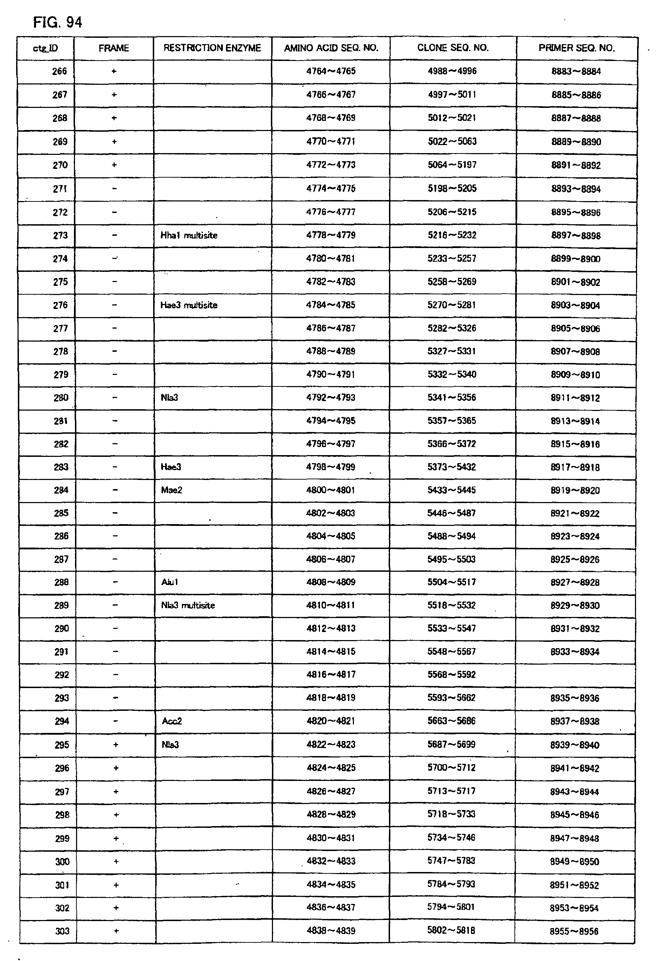

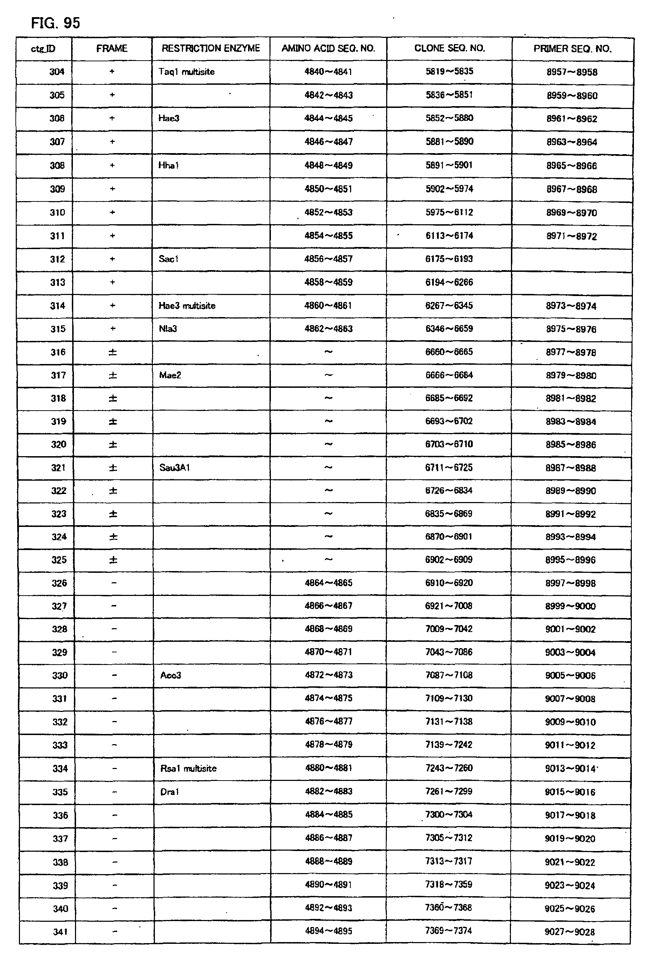

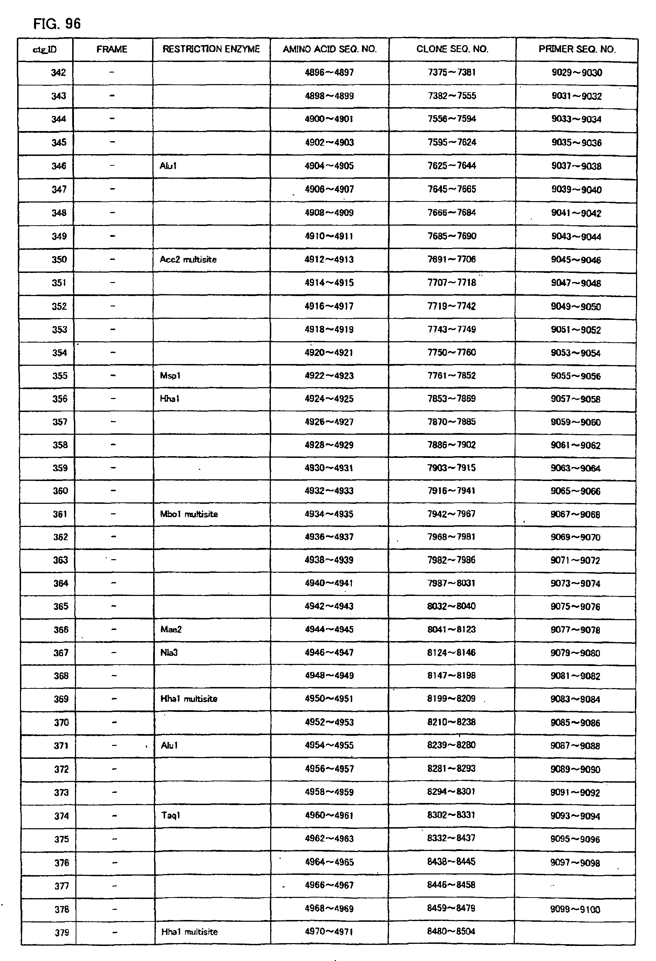

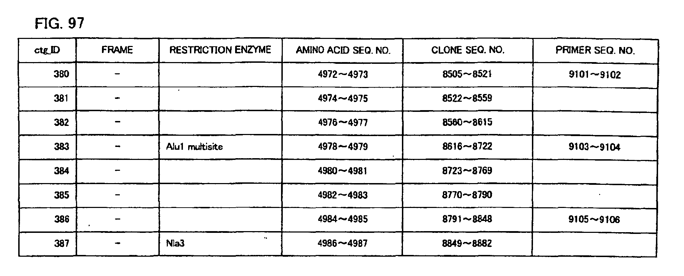

First, a homology search is conducted for the CONTIGS (1) through (387) according to a known method (search result will be described later). Then, a reading frame of the CONTIG is determined by comparing an amino acid sequence that was translated according to a certain reading frame (frame) and an amino acid sequence of a known protein (protein encoded by a known gene). When there is a certain level of homology between the two, this reading frame is determined as the reading frame of the CONTIG. The results are shown in Fig. 85 through Fig. 97.

In Fig. 85 through Fig. 97, the heading "ctg_ID" is the same as in Fig. 1 through Fig. 84. The notation "+" under the heading "FRAME" indicates that a gene is found on the CONTIG, and "-" indicates that a gene found on the complementary sequence of the CONTIG. The notation "±" indicates that the homology search result = None (no hit) (the result of homology search will be described later). Note that, the homology search result "None" indicates a high likelihood that at least one of the CONTIG and its complementary sequence has an unknown gene.

Fig. 85 through Fig. 93 show pseudo polymorphic sites for the CONTIGS (1) through (265). The column under "PSEUDO POLYMORPHISM" indicates the base position on the CONTIG sequences of the CONTIGS (1) through (265) where a pseudo SNP is found, wherein the pseudo SNP is a base variation found by multiple alignment among the cDNA base sequences of the different strains but at a site where sequence reliability is low. The result of sequence analysis at this stage is not so much reliable as to indicate that the pseudo polymorphism is a SNP. Nevertheless, there is a reasonable level of likelihood that the pseudo polymorphism is a SNP.

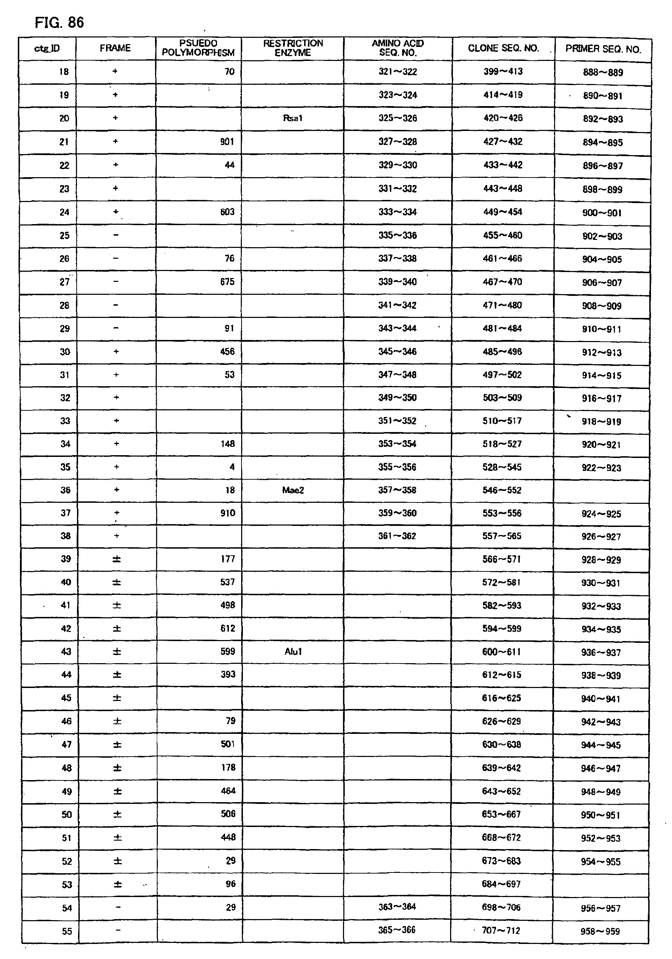

Fig. 86 through Fig. 97 show restriction enzymes for the CONTIGS (18) through (387). The column under "RESTRICTION ENZYME" contains names of restriction enzymes when they are present. The restriction enzyme cuts the polynucleotides of the present invention (described later) in different patterns according to the type of base present at the polymorphic site and/or its complementary site of the CONTIGS (18) through (387) (details will be described later, including the use of the restriction enzyme). Note that, the column is blank when, for example, the presence or absence of restriction enzyme was simply not determined.

Fig. 85 through Fig. 97 show sequence numbers of amino acid sequences encoded by the CONTIGS (1) through (387), referring to the sequence table.

The column under "AMINO ACID SEQUENCE" indicates, referring to the sequence table, sequence numbers of the base sequences of the genes found to be present on the CONTIGS ("+") or on its complementary sequences ("-") by the homology search result (to be described later), and of amino acid sequences encoded by these base sequences. Here, the amino acid sequence is only a portion of the sequence that was translated from a region homologous to an amino acid encoded by a known gene, and it does not necessarily indicate the entire length.

Note that, when the amino acid sequence contains 18 amino acid residues or less, i.e., when one of the 2nd through 19th codons is a stop codon, the stop codon is treated as an unknown amino acid and is translated as "Xaa," as an exception. There were a total of four stop codons that were treated as unknown amino acids:
The 7th stop codon of the amino acid sequence (sequence numbers 373, 374) of the CONTIG (59);
The 2nd and 19th stop codons of the amino acid sequence (sequence numbers 4914, 4915) of the CONTIG (351); and
The 15th stop codon of the amino acid sequence (sequence numbers 4984, 4985) of the CONTIG (386).

In the amino acid sequences of the CONTIGS (1) through (17) and CONTIGS (72) through (387), the base of the polymorphic site represented by a universal code is of a "wild-type." In the amino acid sequences of the CONTIGS (18) through (71), the base of the polymorphic site represented by a universal code is of "Akashinriki."

In Fig. 1 through Fig. 30, the column under "EVALUATION 1" indicates the results of evaluation of SNPs for the CONTIGS (1) through (387) based on the reading frame of the amino acid sequences. The SNPs that are in a coding region of the amino acid sequence and that do not correspond to the "polymorphic site that causes nonsynonymous substitution" are denoted as "SILENT." The SNPs that are not in a coding region of the amino acid sequence are denoted as "OUTSIDE CODING REGION". For the SNPs that are in a coding region of the amino acid sequence and that correspond to the "polymorphic site that causes nonsynonymous substitution," a change in amino acid is indicated that results from the base variations at the polymorphic site.

For the CONTIGS (266) through (387), the SNPs that are not in a coding region of the amino acid sequence are classified under "UPSTREAM OF CODING REGION" and "OUTSIDE CODING REGION." In the base sequence of a gene that is found to be present on the CONTIG ("+") or on its complementary sequence ("-"), SNPs that are closer to the 5' end upstream of the coding region of the amino acid sequence are indicated by "UPSTREAM OF CODING REGION," whereas SNPs that are closer to the 3' end downstream the coding region of the amino acid sequence are indicated by "OUTSIDE CODING REGION."

Fig. 85 through Fig. 97 show sequence numbers of the clone sequences and primers for the CONTIGS (1) through (387), referring to the sequence table. The column under "CLONE SEQUENCE" contains sequence numbers of the base sequences of the respective clone groups for the CONTIGS (1) through (387), referring to the sequence table. The cDNA base sequences that belong to the same clone group are the sequences of cDNA clones obtained from a single expression gene. The CONTIG sequence is obtained by finding portions of overlap in the cDNA sequences of the clustered clone groups, followed by the alignment (multiple alignment) of the overlapping portions. For example, CONTIG (1) is obtained by aligning the cDNA clones (multiple alignment) represented by sequence numbers 42 through 45.

The column under "PRIMER" contains, referring to the sequence table, sequence numbers of base sequences of primers that were actually designed to amplify a region and/or its complementary region in the CONTIGS (1) through (387) including at least one of the polymorphic sites. (Here, the base sequences of the primers are shown by the base sequences of the CONTIGS.) The primers are used to detect SNPs that are stably preserved across strains. In other words, the primers are used for the amplification, for example by PCR, of oligonucleotides or genes for detecting SNPs that are stably preserved across strains. Details as to use of the primers will be described later. Note that, the primers shown here are merely an example of a PCR primer set for amplifying a region and its complementary region including at least one of the polymorphic sites, and the primers are not just limited to those indicated by the sequence table.

In the CONTIGS (388) through (921), the SNPs that were found to be present on the CONTIGS as above are screened for a nonsynonymous substitution SNP in the manner described below. First, a homology search is conducted for the CONTIGS (388) through (921) according to a known method (search result will be described later). Then, a reading frame of the CONTIG is determined by comparing an amino acid sequence that was translated according to a certain reading frame (frame) and an amino acid sequence of a known protein (including proteins encoded by known genes). When there is a certain level of homology between the two, this reading frame is determined as the reading frame of the CONTIG.

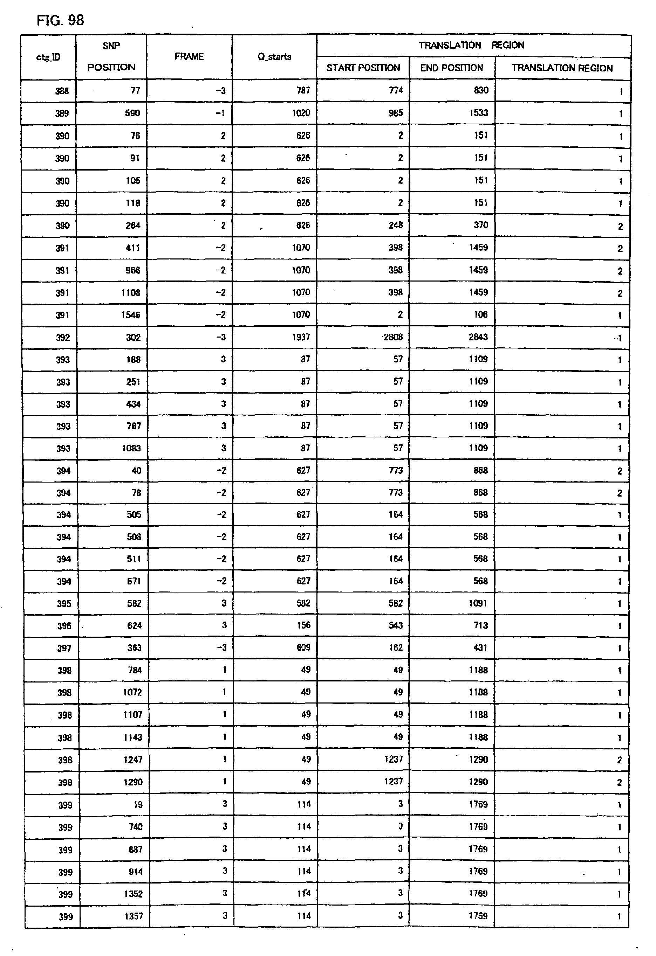

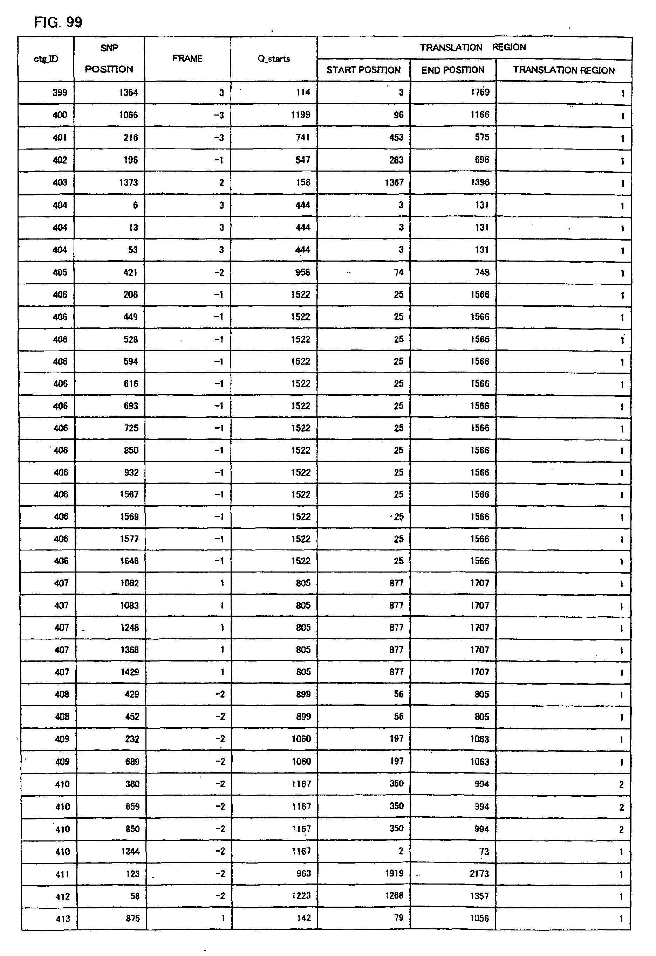

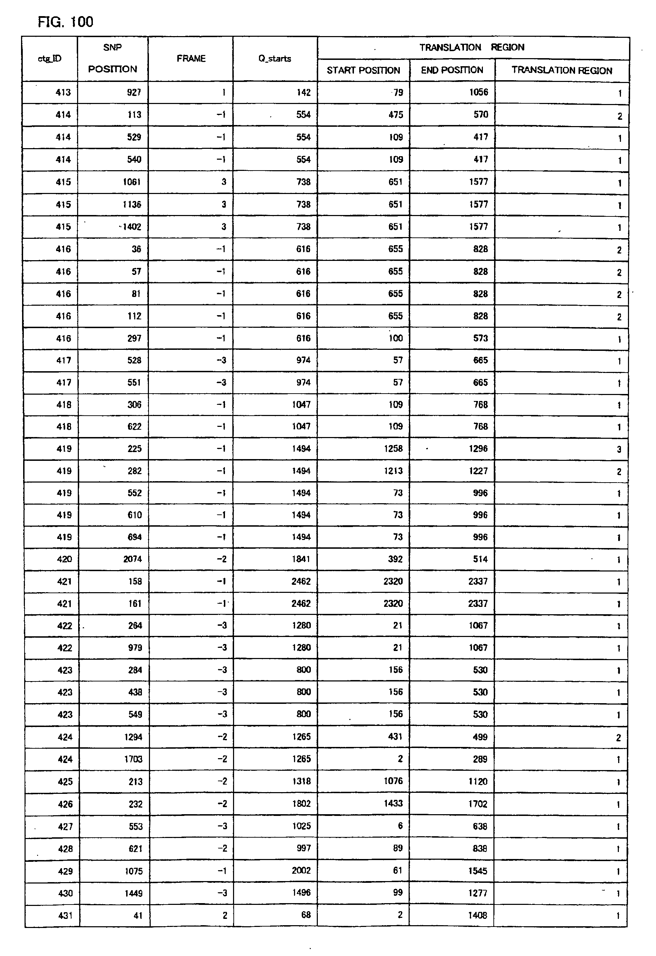

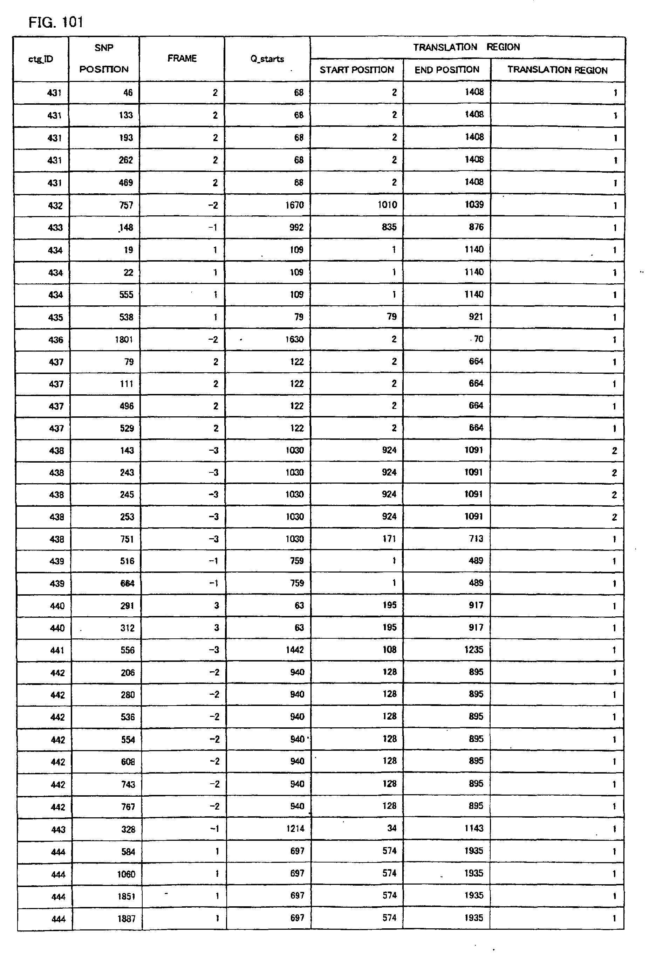

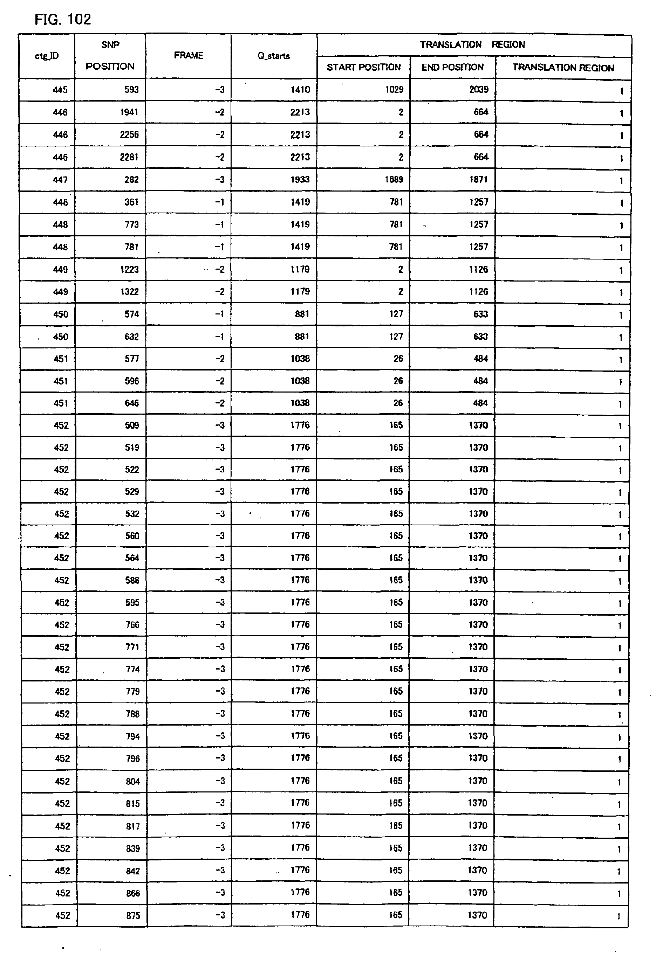

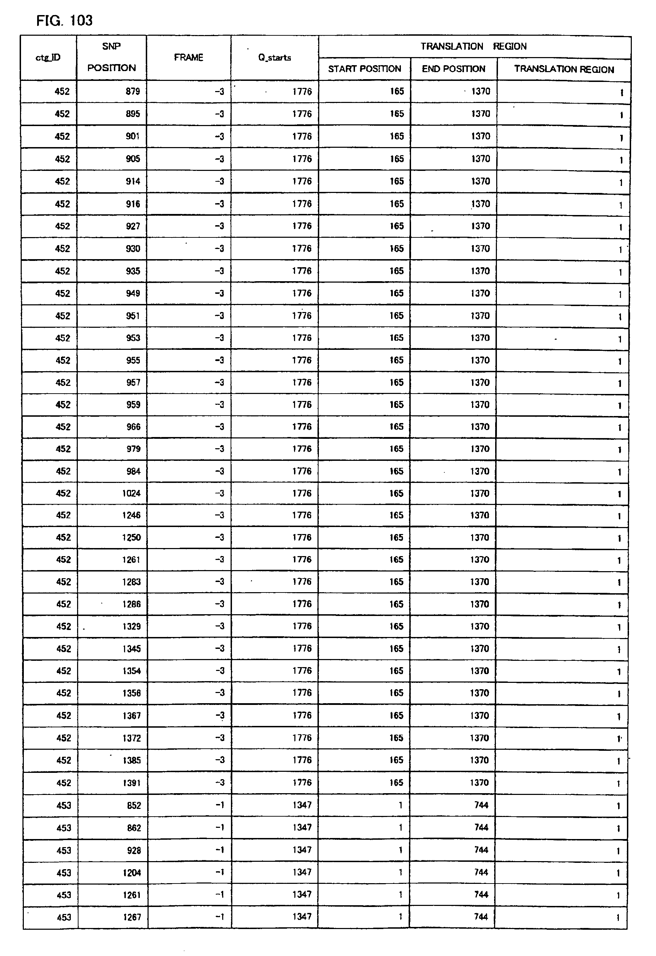

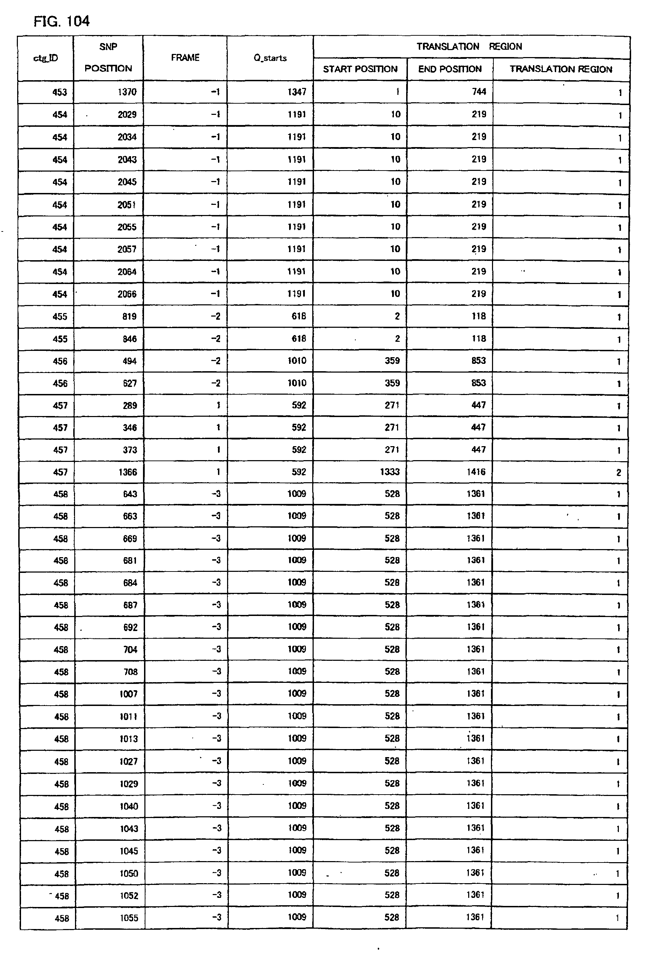

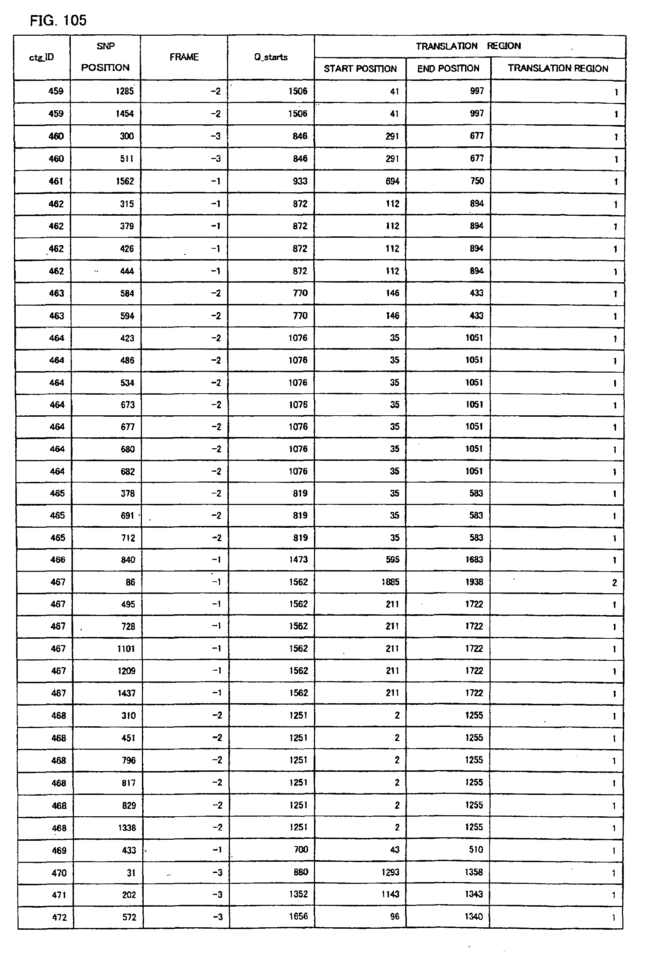

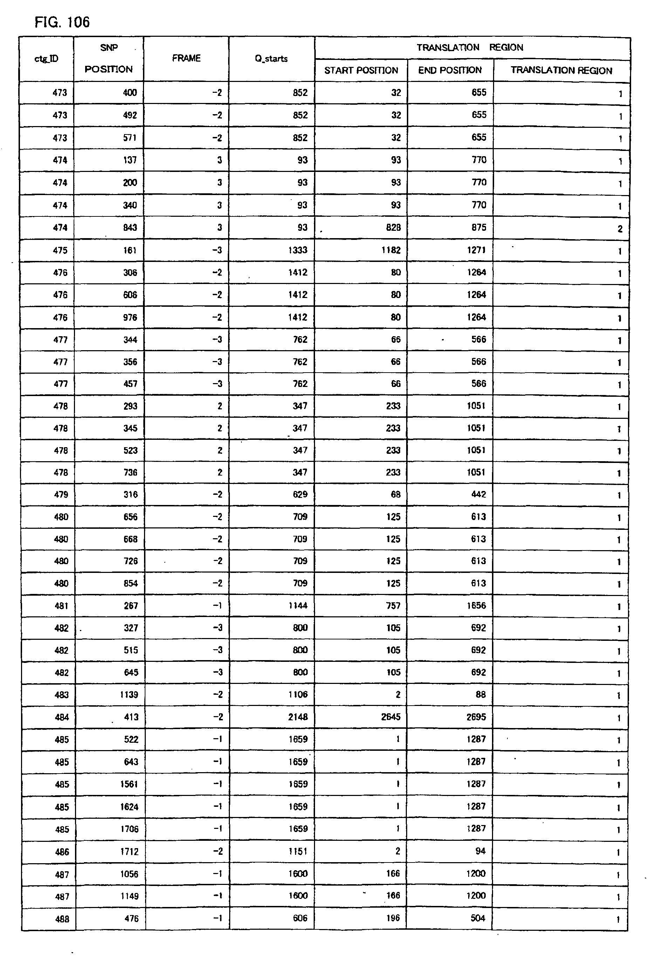

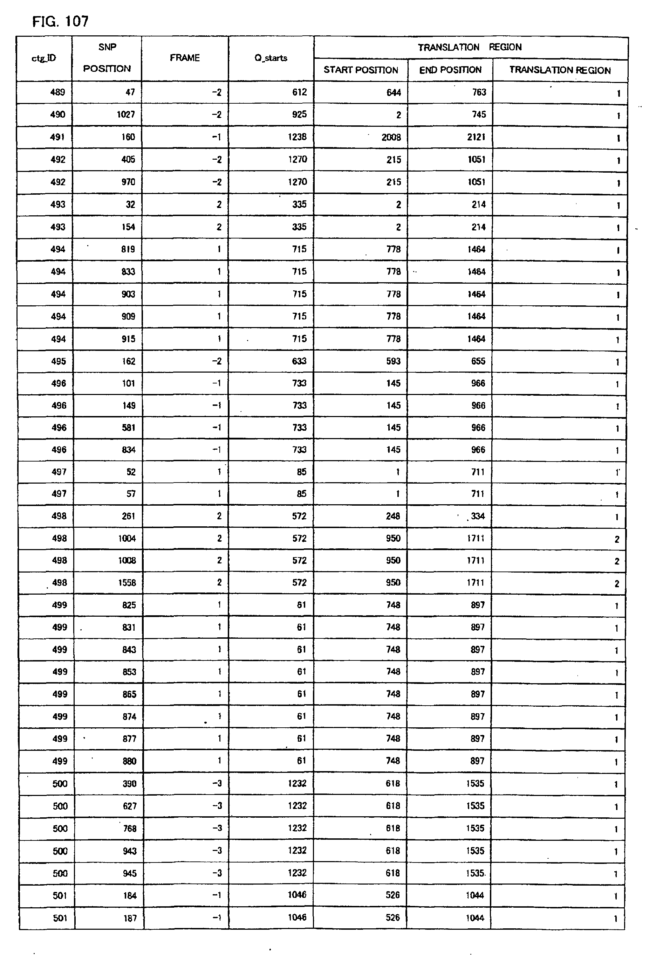

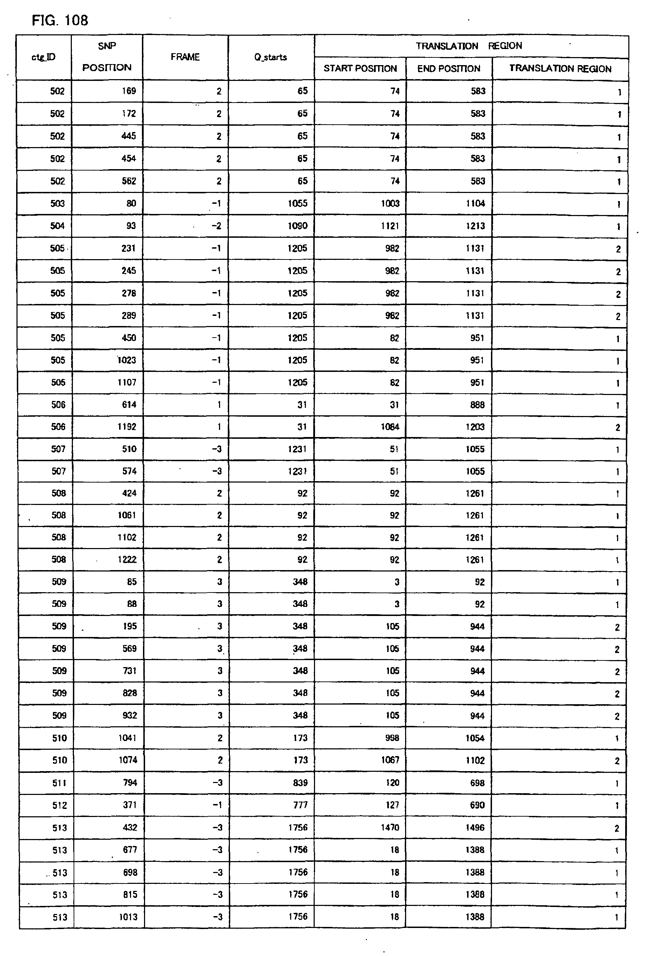

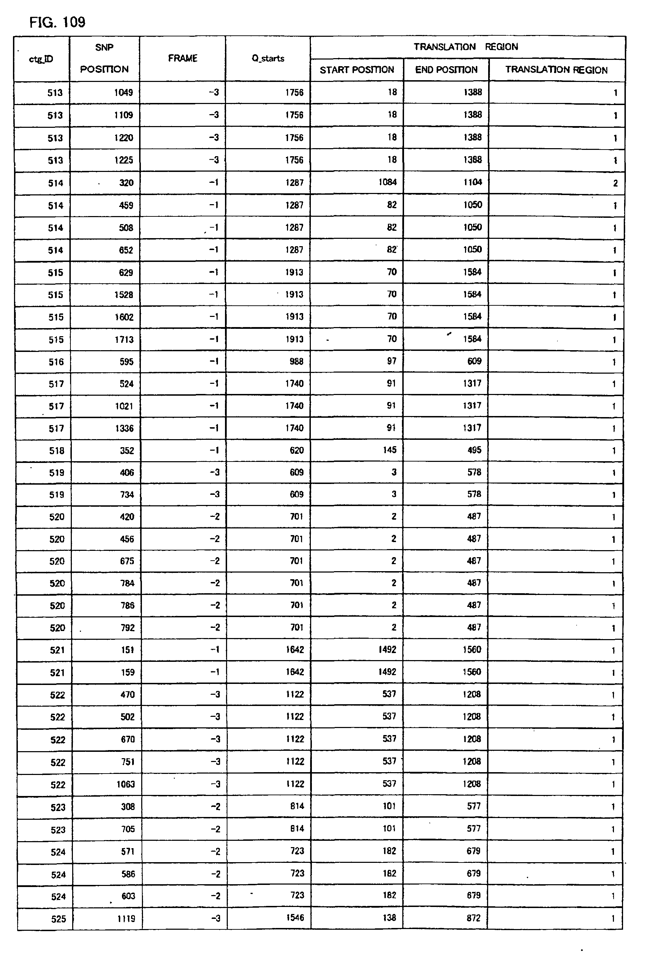

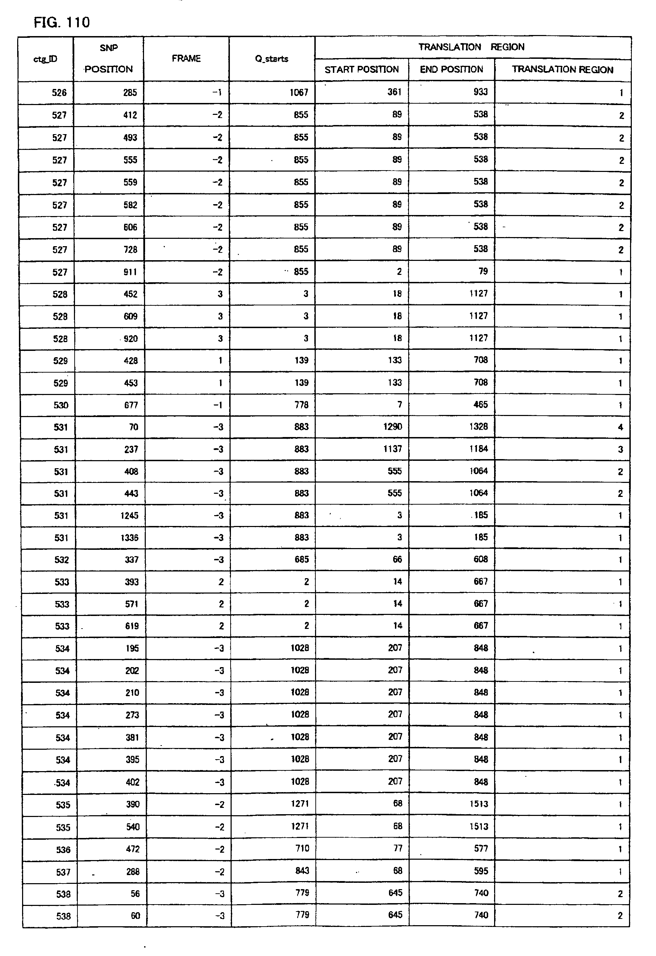

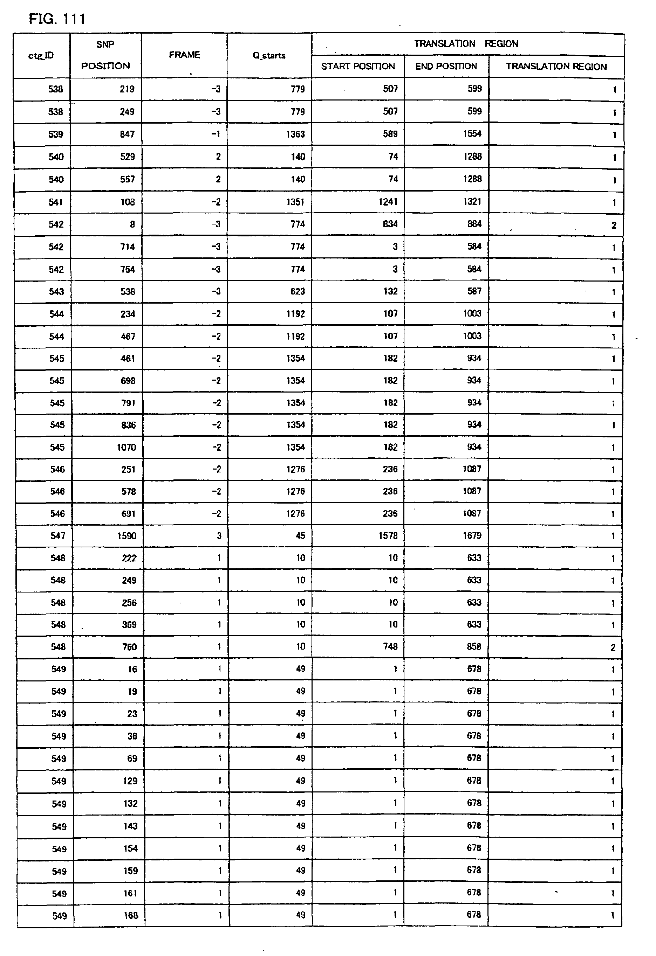

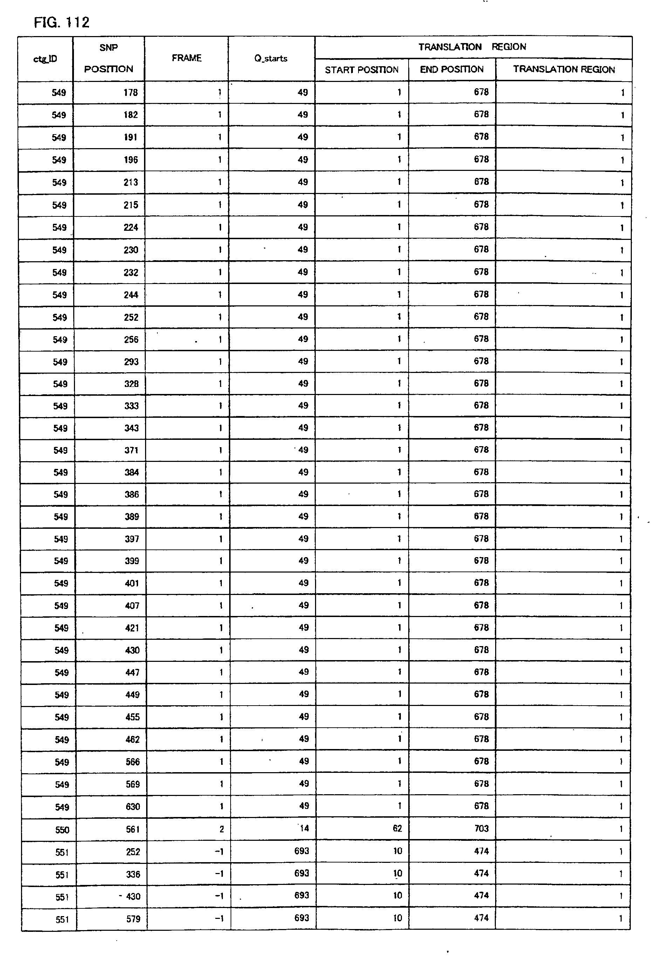

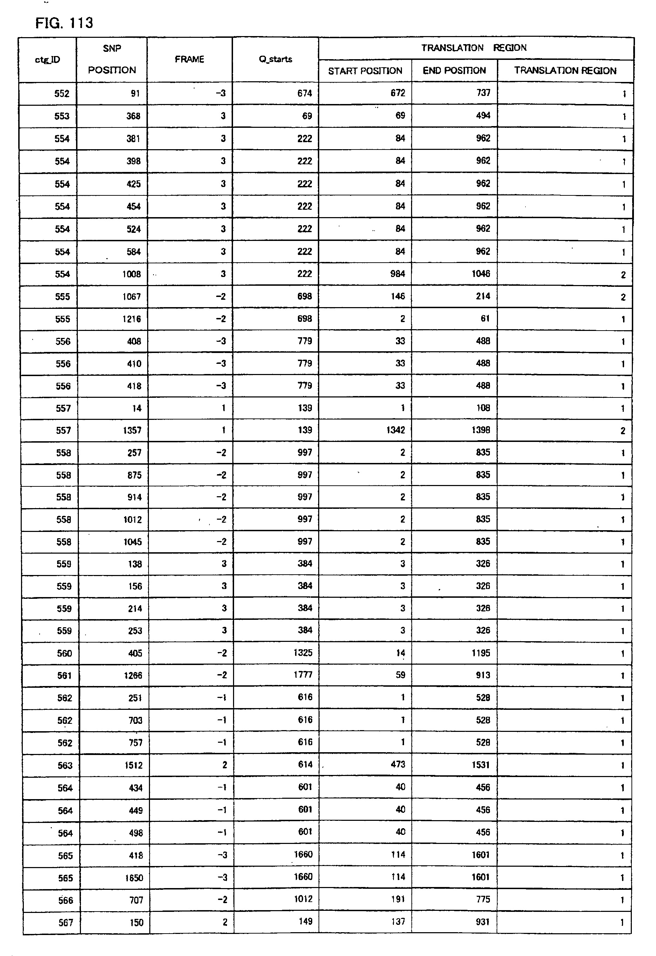

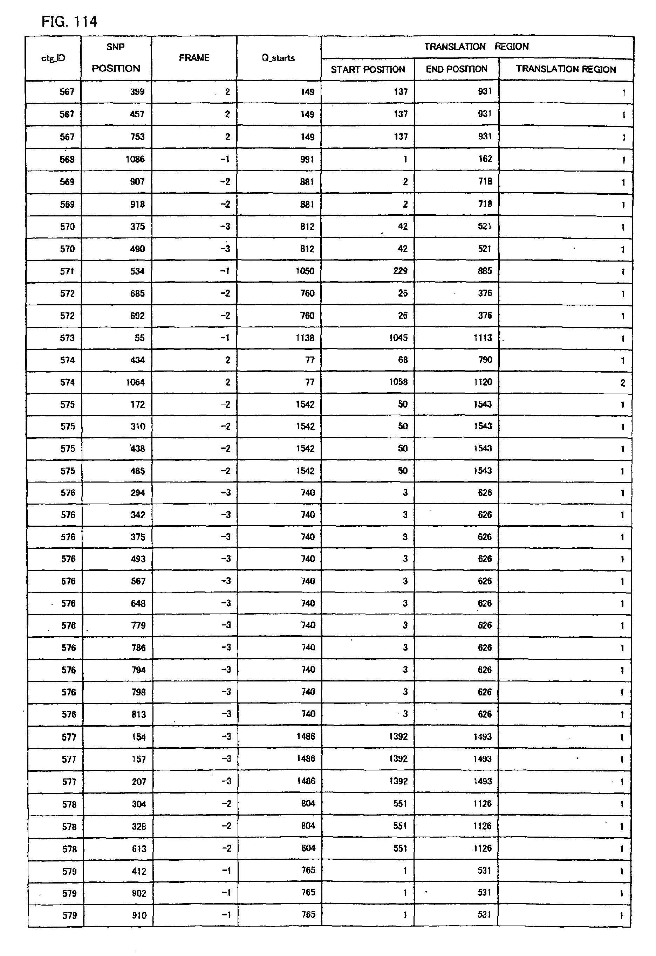

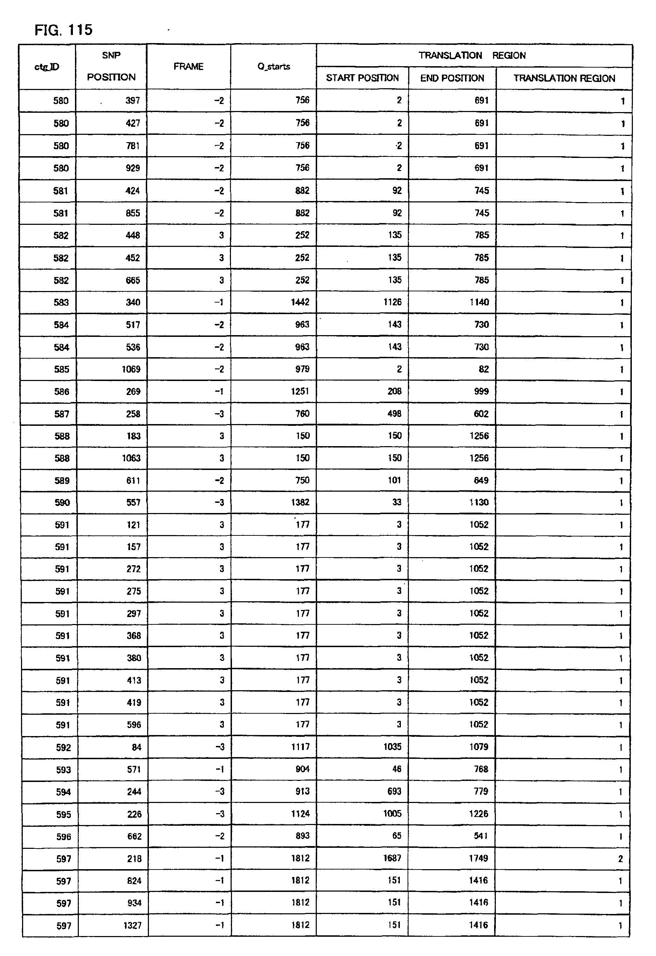

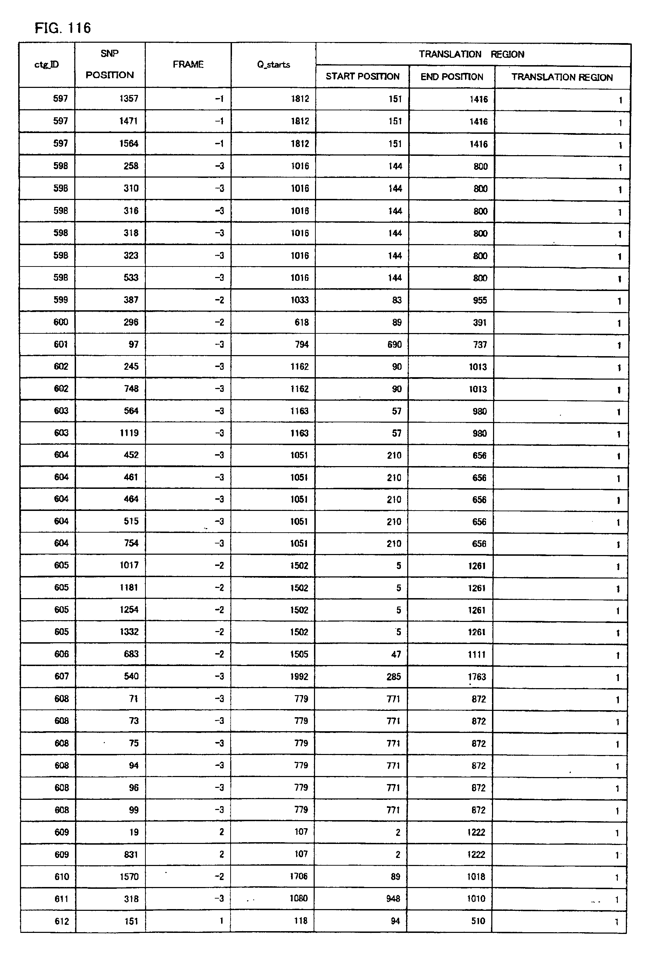

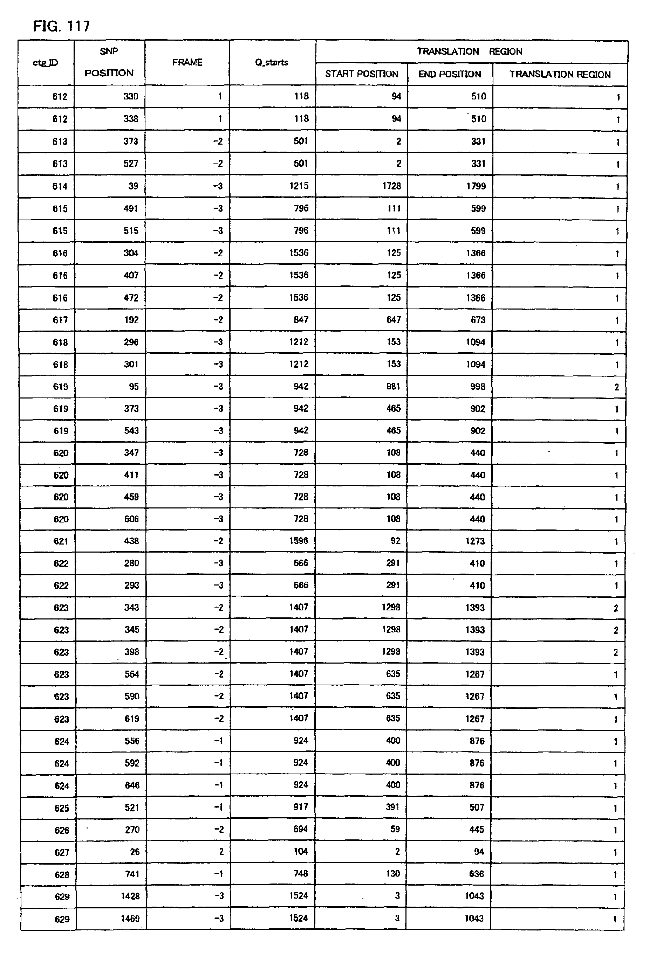

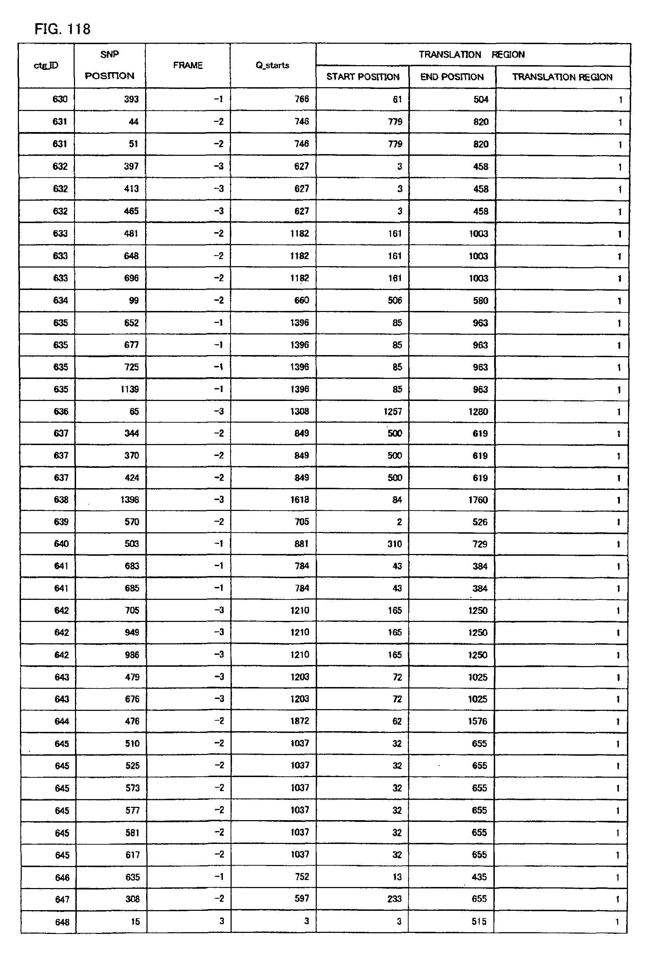

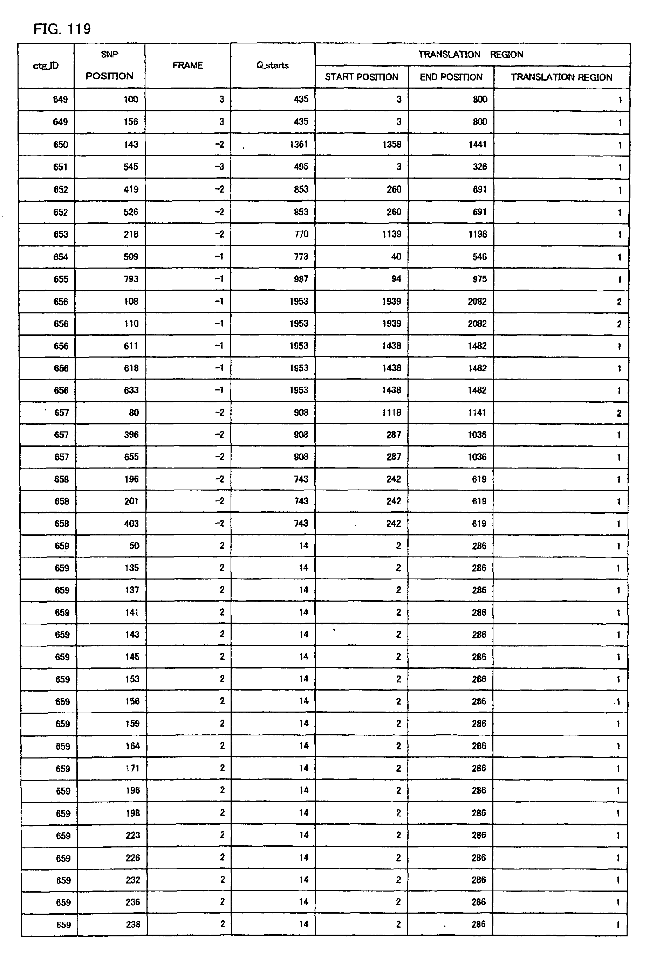

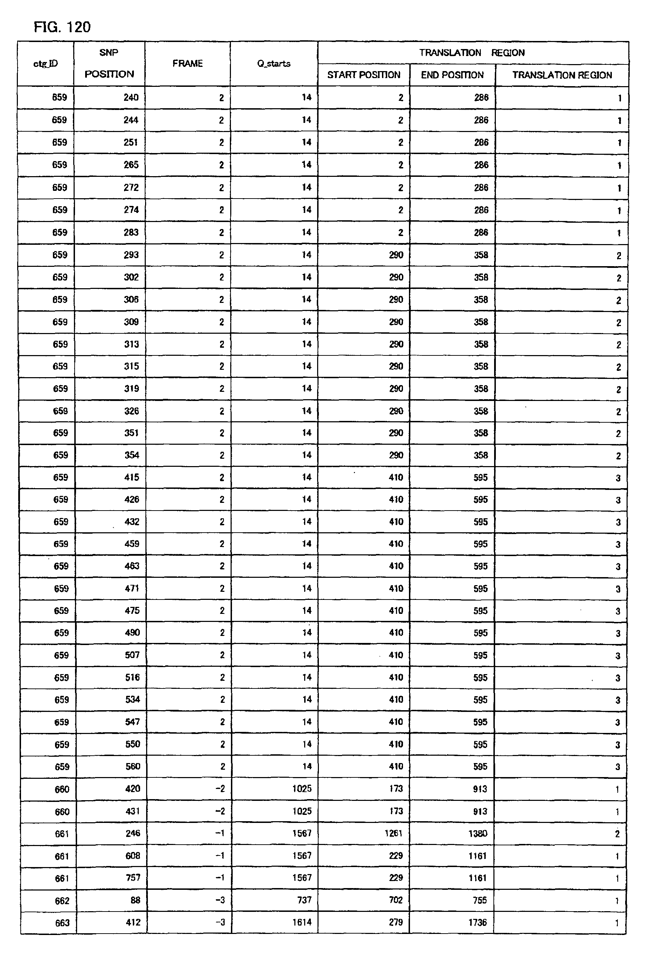

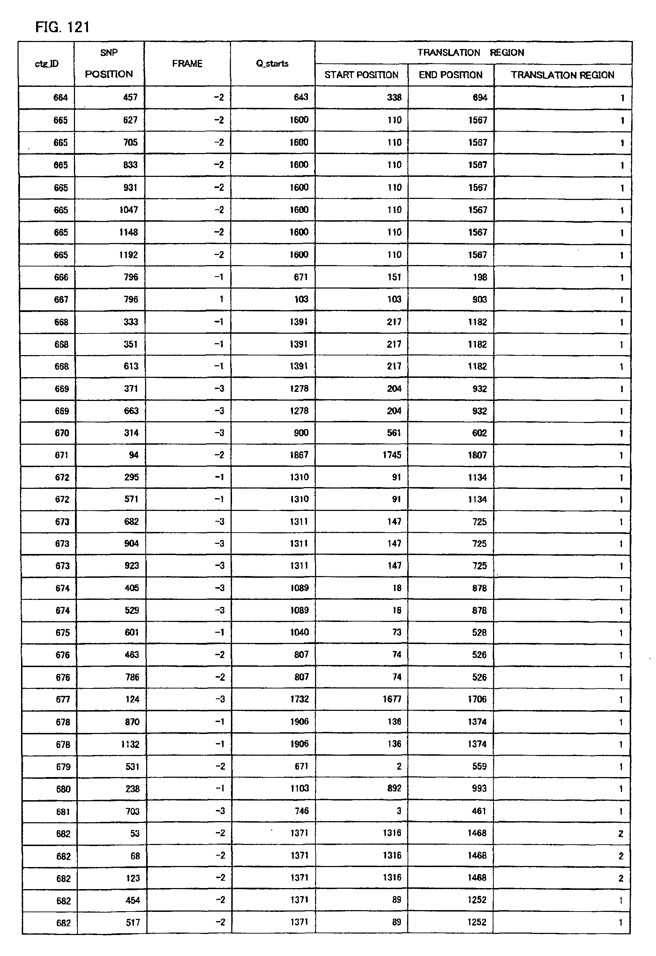

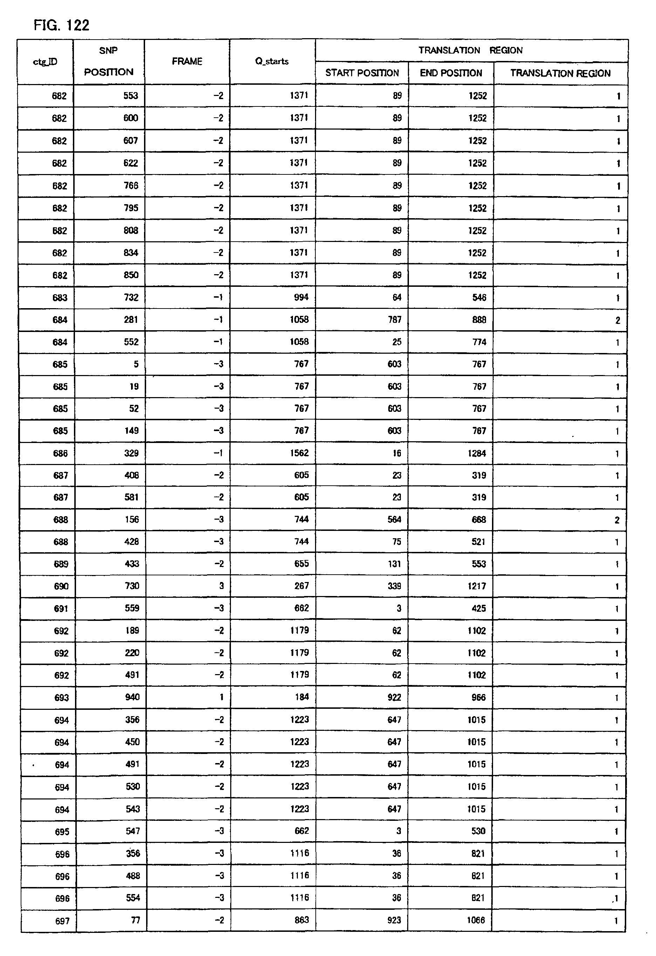

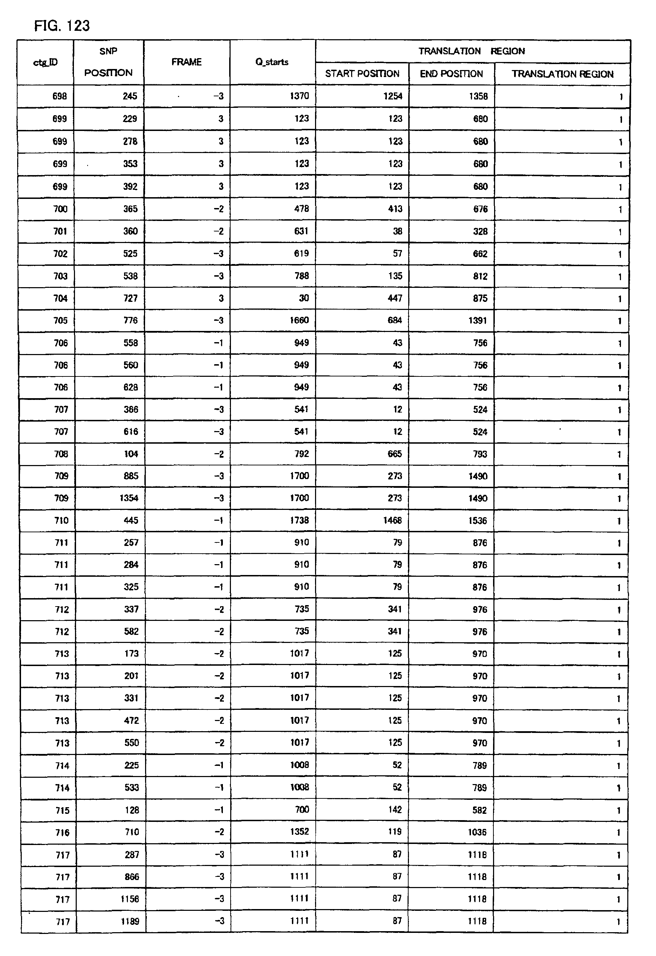

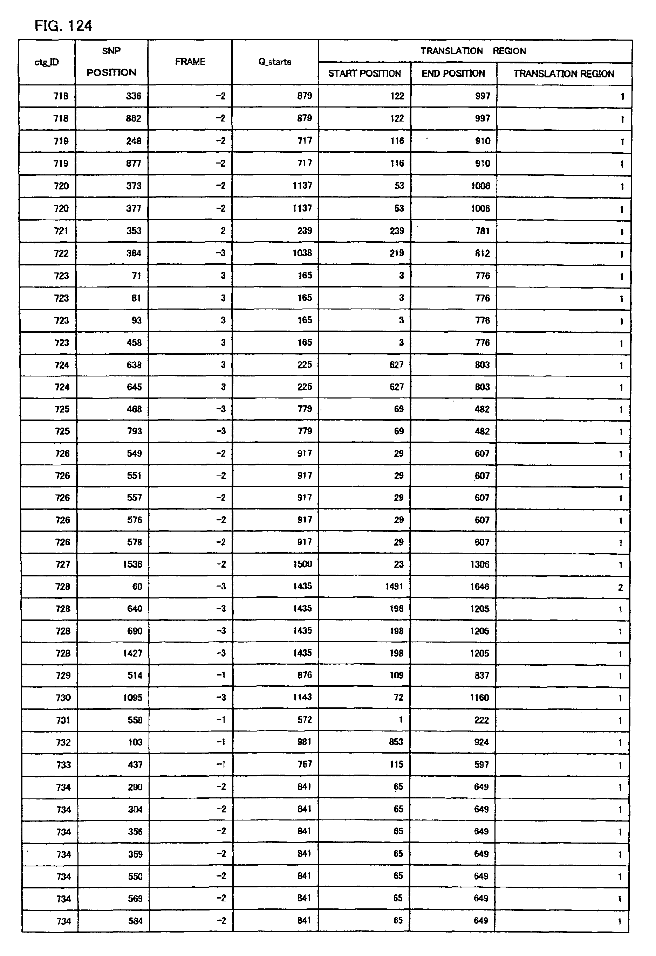

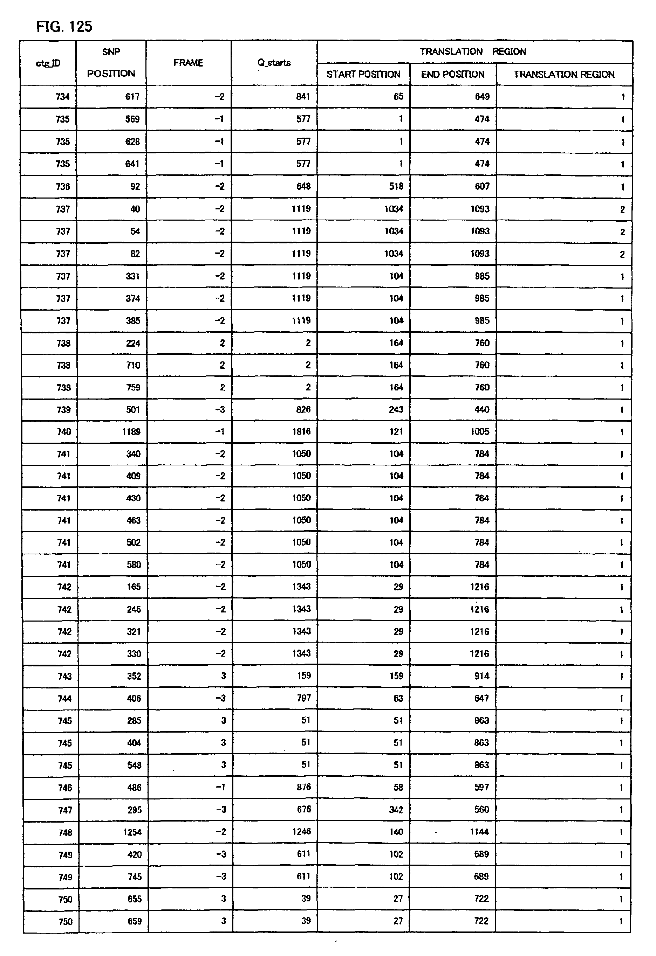

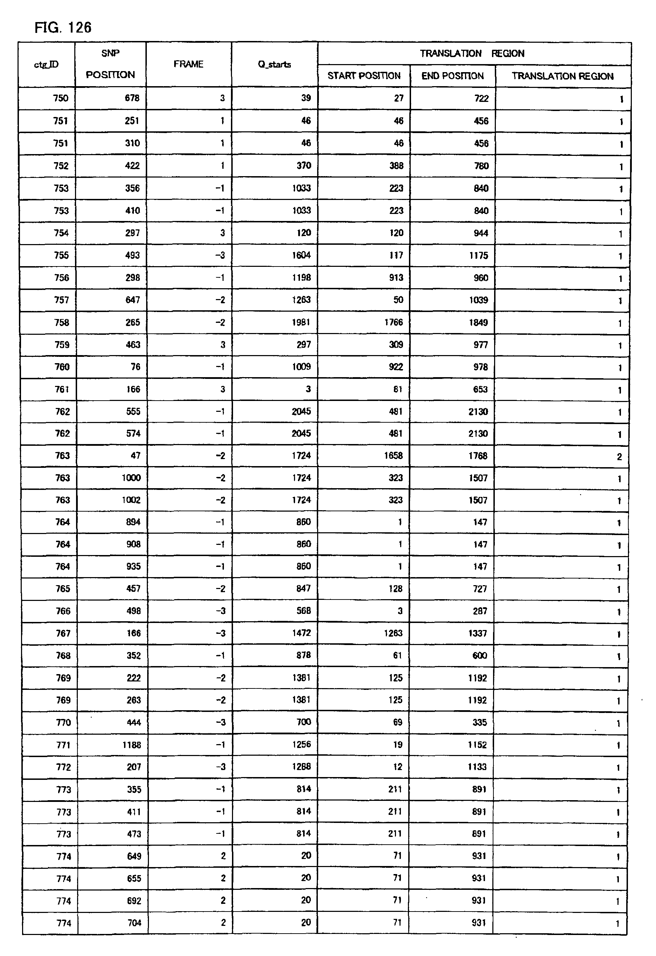

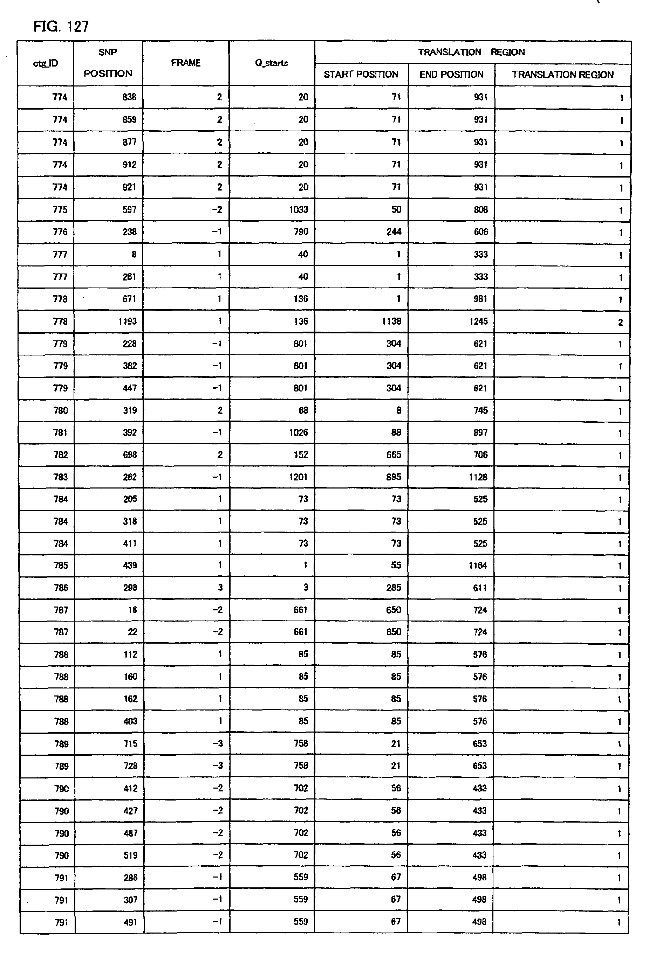

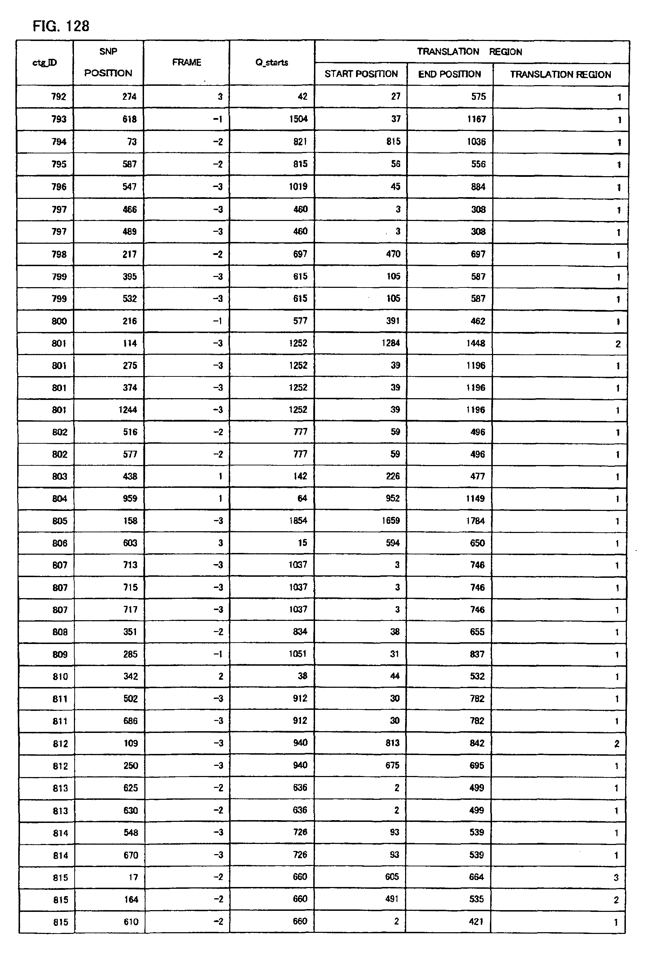

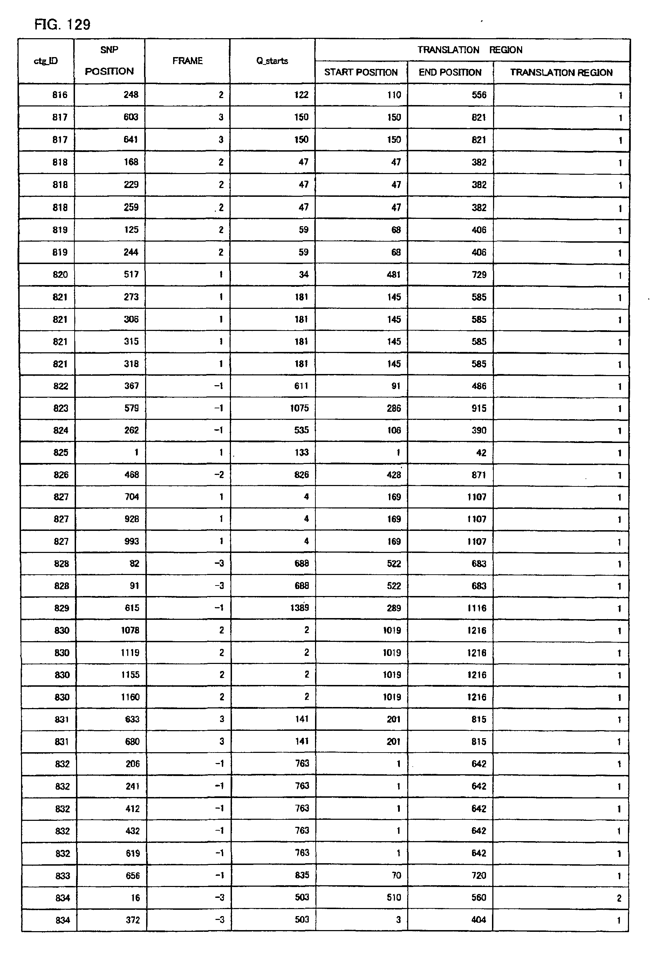

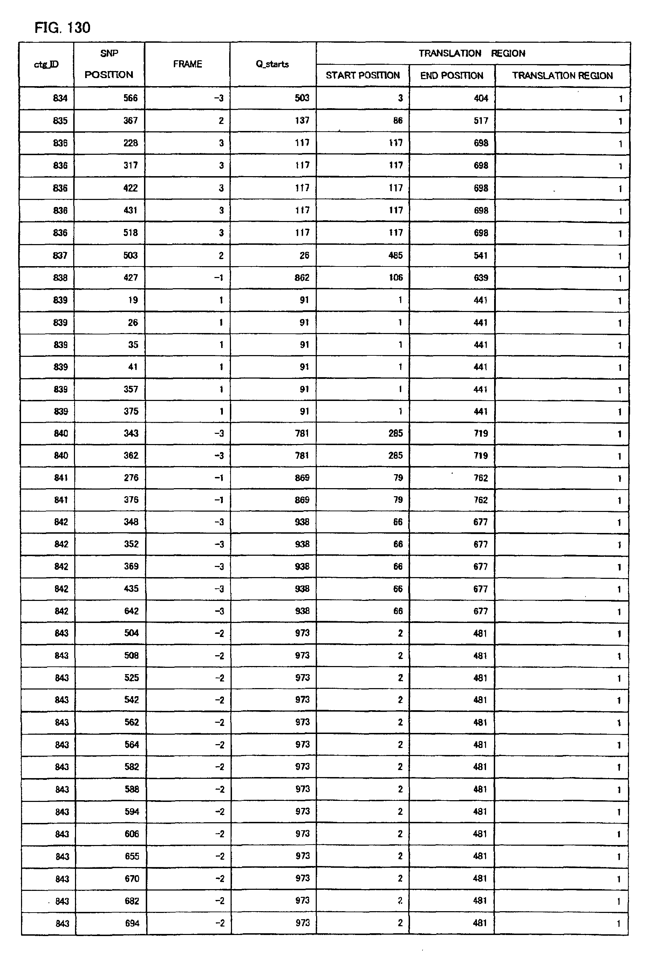

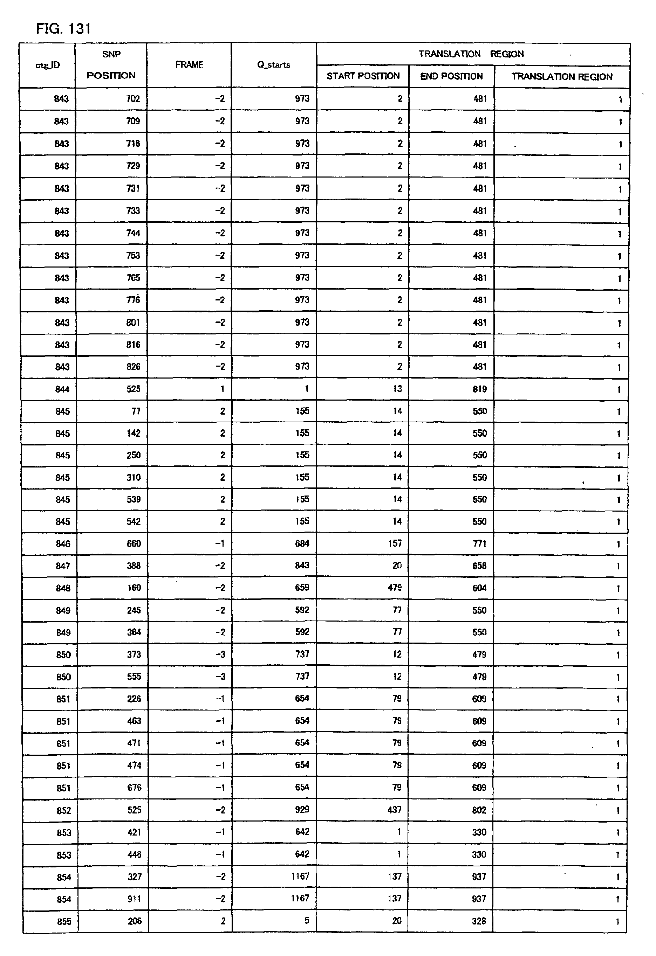

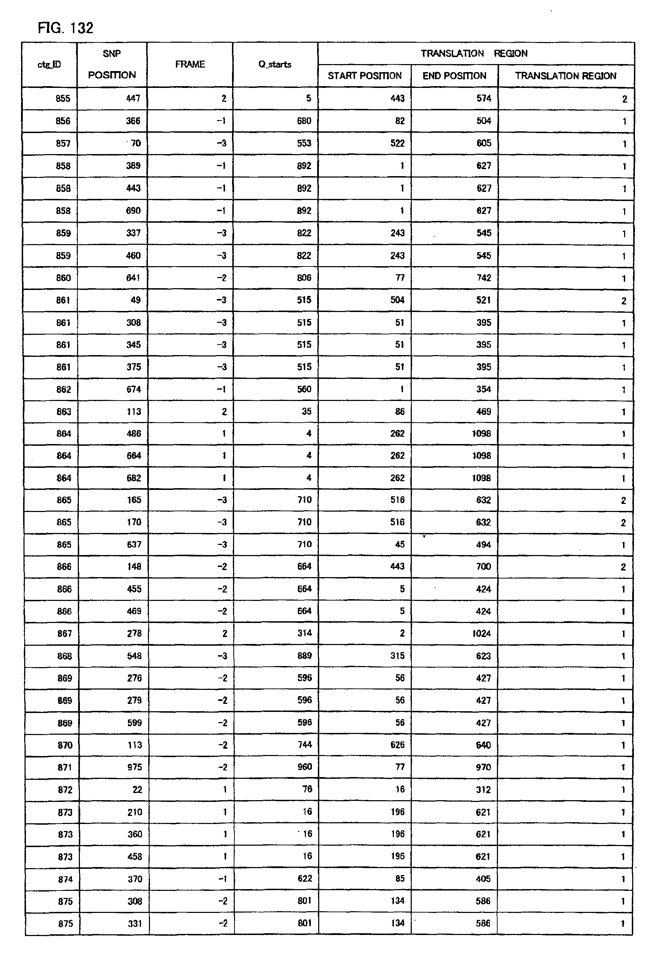

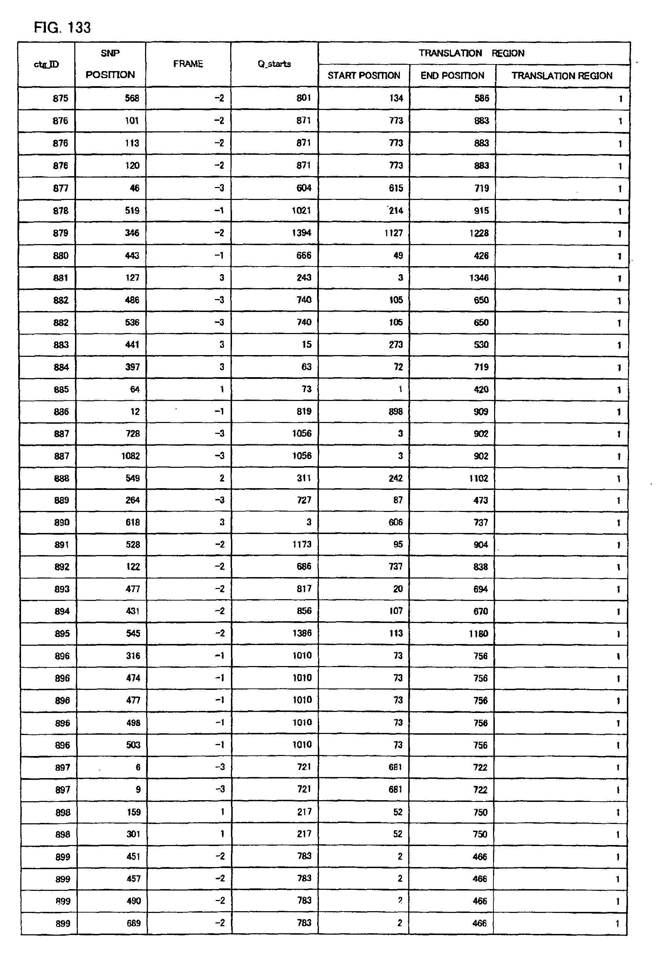

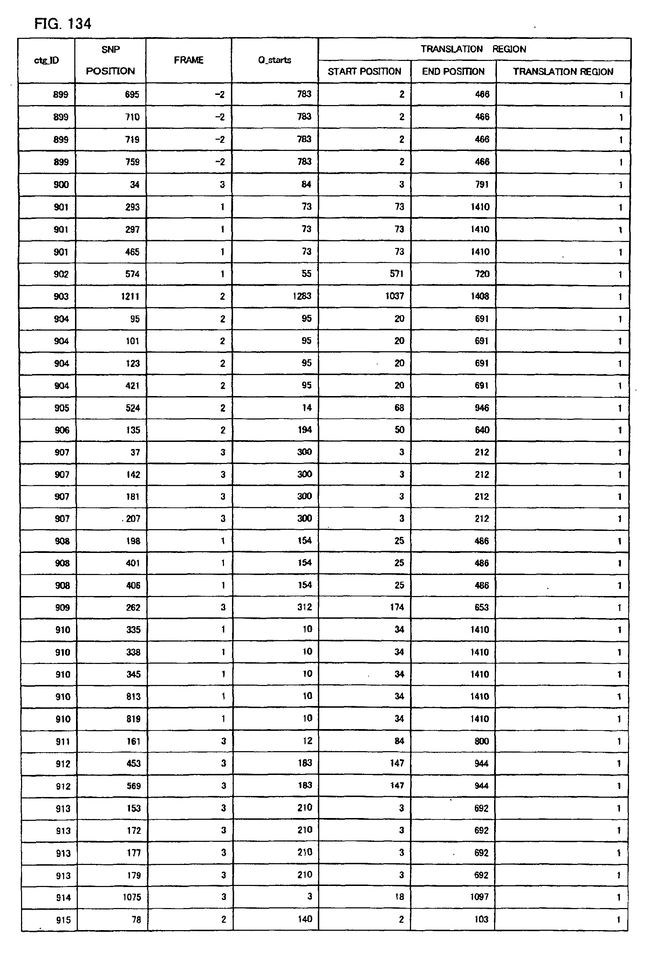

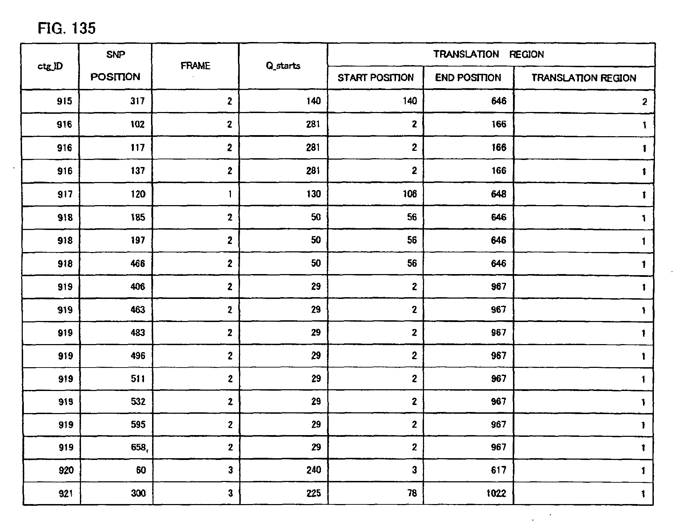

Then, in the amino acid sequence translated according to the reading frame, it is determined whether the translation region (coding region) contains which of the SNPs that cause synonymous substitution to the amino acid according to the base difference among the different strains, i.e., the SNPs that are indicated by "R" under "PRESENCE/ABSENCE OF SUBSTITUTION" in Fig. 31 through Fig. 84. Specifically, SNPs with "R" were decided to fall outside of the translation region when the stop codon appeared before the start codon in propagating toward the 5' end. The other SNPs are decided to fall within the translation region. With the nonsynonymous substitution SNP identified this way, the presence or absence of a start codon and a stop codon upstream and downstream of the SNPs was further determined to identify translation regions in the CONTIGS (388) through (921). The results are shown in Fig. 98 through 135.

In Fig. 98 through Fig. 135, the headings "ctg_ID" and "SNP POSITION" are the same as those used in Fig. 1 through Fig. 84. The column under "FRAME" indicates reading frames for the CONTIGS (388) through (921) determined based on the result of homology search described above. The frame is "+" ("+" is omitted in the Figures) when the CONTIG sequence is the sense strand, and is "-" when the sense strand is the complementary sequence of the CONTIG sequence. The notations "+1", "+2", and "+3" indicate that the reading frame is set in the order of the first, second, and third bases from the 5' end of the CONTIG sequence. The notations "-1", "-2", and "-3" indicate that the reading frame is set in the order of the first, second, and third bases from the 5' end of the complementary sequence of the CONTIG sequence.

In Fig. 98 through Fig. 135, the column under "Q_start" indicates the start position of a region of a base sequence of the sense strand (CONTIG sequence when the frame is "+", or the complementary sequence of the CONTIG sequence when the frame is "-") which was determined to be homologous to a known sequence as a result of homology search (position closest to the 5' end in the homologous region). Thus, when the frame is "-", the start position is the position closest to the 5' end of the complementary sequence in a region which was determined to be a homologous region. However, in the Figures, the column under "Q_start" indicates the start position on the complementary sequence relative to the 3' end of the complementary sequence.

In Fig. 98 through Fig. 135, the column under "TRANSLATION REGION" for the CONTIGS (388) through (921) indicates translation regions. The translation region is defined as a region that contains a nonsynonymous substitution SNP between a start codon and a stop codon, and is determined based on whether the SNP that was found to be a nonsynonymous substitution SNP is on which position of the sense strand, and the reading frame (frame) determined in the described manner. For the CONTIGS (388) through (921), Fig. 98 through Fig. 135 show the "START POSITION" and "STOP POSITION" of the translation region determined as above. However, when the start codon does not appear upstream of the position of nonsynonymous substitution SNP toward the 5' end, the start position of a translation region is determined to be the first base on the 5' end of the sense strand when the reading frame is "+1" and "-1", the second base on the 5' end of the sense strand when the reading frame is "+2" and "-2", and the third base on the 5' end of the sense strand when the reading frame is "+3" and "-3". On the other hand, when the stop codon does not appear downstream of the position of nonsynonymous substitution SNP toward the 3' end, the stop position of a translation region is determined to be a position that provides the longest translation region according to the reading frame determined in the described manner. The translation region so determined does not necessarily encode the entire length of a protein.

When the frame of the CONTIG is "-", the "START POSITION" and "STOP POSITION" in Fig. 98 through Fig. 135 indicate the start position or stop position from the 5' end of the complementary sequence.

For the CONTIGS (395), (397), (488), and (733), the start position of a translation region under "Q_start" was obtained from a comprehensive comparison study by finding homology with a known sequence (in other words, the start position of a region that was determined to be homologous to a known sequence).

In Fig. 98 through Fig. 135, the column under "TRANSLATION REGION NUMBER" contains numbers sequentially assigned to the translation regions when the CONTIG contains more than one region that is determined as a translation region in the described manner. (When there is only one translation region, "1" is assigned to the translation region.)

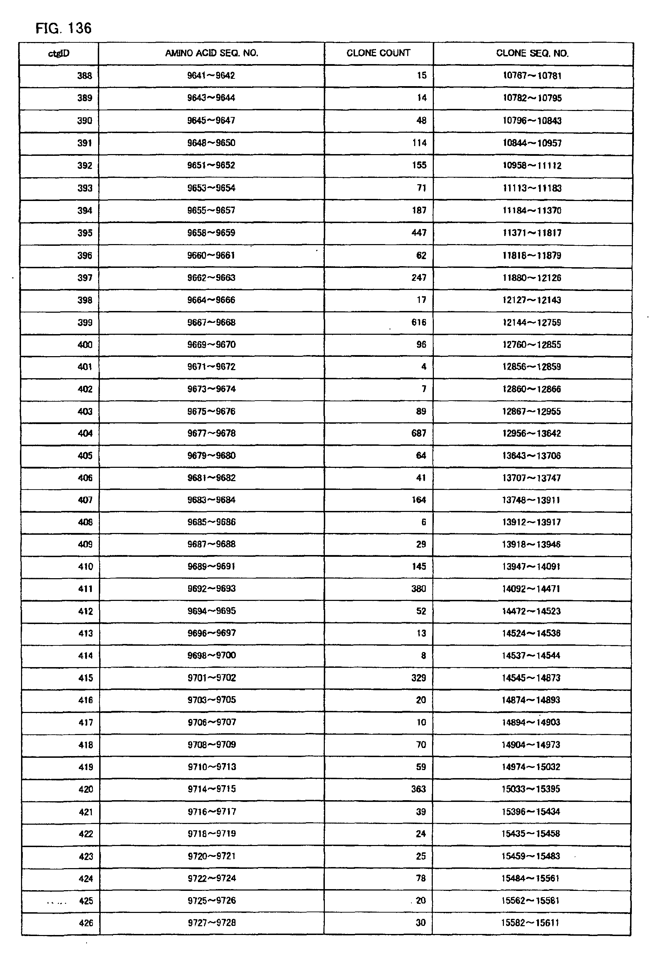

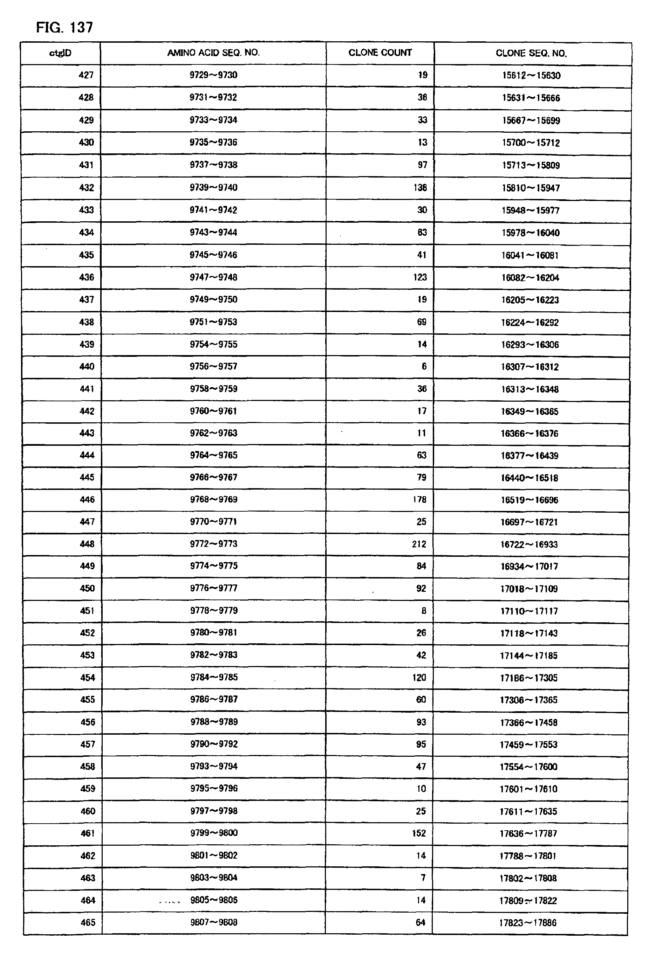

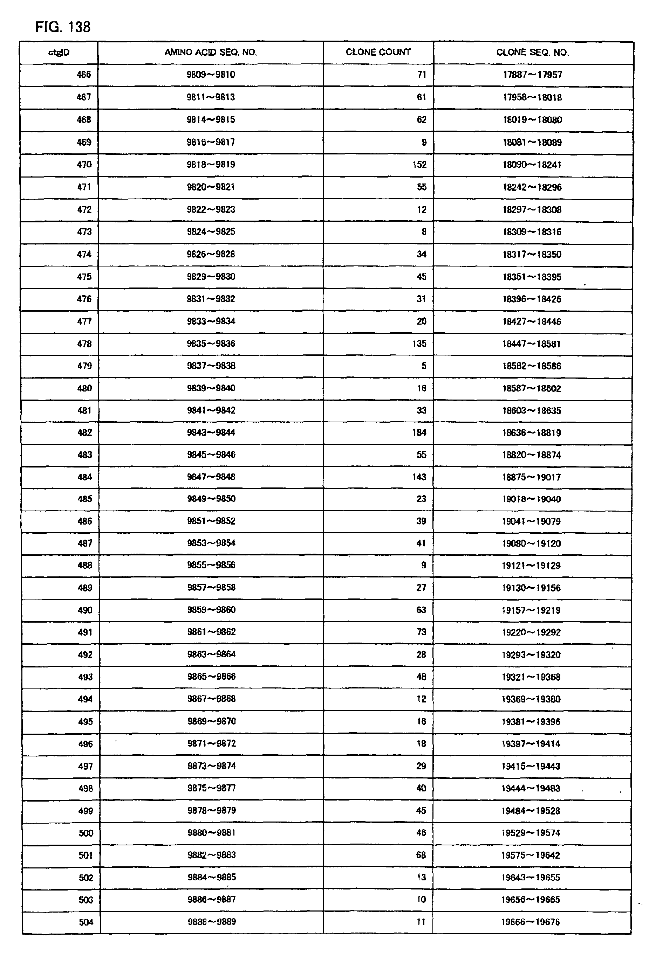

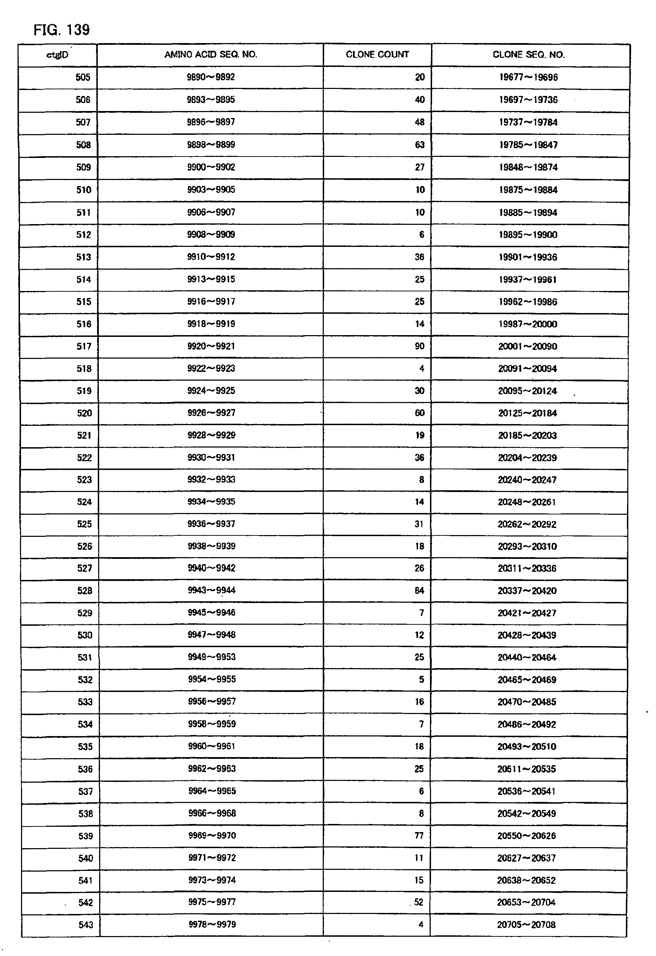

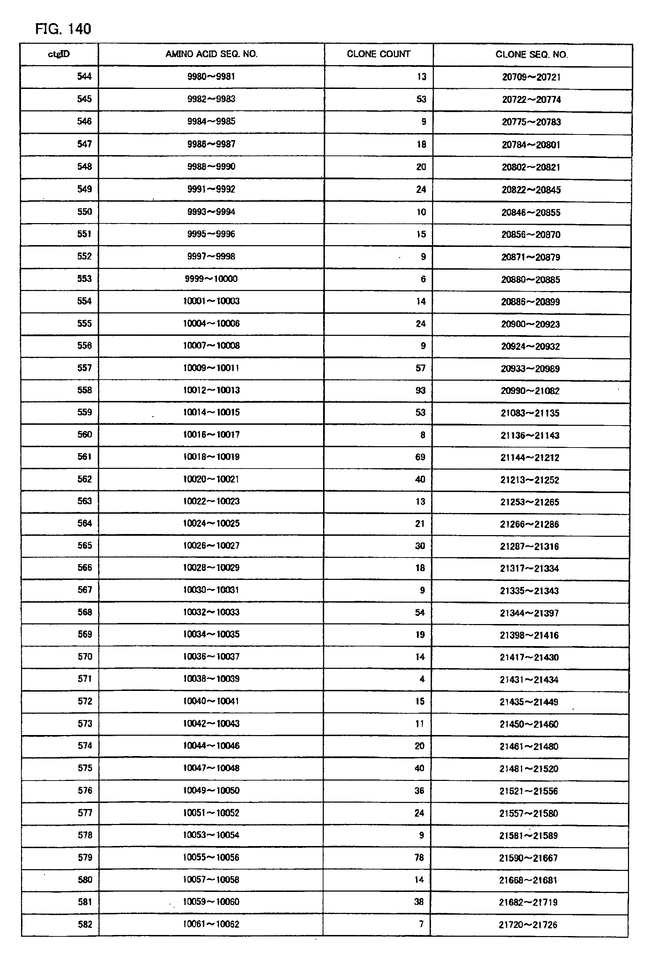

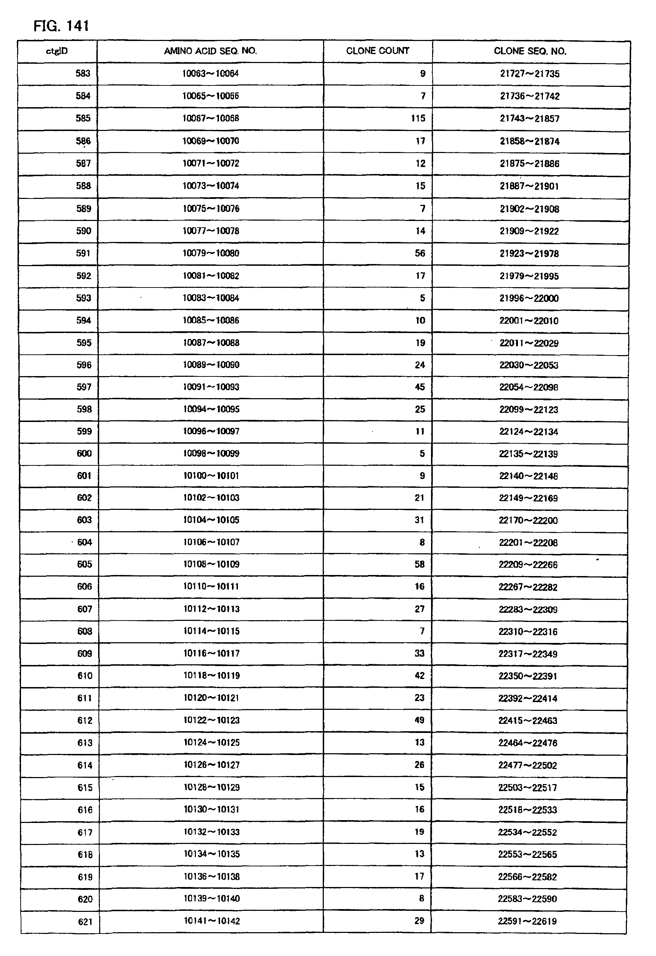

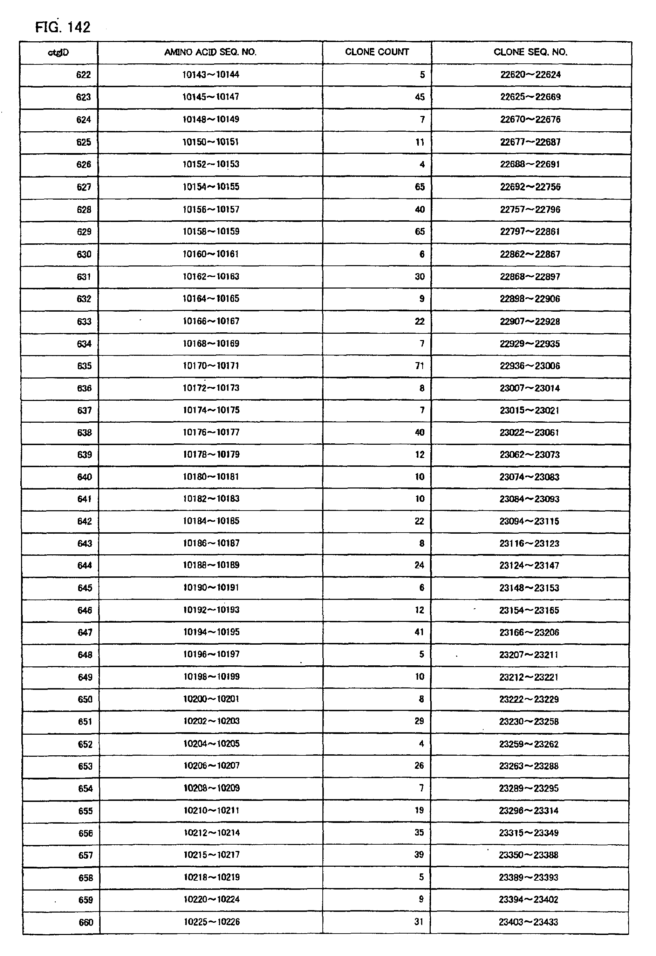

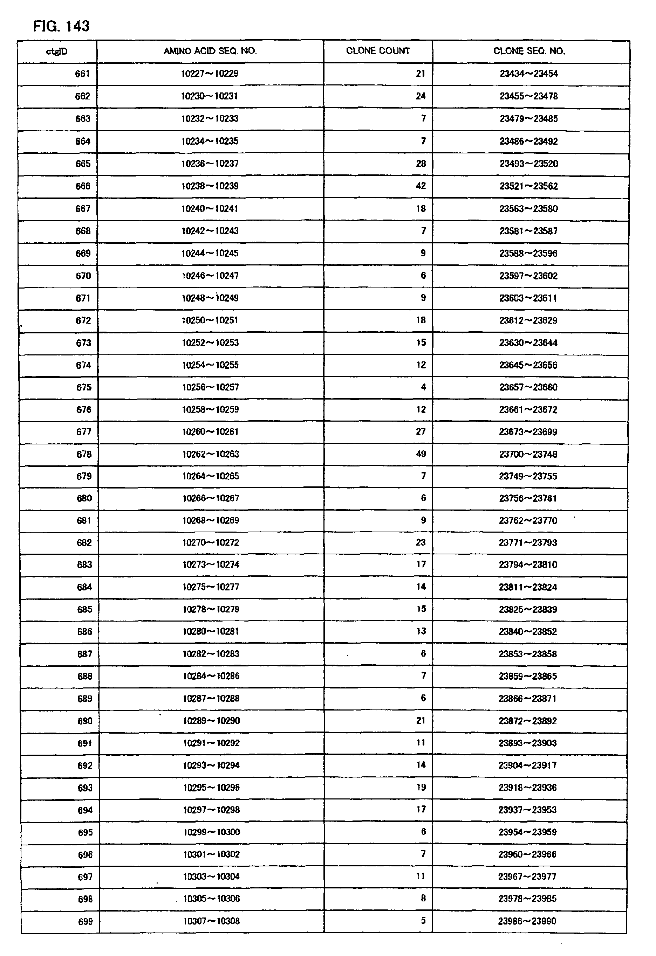

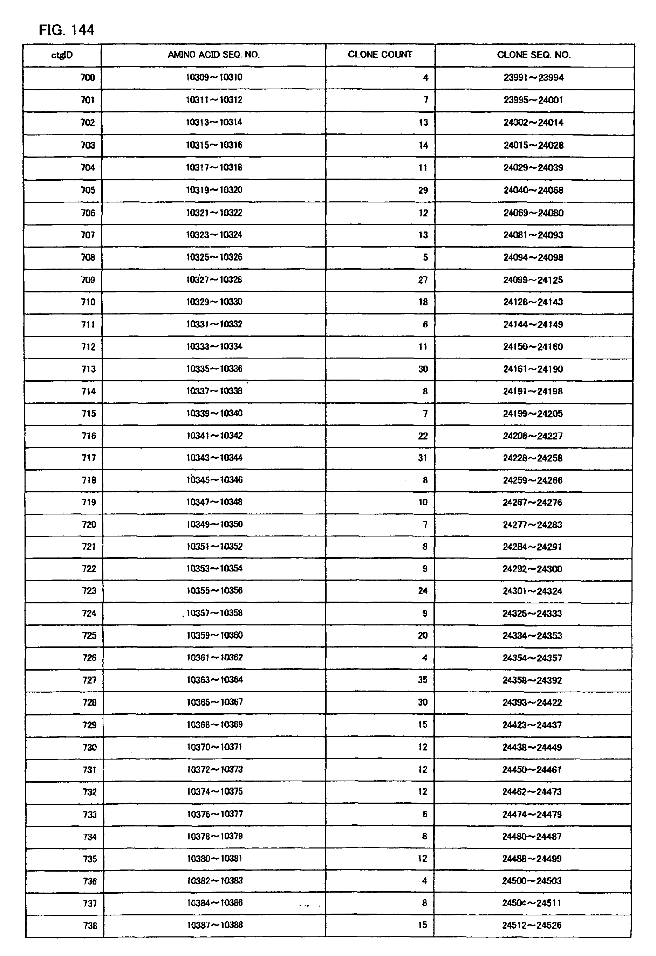

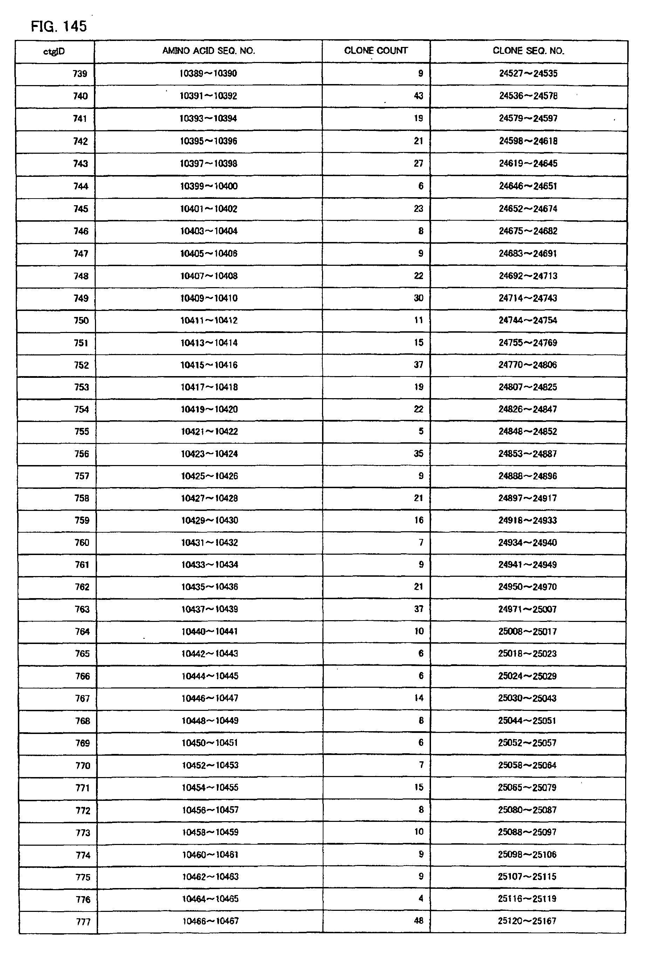

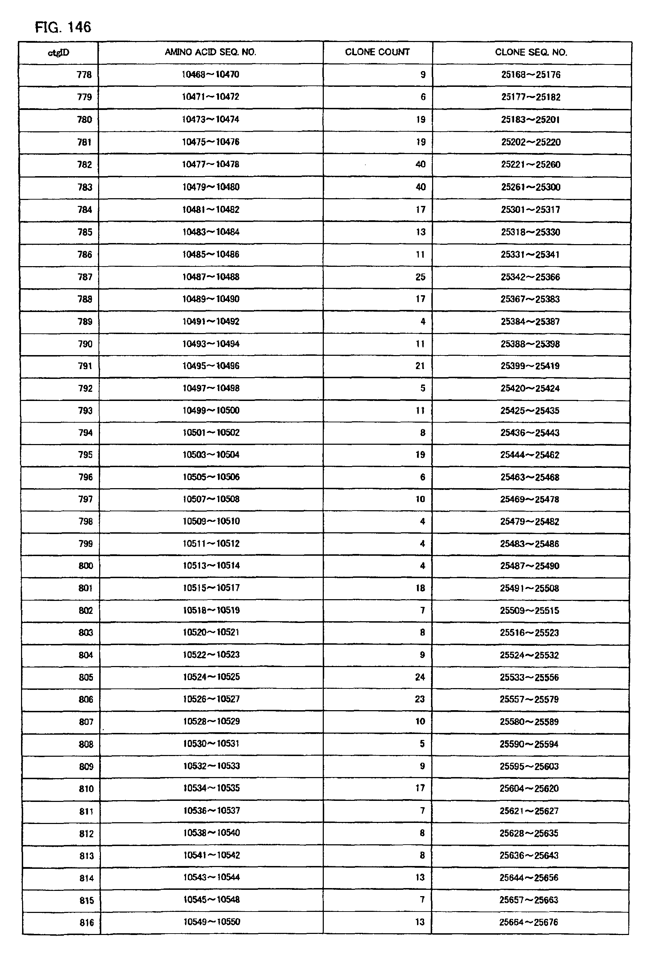

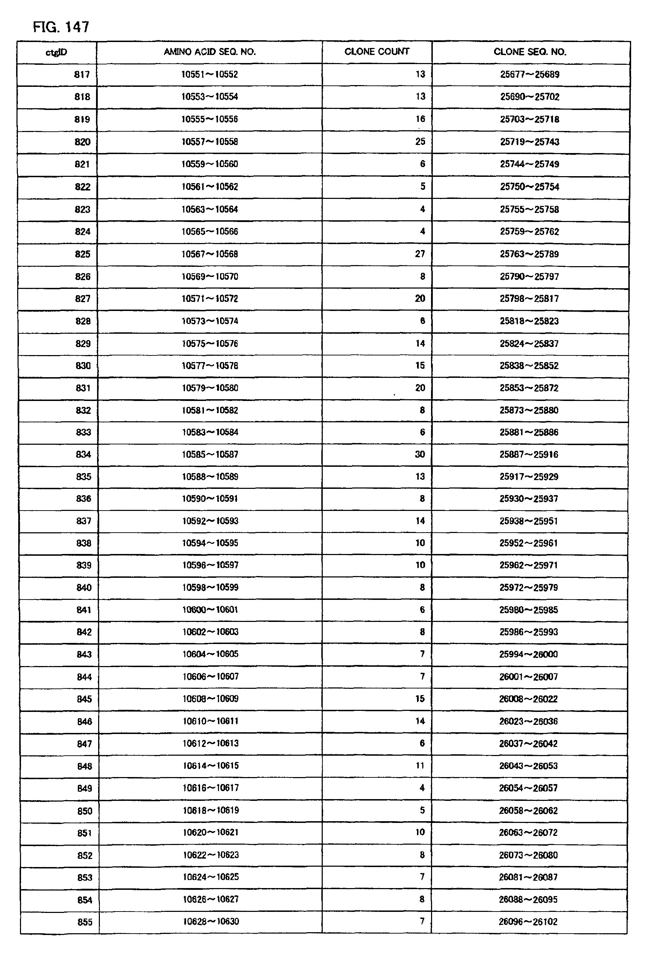

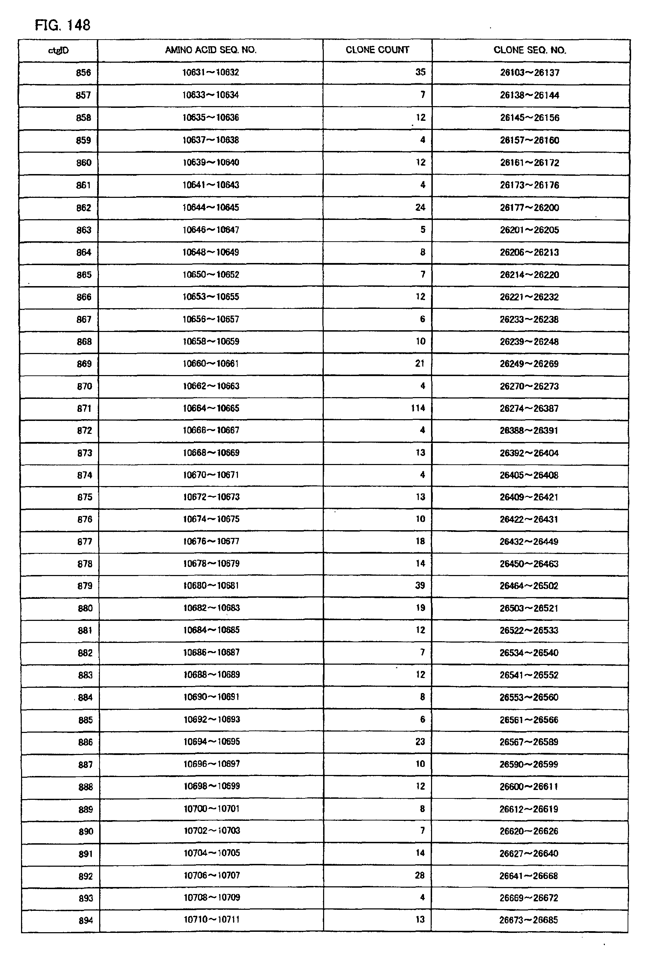

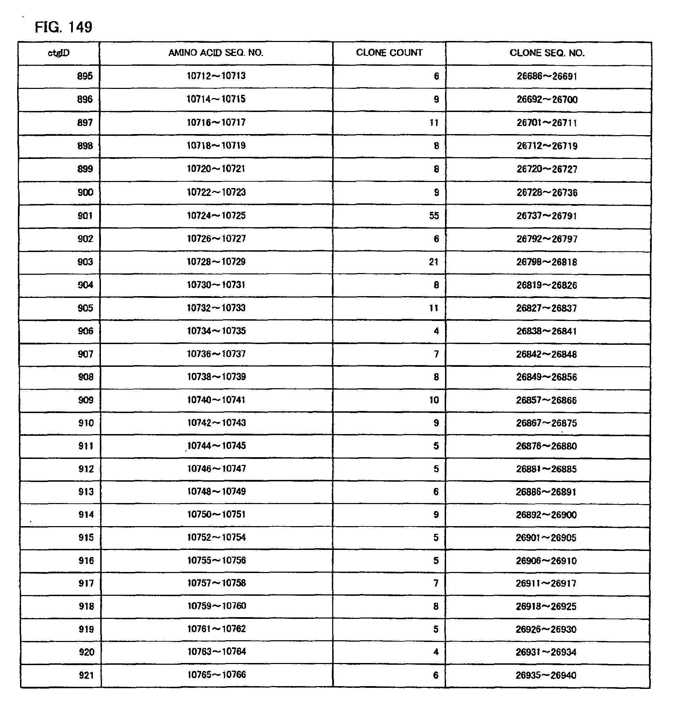

The sequence table shows the amino acid sequences encoded by regions determined as the translation regions of the CONTIGS (388) through (921) in the described manner. Fig. 136 through Fig. 149 show, referring to the sequence table, sequence numbers of the amino acid sequences encoded by the CONTIGS (388) through (921).

In Fig. 136 through Fig. 149, the heading "ctg_ID" is the same as that used in Fig. 1 through Fig. 84. The column under "AMINO ACID SEQUENCE" indicates, referring to the sequence table, sequence numbers of amino acid sequences encoded by the translation regions of the CONTIGS (388) through (921). Note that, for a given amino acid sequence, the sequence table shows both the base sequence and amino acid sequence of the translation region (coding region), or the amino acid sequence alone. The amino acid sequences shown in the sequence table were determined by selecting a base for the polymorphic site or its complementary site, indicated by a universal code, according to the following criteria (a) through (c).
(a) The bases of Haruna, wild-type, or Akashinriki, in which all the SNP bases in a translation region have been determined are selected as the SNP bases. In the event where more than one strain exists in which all the SNP bases in a translation region have been determined, a strain is selected according to the priority order wild-type > Akashinriki > Haruna.
(b) If, however, the translation region contains an SNP that causes replacement of an amino acid to a STOP (stop codon), the strain with a base that causes STOP is not selected, and the bases of a strain that comes next in the priority order are selected as the SNP bases.
(c) If the translation region contains an SNP that causes replacement of an amino acid to a STOP (stop codon), and when the other strains also contain bases that cause replacement of an amino acid to a STOP (stop codon) in their translation regions, SNP bases are selected according to the priority order from the strains in which all the SNP bases in the translation regions have been determined.

It should be noted here that the amino acid sequences in the sequence table are based on the results of determination concerning translation regions, and they do not necessarily indicate the entire length of proteins.

For CONTIGS (388) through (921), Fig. 136 through Fig. 149 also show "CLONE COUNT" and "CLONE SEQUENCE." The column under "CLONE COUNT" indicates the total number of cDNA clones from which the CONTIGS were prepared and constructed. The column under "CLONE SEQUENCE" indicates, referring to the sequence table, sequence numbers of base sequences of the cDNA clones. For example, CONTIG (388) is a sequence that was obtained by aligning the cDNA clones (multiple alignment) indicated by the sequence numbers 10767 through 10781.

### (C) Results of homology search

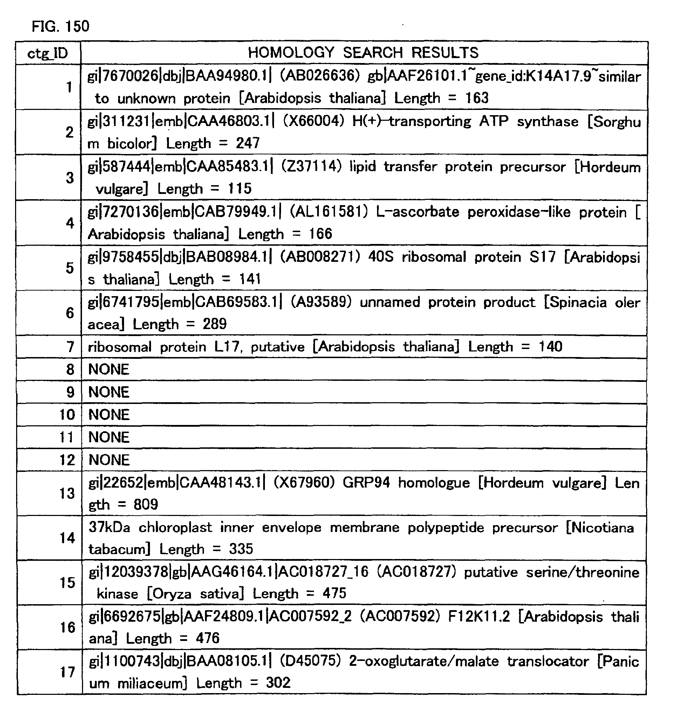

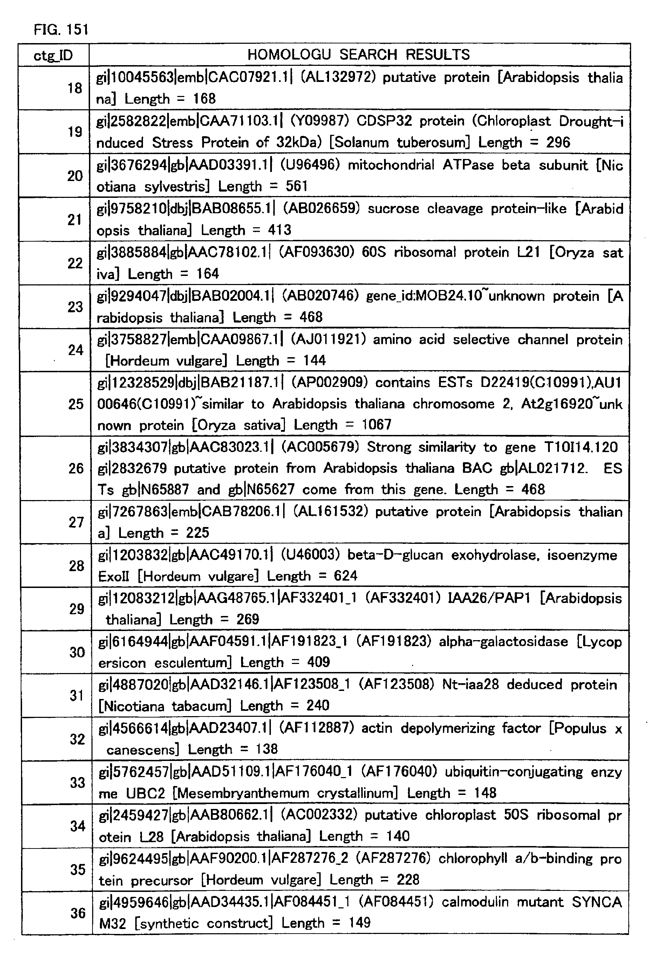

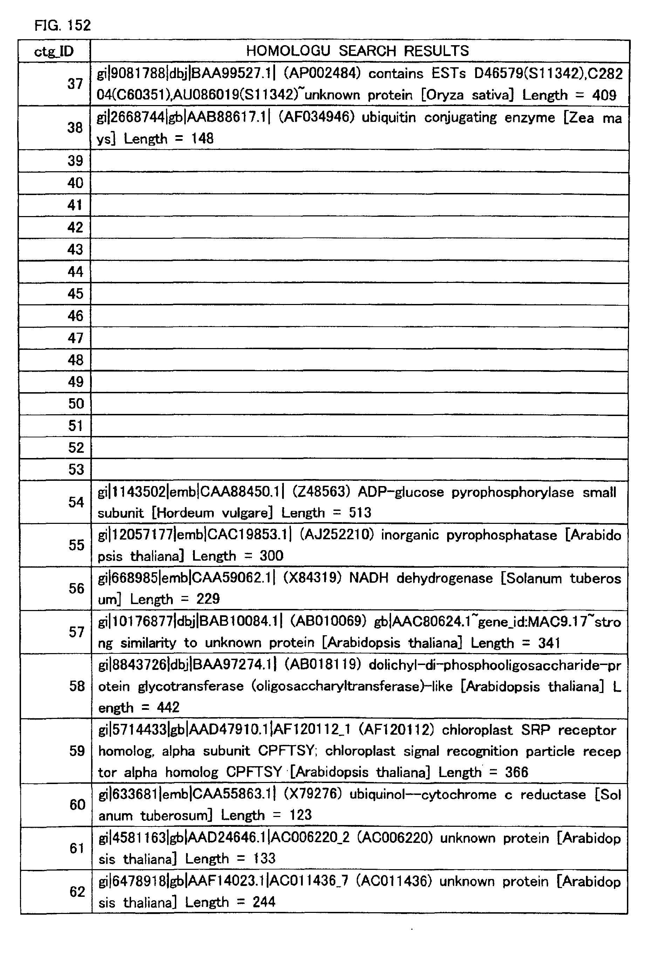

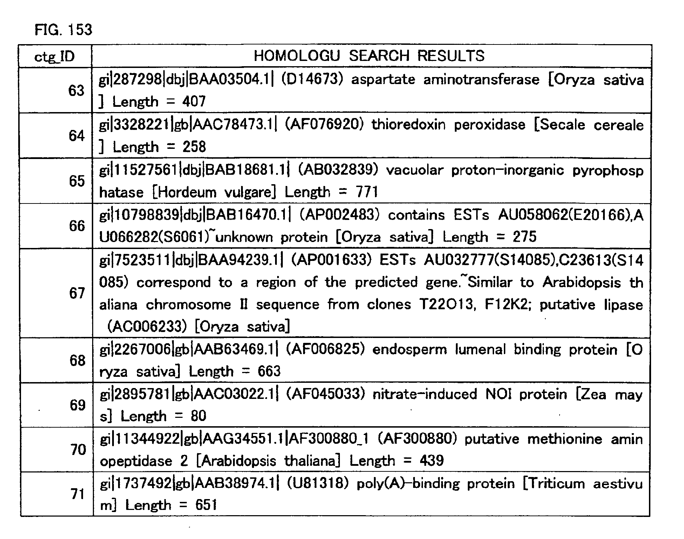

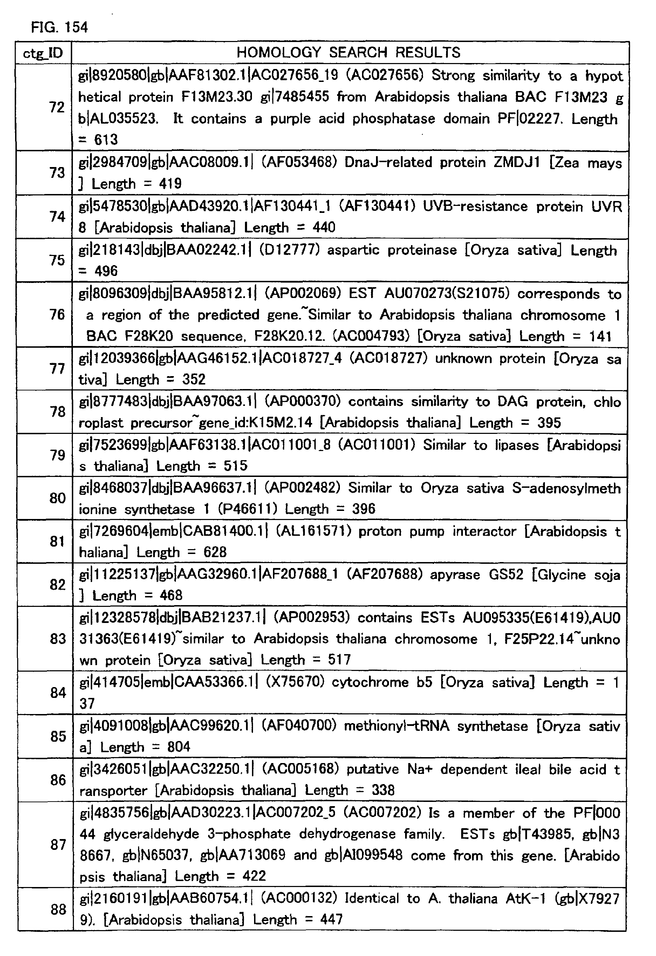

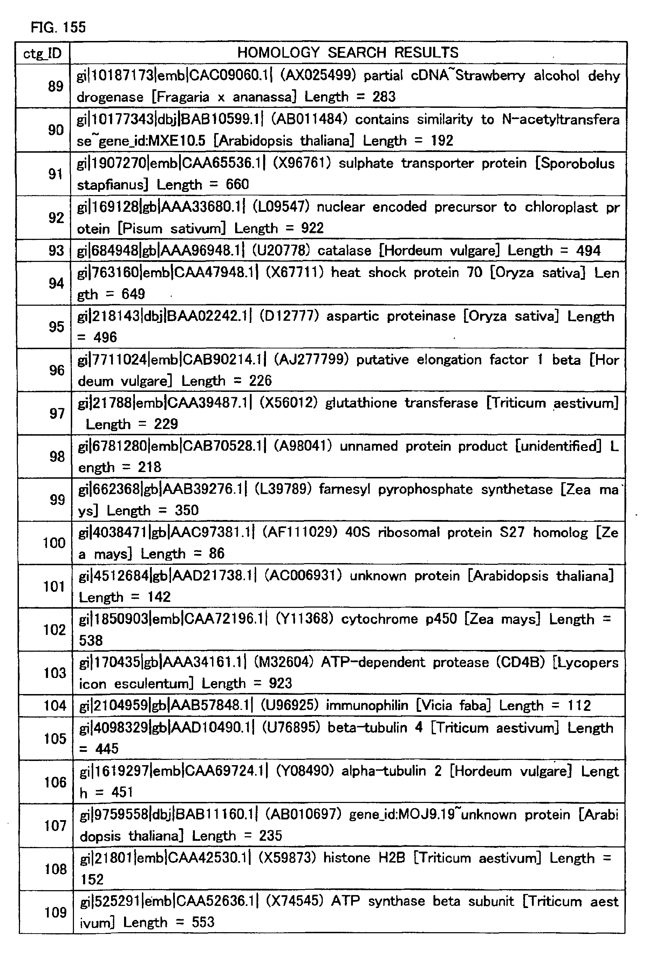

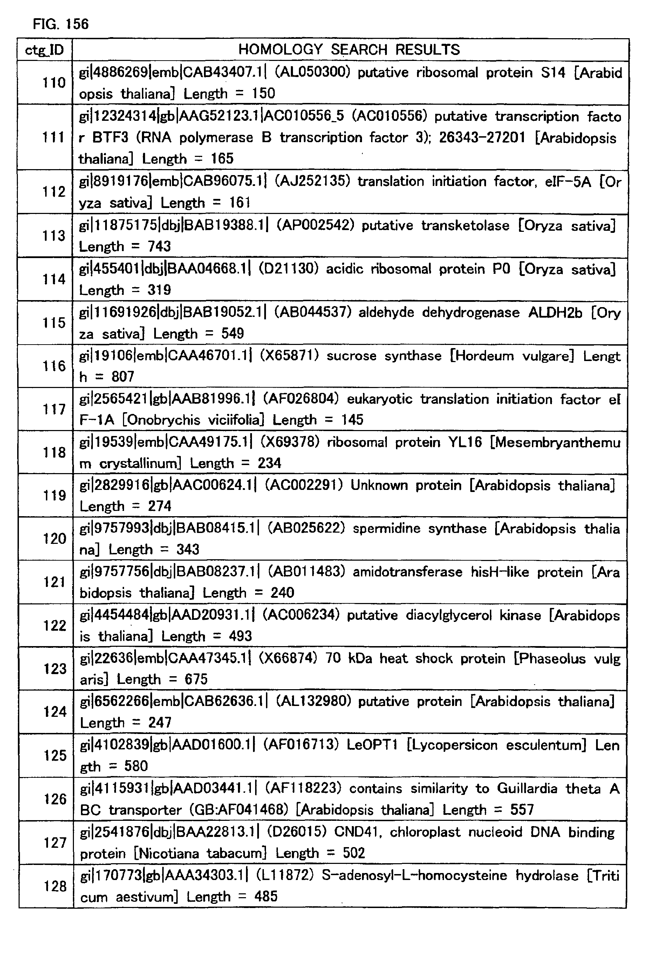

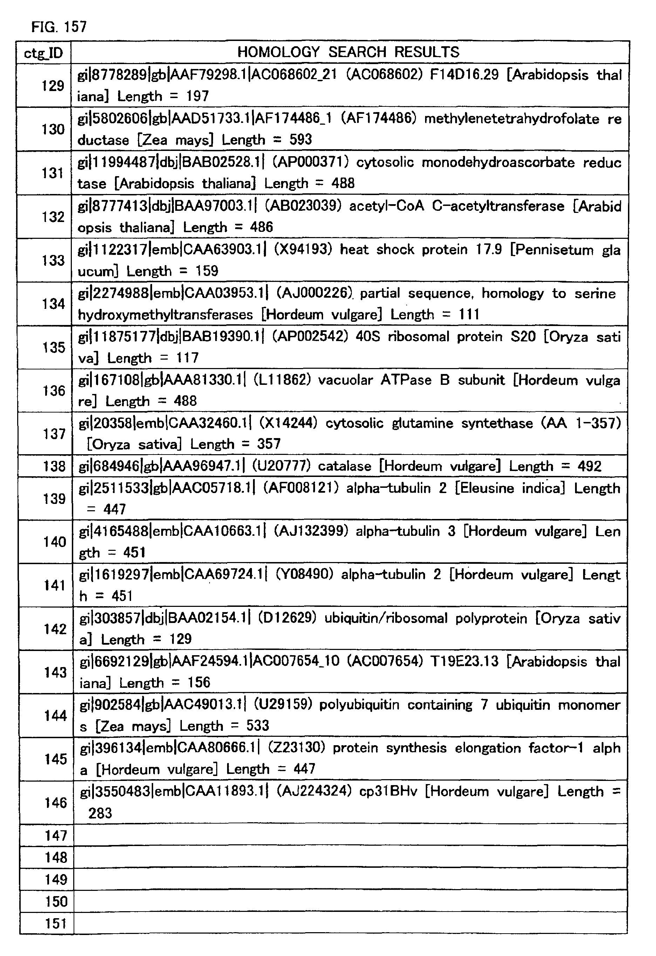

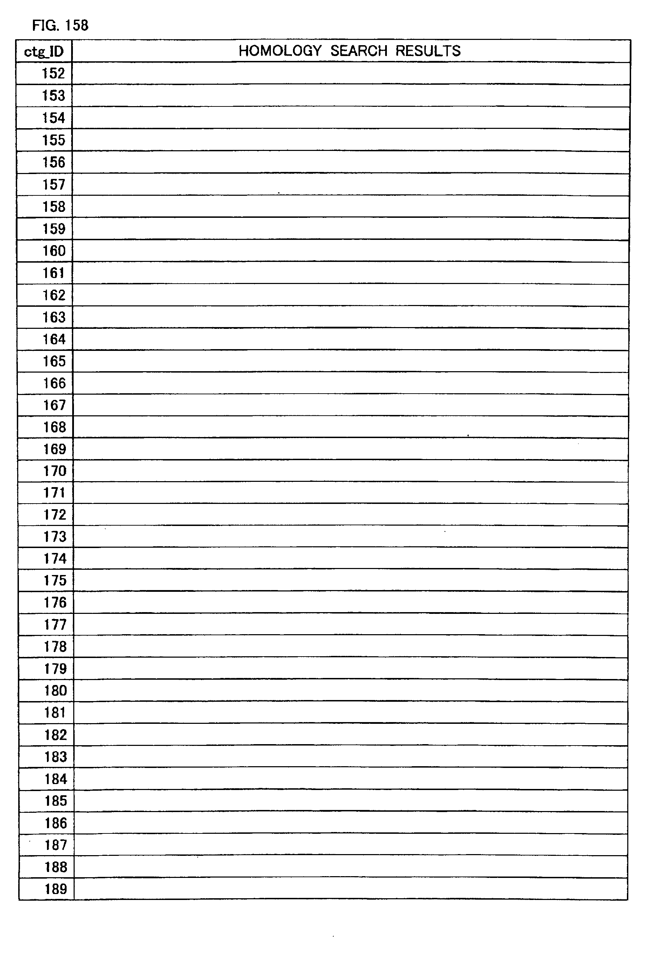

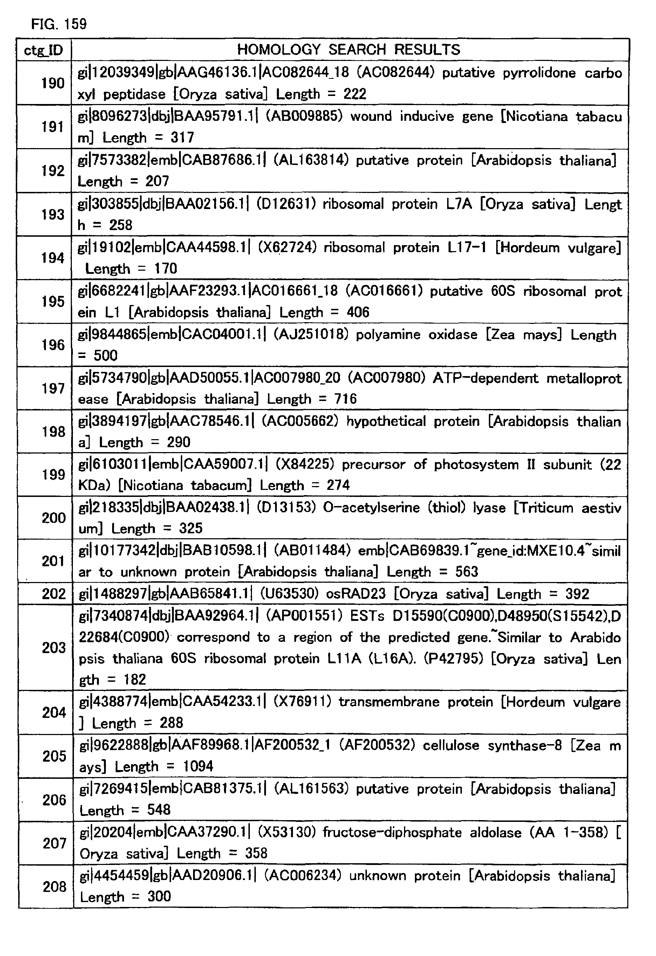

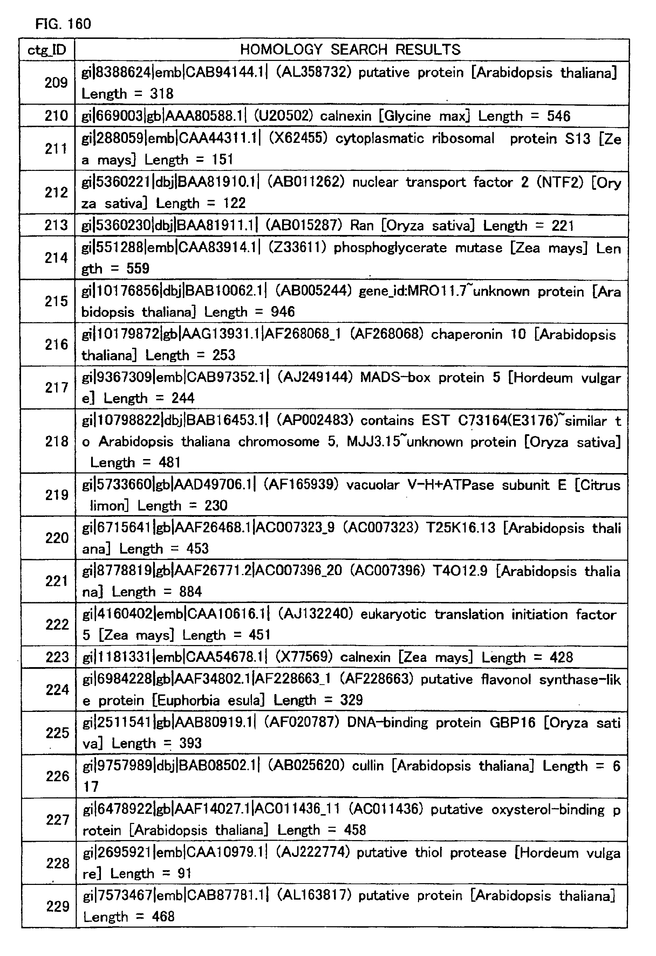

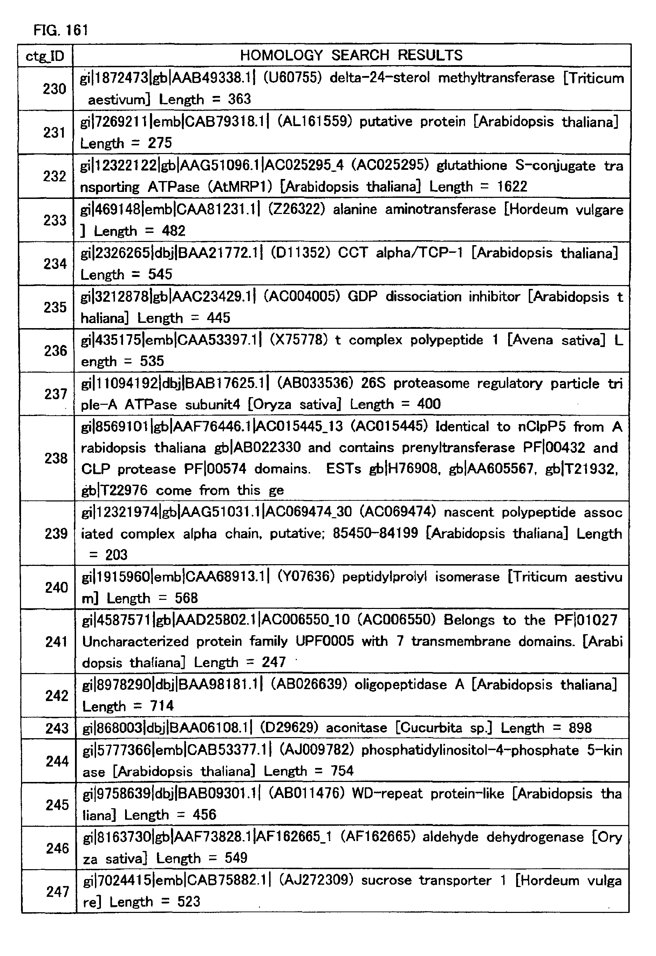

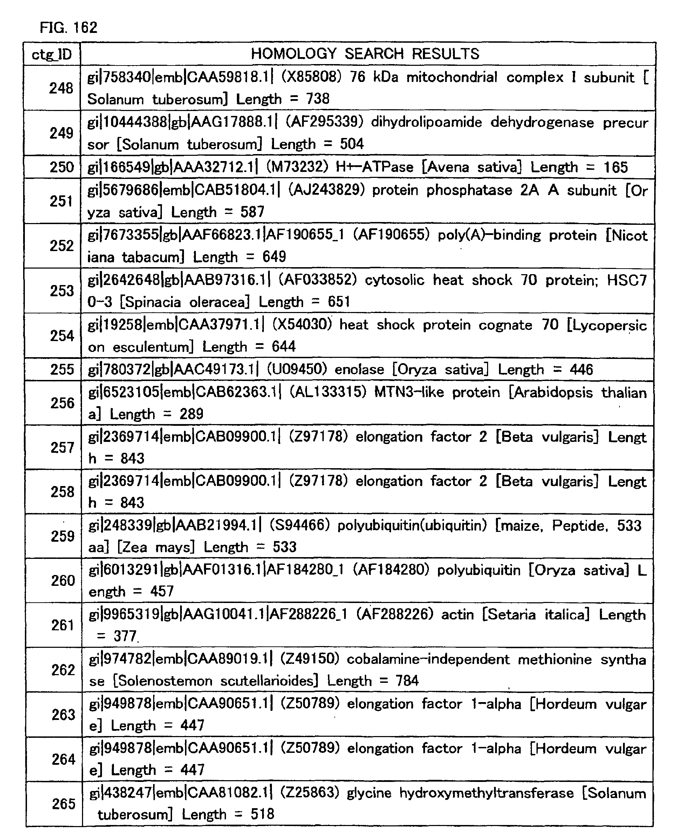

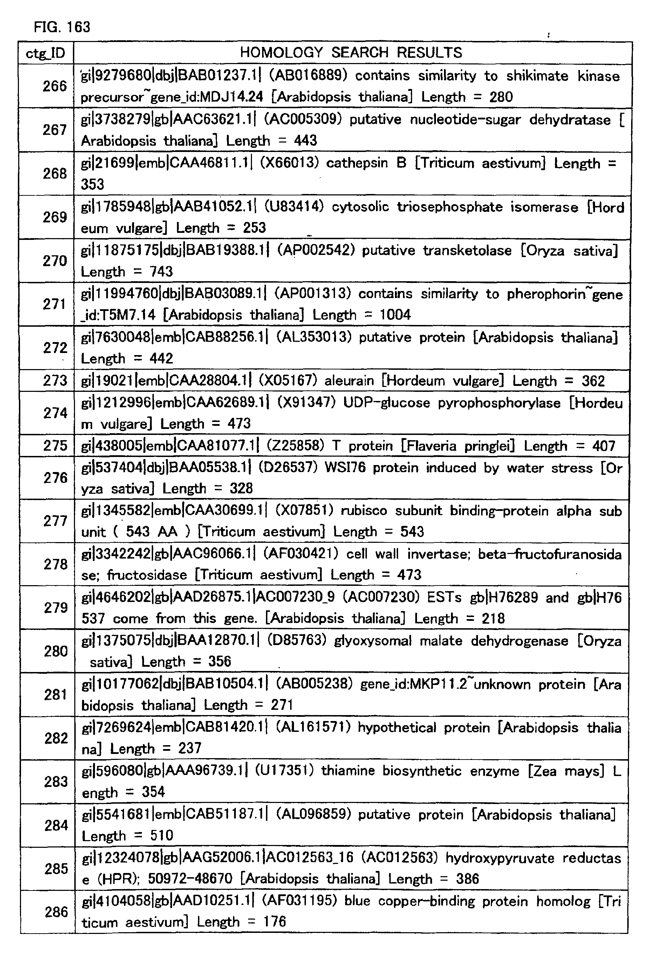

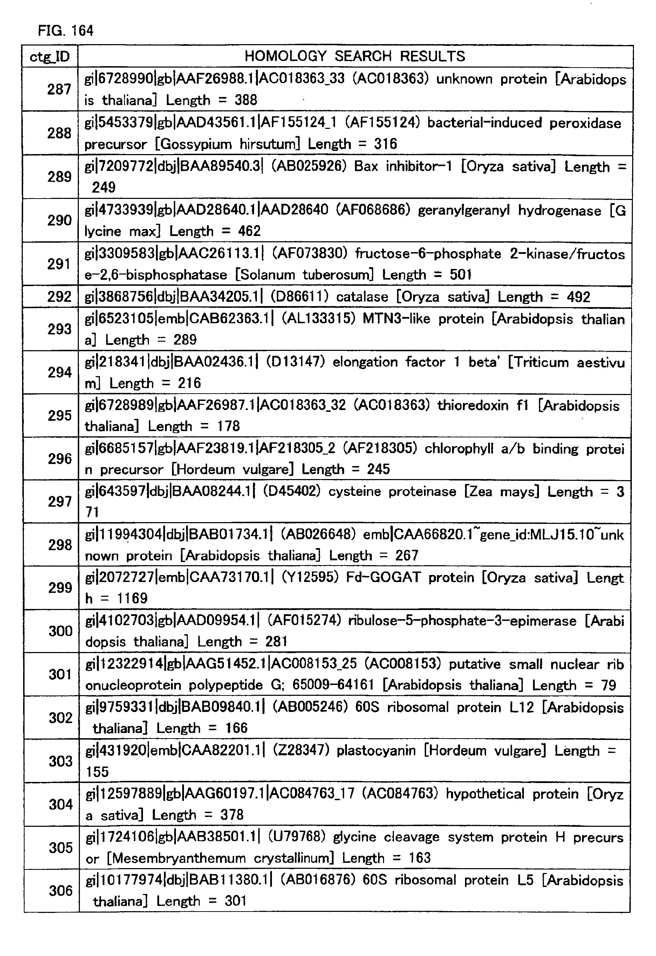

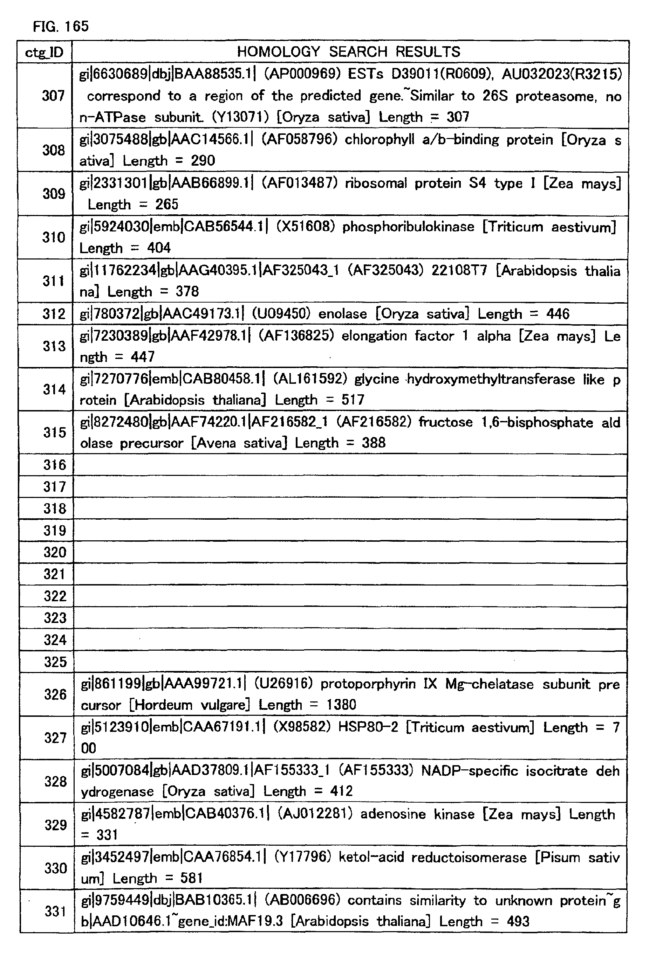

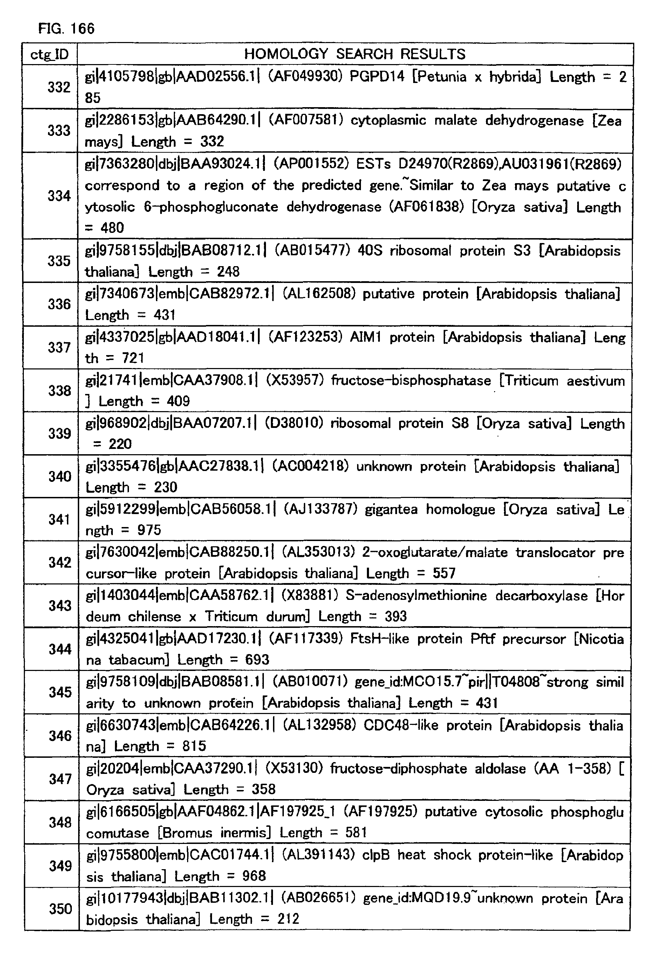

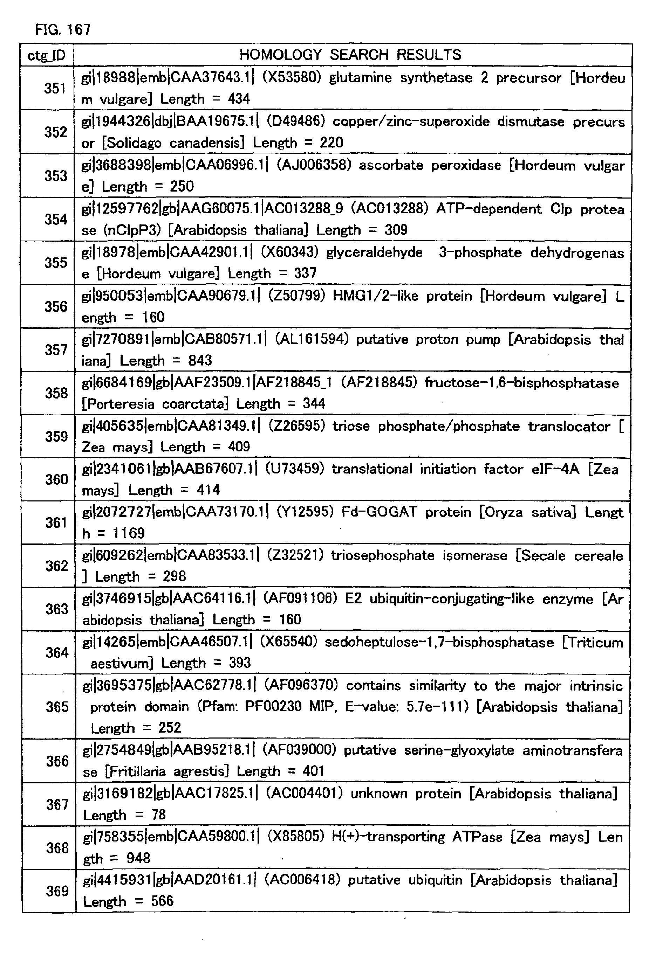

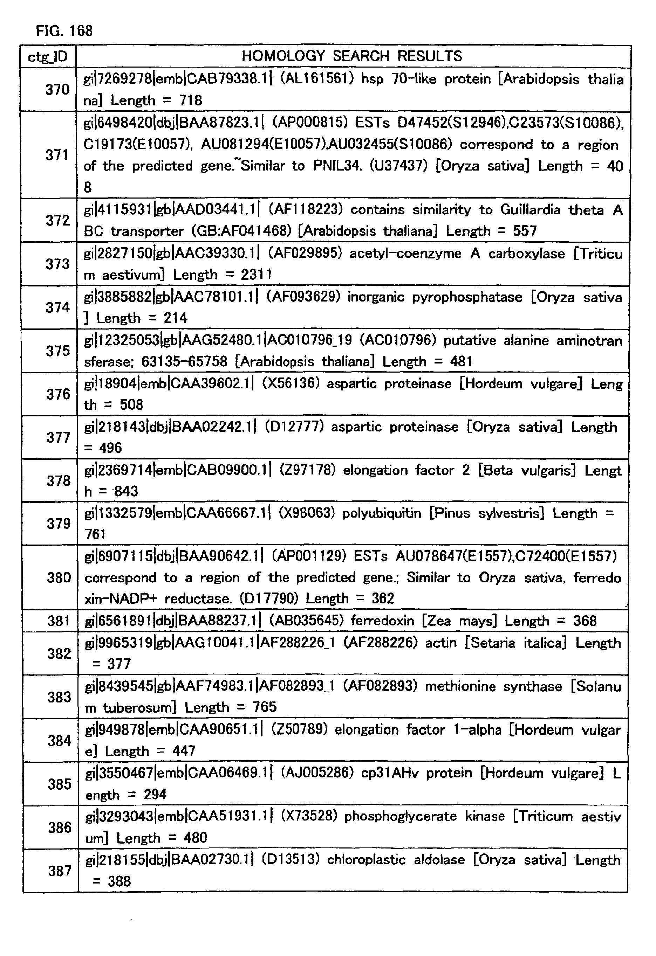

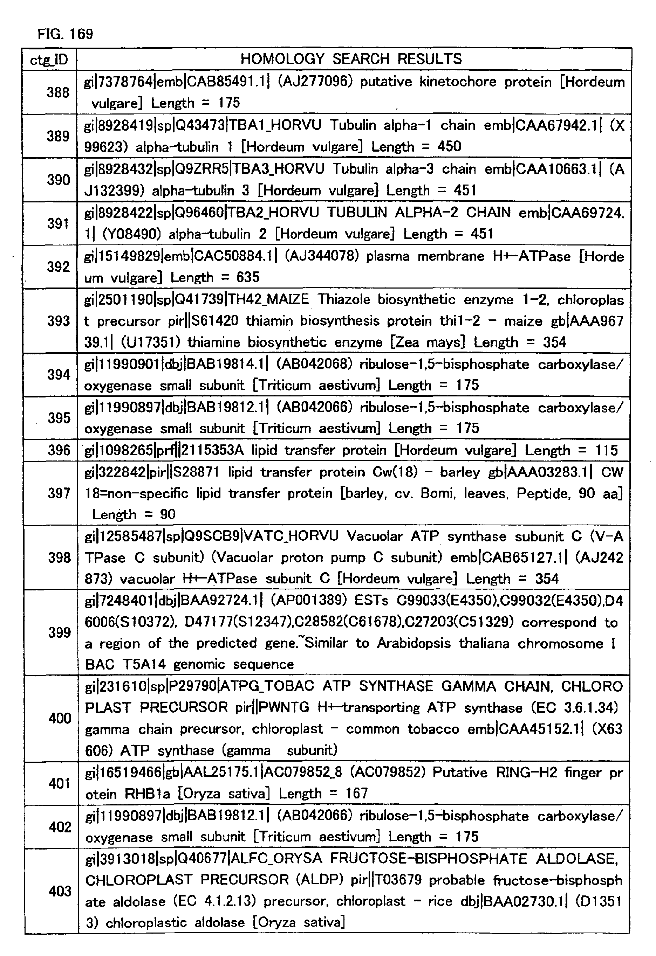

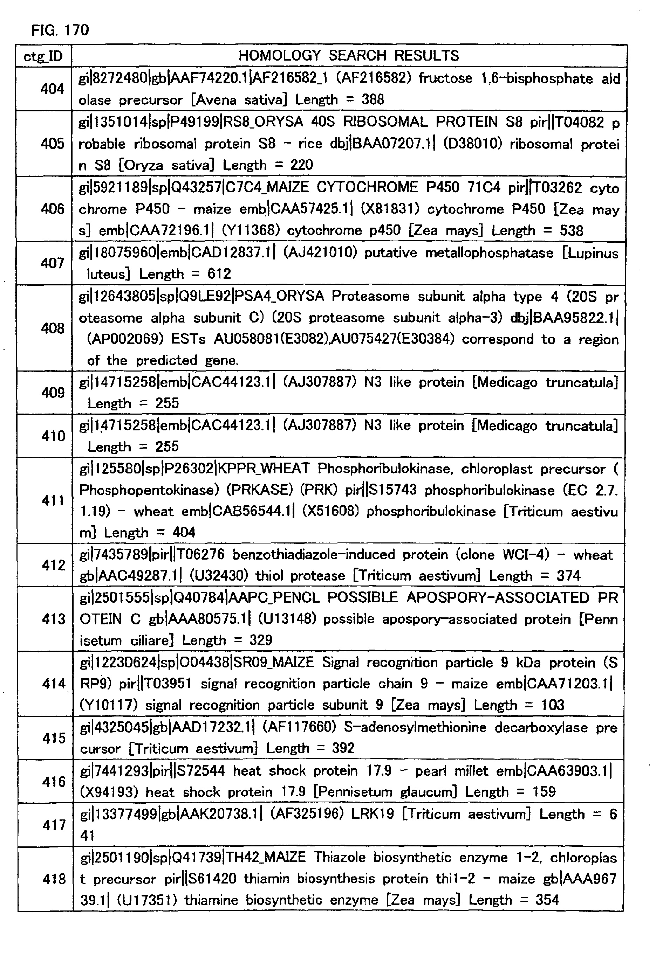

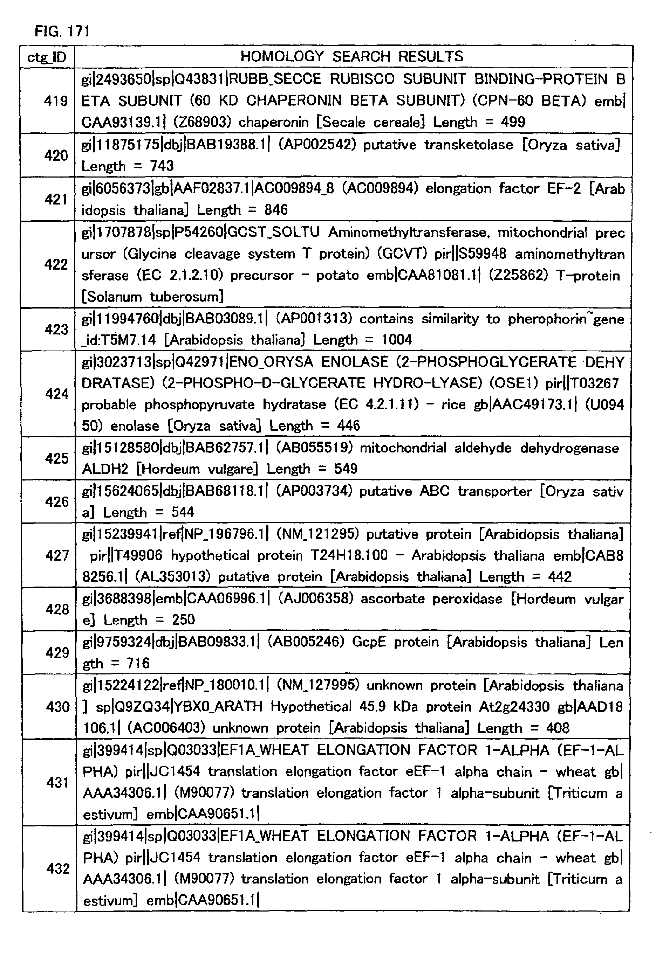

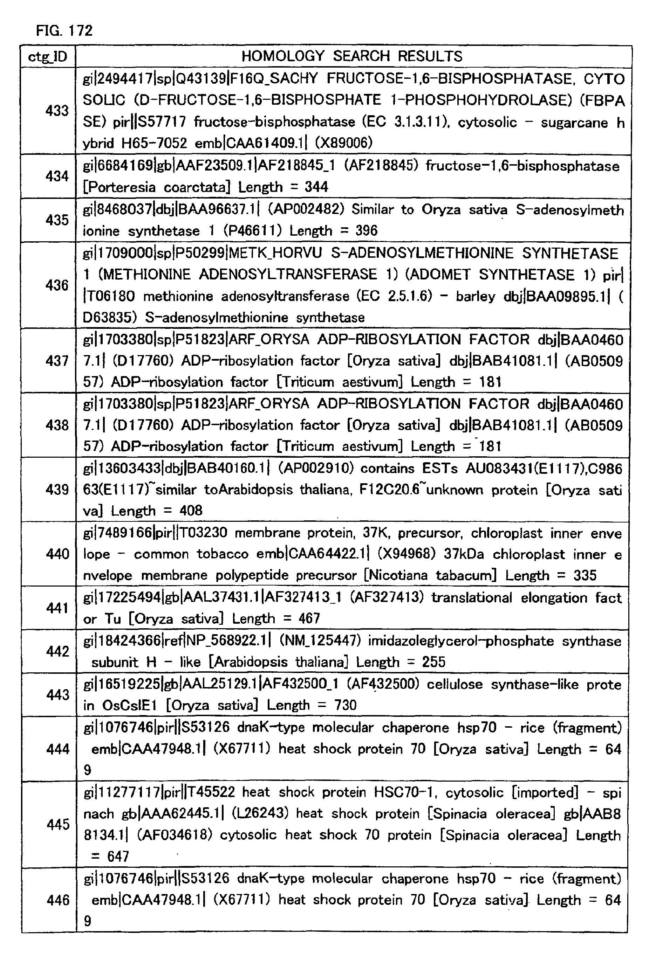

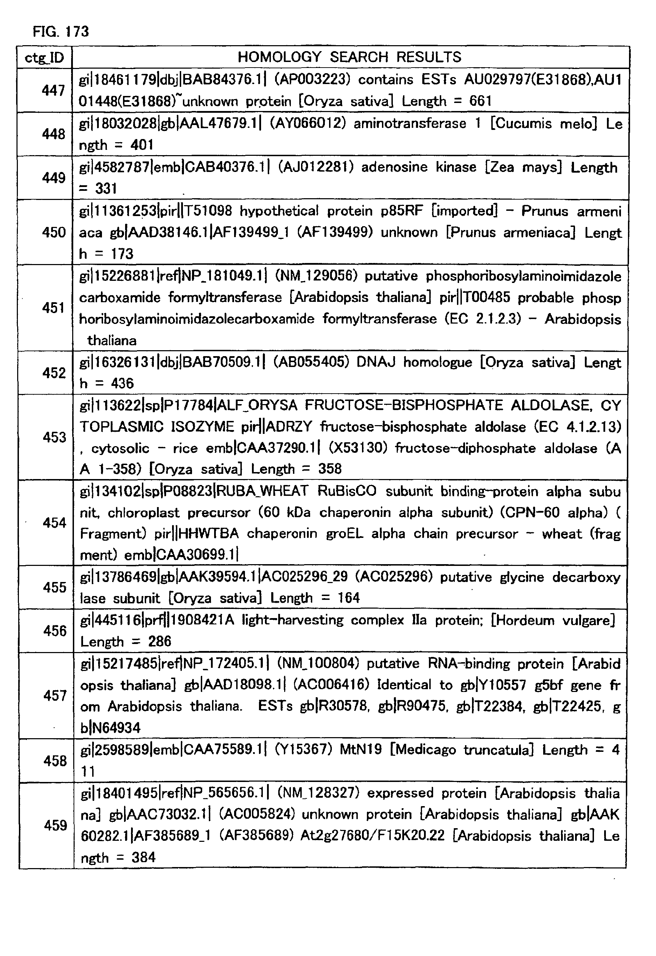

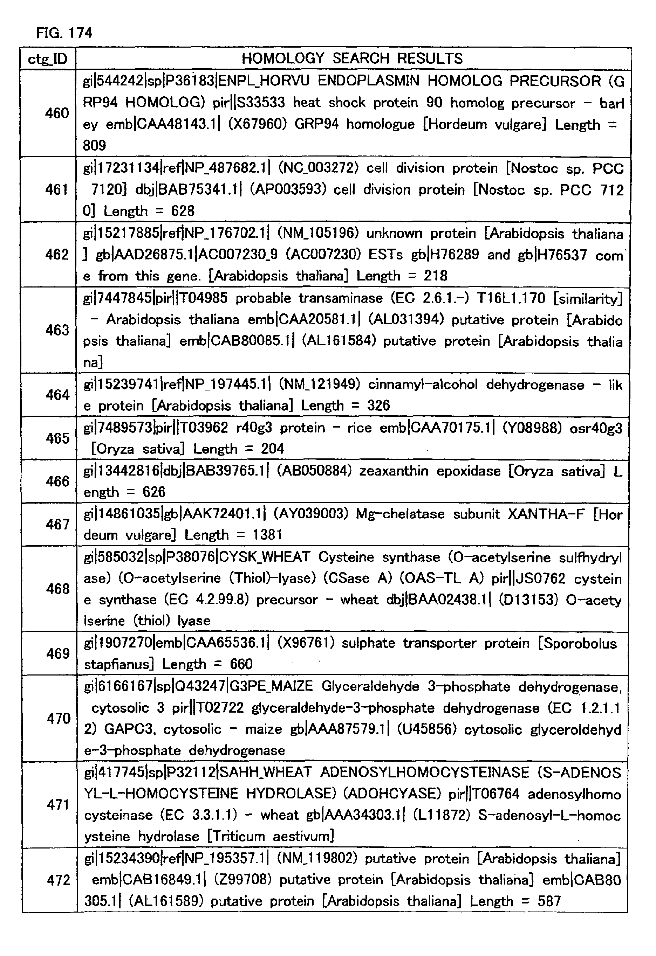

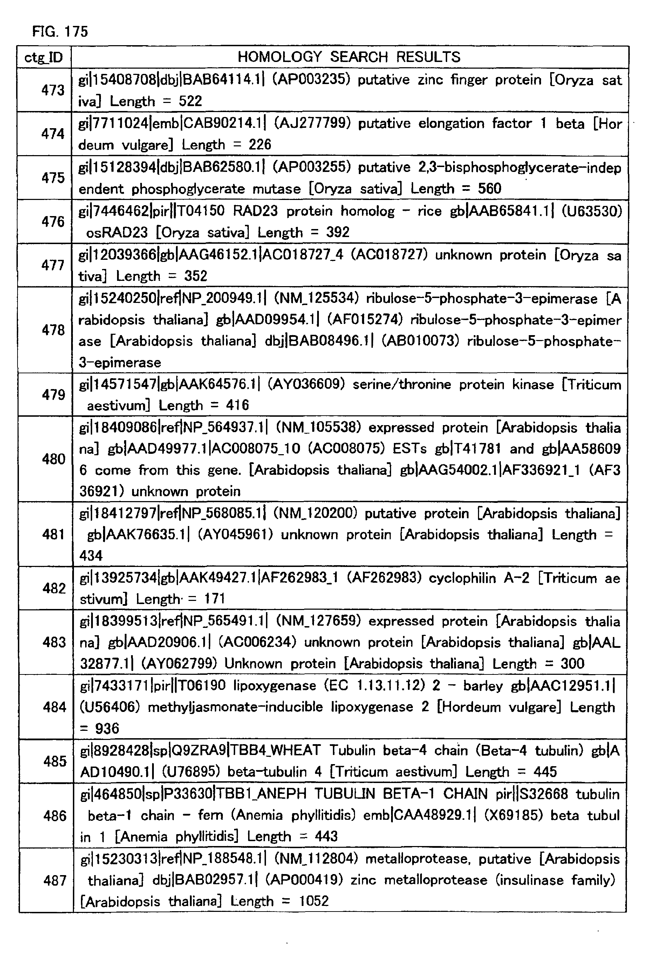

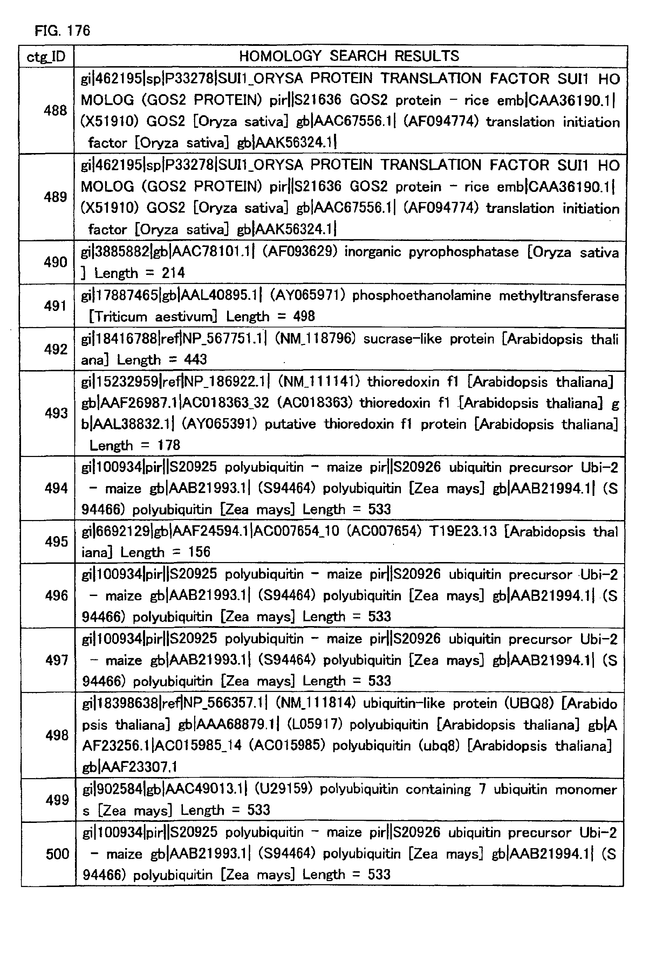

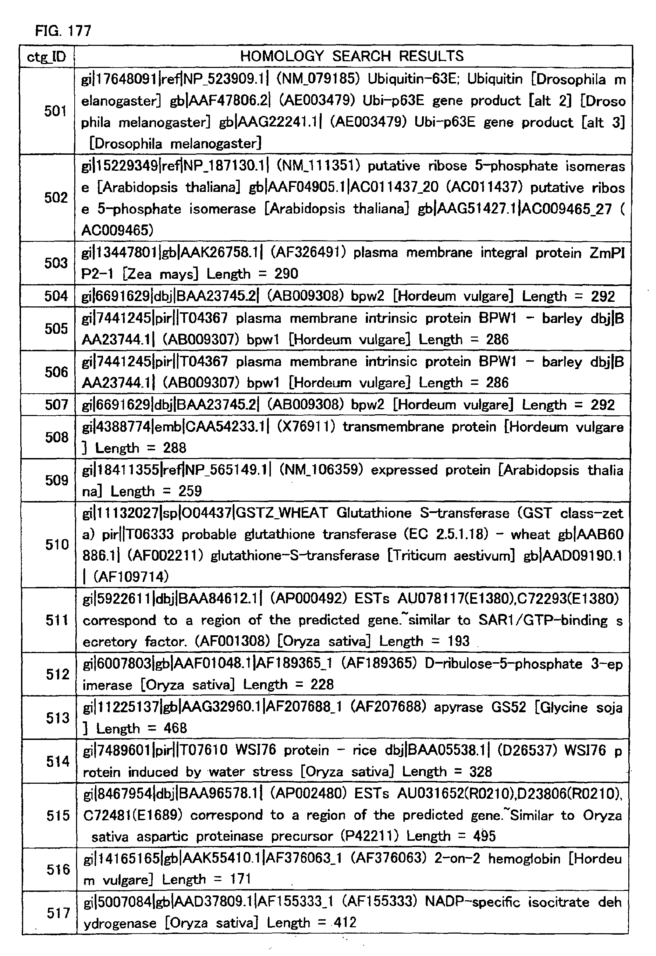

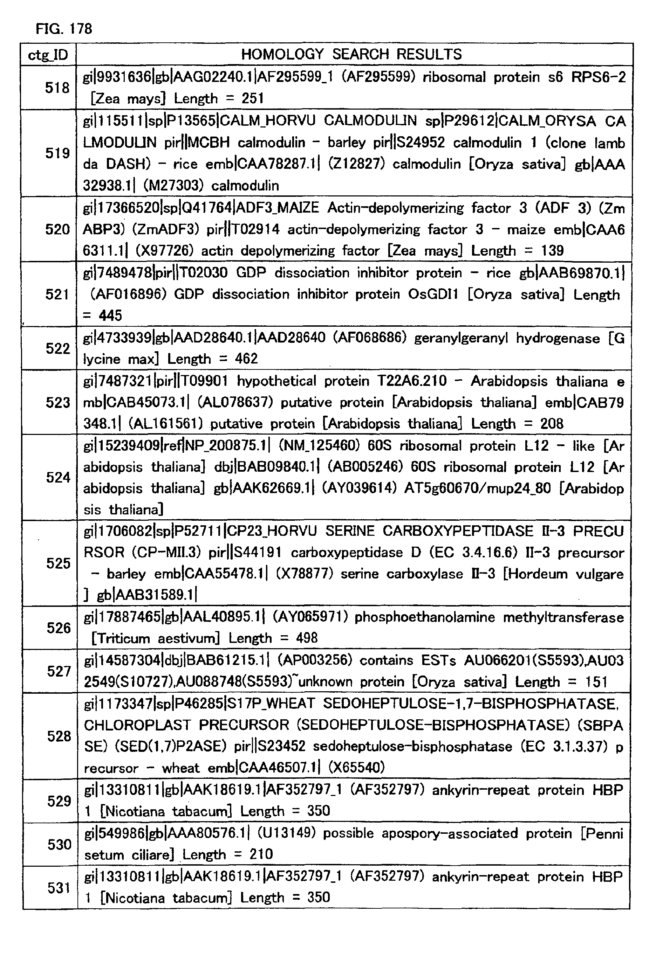

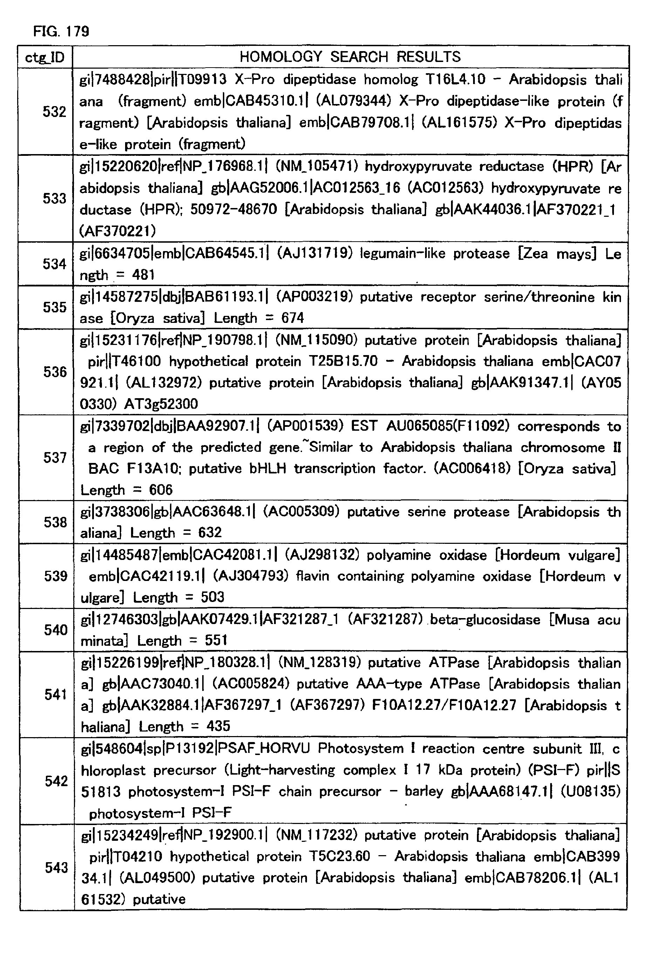

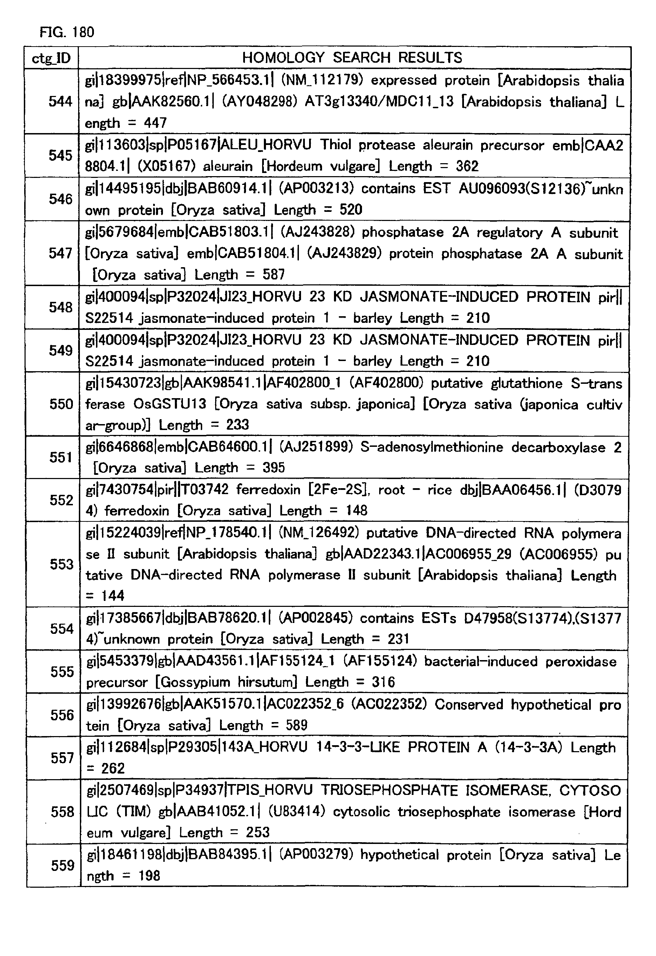

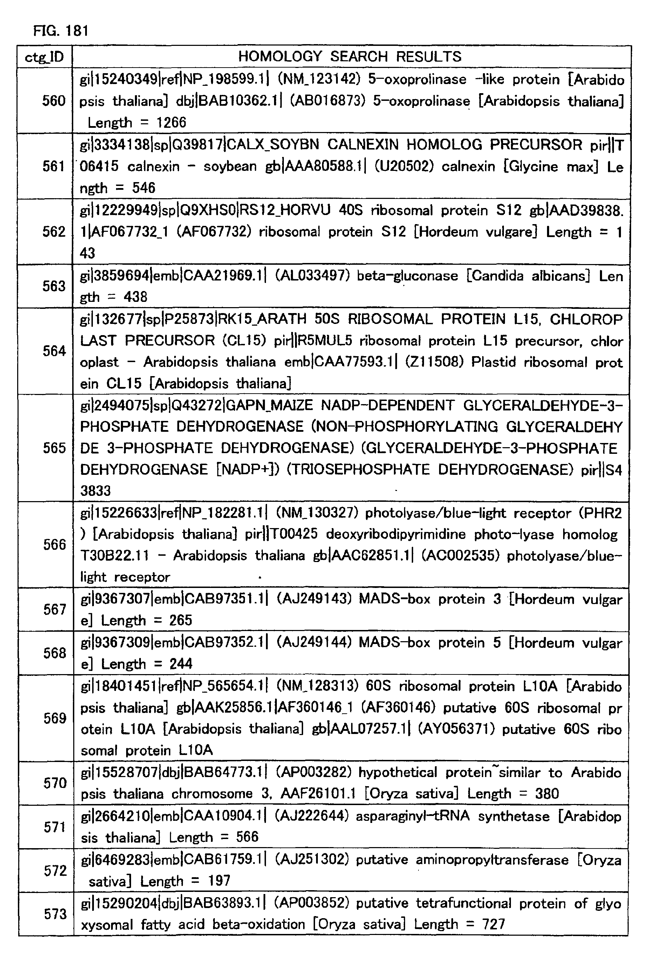

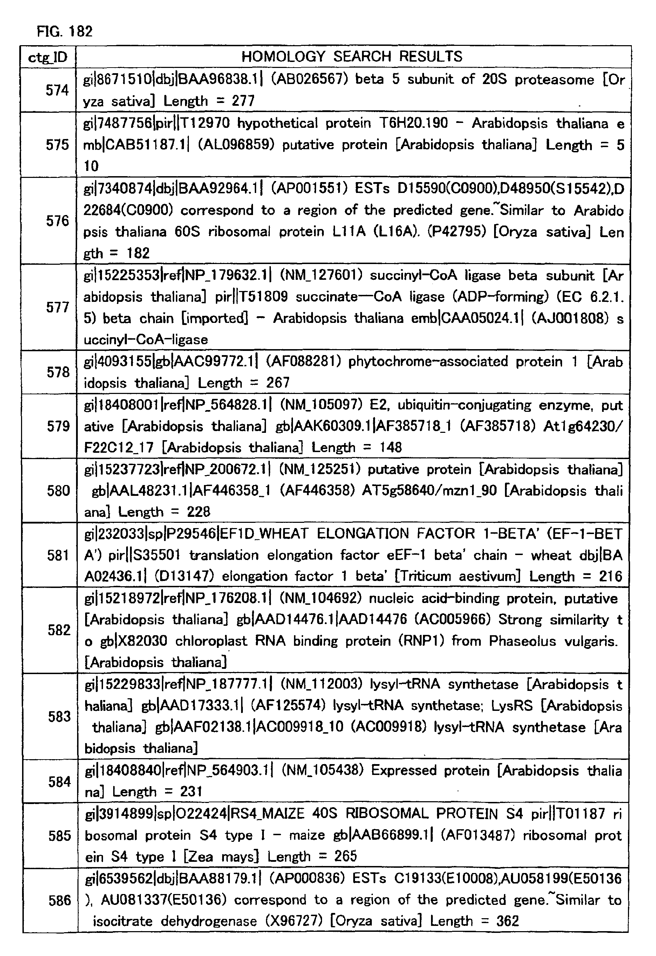

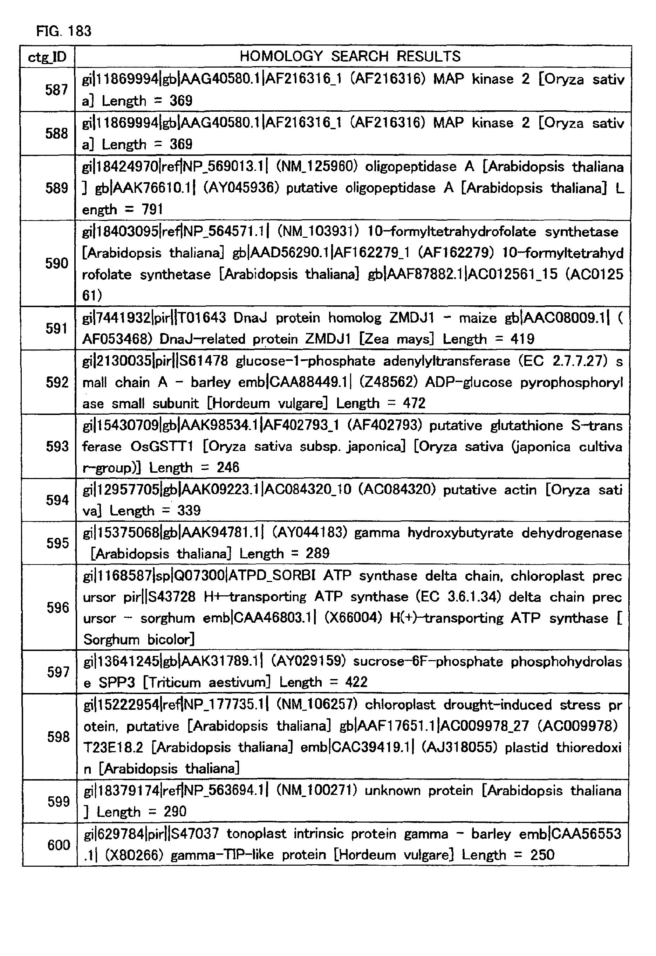

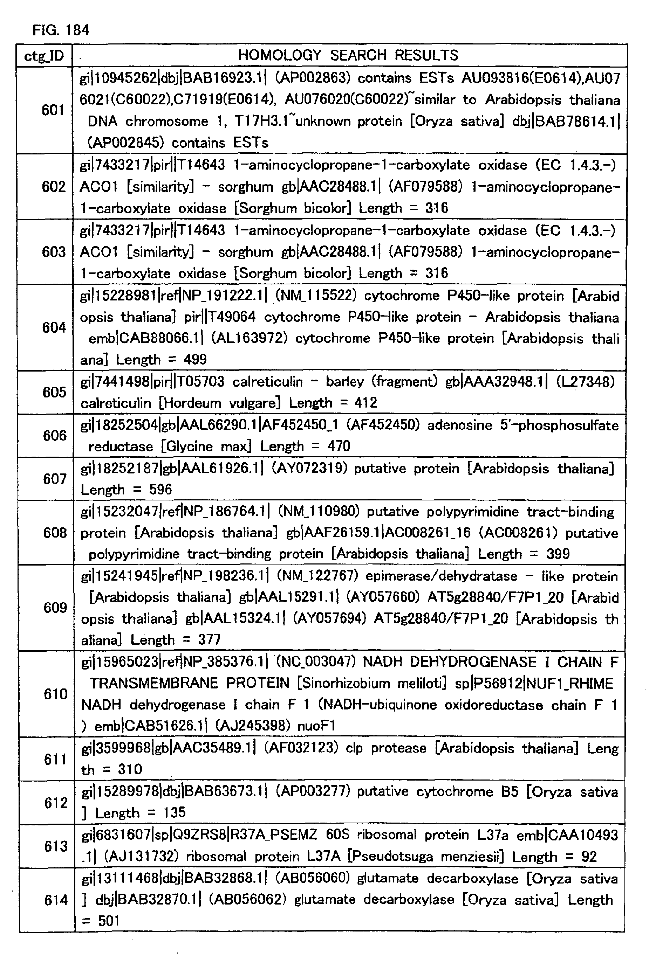

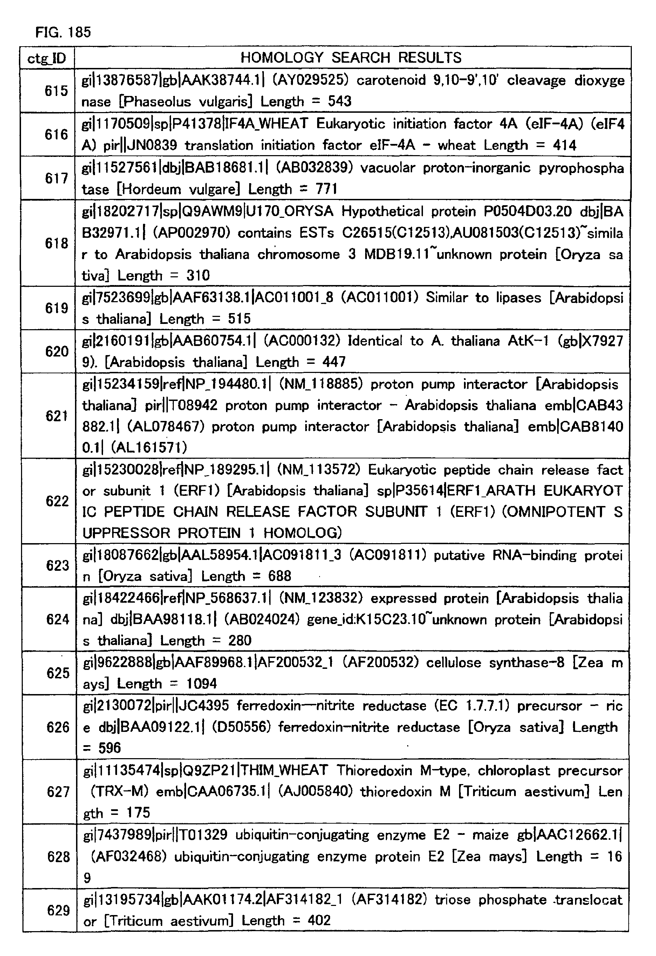

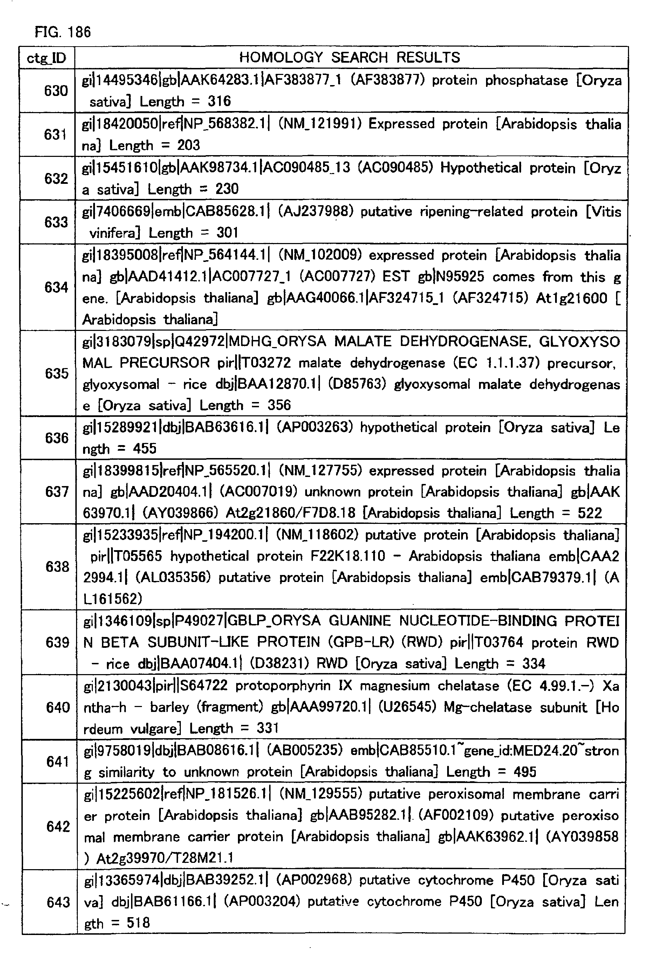

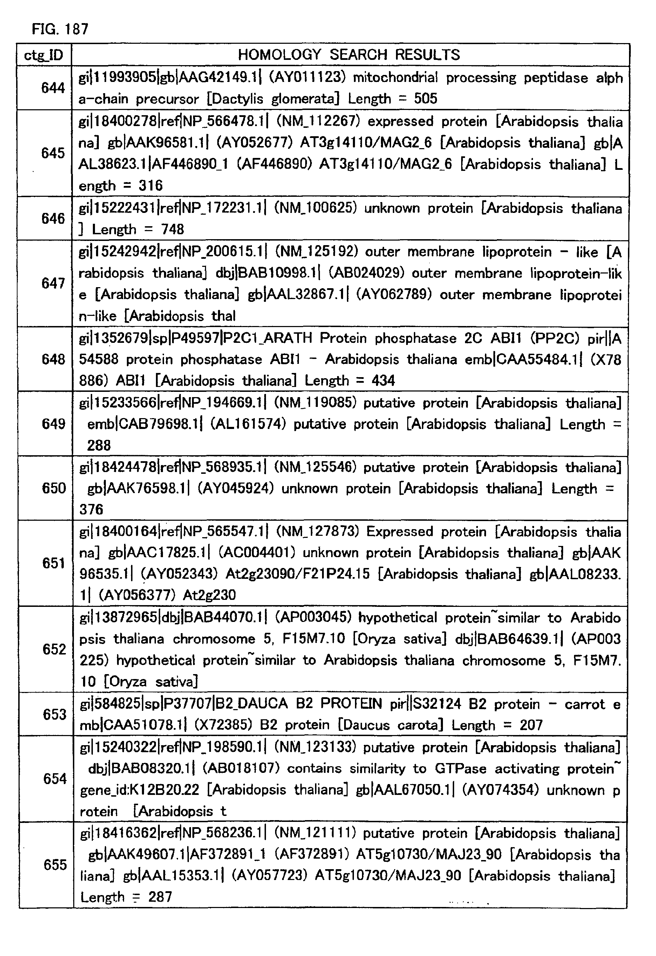

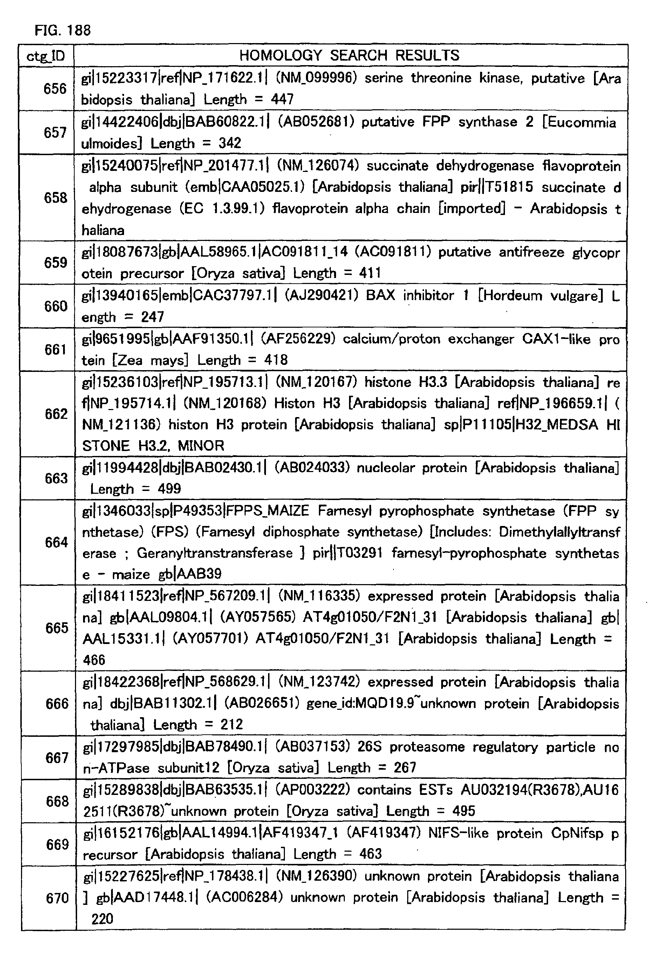

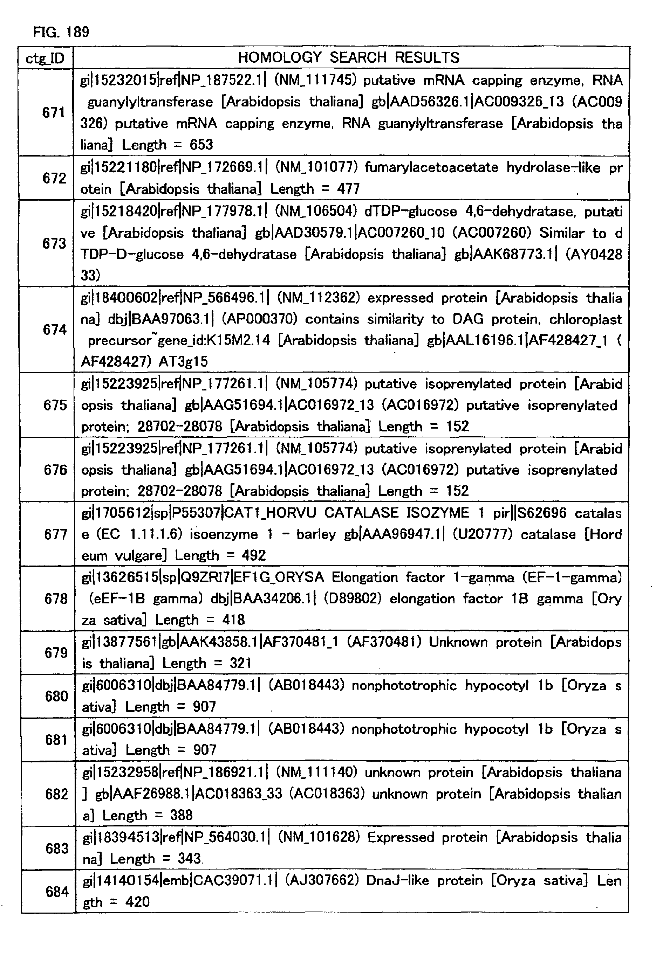

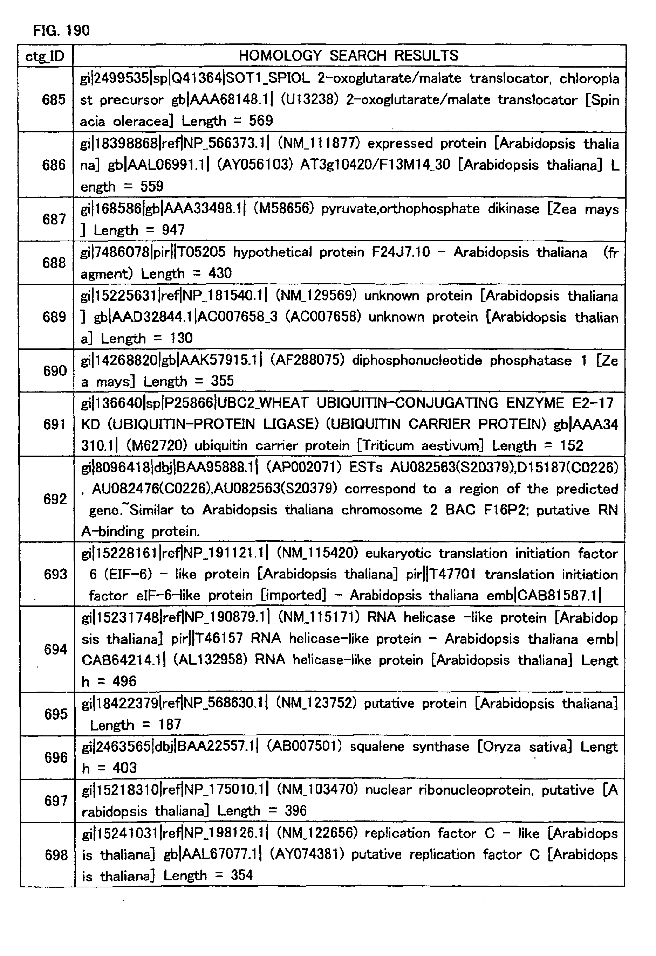

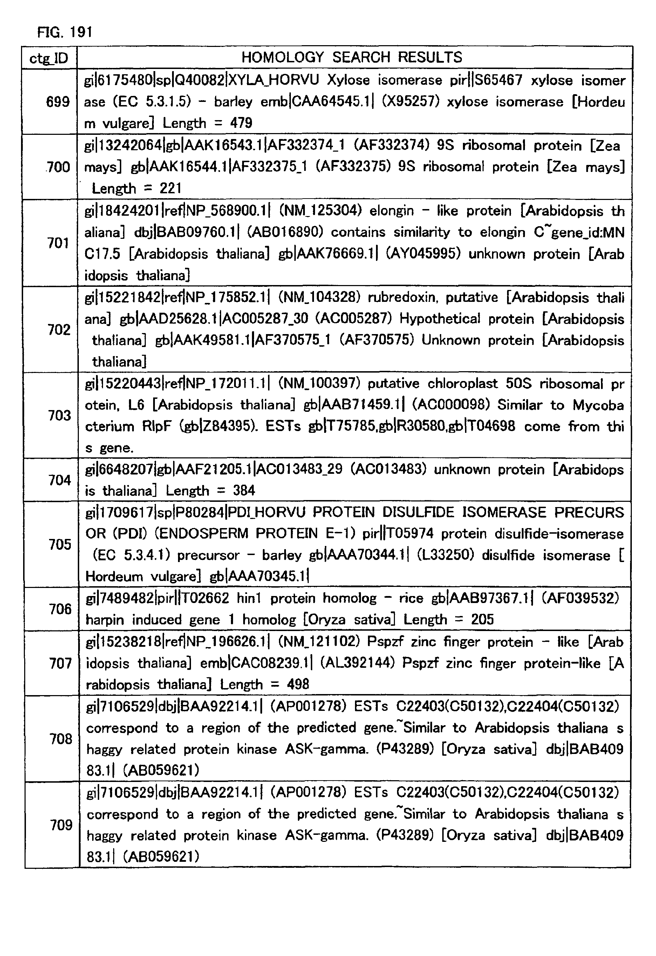

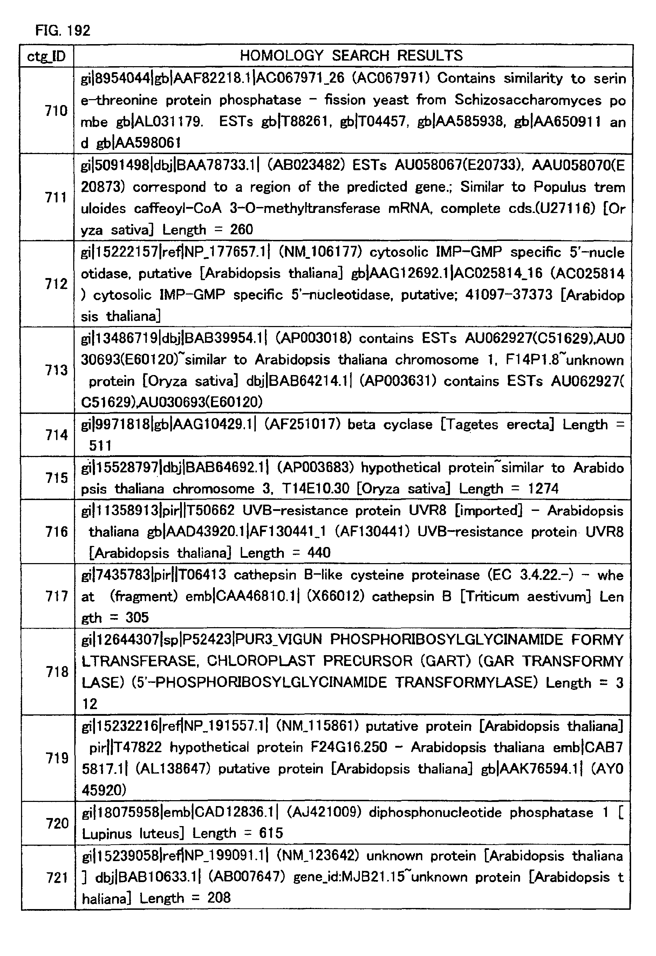

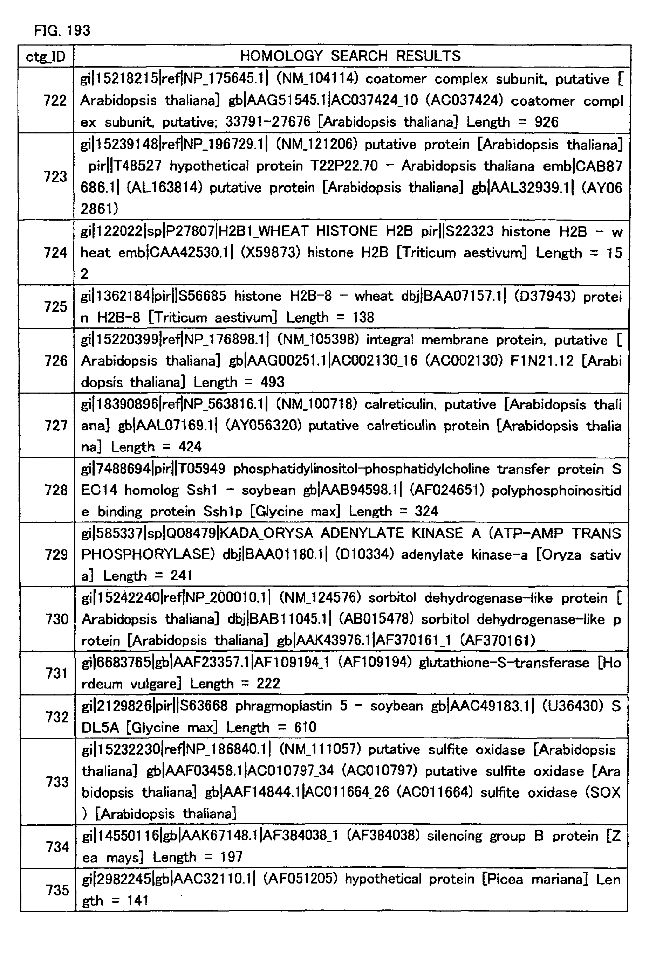

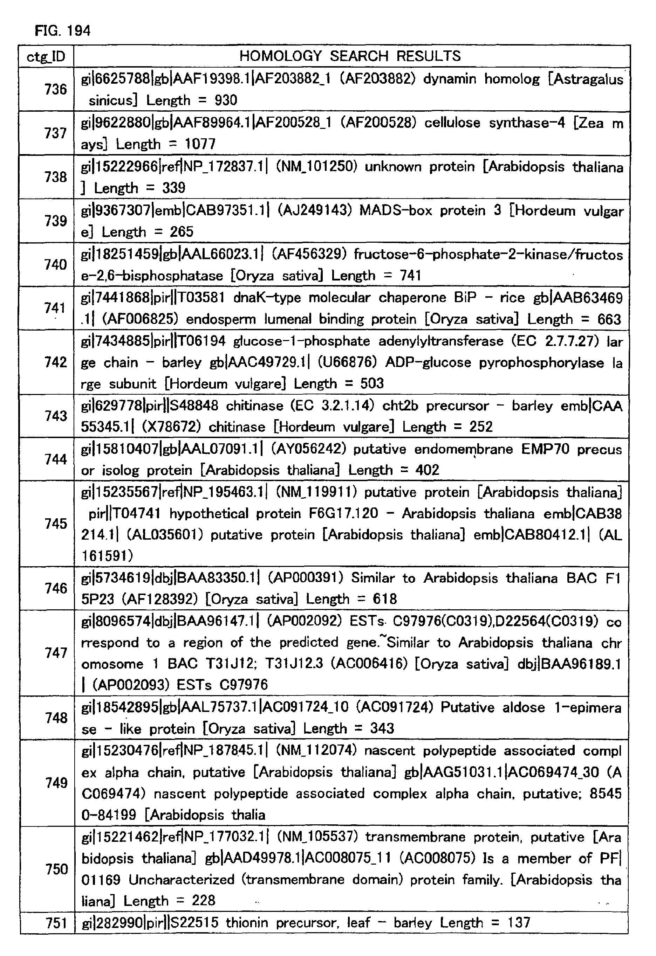

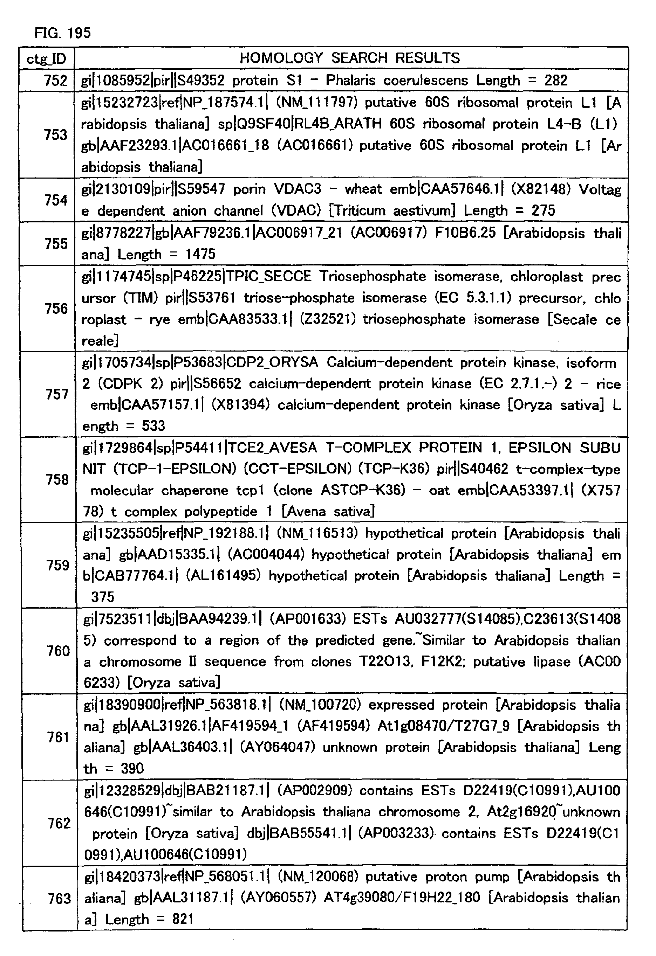

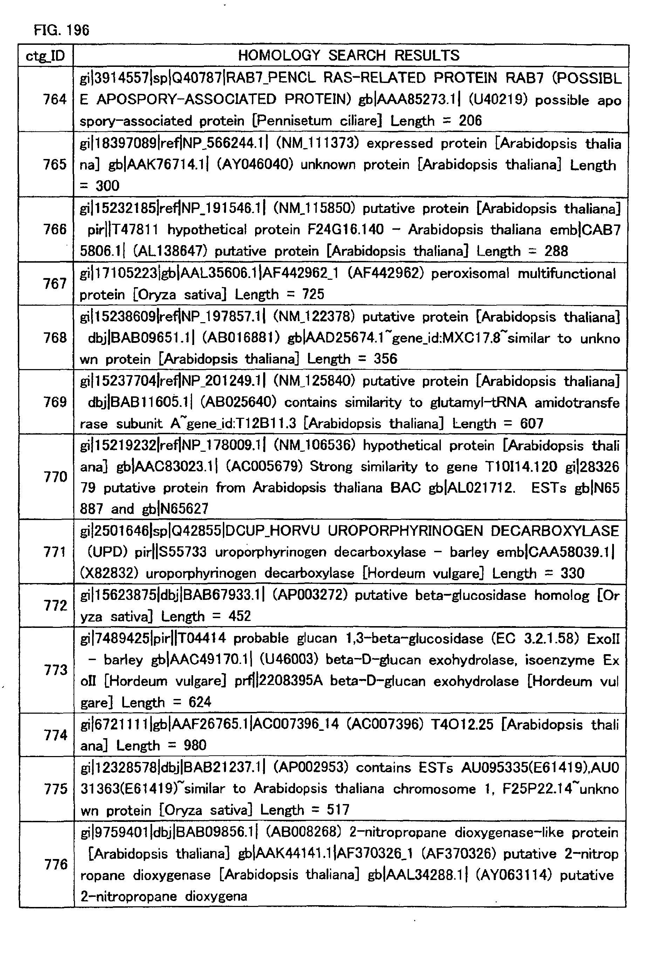

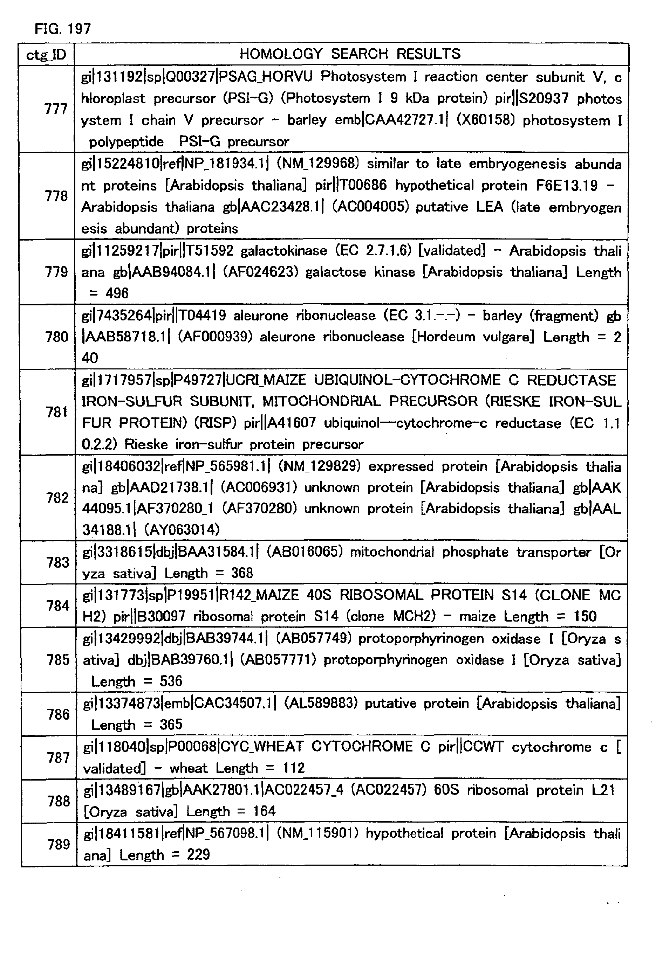

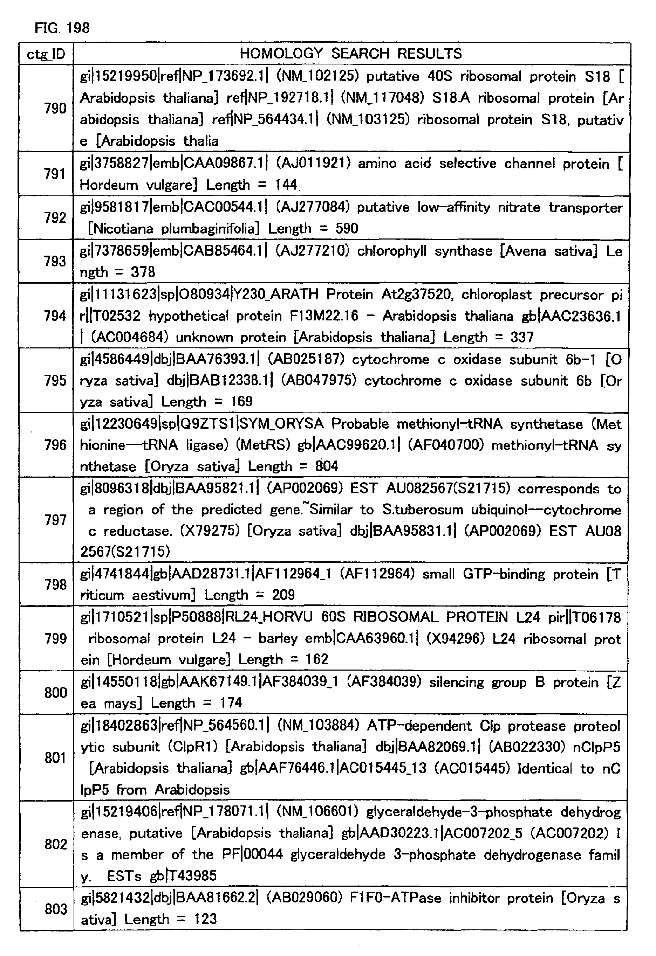

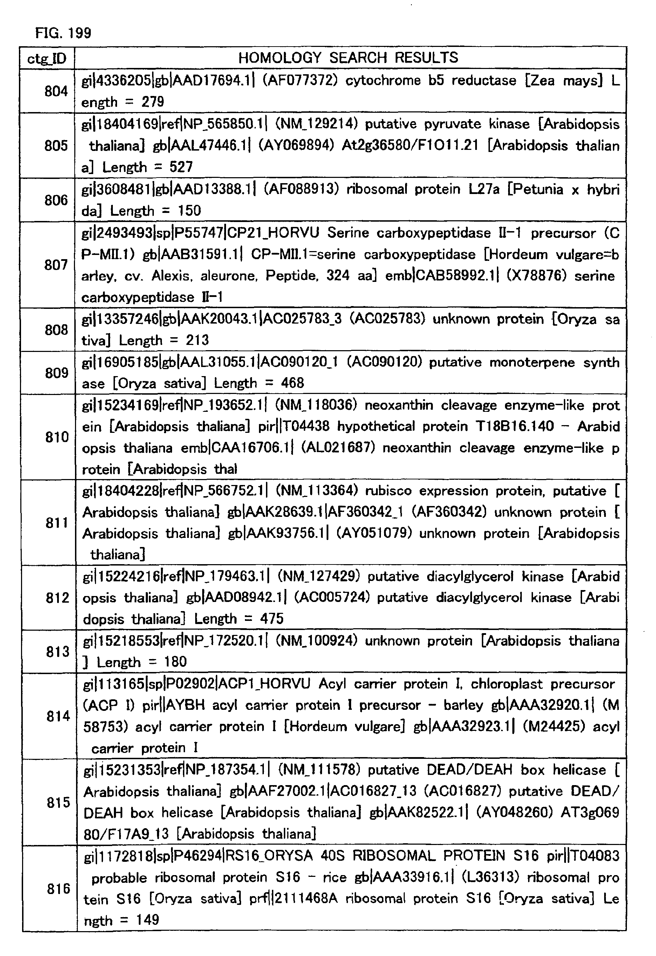

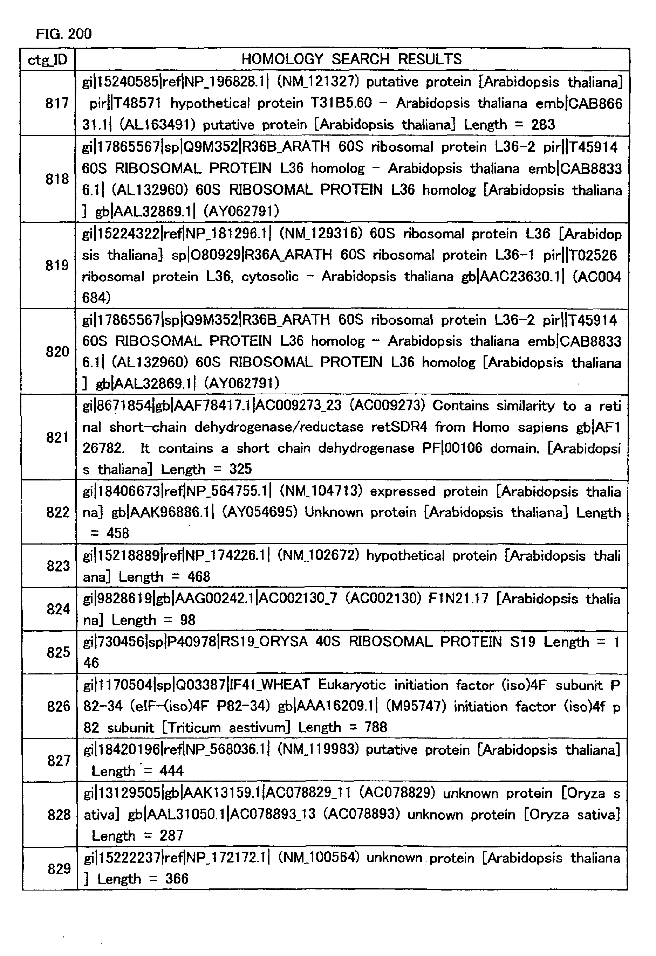

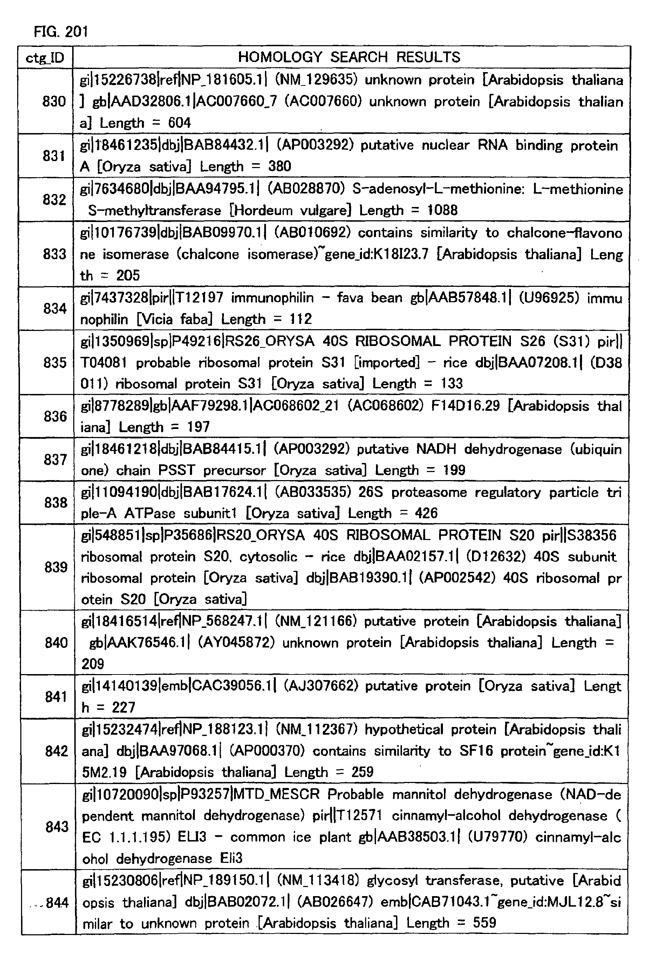

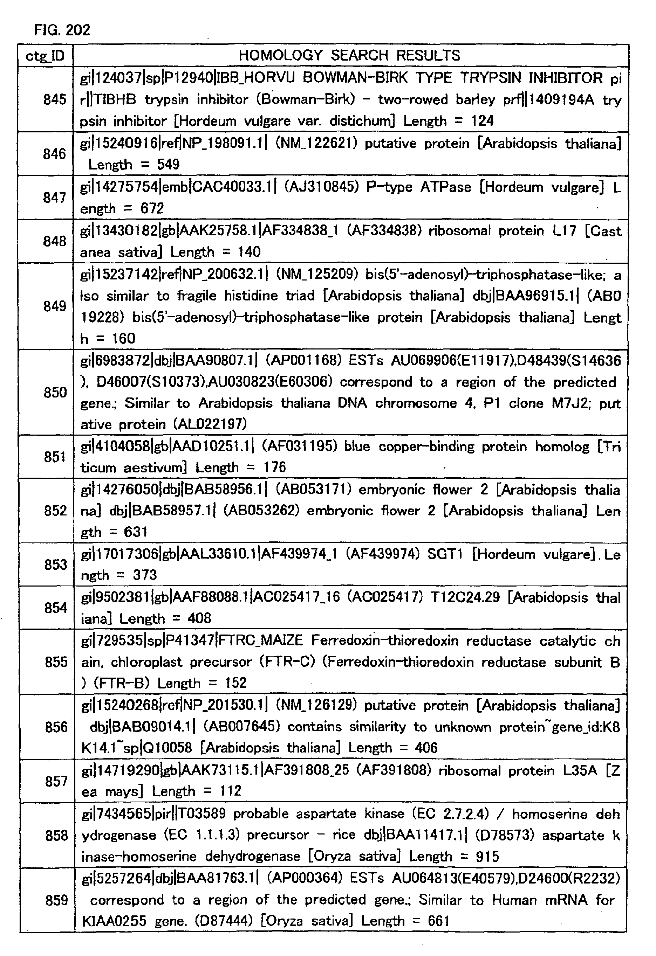

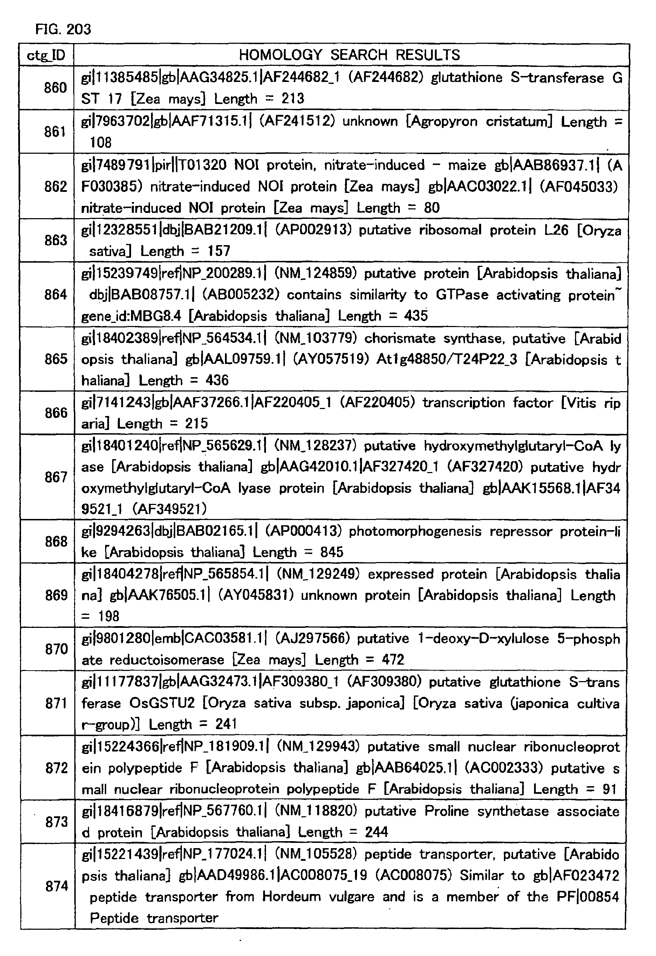

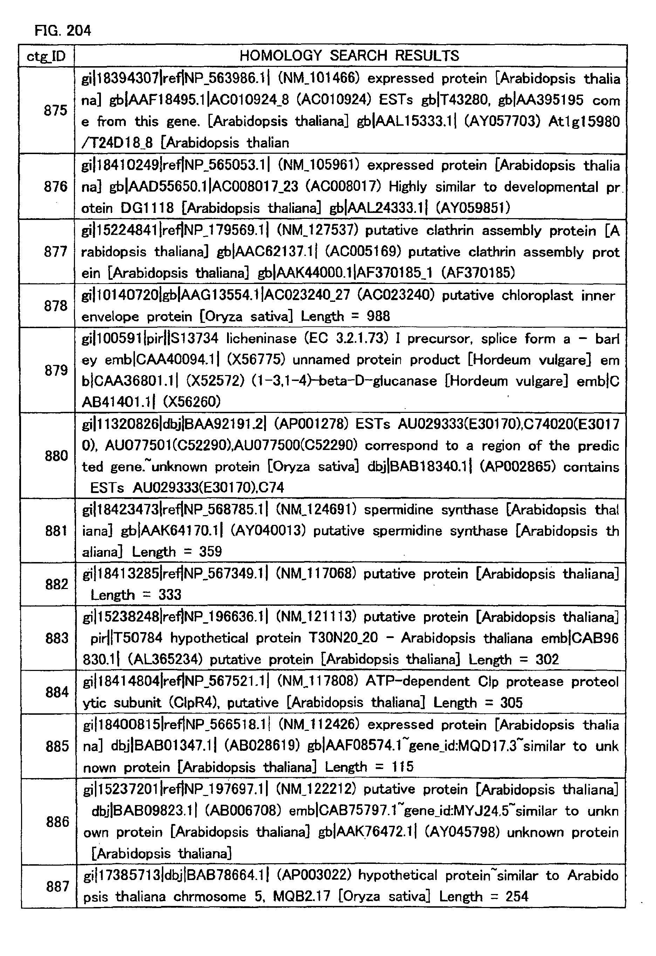

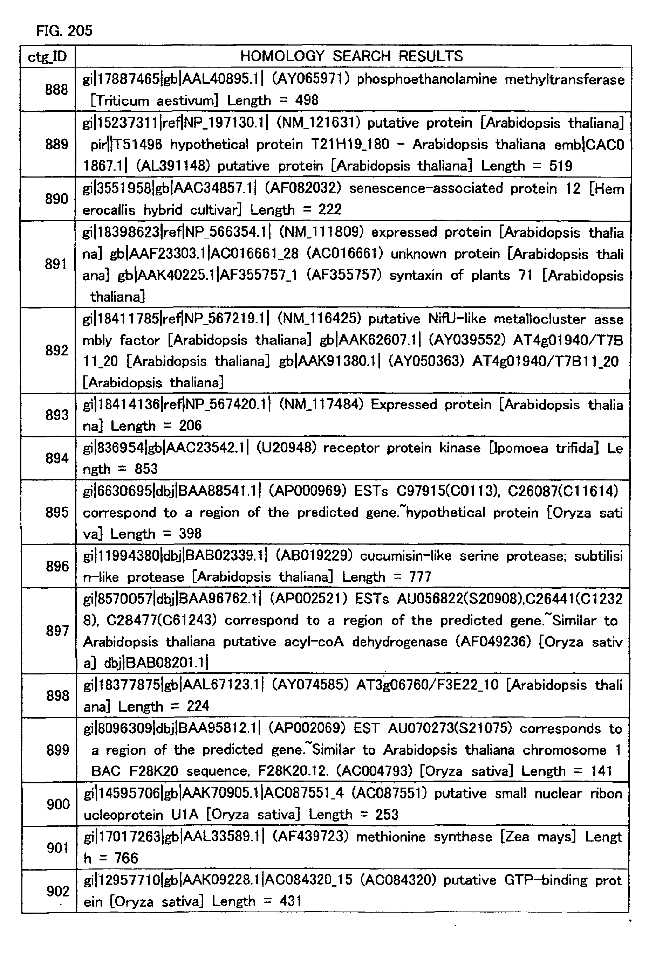

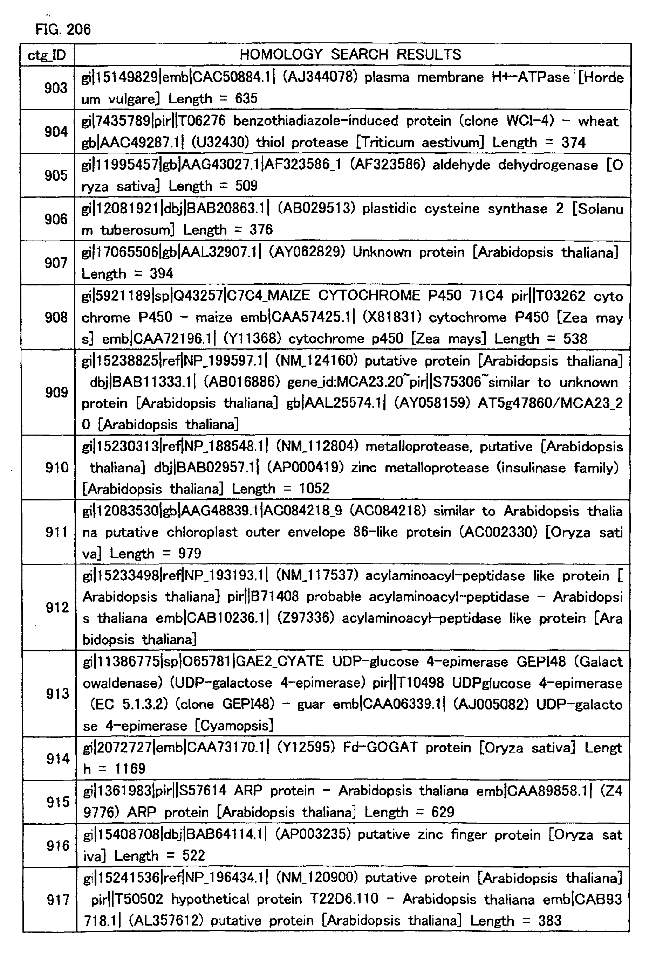

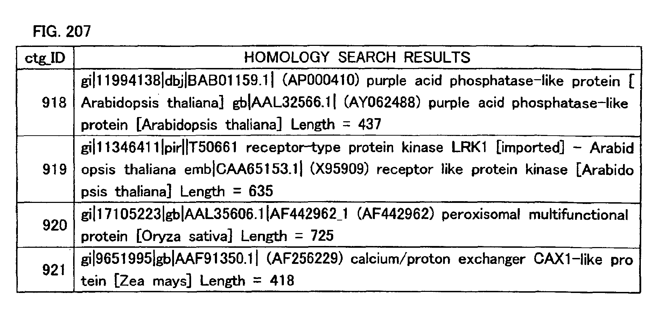

As noted above, the inventors of the present invention conducted a homology search for the CONTIGS (1) through (921) according to a known method. The results are shown in Fig. 150 through Fig. 207.

In Fig. 150 through Fig. 207, the heading "ctg_ID" is the same as that shown in Fig. 1 through Fig. 84. For each CONTIG, the column under "HOMOLOGY SEARCH RESULTS" indicates the name and accession number, etc. of a known gene or protein for which a certain level of homology was observed.

Note that, the "HOMOLOGY SEARCH RESULTS" indicated by "NONE" means that the homology search did not find a sequence that satisfied a predetermined level of homology. This suggests that there is a high probability that the CONTIG and/or its complementary sequence represents an unknown gene.

### (D) Use (usefulness) of the oligonucleotides of the present invention

An oligonucleotide of the present invention is a continuous DNA sequence of seven or more nucleotides, or a complementary sequence thereof, containing at least one SNP on the CONTIG as above. The oligonucleotide is useful in many respects, as described below.

First, because the oligonucleotides of the present invention include a polymorphic site or its complementary site that confers differences among different barley strains, the oligonucleotides or compositions containing the oligonucleotides can be used to test single nucleotide polymorphism that exists among different strains of barley. This enables genetic tagging and genotyping etc. of barley strains to be carried out both quickly and conveniently.

For example, the oligonucleotide may be immobilized on a base plate (support) to make a DNA chip (one form of a composition according to the present invention). Such a DNA chip may be used as an oligonucleotide probe for testing single nucleotide polymorphism in genes of barley. In this case, the oligonucleotide may be of any length, provided that it can test single nucleotide polymorphism. However, the oligonucleotide should preferably be 7 to 50 nucleotide or 10 to 30 nucleotide long, and more preferably 15 to 25 oligonucleotide long.

Other than testing single nucleotide polymorphism using DNA containing the polymorphic site or its complementary site, testing may be indirectly carried out using RNA obtained from the DNA containing the polymorphic site or its complementary site. This enables the subject DNA and RNA to be used as a target of a hybridize test using the DNA chip. In the case of DNA, cDNA or genomic DNA is generally used. For RNA, mRNA or cRNA is generally used.

Further, DNA fragments corresponding to the oligonucleotides of the present invention may be obtained from the subject and may be directly analyzed to test single nucleotide polymorphism in genes of the subject. Specifically, in order to test single nucleotide polymorphism in genes of the subject, the DNA derived from the subject may be amplified using, for example, a primer that is designed to amplify a region containing at least one polymorphic site whose base is represented by a universal code, and/or a complementary site thereof, in a polynucleotide with a DNA sequence set forth in any one of SEQ ID NOs: 1 through 17, 267 through 320, 990 through 1183, 4642 through 4763, and 9107 through 9640.

The primer may be one that amplifies both a region and its complementary region that includes at least one polymorphic site, like a PCR primer, or one that amplifies only one of these regions, like an RCA primer. The DNA fragments so amplified may be analyzed, for example, (1) by directly sequencing the DNA fragments, (2) by digesting the DNA fragments with a restriction enzyme that cuts the fragments in different patterns according to the base of the polymorphic site and/or its complementary site, and then by analyzing the length of each fragment, or (3) by conducting a hybridize test with the oligonucleotides of the present invention. The restriction enzyme may be one that cuts the fragments by recognizing a specific sequence of double-strand DNA (class II restriction enzyme), or one that cuts the fragments by recognizing a specific sequence of single-strand DNA (class I restriction enzyme, and some class II restriction enzyme).

It should be noted here that genes generally exist in the form of double-strand DNA (or cDNA) with a complementary sequence. Therefore, as used herein, "testing single nucleotide polymorphism (single nucleotide polymorphism on a sense strand) in a gene" also means "testing single nucleotide polymorphism on the complementary sequence of the gene (single nucleotide polymorphism on the anti-sense strand), so as to test single nucleotide polymorphism on the complementary sense strand. Further, by "testing single nucleotide polymorphism," it means not only specifying (identifying or detecting) a type of a base at the polymorphic site or its complementary site but also ruling out a particular type of the base (for example, at least adenine may be ruled out).

As used herein, the term "subject" indicates all living organisms with a gene whose base sequence, either partially or completely, is the same as or complementary to the oligonucleotides of the present invention. Suitable examples of such living organisms, other than barley, include plants that belong to the genus Hordeum, and Triticeae plants closely related to the genus Hordeum, for example, such as those belonging to the genus Aegilops, Secale, and Lolium. The present invention has basically adopted the taxonomic system described in W.D. Clayton and S.A. Renvoize: KEW BULLETIN ADDITIONAL SERIES XII, GENERA GRAMINUM, Grasses of the World (3rd Edition), pp. 146-158 (Edited by THE ROYAL BOTANIC GARDENS, KEW, 1999).

The oligonucleotides of the present invention may be used to produce a recombinant and develop a practical system in which a useful gene is introduced. For example, a useful gene may be isolated, using the oligonucleotides of the present invention as a probe or primer, or using a primer for obtaining a region of the oligonucleotides. The useful gene so isolated may be used to produce a recombinant by introducing the gene into a target cell by a genetic engineering technique. The oligonucleotide, when used as a probe for isolating a gene, should have a length sufficient as a probe. For example, the oligonucleotide as a probe should preferably be 7 to 500, 51 to 500, or 70 to 400 nucleotide long, and more preferably 90 to 300 nucleotide long.

Further, for example, where crossbreeding of parent plant A and parent plant B is possible, wherein the parent plant A has a gene which either partially or completely has a sequence of an oligonucleotide of the present invention, and the parent plant B does not have the gene or has an allele that confers single nucleotide polymorphism to the gene (especially that affects the phenotype), a desirable recombinant (recombinant plant) can be produced first by producing hybrid plants by crossbreeding the parent plant A and parent plant B (producing step), and then screening for a hybrid plant with the gene, using the testing method of oligonucleotides or SNP according to the present invention (screening step). Preferably, the allele confers single nucleotide polymorphism to at least one useful gene, so that the expression of a useful trait is suppressed or eliminated. The type of useful gene is not particularly limited. For example, a useful gene (environment resistant gene, for example) in cultivated ancestral wild-type barley is particularly preferable as in usual breeding.

As described above, an oligonucleotide of the present invention has a continuous DNA sequence of seven or more nucleotides, or a complementary sequence thereof, containing at least one polymorphic site whose base is represented by a universal code. More preferably, an oligonucleotide of the present invention has a continuous DNA sequence of seven or more nucleotides, or a complementary sequence thereof, containing at least one polymorphic site at which nonsynonymous substitution occurs and whose base is represented by a universal code. Note that, the oligonucleotide is not necessarily required to be single-stranded, and it may form a double-strand by hydrogen bonding with a complementary sequence. Further, the oligonucleotide may exist by being inserted in the sequence of a padlock probe used for SNP typing by an RCA technique (described later), or in the vector sequence (including expression vector sequence).

The oligonucleotide is a part of or entire sequence of a CONTIG or its complementary sequence that is obtained by aligning the inserts of cDNA clones. It is therefore highly likely that the same sequence is also present in the cDNA and genomic DNA as well. This enables the oligonucleotide to be used, for example, (1) as a probe or primer (probe or primer for SNP typing) for testing single nucleotide polymorphism in a gene, or (2) as a probe or primer for obtaining (isolating) genes. In the following, oligonucleotides used for purpose (1) above will be called oligonucleotide A, and those used for purpose (2) above will be called oligonucleotide B.

Oligonucleotide A is used to detect SNP that is stably preserved among the genomes of different barley strains, and is required to (a) include at least one site of SNP in a gene among different strains of barley, and (b) has a length that is suitable as a probe or primer used for hybridization in testing SNP. The oligonucleotide A may have any length so long as these conditions (a) and (b) are met. However, generally, a range of 7 to 50 nucleotides, or 10 to 30 nucleotides is preferable, and a range of 15 to 25 nucleotides, which provides a sequence with enough specificity, is more preferable.

When the oligonucleotide A has more than one polymorphic site or its complementary site, the base sequence of the oligonucleotide A is specified by fixing the bases of the polymorphic sites or their complementary sites to those found in any one of Haruna Nijo, Akashinriki, and H. spontaneum.

On the other hand, the oligonucleotide B is used as a probe or primer for obtaining a gene, or its homolog, whose sequence is either part of entire sequence of the CONTIG or its complementary sequence. Accordingly, the oligonucleotide B is not particularly limited as long as it has seven or more nucleotides including at least one polymorphic site or its complementary site. However, it is preferable that the oligonucleotide B is 51 to 500 or 70 to 400 nucleotide long, or more preferably 90 to 300 nucleotide long. It should be noted here that since the oligonucleotides of the present invention may be used for the purpose of both (1) and (2) above, the oligonucleotides A and B may have the same base sequence.

The inserts of the clones making up the CONTIGS include regions in which substantially no base difference is observed at a corresponding base among the respective sequence data derived from Haruna Nijo, Akashinriki, and H. spontaneum, except for the polymorphic site.

It is therefore preferable that the oligonucleotides of the present invention, and particularly the oligonucleotide A for detecting a base difference, are confined within a region of a DNA sequence, set forth in one of SEQ ID NOs: 1 through 17, 267 through 320, 990 through 1183, 4642 through 4763, and 9107 through 9640, in which the number of base differences among different strains is small, and has a continuous DNA sequence or its complementary sequence with seven more nucleotides including at least one polymorphic site.

It is more preferable that the oligonucleotides of the present invention, and particularly the oligonucleotide A for detecting a base difference, are confined within a region of a DNA sequence, set forth in one of SEQ ID NOs: 1 through 4, 267 through 278, 990 through 1016, 4642 through 4670, and 9107 through 9640, in which the number of base differences among different strains is small, and has a continuous DNA sequence or its complementary sequence with seven or more nucleotides including at least one polymorphic site at which nonsynonymous substitution occurs.

### (E) Preparation of oligonucleotides of the present invention

A producing method of oligonucleotides according to the present invention is not limited to a particular method, and any ordinary method may be used therefor. For example, the oligonucleotides may be obtained from the genomic DNA, genomic DNA library, or cDNA library of the target organism by cutting the DNA with a restriction enzyme, followed by purification. Alternatively, the oligonucleotides of the present invention may be isolated by setting up amplification using the genomic DNA or cDNA of the target organism as a template, and using a primer (primer according to the present invention) that is designed to amplify a region and/or its complementary region including at least one polymorphic site, represented by a universal code, in an oligonucleotide of the DNA sequence set forth in any one of SEQ ID NOs: 1 through 17, 267 through 320, 990 through 1183, 4642 through 4763, and 9107 through 9640.

Further, the oligonucleotides of the present invention may be isolated by setting up amplification using the genomic DNA or cDNA of the target organism as a template, and using a primer (primer according to the present invention) that is designed to amplify a region and/or its complementary region including at least one polymorphic site, represented by a universal code, at which nonsynonymous substitution occurs, in an oligonucleotide of the DNA sequence set forth in any one of SEQ ID NOs: 1 through 4, 267 through 278, 990 through 1016, 4642 through 4670, and 9107 through 9640. Further, the oligonucleotides may be synthesized by a known method.

The length of the primer used to obtain the oligonucleotide is not particularly limited as long as it does not cause amplification in regions other than a target region by mismatch. For example, when PCR (Polymerase Chain Reaction) is used for amplification, a pair of primer sets of 15 to 25, or 15 to 30 oligonucleotide long is generally used. In Fig. 85 through Fig. 97, the column under "PRIMER SEQUENCE NUMBER" shows examples of primer sets for amplifying regions of CONTIGS including polymorphic sites. The sequence of the primer is not particularly limited either, and any sequence may be designed based on specific and characteristic regions of the DNA sequence.

PCR may be carried out under ordinary reaction conditions (for example, see Saiki, Science, 230, 1350-1354 (1985); PCR Technology, Takara Shuzo Co., Ltd., published in 1990). Specifically, the heat denaturing step for a target double strand DNA (including template) may be carried out in a temperature range of 90°C to 96°C or more preferably 94°C to 96°C for about 1 to 3 minutes or more preferably about 1 to 2 minutes. The annealing step for the primer may be carried out in a temperature range of 40°C to 60°C or more preferably 55°C to 60°C for about 1 to 3 minutes or more preferably about 1 to 2 minutes. The strand extending step using DNA polymerase may be carried out in a temperature range of 70°C to 74°C or more preferably 72°C to 74°C for about 1 to 4 minutes or more preferably about 2 to 3 minutes.

The number of PCR cycles, from the heat denaturing step to the strand extending step, is not particularly limited. Generally, the PCR is carried out in 20 to 40 cycles, or more preferably 25 to 35 cycles. The type or quantity of DNA polymerase (heat-resistant polymerase such as Taq polymerase) or of the chemicals used, and the method by which a DNA template is extracted are no different from those conventionally used, and no explanation will be made therefor. As used herein, the scope of PCR includes an alternative form such as RT-PCR (Reverse Transcribed PCR). Further, the PCR, the product of which is double-strand DNA fragments with polymorphic sites, may produce single-strand DNA fragments including the polymorphic site or its complementary site (oligonucleotides of the present invention) when the reaction is carried out with a suitable primer-template set (cyclic single-strand DNA with a polymorphic site or its complementary site), using a single-strand amplification method such as RCA (Rolling Circle Amplification) for example (Lizardi PM et al. Nat Genet 19,225, 1998).

Suitable examples of organisms from which the oligonucleotides A and B of the present invention are obtained include various barley strains including H. spontaneum, Haruna Nijo, and Akashinriki, and other plants that belong to the genus Hordeum. Other than these plants that belong to the genus Hordeum, Triticeae plants closely related to the genus Hordeum, for example, such as those belonging to the genus Aegilops, Secale, and Lolium may be suitably used.

### (F) Typing method of SNP using the oligonucleotides of the present invention

In the following, description is made as to SNP typing using the oligonucleotides of the present invention.

Single nucleotide polymorphism on a gene of a subject may be tested (SNP typing) using the oligonucleotides of the present invention. The method is broadly classified into the following two methods (A) and (B). In method (A), the oligonucleotide is isolated from the DNA (may be cDNA) of a target organism, and the base of a polymorphic site or its complementary site on the oligonucleotide is directly tested ("direct method"). In method (B), single nucleotide polymorphism on a gene of the subject is indirectly tested using a polymorphic site or its complementary site of the oligonucleotide A of the present invention ("indirect method"). That is, methods (A) and (B) both test single nucleotide polymorphism in a gene of a subject based on the base of a polymorphic site or its complementary site present in the oligonucleotides of the present invention.

Note that, particularly suitable examples of "subject" are the barley strains H. spontaneum, Haruna Nijo, and Akashinriki, and other plants that belong to the genus Hordeum. However, the type of subject is not particularly limited so long as it is an organism possessing a gene whose base sequence is either part of or all of the base sequence of the oligonucleotide or its complementary sequence of the present invention. Specific examples of organisms which are highly likely to possess such a gene include Triticeae plants closely related to the genus Hordeum, for example, such as those belonging to the genus Aegilops, Secale, and Lolium. Further, as used herein, the term "subject" includes a recombinant into which the gene of such organisms was introduced (or attempted to be introduced) by crossbreeding or genetic engineering techniques.

It should be noted here that genes generally exist in the form of double-strand DNA (or cDNA) with a complementary sequence. Therefore, as used herein, "testing single nucleotide polymorphism (single nucleotide polymorphism on a sense strand) in a gene" also means testing single nucleotide polymorphism on the complementary sequence of the gene (single nucleotide polymorphism on the anti-sense strand), so as to test single nucleotide polymorphism on the complementary sense strand.

In the following, the direct method is described in more detail. The direct method is a method by which SNP that has occurred in a gene is tested by first obtaining amplified DNA fragments (oligonucleotides according to the present invention) by amplification of the DNA (genomic DNA or cDNA) derived from a subject, using, for example, a primer (primer according to the present invention) designed to amplify a region and/or its complementary region including at least one polymorphic site on a DNA sequence set forth in one of SEQ ID NOs: 1 through 17, 267 through 320, 990 through 1183, 4642 through 4763, and 9107 through 9640, and then by directly analyzing the amplified DNA fragments so obtained. Note that, as to the specific examples and conditions of amplification used in this method, the foregoing Section (E) Preparation of oligonucleotides of the present invention should be referred to.

More specific examples of the direct method include (1) SNP typing based on mass differences of amplified DNA fragments using a MALDI-TOF/MS (Matrix Assisted Laser Desorption-Time of Flight/Mass Spectrometry), (2) sequencing of the amplified DNA fragments to directly determine the base of a polymorphic site or its complementary site, and (3) direct typing of SNP based on a difference in length of fragments that were obtained by digesting the amplified DNA fragments with a restriction enzyme that cuts the fragments in different patterns according to the type of base present at the polymorphic site and/or its complementary site (also known as RFLP (Restriction Fragment Length Polymorphism)). In method (3), two types of restriction enzymes, one that cuts the sequence by recognizing a specific sequence of double-strand DNA (class II restriction enzyme), and one that cuts the sequence by recognizing a specific sequence of single-strand DNA (class I restriction enzyme, and some class II restriction enzyme), are selectively used depending on whether the amplified DNA fragments are single-stranded (using the RCA method and the like) or double-stranded.

The methods (2) and (3) above are fairly common, and accordingly only method (1) will be described below. In the SNP typing using the MALDI-TOF/MS technique, amplification is carried out using the genomic DNA or cDNA of the subject as a template, and using a primer designed to amplify a region or its complementary region including the polymorphic site. Then, using the resulting amplified DNA fragments as a template, the sequence of DNA is extended with the primer to obtain DNA products that include the polymorphic site or its complementary site at the 3' end. Next, mass differences of the DNA products due to different bases at the polymorphic site or its complementary site are measured with a mass spectrometer, so as to type the base of the polymorphic site or its complementary site (see Post-Sequence Genome Science (1), Strategy on SNP Gene Polymorphism, 1 st impression (Nakayama Shoten, Co., Ltd., pp. 106-117). Note that, a multiplicity of SNPs may be simultaneously tested under multiplex conditions.

On the other hand, the indirect method is an SNP typing method that uses the oligonucleotide A of the present invention. Specifically, the indirect method includes the steps of, for example, (1) preparing DNA (including cDNA) and RNA derived from a subject, (2) obtaining the oligonucleotide A, and (3) determining whether the oligonucleotide A completely hybridizes with the RNA or DNA prepared in step (1). When the oligonucleotide A and the RNA or DNA completely hybridize, the base of a polymorphic site (or its complementary site) in a gene of the subject is determined to be complementary to the corresponding base of the oligonucleotide A. On the other hand, when the oligonucleotide A and the RNA or DNA do not hybridize completely, the base of a polymorphic site (or its complementary site) in a gene of the subject is determined to be non-complementary to the corresponding base of the oligonucleotide A.

It should be noted here that, apparently, when the oligonucleotide A has a DNA sequence that includes at least one polymorphic site, it completely hybridizes with its complementary sequence of the genomic DNA or cDNA. On the other hand, when the oligonucleotide A is the complementary sequence of the DNA sequence, it completely hybridizes with its complementary sequence of the mRNA, cRNA, or genomic DNA.

As used herein, "DNA derived from a subject" includes amplified DNA fragments obtained by PCR, RCA, or other methods, using the DNA as a template. Generally, the DNA or RNA derived from a subject is obtained from various organs, tissues, cells, protoplast, spheroplast, or callus of the subject organism. Other than the subject organism, processed food (including drinks) or fossil may be used as a DNA or RNA source.

For the preparation of subject's DNA or RNA, common DNA or RNA extraction techniques may be used. The type of tissue used for DNA or RNA extraction, the method of cultivating (growing) the subject, and the growth stage of the subject are not particularly limited either. The DNA or RNA may be prepared as cDNA or mRNA with the method used to make CONTIGS, for example. In the case of preparing cRNA, a common method may be used by which cDNA is transcribed with a suitable RNA polymerase. Further, the DNA or RNA, which are the target of hybridization, and/or the oligonucleotide A (probe), are generally tagged with a biotin tag or various fluorescent tags to check the state of hybridization. However, tagging of the target and/or probe is not necessarily required when the state of hybridization is checked by other methods such as intercalation.

Note that, the subject's DNA or RNA (target) and the oligonucleotide A (probe) may be hybridized under the conditions employed by various methods, taking into account the length of oligonucleotide A (for example, see DNA micro array, 1 st edition, 3rd impression, pp. 67-83 (published by Takara Shuzo Co., Ltd.)).

Generally, the hybridization is carried out by preparing a DNA chip (composition) in which the oligonucleotide A (probe) is immobilized on a base plate (support). As used herein, "DNA chip" primarily means a DNA chip that uses a synthetic oligonucleotide as a probe. However, the scope of "DNA chip" also includes a paste DNA micro array that uses the product of PCR, such as cDNA, as a probe.

The DNA chip may be fabricated by a known method. When a synthetic oligonucleotide is used as the oligonucleotide A, the oligonucleotide is synthesized on a base plate by a combination of a photolithography technique and a solid-phase DNA synthesis technique. On the other hand, when cDNA is used as the oligonucleotide A, the oligonucleotide is stuck on a base plate using an array machine. Further, the accuracy of testing SNP may be improved, as in common DNA chips, by positioning a perfect match probe (oligonucleotide A) and a mismatch probe, which is made from a perfect match probe by replacing a single base at a site other than the polymorphic site (or its complementary site). Further, in order to concurrently test SNPs that are present on different genes, a DNA chip may be fabricated that immobilizes different kinds of oligonucleotides A on a single base plate.

Another example of the indirect method is SNP typing using an invader method (see Post-Sequence Genome Science (1), Strategy on SNP Gene Polymorphism, 1st impression (Nakayama Shoten, Co., Ltd., pp. 98-105). In one example, the complementary sequence of a sequence that is part of any one of the CONTIGS (1) through (921) and has a polymorphic site at the 3' end is selected as the oligonucleotide A (probe region), and a certain common sequence (flap region) is linked to the 5' end of the oligonucleotide A, so as to prepare an allele probe. Then, the allele probe and an invader probe, which is part of the CONTIG and has the polymorphic site at the 3' end (the type of base at the 3' end is not limited), are hybridized with the genomic DNA or cDNA of the subject. Then, a flap region is removed using an enzyme (clevase) that cuts the flap region, only when the allele probe completely matches (completely hybridizes with) the genomic DNA or cDNA. The flap region so removed is then hybridized with a probe, labeled with a fluorescent dye and quencher, that can stick to the flap region by complementary pairing. (FRET (Fluorescence Resonance Energy Transfer) probe), and the fluorescent dye is removed with the enzyme (clevase). The SNP typing at the polymorphic site can then be carried out by determining whether the quantity of generated fluorescence varies depending on whether the allele probe has completely hybridized with the genomic DNA or cDNA.

In yet another example of the indirect method, the oligonucleotide A is set to hybridize with the subject's DNA (annealing), and whether or not they have completely hybridized is checked by allowing the DNA to amplify. Specific examples of such a method include conventional SNP typing methods using TaqMan PCR or RCA (see Post-Sequence Genome Science (1), Strategy on SNP Gene Polymorphism, 1 st impression) (Nakayama Shoten, Co., Ltd., pp.94-97, pp. 118-127).

In one example of SNP typing using TaqMan or PCR, the complementary sequence of a sequence of about 20 nucleotides in part of the CONTIGS (1) through (921) and including one polymorphic site is selected as the oligonucleotide A, and a TaqMan probe is prepared by labeling the 5' end and 3' end of the oligonucleotide A in this order with a fluorescent dye and quencher. Here, the TaqMan probe does not emit fluorescence by the presence of the quencher. Then, PCR is run with the TaqMan probe hybridized with the genomic DNA or cDNA of the subject, using a Taq DNA polymerase, and a PCR primer designed to amplify a region and its complementary region of the CONTIG including the polymorphic site. The Taq DNA polymerase has 5' nuclease activity, enabling the fluorescent dye to be removed and fluorescence to be observed when the TaqMan probe has actually hybridized with the genomic DNA or cDNA of the subject. Therefore, with the TaqMan probe, with a suitably selected base that is complementary to the polymorphic site, it is possible to find the type of base present at the polymorphic site or its complementary site of the genomic DNA or cDNA of the subject, by checking the presence or absence of fluorescence (whether the TaqMan probe has completely hybridized with the subject's genomic DNA or cDNA).

In an example of SNP typing using RCA, a single-strand probe (padlock probe) is prepared in which two complementary sequences, each corresponding to a sequence of about 20 nucleotides including a polymorphic site in the subject's genomic DNA or cDNA, are positioned at the ends of the probe by being linked together with a specific sequence called a backbone in between. The padlock probe is designed so that a base complementary to the base of the polymorphic site is positioned at one end (usually 3' end) of the probe. Then, the padlock probe is ligased using the subject's genomic DNA or cDNA as a template, and is allowed to amplify with a combination of a suitable primer and DNA polymerase.

Here, typing of the base at the polymorphic site is enabled when the base of the polymorphic site in the genomic DNA or cDNA used as a template is actually complementary to the base at one end of the probe, because in this case ligation (formation of a ring in this example) and amplification of the padlock probe occur, as opposed to the case where the two bases are non-complementary. Note that, RCA allows for DNA amplification at a constant temperature (generally 65°C), making the method particularly suitable for analyzing large samples. Further, the sequence of the padlock probe (complementary sequences at the ends of the probe) falls within the meaning of oligonucleotide A of the present invention, even though it turns itself to the oligonucleotide A only when a ring is formed.

In still another example of the indirect method, the oligonucleotide A is used as a kind of PCR primer, and is hybridized (annealed) with the subject's DNA. After amplifying the DNA by PCR, the presence or absence of amplified DNA fragments (whether or not complete hybridization has occurred) is determined for the typing of SNP (for example, ASA (allele-specific amplification) method).

The SNP typing kits used in the indirect method all include the oligonucleotide A, and therefore are regarded as the composition of the present invention. That is, the compositions of the present invention encompasses (1) a composition in which the oligonucleotide A is immobilized on a support as in the DNA chip, as well as (2) a composition in which the oligonucleotide A is generally not immobilized, as in SNP typing kits used in TaqMan PCR, Invader, and RCA methods.

As described, the methods using the oligonucleotide A and the other SNP typing methods can be used to determine the base of the polymorphic site or its complementary site in plants that belong to the genus Hordeum (particularly, the barley strains H. spontaneum, Akashinriki, and Haruna Nijo). The type of base present at the polymorphic site or its complementary site is essentially the same within the same barley strain, but is different among particular types of different strains. This enables identification of different barley strains.

For example, any one of the SNP typing methods of the present invention may be used to identify the base of a site corresponding to the polymorphic site of DNA derived from H. spontaneum, Akashinriki, and Haruna Nijo. For example, the base of a site corresponding to the nucleotide No. 1083 (polymorphic site) of the CONTIG (393) (DNA sequence set forth in SEQ ID NO: 9112) may be identified. When the base is aderiine, the strain is identified as H. spontaneum, and when thymine, the strain is Akashinriki or Haruna Nijo.

Strain identification may be possible for organisms other than barley or plants that belong to the genus Hordeum, when these organisms are highly likely to have SNPs similar to those found in barley and its related plants (see a list of subjects for SNP typing). Further, for improved accuracy, identification may be made by combining more than one polymorphic site (or its complementary site), or by using a genetic marker together. Note that, strain identification using polymorphic information such as SNP on a gene is particularly effective in situations where naked eye is not reliable, for example, as in testing of quality level of barley grain or processed products of barley (including starch) (for example, testing for the presence or absence of foreign barley).

### (G) Use of SNP as a genetic marker

When the base of a polymorphic site (or a complementary site) is different between a gene and its allele as to produce different phenotypes, the SNP may be used as a genetic marker for the purpose of, for example, (1) obtaining a gene or its allele associated with a desired phenotype, or (2) screening for a plant having the gene or its allele. These cases (1) and (2) are described below in detail. For convenience of explanation, the term "gene" is used for the gene whose polymorphic site has a base distinct to Akashinriki, Haruna Nijo, or H. spontaneum, whereas the term "allele" is used when its polymorphic site has a base different from that of the "gene" (different genotype). Further, it is assumed here that the gene has a more useful phenotype than its allele ("useful gene").

In case (1), the oligonucleotide (B), which hybridizes with only one of the gene and allele, is used as a probe, and the gene (useful gene) is obtained (isolated) by screening, for example, the genomic DNA library or cDNA library of barley. Alternatively, the gene may be obtained by identifying a clone with the gene, for example, from the genomic DNA library or cDNA library of barley, using a gene amplification technique (such as PCR) that uses the oligonucleotide B as a primer.

The gene so isolated is introduced into a target cell (host cell) by a known genetic engineering technique, and thereby produce a recombinant that can express the gene. One specific example of such a known genetic engineering technique is a method that utilizes infection by agrobacterium, such as a protoplast coculturing method or leaf disk method. Other specific examples include a polyethylene glycol method, electroporetion method, microinjection method, particle gun method, liposome method, DNA direct injection method, and injection method using suitable vectors. A suitable method is selected according the type of target cell.

For the target cell, prokaryotic cells such as Escherichia coli, or eukaryotic cells such as plant cells may be used. However, owning to the fact that the injected gene is derived from barley, the target cell is preferably a plant cell, more preferably cells of Triticeae plants, and particularly preferably cells of plants that belong to the genus Hordeum including barley. When using prokaryotic cells, cDNA is used as the gene.

Note that, when the gene and its allele confer substantially the same phenotype, or when obtaining a homolog of the gene, the oligonucleotide B, which can non-selectively isolate the gene, its allele, and homolog, may be used as a probe or primer for obtaining the gene. The gene, its allele, and homolog are suitably isolated from plants that belong to the genus Hordeum (particularly, the barley strains H. spontaneum, Akashinriki, and Haruna Nijo). Alternatively, they may be isolated from organisms with a gene or its homolog which either partially or completely has a sequence of the oligonucleotides of the present invention (more preferably, the CONTIG or its complementary sequence).

The type of gene (useful gene) is not particularly limited. However, a useful gene in cultivated ancestral wild-type barley (H. spontaneum) is particularly preferable. For the target cell into which the useful gene is injected, cells of cultivated barley are particularly preferable.

In case (2), the base of a polymorphic site (or its complementary site) is determined by any one of the SNP typing methods described above, and a plant with a desired gene or its allele (generally the "gene" (useful gene)) is screened for based on the identified base. The screening of plants is usually carried out in plant breeding, for example, in (1) selecting a hybrid parent with the useful gene, or (2) selecting, from a group of hybrids (recombinant, recombinant plant) obtained in backcrossing, a candidate strain to be crossed with a recurrent parent, or a new strain that stably carries the useful gene.

In the following, description is made as to a specific method of producing a recombinant by crossbreeding. The parents (parent plant A, parent plant B) to be crossed may be of any plants that can be crossbred, with one of the parents (parent plant A) carrying the gene ("useful gene" hereinafter), and the other parent (parent plant B) not carrying the useful gene, or carrying an allele of the useful gene.

In other words, the parent plant A is not particularly limited as long as it is a plant with a useful gene having either partially or completely a sequence of the oligonucleotide of the present invention (more preferably, the CONTIG or its complementary sequence). However, in light of a high probability of the presence of the useful gene, the parent plant A is preferably Triticeae plants, and more preferably plants that belong to the genus Hordeum (for example, barley strains H. spontaneum, Akashinriki, and Haruna Nijo), of which ancestral wild-type barley (H. spontaneum), carrying genes not present in cultivated barley, is particularly preferable.

The parent plant B is not particularly limited as long as it is a plant without the useful gene or with an allele of the useful gene, and that can be crossbred with the parent plant A. However, in light of ease of crossbreeding with Triticeae plants (suitable example of parent plant A), the parent plant B is preferably Triticeae plants, or more preferably plants that belong to the genus Hordeum, including barley. It should be noted here that, evidently, the parent plants A and B are different strains. Known examples of crossbreeding between different strains of Triticeae plants are those between plants that belong to the genus Hordeum, Triticum, and Secale (see Current Biotechnology (1), Growing and Breeding of Grains, Potatoes, and Grasses, P. 112, Nogyo Tosho, published in 1992).

A particularly suitable cross between parent plant A and parent plant B can be obtained when the parent plant A is H. spontaneum (ancestral wild-type barley) and the parent plant B is cultivated barley with the polymorphic site of Haruna Nijo or Akashinriki, because in this case crossbreeding can easily be carried out, and the progeny will not be sterile (even though different strains of the same species are crossed). Further, in view of the fact that the ancestral wild-type plant is known to carry a gene that is expressed only under the genetic background of the cultivated strain, there is great significance in making a cross between ancestral wild-type barley and cultivated barley.

When the crossbreeding is a backcross, the parent plants A and B may be a nonrecurrent parent and a recurrent parent (backcross parent), respectively. Alternatively, the parent plant A may be a hybrid of the backcross, and the parent plant B may be a recurrent parent of the backcross.

The purpose of "crossbreeding" here is to select for and establish a useful lineage with the genetic background of the parent plant B and with the useful gene of the parent plant A. In other words, the purpose here is to select for and establish a useful lineage from which negative traits of the parent plant B have been removed, and into which the useful gene of the parent plant A has been introduced. Therefore, in the process of crossbreeding, there is suitably carried out a step of selecting a hybrid with the useful gene from a group of hybrid plants produced by a cross between the parent plant A and parent plant B (screening step).

The screening step takes advantage of the fact that the useful gene either partially or completely has a sequence of the oligonucleotide of the present invention. For example, when the parent plant B has an allele of the useful gene, a suitable hybrid is selected from a group of hybrid plants using any of the SNP typing methods described above. The screening method using a genetic marker is advantageous in terms of time and convenience because it does not require growing hybrids and actually checking the phenotype but allows for screening using seeds of the hybrids, for example.

When the parent plant B does not carry the useful gene or its allele, whether or not the useful gene has been injected or not can be found, other than using the SNP typing method, by determining whether the hybrid's DNA or RNA hybridizes with the oligonucleotides of the present invention (including the oligonucleotide A for detecting SNP).

Note that, the hybrids screened in the screening step are, for example, (1) a candidate strain to be crossed with a recurrent parent in backcrossing, or (2) a new strain that stably carries the useful gene. Desirably, these hybrids inherit substantially only the useful gene from the parent plant A (negative traits of parent plant A are not inherited). It is therefore particularly preferable, using a genetic marker other than the SNP of the present invention (may be an SNP on a different gene), to create a molecular map (chromosome map) with which various portions of the chromosomes of the parent plants A and B can be identified, and to screen a group of identified hybrids (recombinant chromosome substitution lines) for a hybrid into which substantially only the useful gene has been injected. Note that, the molecular map may be created from a group of doubled haploid lines of the parent plants A and B.

Further, as used herein, the term "recombinant" or "recombinant plant" refers to a hybrid that is produced by crossbreeding or genetic engineering technique and including the useful gene. Further, the scope of "recombinant" or "recombinant plant" includes organisms themselves; various organs such as root, stem, leaf, and reproductive organs (including flower and seed); various tissues; and cells. More broadly, the scope of "recombinant" or "recombinant plant" includes protoplast, spheroplast, induced callus, regenerated organisms and their offsprings.

### (Examples)

In the following the present invention is described in more detail by way of Examples. It should be appreciated that the description in the following Examples does not limit the present invention in any ways.

### [Example 1: Extraction of mRNA from barley and preparation of cDNA libraries]

The barley strains Akashinriki, Haruna Nijo, and H. spontaneum (OUH602) (wild-type barley) were used. In order to obtain a material for RNA extraction, the seeds of these barley strains were germinated and grown according to the procedures described in Cell Technology, Separate Volume, Plant Cell Technology Series 14, Plant Genome Research Protocol, pp. 193-195 (Shujunsha (2000)). For Haruna nijo, a germination shoot, seedling leaf blade and upper three leaves at the flag leaf stage were used. The leaf blade of vegetative stage was used for Akashinriki. For H. spontaneum (OUH602), upper three leaves at the flag leaf stage were used.

Using Sepasol RNA (product of nacalai tesque), total RNA was extracted from each material according to the method in the instruction manual of the kit. Then, using a mRNA purification kit provided by Amersham Pharmacia Biotech, only mRNA was extracted from the total RNA according to the method in the instruction manual of the kit.

The cDNA libraries were constructed using a λZAP-cDNA synthesis kit (product of Stratagene) according to the method described in Genome Analysis Laboratory Manual, pp. 63-75: Springer-Verlag Tokyo, 1988 with the following modifications (a) and (b).
(a) In the second centrifugation in the step of smoothing the cDNA ends (see page 71 of the Genome Analysis Laboratory Manual), 20 µl of sodium acetate solution (3M) and 400 µl of 100% ethanol were added to the aqueous layer containing cDNA and were adequately mixed therein by a vortex. The whole was allowed to stand overnight at -20°C, and was centrifuged for 60 minutes at 15,000 rpm at 4°C.
(b) The product of the reaction in the cutting step with XhoI (see page 72 of Genome Analysis Laboratory Manual) was allowed to stand overnight at -20°C, and was centrifuged for 60 minutes at 15,000 rpm at 4°C. With the supernatant removed and the pellet completely dried, the product was dissolved in 14 µl STE(1×) and 3.5 µl of column loading dye was added for a ligation step into a vector.

The titer of the cDNA so constructed was 10⁶ pfu or greater. The measurement was carried out using Insert Check - Ready- (Product of TOYOBO) according to the method provided by the kit. The average size of the inserts (cDNA insertion fragments) in the clones was about 1.5 kb, and the maximum size was 6 kb or greater.

### [Examples 2: Analysis of cDNA inserts]

By a mass excision method (ZAP-cDNA Gigapack III (product of Stratagene) Gold cloning kit, see instruction manual), the clones excised from the cDNA library were cultured overnight on an LB medium to extract plasmid DNA.

Then, the cDNA inserts in the plasmid DNA were sequenced in two directions from the 5' end and 3' end. For the sequence analysis, the ABI PRISM TM3700 Genetic Analyzer (product of PE Biosystems) was used. Next, in order to examine the extent of overlap of the clones from the result of sequence analysis, the clones were clustered. Concurrently, the inserts of the clones were assembled to prepare CONTIGS (1) through (921). The inserts were then multiple-aligned to find SNP among different clones.

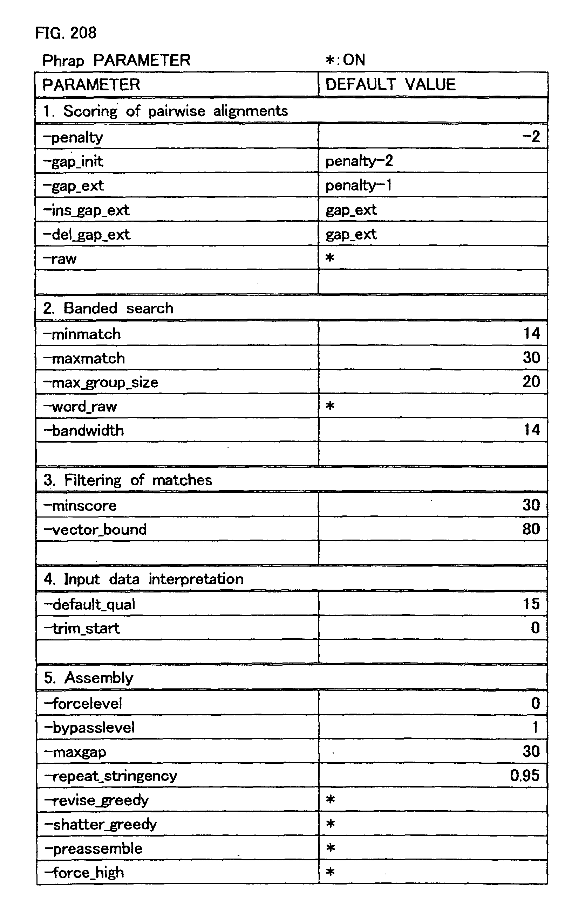

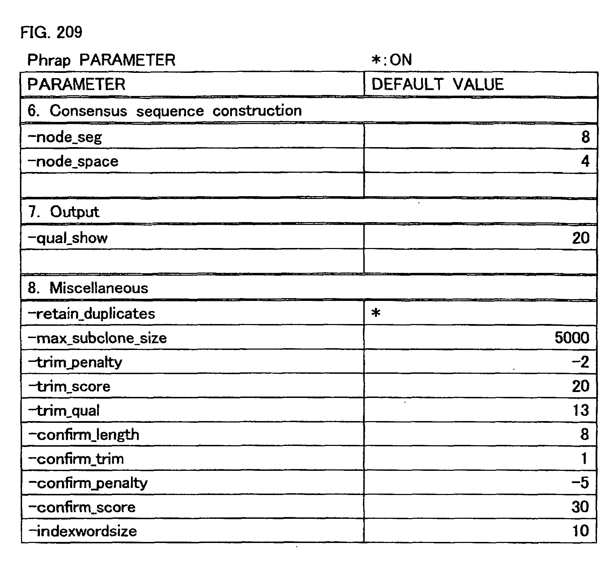

For the clustering of the clones, assembly of the inserts, and detection of SNP, the software dynaclust SNP explorer (dynaclust ver. 4, additional module, product of Dynacom) was used. More specifically, the clones were clustered using the blastn program installed in the software, and by setting the Expect Value to 1e⁻¹⁰. The inserts were then assembled according to the parameter settings shown in Fig. 208 and Fig. 209, by setting the reading error probability of the bases in base-calling constant (Phred score = 15 (default value)), and by using Phrap as an assembly tool.

The invention being thus described by way of specific embodiments and examples in the BEST MODE FOR CARRYING OUT THE INVENTION section, it will be obvious that the same way may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, an oligonucleotide according to the present invention is a DNA sequence of seven or more nucleotides, or a complementary sequence thereof, containing at least one polymorphic site represented by a universal code, the oligonucleotide being identified as a partial or entire sequence of a DNA sequence of any one of SEQ ID NOs: 1 through 17, 267 through 320, 990 through 1183, 4642 through 4763, and 9107 through 9640.

As described above, an oligonucleotide according to the present invention is a DNA sequence of seven or more nucleotides, or a complementary sequence thereof, containing at least one polymorphic site represented by a universal code and at which nonsynonymous substitution occurs, the oligonucleotide being identified as a partial or entire sequence of a DNA sequence set forth in any one of SEQ ID NOs: 1 through 4, 267 through 278, 990 through 1016, 4642 through 4670, and 9107 through 9640.

The oligonucleotide includes a polymorphic site or its complementary site that confers variations among different barley strains, and therefore can be used to test single nucleotide polymorphism that exists among different barley strains, for example. Further, the oligonucleotide enables genetic identification of different barley strains, and genotyping of barley, for example.

Further, a desirable recombinant can be produced with the oligonucleotide by the following methods. In one method, a useful gene is isolated using the oligonucleotide as a primer or probe, and the useful gene is introduced into a target cell by a genetic engineering technique. In another method, the oligonucleotide is used to select an individual with a useful gene from a group of hybrid plants obtained by crossbreeding parent plants.

## Claims

1. An oligonucleotide with a continuous DNA sequence of seven or more nucleotides, or a complementary sequence thereof, containing at least one polymorphic site whose base is represented by a universal code, said oligonucleotide being identified by a DNA sequence set forth in any one of SEQ ID NOs: 1 through 17, 267 through 320, 990 through 1183, 4642 through 4763, and 9107 through 9640.

2. An oligonucleotide with a continuous DNA sequence of seven or more nucleotides, or a complementary sequence thereof, containing at least one polymorphic site at which nonsynonymous substitution occurs and whose base is represented by a universal code, said oligonucleotide being identified by a DNA sequence set forth in any of SEQ ID NOs: 1 through 4, 267 through 278, 990 through 1016, 4642 through 4670, and 9107 through 9640.

3. A composition containing the oligonucleotide set forth in claim 1 or 2, for testing or identifying single nucleotide polymorphism in a gene.

4. A method for testing or identifying single nucleotide polymorphism in a gene of a subject based on at least one polymorphic site, or a complementary site thereof, whose base is represented by a universal code, said at least one polymorphic site being identified by a DNA sequence set forth in any one of SEQ ID NOs: 1 through 17, 267 through 320, 990 through 1183, 4642 through 4763, and 9107 through 9640.

5. A method of testing or identifying single nucleotide polymorphism in a gene of a subject based on at least one polymorphic site, or a complementary site thereof, at which nonsynonymous substitution occurs and whose base is represented by a universal code, said at least one polymorphic site being identified by a DNA sequence set forth in any one of SEQ ID NOs: 1 through 4, 267 through 278, 990 through 1016, 4642 through 4670, and 9107 through 9640.

6. A method of producing a recombinant, comprising the steps of:
isolating a gene that contains at least one polymorphic site, or a complementary site thereof, whose base is represented by a universal code, said at least one polymorphic site being identified by a DNA sequence set forth in any one of SEQ ID NOs: 1 through 17, 267 through 320, 990 through 1183, 4642 through 4763, and 9107 through 9640; and
introducing the gene so obtained in a target cell, using a genetic engineering technique.

7. A method of producing a recombinant, comprising the steps of:
isolating a gene that contains at least one polymorphic site, or a complementary site thereof, at which nonsynonymous substitution occurs, and whose base is represented by a universal code, said at least one polymorphic site being identified by a DNA sequence set forth in any one of SEQ ID NOs: 1 through 4, 267 through 278, 990 through 1016, 4642 through 4670, and 9107 through 9640; and
introducing the gene so obtained in a target cell, using a genetic engineering technique.

8. A method of producing a recombinant, comprising the steps of:
producing hybrid plants by crossbreeding (i) a parent plant A having a gene that contains at least one polymorphic site, or a complementary site thereof, whose base is represented by a universal code, said at least one polymorphic site being identified by a DNA sequence set forth in any one of SEQ ID NO: 1 through 17, 267 through 320, 990 through 1183, 4642 through 4763, and 9107 through 9640, and (ii) a parent plant B without the gene or with an allele that confers single nucleotide polymorphism to the gene; and
screening for a hybrid plant with the gene, using the oligonucleotide set forth in claim 1, or the testing method set forth in claim 4.

9. A method of producing a recombinant, comprising the steps of:
producing hybrid plants by crossbreeding (i) a parent plant A having a gene that contains at least one polymorphic site, or a complementary site thereof, at which nonsynonymous substitution occurs and whose base is represented by a universal code, said at least one polymorphic site being identified by a DNA sequence set forth in any one of SEQ ID NOs: 1 through 4, 267 through 278, 990 through 1016, 4642 through 4670, and 9107 through 9640, and (ii) a parent plant B without the gene or with an allele that confers single nucleotide polymorphism to the gene; and
screening for a hybrid plant with the gene, using the oligonucleotide set forth in claim 2, or the testing method set forth in claim 5.

10. A recombinant produced by the method set forth in any one of claims 6 through 9.

11. A primer designed to amplify a region of a polynucleotide with a DNA sequence set forth in any one of SEQ ID NOs: 1 through 17, 267 through 320, 990 through 1183, 4642 through 4763, and 9107 through 9640, containing at least one polymorphic site, and/or a complementary site thereof, whose base is represented by a universal code.

12. A primer designed to amplify a region of a polynucleotide with a DNA sequence set forth in any one of SEQ ID NOs: 1 through 4, 267 through 278, 990 through 1016, 4642 through 4670, and 9107 through 9640, containing at least one polymorphic site, and/ or a complementary site thereof, at which nonsynonymous substitution occurs and whose base is represented by a universal code.
